# EUROPEAN PATENT SPECIFICATION

(11) **EP 4 504 723 B1**
(45) Date of publication and mention of the grant of the patent: **14.10.2026**
(21) Application number: 23718214.2
(22) Date of filing: 05.04.2023
(51) Int. Cl.: C07D 409/12, C07D 405/12, C07D 409/14, C07D 513/04, C07D 407/12, A61P 35/00, A61P 25/00, A61P 25/28, A61P 3/00, A61P 3/04, A61P 3/10, A61P 31/00, A61P 31/12, A61K 31/357, A61K 31/381, A61K 31/404, A61K 31/4436, A61K 31/4025, A61K 31/4365, A61K 31/4985, A61K 31/343

(54) **N-(3-(BENZO[B]THIOPHENE-2-CARBOXAMIDO)-PHENYL)-2,3-DIHYDROBENZO[B][1,4]DIOXINE-6-CARBOXAMIDE DERIVATIVES AND RELATED COMPOUNDS AS LACTATE/ATP PRODUCTION INHIBITORS FOR THE TREATMENT OF CANCER**
N-(3-(BENZO[B]THIOPHENE-2-CARBOXAMIDO)-PHENYL)-2,3-DIHYDROBENZO[B][1,4]DIOXINE-6-CARBOXAMID-DERIVATE UND ÄHNLICHE VERBINDUNGEN ALS INHIBITOREN DER LACTAT/ATP-PRODUKTION ZUR BEHANDLUNG VON KREBS
DÉRIVÉS DE N-(3-(BENZO[B]THIOPHÈNE-2-CARBOXAMIDO)-PHÉNYL)-2,3-DIHYDROBENZO[B][1,4]DIOXINE-6-CARBOXAMIDE ET COMPOSÉS SIMILAIRES EN TANT QU'INHIBITEURS DE LA PRODUCTION DE LACTATE/ATP POUR LE TRAITEMENT DU CANCER

(30) Priority: 05.04.2022 EP 22166685
(43) Date of publication of application: 12.02.2025
(73) Proprietor: WMT AG, 69123 Heidelberg (DE)
(72) Inventor: TARASEWICZ, Anika, 64673 Zwingenberg (DE); STEENECK, Christoph, 69120 Heidelberg (DE); LESZCZYNSKI, Bartosz, 30167 Hannover (DE); VIEIRA, Andreia, 64295 Darmstadt (DE); SECHI, Stefano, 69226 Nußloch (DE); REID, George, Dundee DD2 1BN (GB); CHARAPITSA, Iryna, 69198 Schriesheim (DE); FABIAN, Johannes, 69723 Schwetzingen (DE); KROL, Helen, 64625 Bensheim (DE); KREMOSER, Claus, 69121 Heidelberg (DE)
(74) Representative: Ullrich & Naumann PartG mbB
(86) International application number: PCT/EP2023/058978
(87) International publication number: WO 2023/194458

(56) References cited:
- WO-A1-2011/011514
- WO-A1-2015/049535
- WO-A1-2018/065787

## Description

### Field of the Invention

The present invention relates to new and inventive compounds, especially compounds useful in the treatment of cancer. The present invention further relates to a pharmaceutical composition comprising such a compound. The present invention further relates to such a compound for use as a medicament. The present invention further relates to such a compound or such a pharmaceutical composition for use in a method of preventing and/or treating a disease or condition, in particular cancer. The present invention further relates to compounds for use in a method of treating a disease or condition mediated by the lactate/ATP mechanism, in particular cancer, in which an effective amount of such a compound or of such a pharmaceutical composition is administered to a subject in need thereof. The present invention further relates to the use of such a compound or such a pharmaceutical composition in the manufacture of a medicament for treating a disease or condition mediated by the lactate/ATP mechanism, in particular cancer.

References in the present description to methods of medical treatment or medical uses have to be understood as references to the compounds of the present invention for use in methods of medical treatment. Methods of medical treatment or medical uses are not encompassed by the claimed invention.

### Background of the Invention

Cancer remains the second leading cause of mortality in mankind. Deadly cancers are those that are either diagnosed too late, i.e. at a very late stage of disease progression, or cancers that have been treated but could not be cured. After repeated relapses or at late stage, i.e. typically stage 3B or higher, many solid cancers, in particular, tend to acquire a chemo- and radiotherapy-resistant phenotype. Such advanced and treatment-resistant cancers are often characterized by a high degree of metastasis where the original tumours and / or the metastases employ a high degree of so-called Warburg metabolism (Weber et al., Front Oncol. (2016) 6:257).

Otto Warburg, after whom this effect is named, discovered about 100 years ago that cancer cells utilize anaerobic glycolysis to convert glucose into lactate. This metabolic phenotype generates ATP at a low efficiency compared to oxidative phosphorylation, i.e. mitochondrial respiration. Warburg originally ascribed this metabolic switch from respiration towards anaerobic glycolysis as a consequence of a lack of oxygen in the core of advanced cancers (Warburg et al., Biochem Zeitschr. (1924) 152:309-44; Warburg et al., J Gen Physiol (1927) 8(6):519-30). Research over the past 20 years has elucidated, however, that this metabolic switch towards a predominance of anaerobic glycolysis occurs in order to meet the huge demand for carbon-containing building blocks for malignant cell proliferation, rather than to achieve efficient ATP generation. Multiple intermediates of the glycolytic or the pentose phosphate pathways are used by the cell to synthesize nucleosides, lipids, different carbohydrates or amino acids or to regenerate NADPH, which is needed for either lipid and cholesterol biosynthesis or for regenerating defense systems that protect against reactive oxygen species (ROS) (Vander et al., Cell (2017) 168(4):657-669). ROS are dangerous to the cell and their rate of generation is drastically increased in cancer cells (Hosios et al., J Biol Chem. (2018) 293(20):7490-7498). Thus, pathways such as the glutathione or Thioredoxin systems, that detoxify ROS species, are absolutely required in rapidly proliferating cells, which place a demand on the provision of NADPH-reducing equivalents, generally supplied by the first oxidative steps of the pentose phosphate pathway, using glucose as a substrate (Wang et al., Theranostics. (2011) 11(10):4839-4857).

ROS stress can also lead to damaged proteins where misfolded or chemically impaired proteins induce a so-called proteotoxic stress or unfolded protein response (UPR). If this stress situation occurs in the endoplasmic reticulum (ER), it is recognized as ER stress, a sub-phenomenon of proteotoxic stress (Radanović et al., Cells. (2021) 10(11):2965). As part of the proteotoxic stress response, certain transcription factors, in particular Heat Shock Factor -1 (HSF-1), are well known as master regulators of a transcriptional program that occurs to compensate and resolve proteotoxic stress. HSF-1 is one of the first proteins to be induced upon heat shock or chemically induced proteotoxic stress response, where it coordinates transcription and hence production of proteins and systems that counteract this specific stress situation e.g. certain chaperones, ROS-detoxifying systems, certain membrane transporters or pumps (Dai et al., Philos Trans R Soc Lond B Biol Sci. (2018) 373(1738):20160525).

All of the aforementioned elements, increased anaerobic glycolysis, increased flux through the pentose phosphate pathway, increased need for ROS protection and for counteracting proteotoxic stress, can be defined as hallmarks of the Warburg metabolism in a wider, more modern definition.

The final key consequence of Warburg metabolism is high-level lactate production, an inevitable consequence of not utilizing oxidative phosphorylation. Lactate can be regarded as the waste product of anaerobic glycolysis, resulting from the action of Lactate Dehydrogenase (LDH), which reduces pyruvate into lactate by the concomitant oxidation of NADH into NAD+. This NAD+ needs to be regenerated to permit an intermediate transformation in the midst of glycolysis, catalyzed by GAPDH. Thus, converting pyruvate to lactate closes the loop for NAD-regeneration in anaerobic glycolysis, and does not allow for entry of pyruvate into mitochondria for oxidative phosphorylation metabolism. Importantly, however, cancer cells, even when predominately using Warburg metabolism and deprived of oxygen, do not fully shut down mitochondrial metabolism. This is essential as mitochondrial metabolism, in particular the citric acid cycle, allows for the use of alternative Warburg fuels, i.e. amino acids such as asparagine or glutamine, to use this central metabolic turntable to yield other amino acids and pyruvate. These can partly be converted into lactate for the aforementioned reasons or can undergo "reverse" glycolysis, i.e. gluconeogenesis and the pentose phosphate pathway, to meet the demand for carbon building blocks (Baltazar et al., Front Oncol. (2020) 10:231).

Thus, lactate is the common end product of various aspects of the complex Warburg metabolic state. Moreover, it has a further function by suppressing an immune response within a heavily lactate-producing tissue environment. The primary physiological consequence of the immunosuppressive role of lactate, and the local acidic pH that comes with it, is that skeletal muscle, in particular, produces large amounts of lactate when oxygen supply is insufficient under physical exercise. In untrained muscles, microfibers tend to disrupt and intracellular components are exposed to the extracellular environment. This is a typical stimulus for tissue-resident antigen presenting cells and other myeloid cells to define the damage muscle as target for the immune system. In order to prevent this, lactate and the acidic pH, both in their own right, act as immunosuppressants, thereby precluding an undesirable and unnecessary immune attack on muscle.

This physiological immunosuppressive role of lactate is used by solid tumors which release considerable amounts of lactate in their microenvironment, thereby preventing immune destruction of the tumor. In this sense, such tumor compartments resemble a "sore" muscle, with several publications that demonstrate immunosuppressive activities on basically every relevant immune cell subtype.

In summary, lactate is foremost the waste product of Warburg metabolism. Determining its levels in the context of cancer cell proliferative capacity is probably the best indicator of the Warburg effect, as it integrates carbon flux from different sources under anaerobic and aerobic conditions.

Secondly, as lactate plays a key role as a pan-immunosuppressant in the tumor microenvironment, reducing tumor lactate production is an attractive target to achieve in clinical cancer treatment (Vaupel et al., J Physiol. (2021) 599(6):1745-1757; de la Cruz-López et al., Oncol. (2019) 9:1143).

However, it has to be considered that cancer cells and transiently active skeletal muscle cells are not the only cell types in the human body that employ Warburg metabolism. It is well documented that all cell types that undergo several proliferation cycles upon certain mitogenic stimuli employ this metabolic phenotype, since it is an evolutionary predetermined pattern to support cell growth and division. In particular, T-cells that have been antigen-primed and that have received a strong proliferative signal, also consume huge amounts of glucose and turn it into lactate. It needs to be considered, though, that T-cells, whose activity is very instrumental in an immune anti-tumor response, typically proliferate in compartments where they come in close contact with antigen-presenting cells, i.e. tumor-draining lymph nodes, for example. Although it can be clearly demonstrated that T-cell proliferation upon a CD3/CD28 stimulus, for example, can be completely blunted by glycolysis inhibitors such as 2-deoxyglucose or others, it might be possible to separate anti-glycolytic effects on tumor cells from those on expanding T-cells by directing the respective pharmaceutical agent towards the tumor to exclude lymph nodes or the blood compartment. Once T-cells have proliferated and have become CD8-positive anti-tumor effector effector cells, they can enter the tumor and thereafter, attack cancer cells. This anti-tumor effectivity is not necessarily blunted by lowering intratumoral lactate, as recent publications have illustrated (Renner et al., Cell Rep. (2019) 29(1):135-150.e9).

To date, there is no approved drug available that would inhibit lactate production in the context of cancer treatment, other than as a side effect of cytotoxic activity, in general. There have been several attempts, using various different chemical compounds, to either inhibit one or more of the glycolytic enzymes directly, or to downregulate or inhibit key transcription factors or master regulators of the Warburg program. Prominent examples of the latter mode of action or members of the family of Hypoxia-induced factors (HIF), foremost Hif-1alpha or Hif-2alpha. Hif-1, -2 or -3 alpha heterodimerize with a common partner, Hif-1beta, which is also known as ARNT (Aryl Hydrocarbon Receptor Nuclear Translocator). All three members of the Hif-family are well described to act as transcription factors inducing the key genes that mediate the Warburg effect, i.e. GLUT-1 (glucose transporter), HK2, PK-M, LDH-A, MCT-4, the latter being hypoxia and tumor-specific isoforms of glycolytic enzymes or a lactate transporter (MCT-4) (Yu et al., J Cancer. (2017) 8(17):3430-3440).

Most of these drugs failed during clinical development, either due to a lack of efficacy with respect to anticancer effects, or because they exhibited serious and intolerable side effects.

The premier untransformed cell type that is susceptible to glycolytic inhibition is the erythrocyte, which relies solely on glycolysis as its energy source. Consequently, direct glycolysis inhibition leads to erythrocyte lysis, clinically manifesting as hemolytic anemia. Moreover, cancer patients under chemotherapy frequently suffer from anemia, which makes this side effect even more intolerable in the context of cancer treatment (Oshima et al., Cell Rep. (2020) 30(6):1798-1810.e4).

Another approach to limit cancer cell proliferation targets the proteotoxic stress response. This can be achieved in manifold ways, including but not limited to inhibitors of the proteasome such as bortezomib or carfilzomib. Limiting proteasomal protein degradation leads to an accumulation of misfolded protein, ultimately leading to apoptosis.

There have been attempts to inhibit HSF-1 as a key regulator of the proteotoxic stress response in order to suppress measures by the cell to mitigate the anti-proliferative effects of proteotoxic stress. One such chemical compound that was found to downregulate HSF-1 is CCT251236, a small molecule chemical compound derived from a screen for the aforementioned effect. CCT251236 is published to limit cell proliferation in multiple different cancer cell lines. This compound also inhibited tumor growth in an ovarian cancer xenograft mouse model. CCT251236, and its closely related PROTAC analogue CCT367766, were found to tightly bind to Pirin. However, it remains unproven that Pirin is the functionally relevant target of these bisamide compounds, also based on published results that show the CCT251236 displays micromolar inhibitors activity on other relevant proteins such as Acetylcholinesterase or certain kinases, as well. Nevertheless, CCT251236 has potent antitumor efficacy at a dose of 30 mg/kg every other day upon oral delivery (Cheeseman et al., J Med Chem. (2017) 60(1):180-201; Chessum et al., J Med Chem. (2018) 61(3):918-933). WO 2015/049535 and WO 2016/156872 disclose a series of bisamides with overlaps with certain compounds named CCT245232 and CCT251236 that were described in more detail in Cheeseman et al.; J Med Chem (2017), 60(1) 180-201 and in Chessum et al. J Med Chem. (2018);61(3):918-933. CCT245232 was identified in a screen for inhibitors of the HSF-1 proteotoxic stress pathway. Using medicinal chemistry efforts, CCT245232 was developed further into CCT251236 which demonstrates better solubility and oral bioavailability in mice. The compounds of this bisamide series demonstrate potent antiproliferative activities against cancer cells with SK-OV-3 as the reference cell line to determine exact growth IC₅₀ values. CCT251236 also shows significant anti-cancer effects in a mouse xenograft model of cancer.

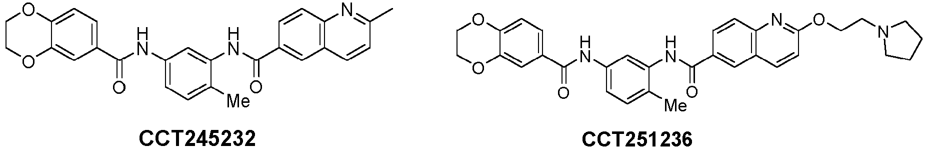

The anticancer effects of this bisamide type of compounds were attributed to their inhibitory effects on the HSF-1 proteotoxic stress pathway.

WO 2011/011514 A1 discloses N-(pyridin-3-yl)-benzamide derivatives as inhibitors of hypoxia-inducible factor 1 alpha (Hif-1a) mediated induction of PDK1 for the treatment of cancer.

WO 2018/065787 A1 discloses N-(5-(2,3-dihydrobenzo[b][1,4]dioxine-6-carboxamido)-2-fluorophenyl)-2-((4-ethylpiperazin-1-yl)methyl)quinoline-6-carboxamide and deuterated derivatives thereof as inhibitors of heat shock factor 1 (HSF1) for the treatment of cancer and viral infections.

Chessum et al., J Med Chem. (2018) 61(3):918-933) disclose these compounds to bind very potently to Pirin, a non-heme iron containing cytosolic protein with so far only poorly described cellular functions. In Meyers et al. (ACS Med Chem Lett (2018) ;9(12):1199-1204.) it is demonstrated that these bisamide compounds promiscuously bind to different kinases as protein targets. Thus, it is not entirely clear whether these bisamide compounds really exert the anti-cancer effects solely by binding to Pirin.

Autophagy is a highly conserved catabolic mechanism that degrades cellular components to provide intermediate metabolites during cell stress, for example, starvation (J Pathol. (2010) 221: 3-12). While autophagy can act to overcome temporary nutrient deprivation in tumor cells, uncontrolled or inappropriate autophagy can induce ferroptosis, a type of regulated cell death driven by iron dependent lipid peroxidation (Cell Research (2016) 26 1021-1032). Ferroptosis is dependent on autophagy, primarily through the release of iron bound to intracellular proteins, resulting in biotoxicity.

Autophagy is regarded as a key mechanism which is impaired in certain diseases, in particular in ageing- and lifestyle-related diseases (Madeo et al., Cell Metab. 5;29(3):592-610 (2019). It is believed that restoring the autophagy capacity in the respective organs and cells would overcome the accumulation of fibrillary protein tangles of beta-amyloid, alpha-synuclein and other proteins that are involved in the etiology of Alzheimer's disease, Parkinson's disease, Amyotrophic Lateral Sclerosis (ALS) but also in age-related memory decline, in general. Beyond applications in neurodegenerative diseases, activation of autophagy could show beneficial effects in fibrotic pulmonary diseases, obesity and diabetes and even in viral infections, induction of autophagy might help (Carmona-Gutierrez et al., Microb Cell, 7(5): 119-128. (2020).

Cancer therapy with small molecules is so far mainly restricted to cytotoxic drugs, i.e. substances that target vital enzymes, structural proteins such as microtubule or the DNA for preventing cancerous proliferation at the expense of drastic side effects, in particular to normal, healthy proliferating tissues such as intestinal epithelium or hair follicles.

The so-called biguanides, exemplified by the drugs Metformin, Buformin and Phenformin, are a class of drugs known to lead to side effects resulting from stimulation of glycolysis. These three drugs, with Phenformin being the most potent and Metformin the weakest, are or were antidiabetic medications to improve insulin sensitivity and therefore ameliorate hyperglycemia as a key hallmark of Type 2 Diabetes. All biguanides are believed to inhibit the Complex I of the mitochondrial respiratory chain resulting in lowered mitochondrial ATP production (Bridges et al., BMC Biol. 14, 65 (2016)) and this fosters an increased ATP production through upregulation of glycolysis. This explains the key issue of biguanides in clinical use, the potential occurrence of lactic acidosis. The incidence of lactic acidosis, which has a fatality of approximately 50%, is 64 cases in 100,000 patients treated with Phenformin and 3 cases in 100,000 patients treated with Metformin. While Phenformin is clearly the more potent drug and was in widespread use as an antidiabetic medication in the 1960s and 70s, it was replaced by the weaker but safer Metformin. Several studies that investigated the incidence of different cancers in Type 2 diabetes patients receiving or not receiving Metformin treatment showed that Metformin not only reduced overall mortality but also the incidence of cancer in these patients. However, clinical trials showed a slight benefit of Metformin treatment of cancer patients but there are no data from prospective trials comparing standard-of-care chemotherapy with or without additional Metformin treatment with regard to survival or cancer responsiveness, although preclinical data and data from retrospective studies suggest this (Top et al., Pharmaceuticals (Basel).15(3):312 (2022)).

A key limitation of the use of Metformin as an adjunct to existing chemotherapies is its mild effectiveness on cancer at the doses used for antidiabetic treatment. Further increases in dosing of Metformin or alternatively, the use of the much more powerful Phenformin might be indicated for the treatment of cancer, but the known side effect of lactic acidosis with Phenformin, and the fact that it was withdrawn from the market, prevent this.

Thus, in addition to new cancer therapies, there also exists a need for a co-treatment which lowers the lactate production which otherwise limits the doses of biguanide drugs that can be given to the respective patients. The subject matter of the present invention addresses both of these needs.

### Summary of the Invention

Unexpectedly, it has been found, that in addition to reducing intracellular ATP and the production of extracellular lactate, a reduction in intracellular HIF1α and the induction of endoplasmic reticulum stress genes, in particular CHAC and CHOP, the compounds of the present invention activate autophagy in cells treated with such compounds.

In experimental results, the compounds of the present invention also show a broad anti-proliferative activity against several cancer cell lines *in vitro.* Compounds of the present invention were successfully used to limit tumor growth in certain mouse models of cancerous growth.

The compounds of the present invention also demonstrate the potential to potently lower lactate production, and thus can be suitably co-administered with a biguanide drug to achieve otherwise unattainable biguanide levels *in vivo*. In this way, one can effectively treat the tumor while reducing or eliminating the risk of inducing a biguanide dose-limiting lactic acid acidosis.

Compounds of the present invention target an as yet unexplored mechanism which is the induction of proteotoxic stress in the endoplasmic reticulum, exemplified by well-known markers of this response such as Chac1 or Chop. In consequence, the cell reacts by the upregulation of genes the function of which could help to overcome the proteotoxic stress by producing more glutathione, for example. SLC7A11, also known as System Xc⁻, is a cystine-glutamate antiporter which is indicative of the upregulation of such countermeasures.

The induction of autophagy which is exerted by compounds of the present invention, is likely downstream of the primary proteotoxic stress response and is a reflection of the general shutdown of most metabolic pathways by compounds of the present invention. The sequence of events as established using these compounds is that they first induce the proteotoxic stress response, resulting in a general hibernation, i.e. shutdown of the cell in terms of metabolic activities resulting in less lactate generation. This simulates a kind of cellular starvation and the cell induces autophagy as a countermeasure to again supply more metabolic fuel.

The significant reduction in lactate production can be used not only to directly target proliferation of cancer cells that show the features of Warburg metabolism, i.e. high glycolysis rate and high lactate output. This intense but not cytotoxic shutdown of cellular proliferation and metabolism can also be exploited to counteract side effects of other drugs that would stimulate glycolysis and, by extension, can dangerously increase lactate concentrations in the blood resulting in lactic acidosis as a prominent side effect.

In a first aspect, the present invention relates to a compound according to formula (I) 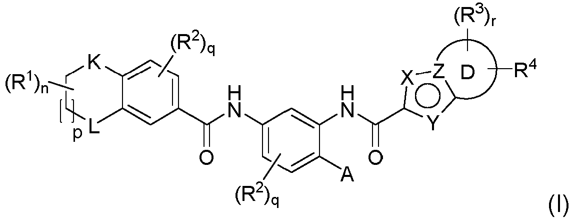 or an enantiomer, diastereomer, N-oxide, solvate or pharmaceutically acceptable salt thereof, wherein A, D, K, L, X, Y, Z, R¹-R⁴ and the indices n, p, q and r are defined as disclosed below.

In a further aspect, the present invention relates to a pharmaceutical composition comprising the compound according to formula (I) and a pharmaceutically acceptable excipient.

In a further aspect, the present invention relates to a compound according to formula (I) for use as a medicament.

In a further aspect, the present invention relates to the compound according to formula (I) or the pharmaceutical composition comprising the compound according to formula (I) for use in a method of preventing and/or treating a disease or condition mediated by the lactate/ATP mechanism.

In a further aspect, the present invention relates to a method of preventing and/or treating a disease or condition mediated by the lactate/ATP mechanism, the method comprising administering to a subject in need thereof an effective amount of the compound according to formula (I) or the pharmaceutical composition comprising the compound according to formula (I).

In a further aspect, the present invention also relates to the use of a compound according to formula (I) in the preparation of a medicament for the prophylaxis and/or treatment of a disease or condition mediated by the lactate/ATP mechanism.

In a further aspect, the present invention relates to a compound according to formula (I), or a pharmaceutical composition comprising the compound according to formula (I), for use in a method of preventing and/or treating a disease or condition, preferably cancer, by inducing endoplasmic reticulum stress in cells affected by said disease or condition, thereby reducing intracellular ATP and lactate production in said cells.

In a further aspect, the present invention relates to a method of preventing and/or treating a disease or condition, preferably cancer, said method comprising administering a compound according to formula (I), or a pharmaceutical composition comprising the compound according to formula (I), to a subject, said preventing and/or treating being achieved by inducing endoplasmic reticulum stress in cells affected by said disease or condition, thereby reducing intracellular ATP and lactate production in said cells.

In a further aspect, the present invention relates to the use of a compound according to formula (I), or a pharmaceutical composition comprising the compound according to formula (I), in the manufacture of a medicament for preventing and/or treating a disease or condition, preferably cancer, by inducing endoplasmic reticulum stress in cells affected by said disease or condition, thereby reducing intracellular ATP and lactate production in said cells.

In a further aspect, the present invention relates to a compound according to formula (I), or a pharmaceutical composition comprising the compound according to formula (I), for use in a method of preventing and/or treating cancer, preferably a cancer cell and/or a cancerous tumor, wherein said compound reduces Hif1-alpha at the protein level in hypoxic areas of said cancer, thereby downregulating ATP and lactate production in these areas and ultimately leading to reduction, preferably efficient reduction, of hypoxic areas and of growth of said cancer.

In a further aspect, the present invention relates to a method of preventing and/or treating cancer, preferably a cancer cell and/or a cancerous tumor, said method comprising administering a compound according to formula (I), or a pharmaceutical composition comprising the compound according to formula (I), to a subject, said preventing and/or treating being achieved by reducing Hif1-alpha at the protein level in hypoxic areas of said cancer, thereby downregulating ATP and lactate production in these areas and ultimately leading to reduction, preferably efficient reduction, of hypoxic areas and of growth of said cancer.

In a further aspect, the present invention relates to the use of a compound according to formula (I), or a pharmaceutical composition comprising the compound according to formula (I), in the manufacture of a medicament for preventing and/or treating cancer, preferably a cancer cell and/or a cancerous tumor, by reducing Hif1-alpha at the protein level in hypoxic areas of said cancer, thereby downregulating ATP and lactate production in these areas and ultimately leading to reduction, preferably efficient reduction, of hypoxic areas and of growth of said cancer.

In a further aspect, the present invention relates to a compound according to formula (I), or a pharmaceutical composition comprising the compound according to formula (I), for use in a method of preventing and/or treating a disease or condition which is susceptible to the induction of endoplasmic reticulum stress, wherein administration of said compound or of said pharmaceutical composition to a subject having or suspected of having said disease or condition results in a reduction of the protein level of Hif-1alpha.

In a further aspect, the present invention relates to a method of preventing and/or treating a disease or condition which is susceptible to the induction of endoplasmic reticulum stress, said method comprising administering a compound according to formula (I), or a pharmaceutical composition comprising the compound according to formula (I), to a subject, wherein administration of said compound to a subject having or suspected of having said disease or condition results in a reduction of the protein level of Hif-1alpha.

In a further aspect, the present invention relates to the use of a compound according to formula (I), or a pharmaceutical composition comprising the compound according to formula (I), in the manufacture of a medicament for preventing and/or treating a disease or condition which is susceptible to the induction of endoplasmic reticulum stress, by reducing the protein level of Hif-1alpha.

In a further aspect, the present invention relates to a compound according to formula (I), or a pharmaceutical composition comprising the compound according to formula (I), for use in a method of preventing and/or treating an autophagy-related disease selected from neuro-degenerative diseases such as Parkinson's disease, Alzheimer's disease and Huntington's disease, amyotrophic lateral sclerosis, metabolic disorders such as obesity and diabetes type I and II, viral infections and diseases which lead to accumulation of certain debris particles such as fibrotic lung diseases.

In a further aspect, the present invention relates to a method of preventing and/or treating an autophagy-related disease selected from neuro-degenerative diseases such as Parkinson's disease, Alzheimer's disease and Huntington's disease, amyotrophic lateral sclerosis, metabolic disorders such as obesity and diabetes type I and II, viral infections and diseases which lead to accumulation of certain debris particles such as fibrotic lung diseases, said method comprising administering a compound according to formula (I), or a pharmaceutical composition comprising the compound according to formula (I), to a subject.

In a further aspect, the present invention relates to the use of a compound according to formula (I), or a pharmaceutical composition comprising the compound according to formula (I) , in the manufacture of a medicament for preventing and/or treating an autophagy-related disease selected from neuro-degenerative diseases such as Parkinson's disease, Alzheimer's disease and Huntington's disease, amyotrophic lateral sclerosis, metabolic disorders such as obesity and diabetes type I and II, viral infections and diseases which lead to accumulation of certain debris particles such as fibrotic lung diseases, said method comprising administering a compound according to formula (I), or a pharmaceutical composition comprising the compound according to formula (I), to a subject.

In a further aspect, the present invention relates to the use of a compound according to formula (I) in the preparation of a medicament for the prophylaxis and/or treatment of cancer.

In further aspects, the present invention relates to a composition, preferably a pharmaceutical composition, comprising a compound according to formula (I) and a biguanide, as well as this composition for use as a medicament.

In a further aspect, the present invention relates to a combination of a compound according to formula (I) and a biguanide, for use in therapy.

In a further aspect, the present invention relates to a method of preventing and/or treating a disease, said method comprising administering a combination of a compound according to formula (I), or a pharmaceutical composition comprising the compound according to formula (I), and a biguanide, to a subject.

In a further aspect, the present invention relates to a combination of a compound according to formula (I), or a pharmaceutical composition comprising the compound according to formula (I), and a biguanide, for use in a method of preventing and/or treating cancer.

In a further aspect, the present invention relates to a method of preventing and/or treating cancer, said method comprising administering a combination of a compound according to formula (I), or a pharmaceutical composition comprising the compound according to formula (I), and a biguanide, to a subject.

In a further aspect, the present invention relates to the use of a combination of a compound according to formula (I), or a pharmaceutical composition comprising the compound according to formula (I), and a biguanide, in the manufacture of a medicament for preventing and/or treating cancer.

Herein, administering a compound according to formula (I) or a pharmaceutical composition comprising a compound according to formula (I) to a subject is to be understood as administering an effective amount of said compound or said pharmaceutical composition to said subject,

In further aspects of the treatments and uses set out above, the compound according to formula (I) is comprised in a pharmaceutical composition together with a pharmaceutically acceptable excipient.

### Description of the Figures

**Figure 1** shows ATP levels downregulated in HeLa, 4T1, LLC1, A549, MDA-MB-231 and MiaPaCa2 cell cultures after 48 hours treatment with the compounds of example 3/16, 3/8 and 10.
**Figure 2** shows extracellular lactate levels which are downregulated in HeLa, 4T1, LLC1, A549, MDA-MB-231 and MiaPaCa2 cell cultures after 48 hours treatment with the compounds of example 3/16, 3/8 and 10.
**Figure 3** shows ER stress genes induced in HeLa cells over a four hour period of treatment with 10 nM of the compounds of example 3/16, 3/8 and 10.
**Figure 4** shows the compounds of example 3/8 and 10 downregulating HIF1a protein levels in HeLa cells after 24 hours treatment under normoxia and hypoxia.
**Figure 5** shows that compounds of example 3/8, 3/16 and 10 activate autophagy in HeLa cells, also where autophagy is prevented by the application of Bafilomycin A. Similarly, examples 3/8, 3/16 and 10 induce phosphorylation of p70-S6K (Thr389) in HeLa cells and in cells concomitantly treated with Bafilomycin A.
**Figure 6** shows that compounds of example 3/16, 3/8 and 10 directly induce autophagy in a HeLa reporter cell line expressing eGFP-LC3 (ns, not significant; ****, p < 0.0001).
**Figure 7** shows that compounds of example 3/16, 3/8 and 10 reduce the formation of lactate in a dose-dependent manner produced by HeLa cells when treated with phenformin.
**Figure 8** shows a comparison of activities of compounds of example 3/16, 3/8, 10, 3/19, 30 and 30/2 in downregulation of ATP (Figures 8A and 8B), extracellular lactate levels (Figures 8C and 8D) and induction of ER stress genes (Figures 8E-8AB) in Panc02 and SKOV3 cells.

### Detailed description of the invention

The present invention relates to a compound of formula (I) 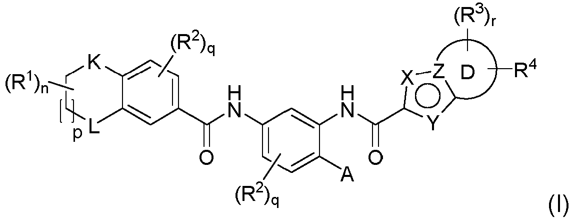
or an enantiomer, diastereomer, N-oxide, solvate or pharmaceutically acceptable salt thereof, wherein
K is O, S or C(R⁵)₂;
L is O, S or C(R⁵)₂;
n is 0, 1, 2, 3 or 4;
p is 1 or 2;
q is independently from each other 0, 1 or 2;
r is 1 or 2;
A is C₁₋₆-alkyl, halogen, CN, halo-C₁₋₆-alkyl, C₃₋₆-cycloalkyl, halo-C₃₋₆-cycloalkyl, OH, -OC₁₋₆-alkyl or O-halo-C₁₋₆-alkyl;
X is S, O, NR⁶, N or CR⁷;
Y is S, O, NR⁶, N or CR⁷;
Z is C or N;
D represents a 6-membered aryl or 5- to 6-membered heteroaryl containing 1 to 4 heteroatoms independently selected from O, N and S;
R¹ is F, C₁₋₃-alkyl or spirocyclic fused C₃₋₆-cycloalkyl;
R² is independently halogen or C₁₋₆-alkyl;
R³ is hydrogen, halogen, C₁₋₆-alkyl, halo-C₁₋₆-alkyl, C₃₋₆-cycloalkyl, halo-C₃₋₆-cycloalkyl, -OC₁₋₆-alkyl or O-halo-C₁₋₆-alkyl;
R⁴ is hydrogen, halogen or V¹-V²-V³-V⁴-V⁵
   wherein
   V¹ is absent, a bond or C₁₋₆-alkylene;
   V² is absent, a bond or O, S, S(O)ₓ, S(O)(=NR^{a}), S(O)₂NR^{a}, NR^{a}, NR^{a}C(O) or C(O)NR^{a};
   V³ is absent, a bond or C₁₋₆-alkylene;
      wherein C₁₋₆-alkylene in V¹ or V³ is unsubstituted or substituted with 1 to 4 substituents independently selected from the group consisting of OH, C₁₋₆-alkyl, C₁₋₆-haloalkyl, halogen and oxo,
         or
      wherein two C₁₋₆-alkyl groups on the C₁₋₆-alkylene in V¹ or V³, together with the carbon atom to which they are attached, form a C₃₋₆-cycloalkyl group;
   V⁴ is absent, a bond or O, S, S(O)ₓ, S(O)(=NR^{a}), S(O)₂NR^{a}, NR^{a}, NR^{a}C(O) or C(O)NR^{a};
   V⁵ is hydrogen, halogen, OH, CN, CO₂H, CO₂-C₁₋₆-alkyl, N(R^{a})₂, C₁₋₆-alkyl, C₃₋₈-cycloalkyl, 4- to 8-membered mono or bicyclic heterocycloalkyl containing 1, 2 or 3 heteroatoms independently selected from the group consisting of O, S and N, phenyl or 5- to 6-membered heteroaryl containing 1, 2 or 3 heteroatoms independently selected from the group consisting of O, S and N,
      wherein alkyl, cycloalkyl, heterocycloalkyl, phenyl or heteroaryl are unsubstituted or substituted with 1 to 4 substituents R^{V5} independently selected from the group consisting of CN, CO₂H, CO₂-C₁₋₆-alkyl, N(R^{a})₂, OH, C₁₋₆-alkyl, O-C₁₋₆-alkyl, C(O)-C₁-₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N;
R⁵ is hydrogen, F, C₁₋₃-alkyl or spirocyclic fused C₃₋₆-cycloalkyl;
R⁶ is hydrogen or C₁₋₆-alkyl;
R⁷ is hydrogen, halogen or C₁₋₆-alkyl;
R^{a} is hydrogen or C₁₋₃-alkyl; and
x is 1 or 2.

In an embodiment in combination with any of the above or below embodiments, the compound is represented by formula (I) 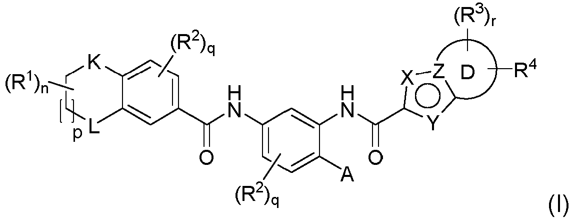 or an enantiomer, diastereomer, N-oxide, solvate or pharmaceutically acceptable salt thereof, wherein
K is O, S or C(R⁵)₂;
L is O, S or C(R⁵)₂;
n is 0, 1, 2, 3 or 4;
p is 1 or 2;
q is independently from each other 0, 1 or 2;
r is 1 or 2;
A is C₁₋₆-alkyl, halogen, CN, halo-C₁₋₆-alkyl, C₃₋₆-cycloalkyl, halo-C₃₋₆-cycloalkyl, OH, -OC₁₋₆-alkyl or O-halo-C₁₋₆-alkyl;
X is S, O, NR⁶, N or CR⁷
Y is S, O, NR⁶, N or CR⁷;
Z is C or N;
D represents a 6-membered aryl or 5- to 6-membered heteroaryl containing 1 to 4 heteroatoms independently selected from O, N and S;
R¹ is F, C₁₋₃-alkyl or spirocyclic fused C₃₋₆-cycloalkyl;
R² is independently halogen or C₁₋₆-alkyl;
R³ is hydrogen, halogen, C₁₋₆-alkyl, halo-C₁₋₆-alkyl, C₃₋₆-cycloalkyl, halo-C₃₋₆-cycloalkyl, -OC₁₋₆-alkyl or O-halo-C₁₋₆-alkyl;
R⁴ is hydrogen, halogen or V¹-V²-V³-V⁴-V⁵
   wherein
   V¹ is absent, a bond or C₁₋₆-alkylene;
   V² is absent, a bond or O, S, S(O)ₓ, S(O)(=NR^{a}), S(O)₂NR^{a}, NR^{a}, NR^{a}C(O) or C(O)NR^{a};
   V³ is absent, a bond or C₁₋₆-alkylene;
      wherein C₁₋₆-alkylene in V¹ or V³ is unsubstituted or substituted with 1 to 4 substituents independently selected from the group consisting of C₁₋₆-alkyl, C₁₋₆-haloalkyl, halogen and oxo,
         or
      wherein two C₁₋₆-alkyl groups on the C₁₋₆-alkylene in V¹ or V³, together with the carbon atom to which they are attached, form a C₃₋₆-cycloalkyl group;
   V⁴ is absent, a bond or O, S, S(O)ₓ, S(O)(=NR^{a}), S(O)₂NR^{a}, NR^{a}, NR^{a}C(O) or C(O)NR^{a};
   V⁵ is hydrogen, halogen, OH, CN, CO₂H, CO₂-C₁₋₆-alkyl, N(R^{a})₂, C₁₋₆-alkyl, C₃₋₈-cycloalkyl, 4- to 8-membered mono or bicyclic heterocycloalkyl containing 1, 2 or 3 heteroatoms independently selected from the group consisting of O, S and N, phenyl or 5- to 6-membered heteroaryl containing 1, 2 or 3 heteroatoms independently selected from the group consisting of O, S and N,
      wherein alkyl, cycloalkyl, heterocycloalkyl, phenyl or heteroaryl are unsubstituted or substituted with 1 to 4 substituents R^{V5} independently selected from the group consisting of CN, CO₂H, CO₂-C₁₋₆-alkyl, N(R^{a})₂, OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N;
R⁵ is hydrogen, F, C₁₋₃-alkyl or spirocyclic fused C₃₋₆-cycloalkyl;
R⁶ is hydrogen or C₁₋₆-alkyl;
R⁷ is hydrogen, halogen or C₁₋₆-alkyl;
R^{a} is hydrogen or C₁₋₃-alkyl; and
x is 1 or 2.

In an embodiment in combination with any of the above or below embodiments, the compound is represented by formula (II) 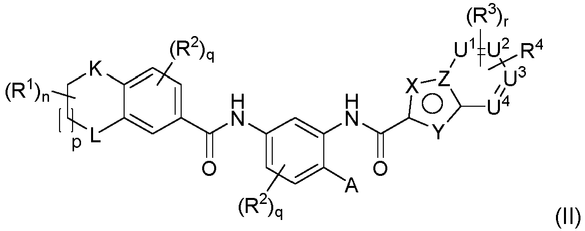 wherein U¹, U² ,U³ and U⁴ are independently selected from the group consisting of N and C.

In an embodiment in combination with any of the above or below embodiments, the compound is represented by formula (III) 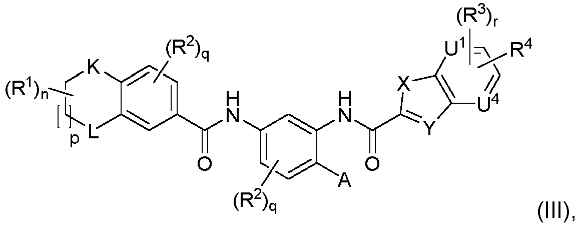 wherein
U¹ and U⁴ are independently selected from N and C;
X is S, O, NR⁶ or N; and
Y is CR⁷ or N.

In an embodiment in combination with any of the above or below embodiments, the index n is 0.

In an embodiment in combination with any of the above or below embodiments, the index p is 1.

In an embodiment in combination with any of the above or below embodiments, the compound is represented by formula (IV) 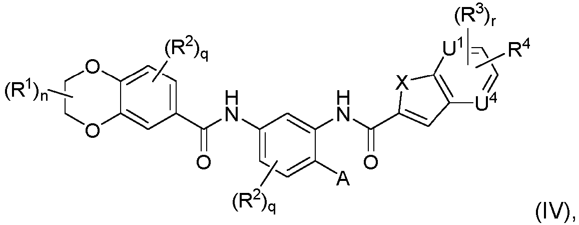 wherein
A is C₁₋₆-alkyl or halogen; and
X is S or NH.

In an embodiment in combination with any of the above or below embodiments, R⁴ is hydrogen, halogen or V¹-V²-V³-V⁴-V⁵, wherein
V¹ is absent, a bond or C₁₋₄-alkylene;
V² is absent, a bond or O, S, S(O)ₓ, NR^{a};
V³ is absent, a bond or C₁₋₄-alkylene,
V⁴ is absent, a bond or O, S, S(O)ₓ, NR^{a},
   wherein C₁₋₄-alkylene in V¹ or V³ is unsubstituted or substituted with 1 to 4 substituents independently selected from the group consisting of C₁₋₃-alkyl, C₁₋₃-haloalkyl, halogen and oxo,
      or
   wherein two C₁₋₆-alkyl groups on the C₁₋₄-alkylene in V¹ or V³, together with the carbon atom to which they are attached, form a C₃₋₆-cycloalkyl group;
V⁵ is hydrogen, halogen, OH, CN, C₁₋₆-alkyl, C₃₋₆-cycloalkyl, 4- to 8-membered mono or bicyclic heterocycloalkyl containing 1, 2 or 3 heteroatoms independently selected from the group consisting of O, S and N, or 5- to 6-membered heteroaryl containing 1, 2 or 3 heteroatoms independently selected from the group consisting of O, S and N,
   wherein alkyl, cycloalkyl, heterocycloalkyl or heteroaryl are unsubstituted or substituted with 1 to 4 substituents R^{V5} independently selected from the group consisting of OH, C₁₋₆-alkyl, O-C₁₋₆-alkyl, C(O)-C₁₋₆-alkyl, C₁₆₃-haloalkyl, halogen, oxo, spirocyclic fused C₃₋₆-cycloalkyl and spirocyclicly fused 3-7 membered heterocycloalkyl containing 1 heteroatom selected from the group consisting of O, S and N.

In an embodiment in combination with any of the above or below embodiments, R⁴ is hydrogen, halogen or V²-V³-V⁵, wherein
V² is absent, a bond or O,
V³ is absent, a bond or C₁₋₄-alkylene,
   wherein C₁₋₄-alkylene in V³ is unsubstituted or substituted with 1 to 4 substituents independently selected from the group consisting of C₁₋₃-alkyl, and
V⁵ is hydrogen, halogen, OH, CN, C₁₋₆-alkyl, 4- to 8-membered mono or bicyclic heterocycloalkyl containing 1, 2 or 3 heteroatoms independently selected from the group consisting of O, S and N, or 5- to 6-membered heteroaryl containing 1, 2 or 3 heteroatoms independently selected from the group consisting of O, S and N,
   wherein alkyl, cycloalkyl, heterocycloalkyl or heteroaryl are unsubstituted or substituted with 1 to 4 substituents R^{V5} independently selected from the group consisting of OH, C₁₋₆-alkyl, O-C₁₋₆-alkyl, C₁₋₆-haloalkyl, halogen and oxo.

In an embodiment in combination with any of the above or below embodiments, the compound is selected from the following group: 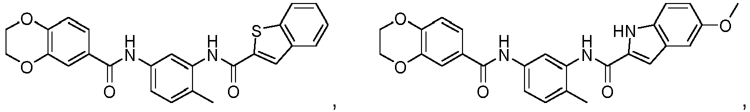 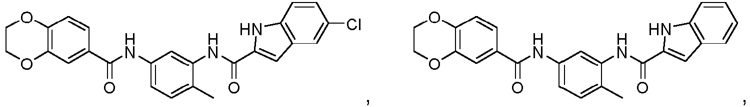 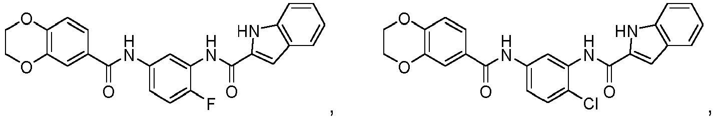 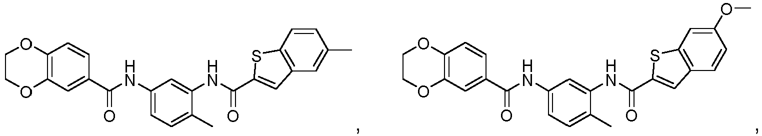 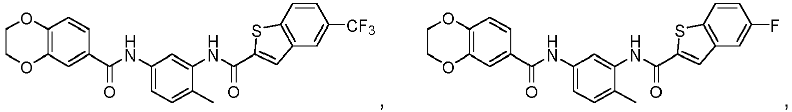 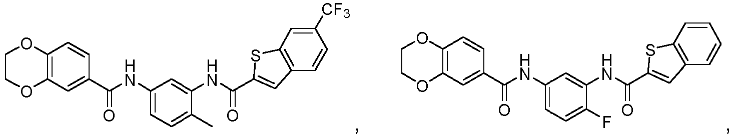 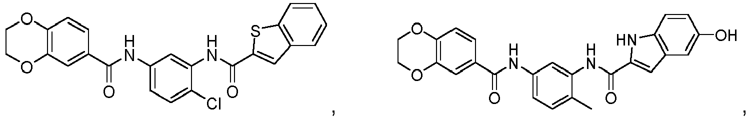 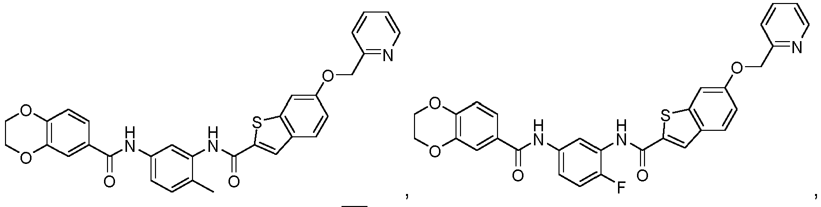 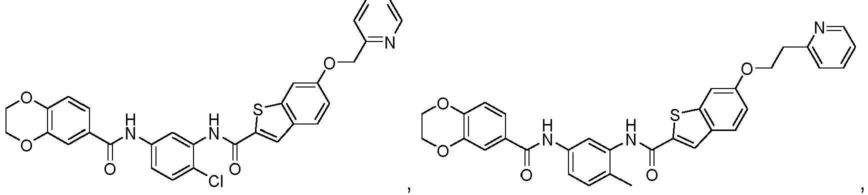 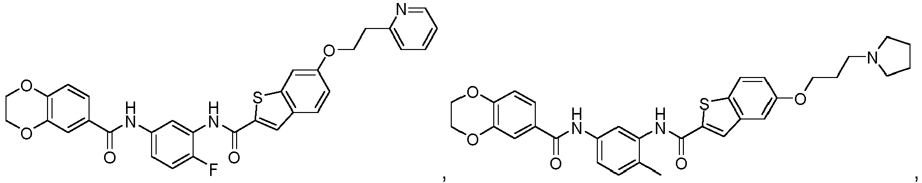 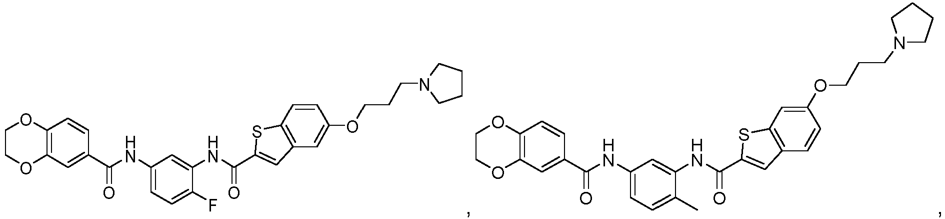 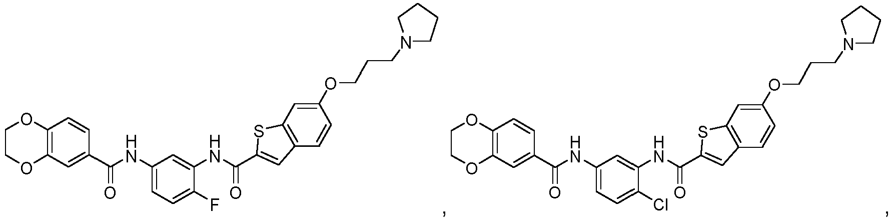 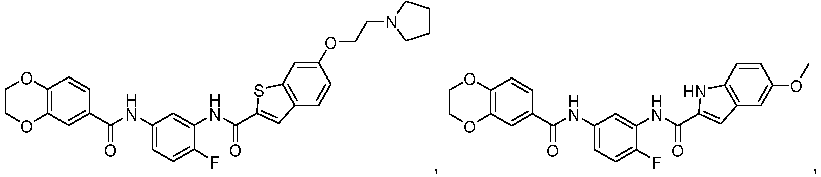 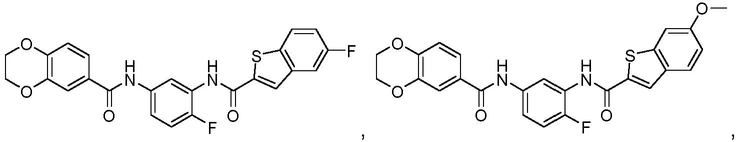 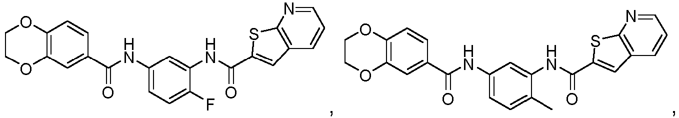 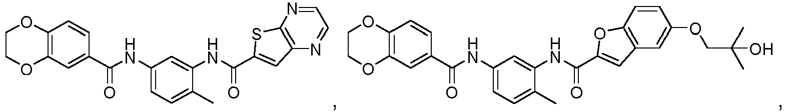 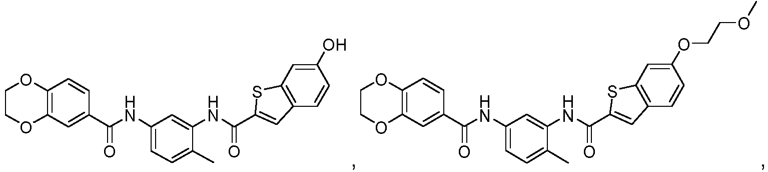 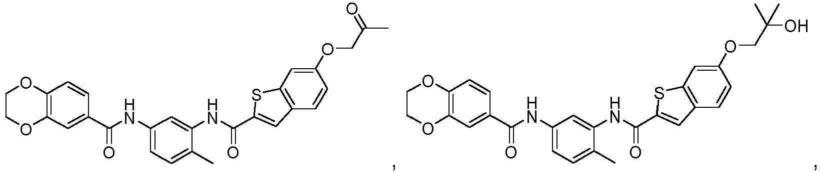 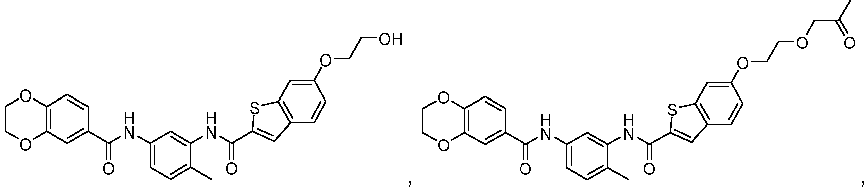 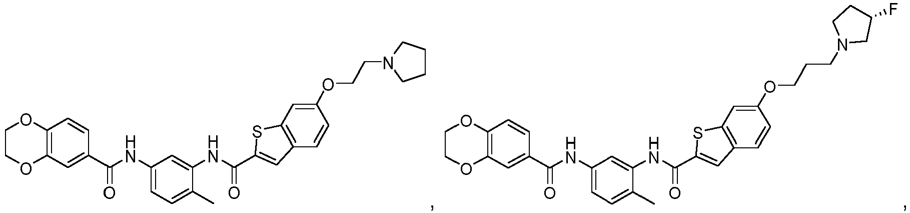 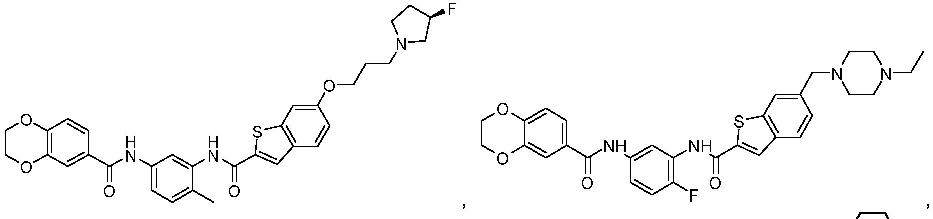 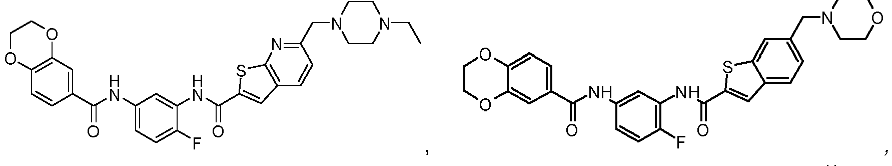 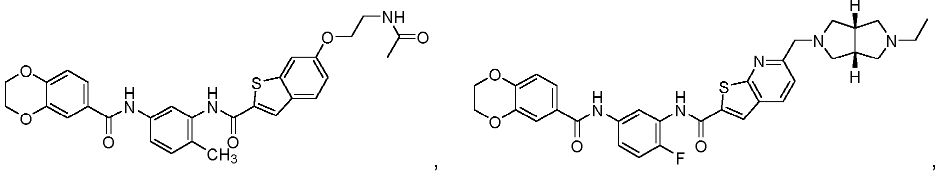 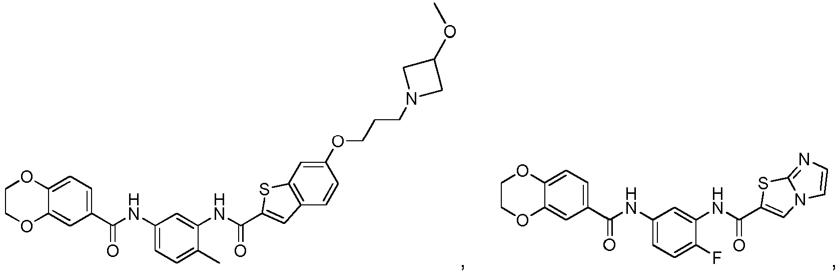 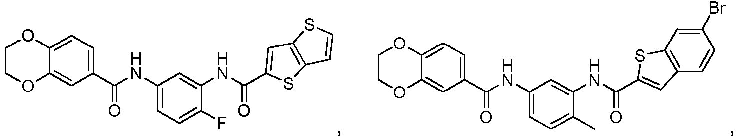 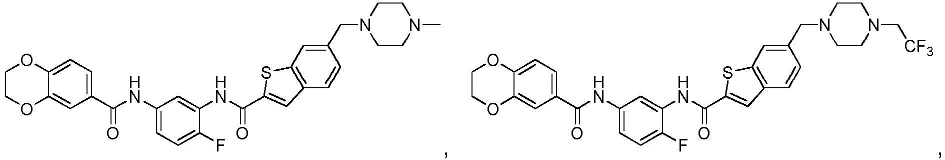 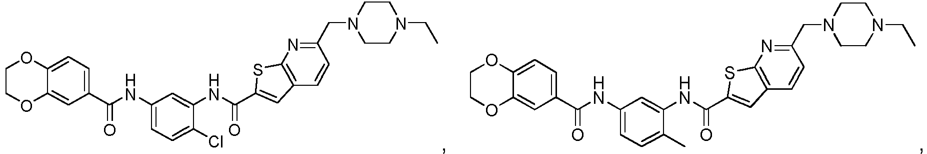 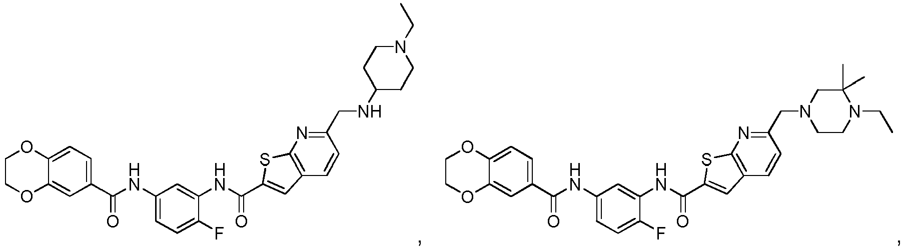 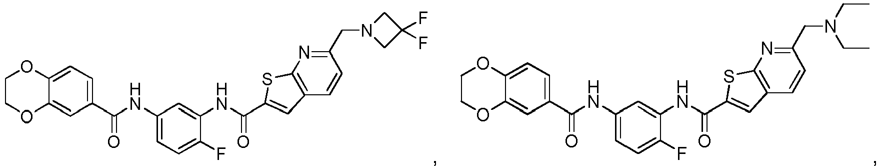 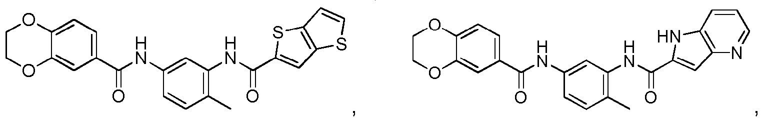 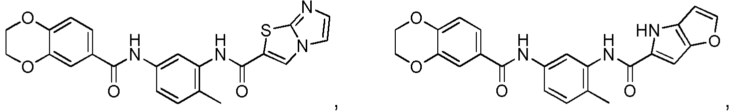 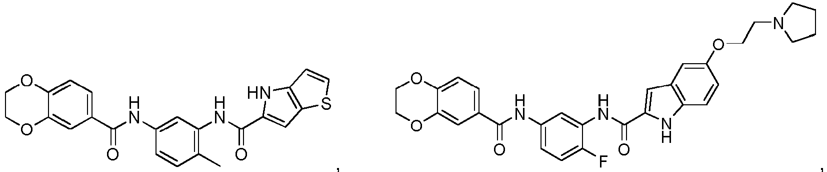 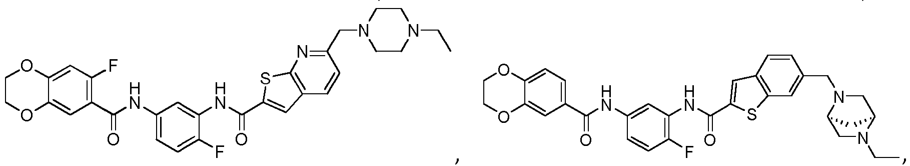 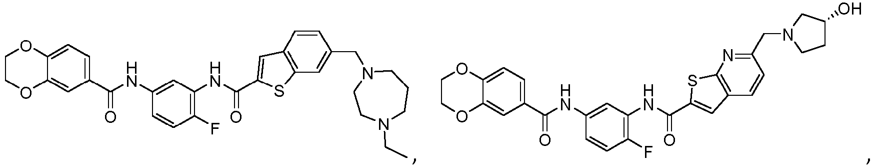 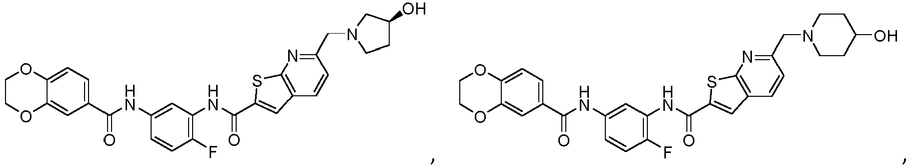 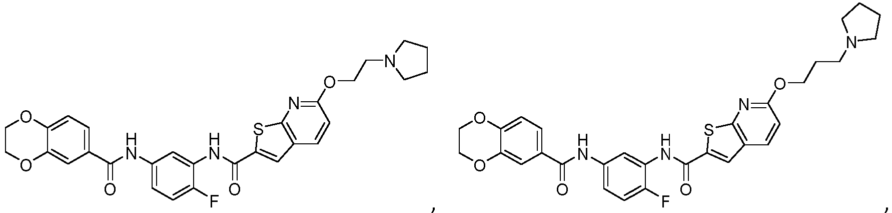 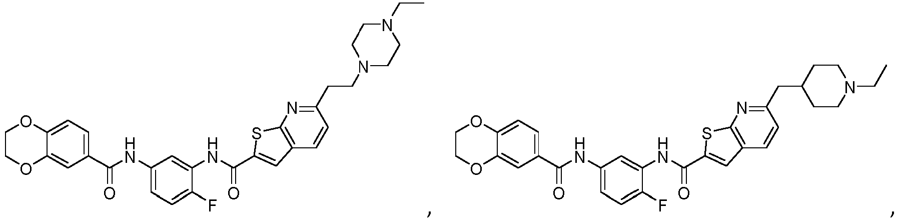 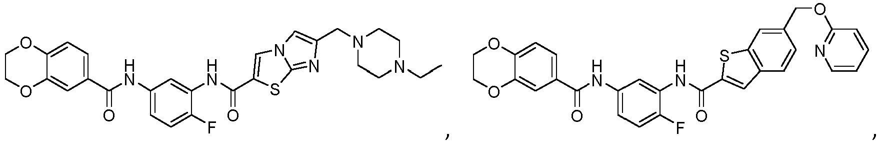 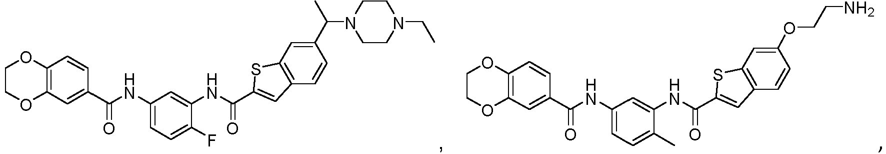 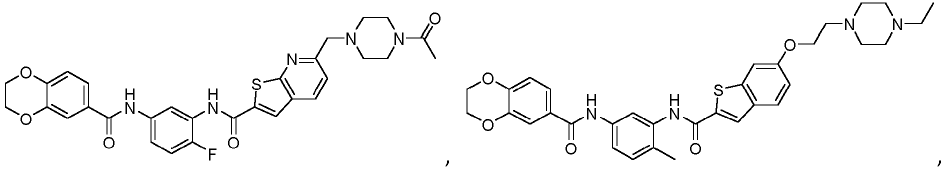 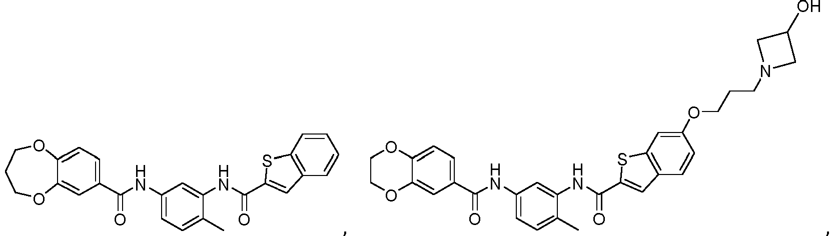 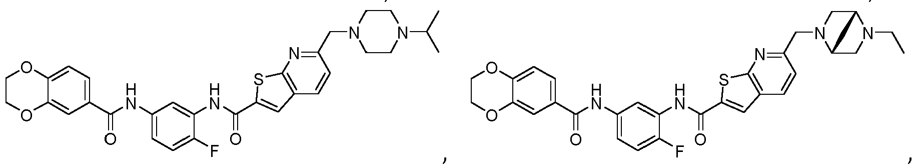 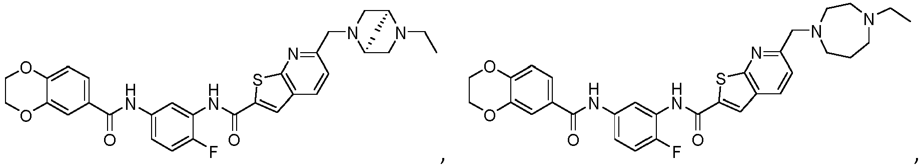 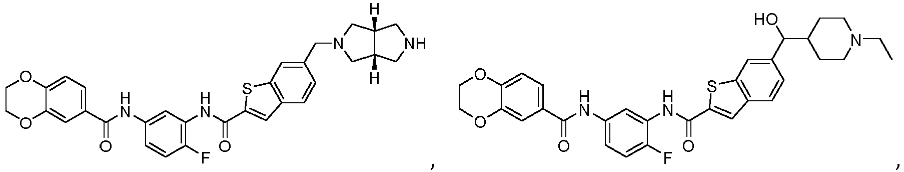 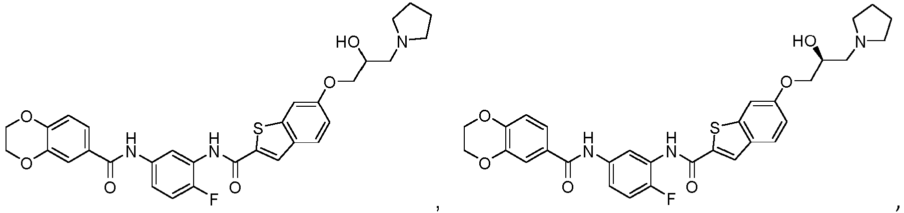 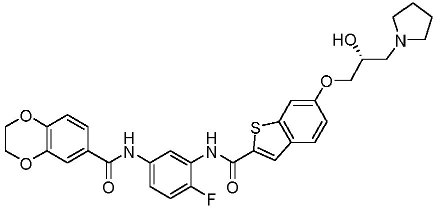 and the N-oxide, solvate or pharmaceutically acceptable salt thereof.

In formula (I), the variables K and L independently represent O, S or C(R⁵)₂. In a preferred embodiment in combination with any of the above or below embodiments, one of the variables K and L represents O and the other represents S, or both variables represent O, or both variables represent S, more preferably both of K and L represent O. In a further preferred embodiment in combination with any of the above or below embodiments, one of K and L represents O and the other C(R⁵).

In formula (I), the variable A is C₁₋₆-alkyl, halogen, CN, halo-C₁₋₆-alkyl, C₃₋₆-cycloalkyl, halo-C₃₋₆-cycloalkyl, OH, -OC₁₋₆-alkyl or O-halo-C₁₋₆-alkyl. In an embodiment in combination with any of the above or below embodiments, the variable A is C₁₋₆-alkyl, halogen, halo-C₁₋₆-alkyl, C₃₋₆-cycloalkyl, halo-C₃₋₆-cycloalkyl, OH, -OC₁₋₆-alkyl or O-halo-C₁₋₆-alkyl. In an embodiment in combination with any of the above or below embodiments, the variable A is C₁₋₆-alkyl, halogen, halo-C₁₋₆-alkyl, OH, -OC₁₋₆-alkyl or O-halo-C₁₋₆-alkyl. In a preferred embodiment in combination with any of the above or below embodiments, the variable A is C₁₋₆-alkyl or halogen.

In a more preferred embodiment in combination with any of the above or below embodiments, the variable A is CH₃, F or CI. In a most preferred embodiment in combination with any of the above or below embodiments, the variable A is F.

In formula (I), the index q is independently selected from 0, 1 and 2. In a preferred embodiment in combination with any of the above or below embodiments, the index q is independently selected from 0 or 1. In a more preferred embodiment in combination with any of the above or below embodiments, the index q is 0.

In formula (I), the index p is 1 or 2. In a preferred embodiment in combination with any of the above or below embodiments, the index p is 1.

In formula (I), index n is 0, 1, 2, 3 or 4. In a preferred embodiment in combination with any of the above or below embodiments, the index n is 0, 1 or 2. In a more preferred embodiment in combination with any of the above or below embodiments, the index n is 0 or 1. In a most preferred embodiment in combination with any of the above or below embodiments, the index n is 0.

In an embodiment in combination with any of the above or below embodiments, the variable A is F and the index q is 0.

In an embodiment in combination with any of the above or below embodiments, the variables K and L both represent O, n is 0, p is 1 and q is 0.

In formula (I), the ring D in the structural moiety 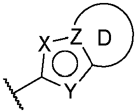 represents a 6-membered aryl or 5- to 6-membered heteroaryl containing 1 to 4 heteroatoms independently selected from O, N and S.

In a preferred embodiment in combination with any of the above or below embodiments, the structural unit 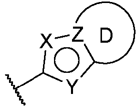 is selected from the group consisting of 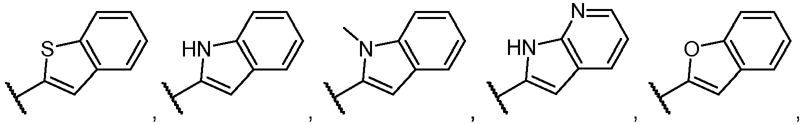 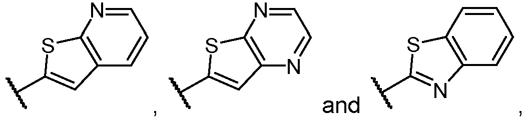 wherein each of the above groups representing the ring D is substituted with R⁴ and 1 or 2 R³.

In a preferred embodiment in combination with any of the above or below embodiments, the structural unit 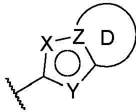 is selected from the group consisting of 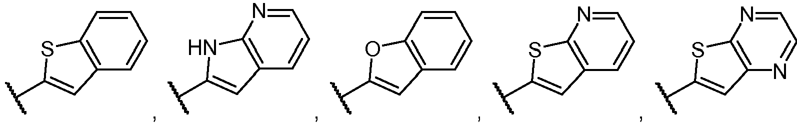 and 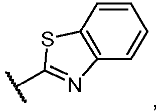 wherein each of the above groups representing the ring D is substituted with R⁴ and 1 or 2 R³.

In a further embodiment in combination with any of the above or below embodiments, the structural unit 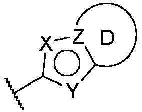 is selected from the group consisting of 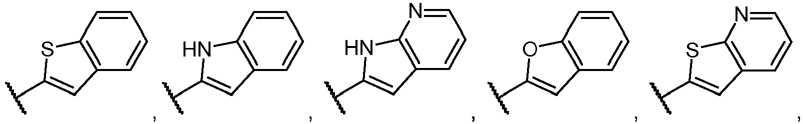 and 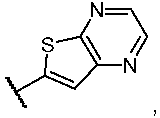 wherein each of the above groups representing the ring D is substituted with R⁴ and 1 or 2 R³.

In an embodiment in combination with any of the above or below embodiments, the structural unit 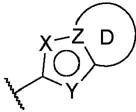 is selected from the group consisting of 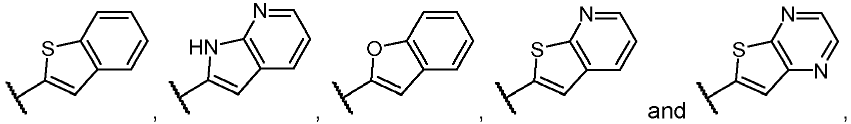 wherein each of the above groups representing the ring D is substituted with R⁴ and 1 or 2 R³.

In a preferred embodiment in combination with any of the above or below embodiments, the structural unit 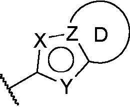 is selected from the group consisting of 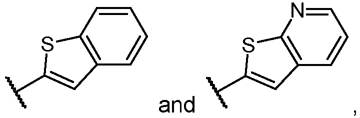 wherein each of the above groups representing the ring D is substituted with R⁴ and 1 or 2 R³.

In a more preferred embodiment in combination with any of the above or below embodiments, the structural unit 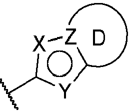 is 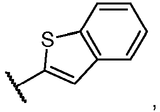 wherein the phenyl ring representing the ring D is substituted with R⁴ and 1 or 2 R³.

In an equally more preferred embodiment in combination with any of the above or below embodiments, the structural unit 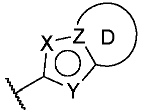 is 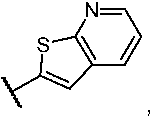 wherein the pyridine ring representing the ring D is substituted with R⁴ and 1 or 2 R³.

In an embodiment in combination with any of the above or below embodiments, the structural unit 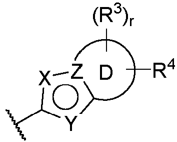 is selected from the group consisting of

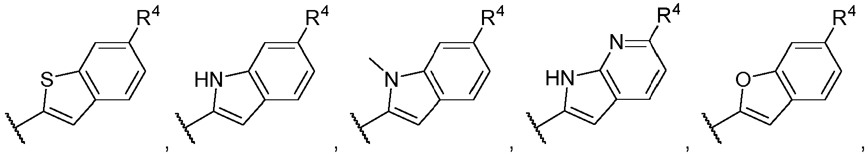

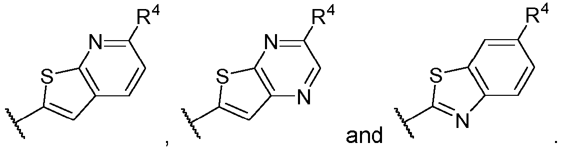

In a preferred embodiment in combination with any of the above or below embodiments, the structural unit 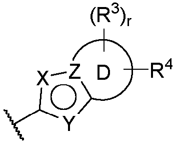 is selected from the group consisting of 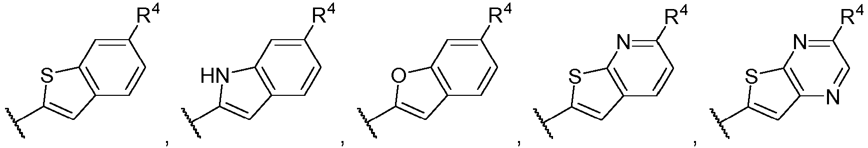 and

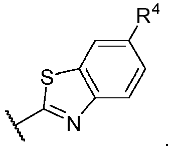

In a further embodiment in combination with any of the above or below embodiments, the structural unit 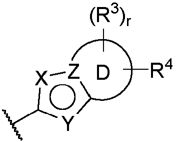 is selected from the group consisting of 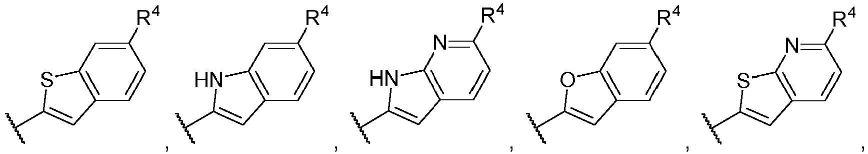 and

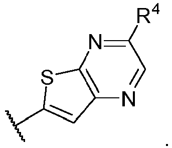

In an embodiment in combination with any of the above or below embodiments, the structural unit 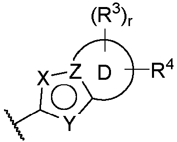 is selected from the group consisting of

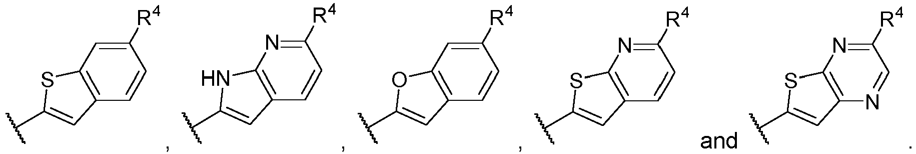

In a preferred embodiment in combination with any of the above or below embodiments, the structural unit 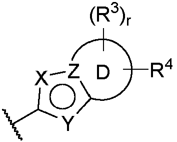 is selected from the group consisting of

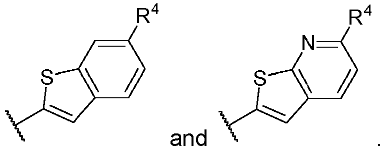

In a more preferred embodiment in combination with any of the above or below embodiments, the structural unit 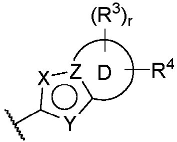 is

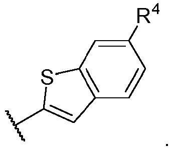

In an equally more preferred embodiment in combination with any of the above or below embodiments, the structural unit 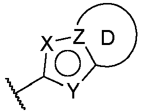 is

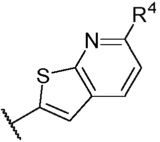

In formula (I), R¹ is F, C₁₋₃-alkyl or spirocyclic fused C₃₋₆-cycloalkyl.

In a preferred embodiment in combination with any of the above or below embodiments, R¹ is F or C₁₋₃-alkyl.

In formula (I), R² is halogen or C₁₋₆-alkyl.

In a preferred embodiment in combination with any of the above or below embodiments, R² is F, Cl or C₁₋₃-alkyl.

In formula (I), R³ is hydrogen, halogen, C₁₋₆-alkyl, halo-C₁₋₆-alkyl, C₃₋₆-cycloalkyl, halo-C₃₋₆-cycloalkyl, -OC₁₋₆-alkyl or O-halo-C₁₋₆-alkyl.

In a preferred embodiment in combination with any of the above or below embodiments, R³ is hydrogen, F, Cl, Br, C₁₋₆-alkyl, halo-C₁₋₆-alkyl, C₃₋₆-cycloalkyl or -OC₁₋₆-alkyl, more preferably R³ is F, Cl or C₁₋₆-alkyl.

In formula (I), R⁴ is hydrogen, halogen or V¹-V²-V³-V⁴-V⁵, wherein
V¹ is absent, a bond or C₁₋₆-alkylene;
V² is absent, a bond or O, S, S(O)ₓ, S(O)(=NR^{a}), S(O)₂NR^{a}, NR^{a}, NR^{a}C(O) or C(O)NR^{a};
V³ is absent, a bond or C₁₋₆-alkylene;
   wherein C₁₋₆-alkylene in V¹ or V³ is unsubstituted or substituted with 1 to 4 substituents independently selected from the group consisting of OH, C₁₋₆-alkyl, C₁₋₆-haloalkyl, halogen and oxo, or
   wherein two C₁₋₆-alkyl groups on the C₁₋₆-alkylene in V¹ or V³, together with the carbon atom to which they are attached, form a C₃₋₆-cycloalkyl group;
V⁴ is absent, a bond or O, S, S(O)ₓ, S(O)(=NR^{a}), S(O)₂NR^{a}, NR^{a}, NR^{a}C(O) or C(O)NR^{a};
V⁵ is hydrogen, halogen, OH, CN, CO₂H, CO₂-C₁₋₆-alkyl, N(R^{a})₂, C₁₋₆-alkyl, C₃₋₈-cycloalkyl, 4- to 8-membered mono or bicyclic heterocycloalkyl containing 1, 2 or 3 heteroatoms independently selected from the group consisting of O, S and N, phenyl or 5- to 6-membered heteroaryl containing 1, 2 or 3 heteroatoms independently selected from the group consisting of O, S and N,
   wherein alkyl, cycloalkyl, heterocycloalkyl, phenyl or heteroaryl are unsubstituted or substituted with 1 to 4 substituents R^{V5} independently selected from the group consisting of CN, CO₂H, CO₂-C₁₋₆-alkyl, N(R^{a})₂, OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N;

In a preferred embodiment in combination with any of the above or below embodiments, R⁴ is hydrogen, halogen or V¹-V²-V³-V⁴-V⁵, wherein
V¹ is absent, a bond or C₁₋₄-alkylene;
V² is absent, a bond or O, S, S(O)ₓ, NR^{a};
V³ is absent, a bond or C₁₋₄-alkylene,
V⁴ is absent, a bond or O, S, S(O)ₓ, NR^{a},
   wherein C₁₋₄-alkylene in V¹ or V³ is unsubstituted or substituted with 1 to 4 substituents independently selected from the group consisting of OH, C₁₋₃-alkyl, C₁₋₃-haloalkyl, halogen and oxo, or
   wherein two C₁₋₆-alkyl groups on the C₁₋₄-alkylene in V¹ or V³, together with the carbon atom to which they are attached, form a C₃₋₆-cycloalkyl group;
V⁵ is hydrogen, halogen, OH, CN, C₁₋₆-alkyl, C₃₋₆-cycloalkyl, 4- to 8-membered mono or bicyclic heterocycloalkyl containing 1, 2 or 3 heteroatoms independently selected from the group consisting of O, S and N, or 5- to 6-membered heteroaryl containing 1, 2 or 3 heteroatoms independently selected from the group consisting of O, S and N,
   wherein alkyl, cycloalkyl, heterocycloalkyl or heteroaryl are unsubstituted or substituted with 1 to 4 substituents R^{V5} independently selected from the group consisting of OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N.

In a preferred embodiment in combination with any of the above or below embodiments, R⁴ is H, -CH₃, -CF₃, -OCH₃, F, Cl, Br,

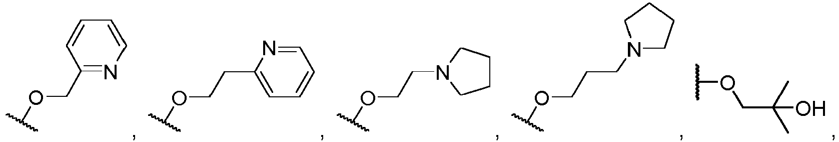

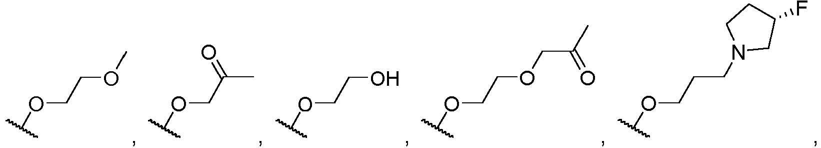

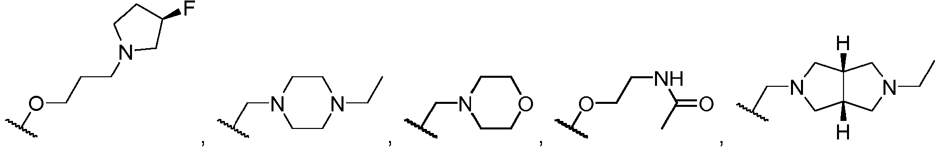

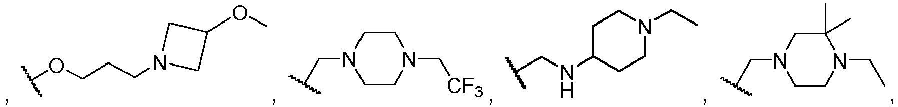

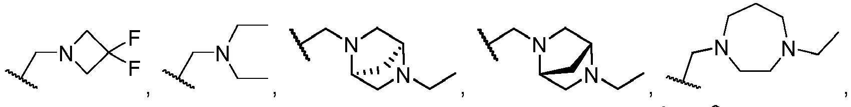

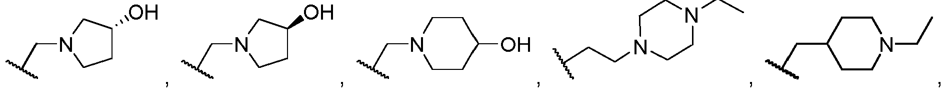

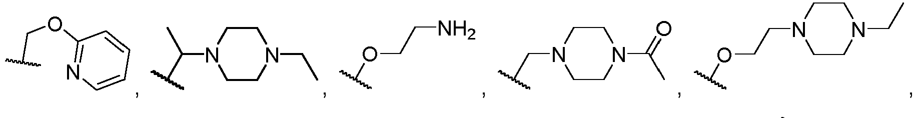

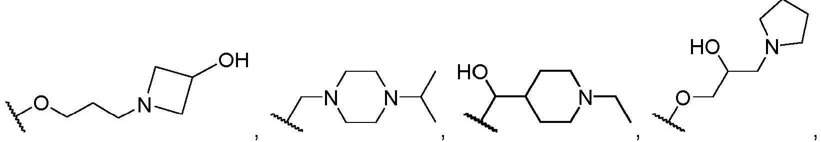

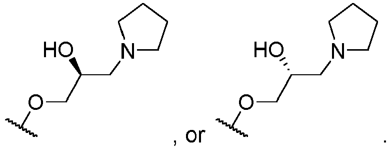

In a more preferred embodiment in combination with any of the above or below embodiments, R⁴ is

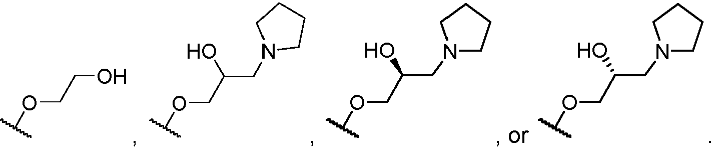

In an equally more preferred embodiment in combination with any of the above or below embodiments, R⁴ is

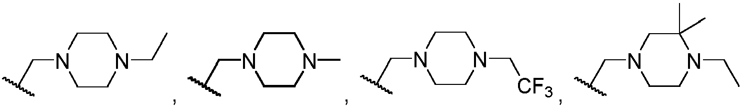

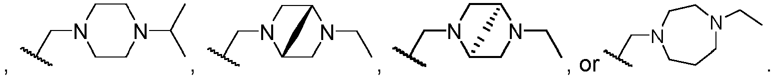

In a preferred embodiment in combination with any of the above or below embodiments, the compound is selected from the following group: 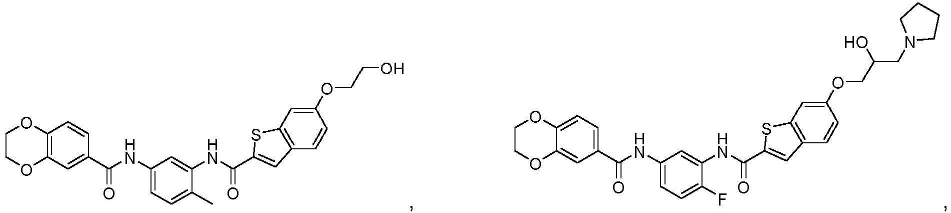 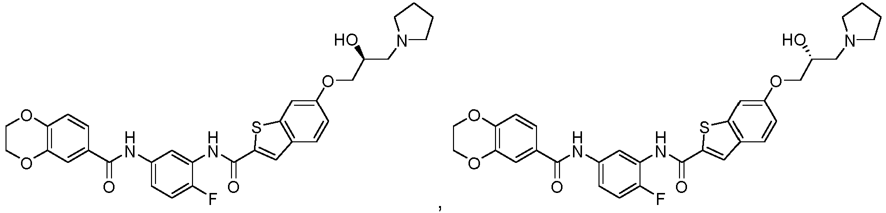 and the N-oxide, solvate or pharmaceutically acceptable salt thereof.

In an equally preferred embodiment in combination with any of the above or below embodiments, the compound is selected from the following group: 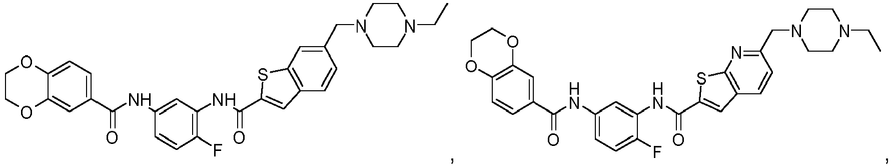 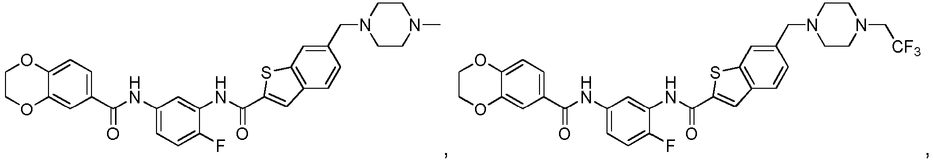 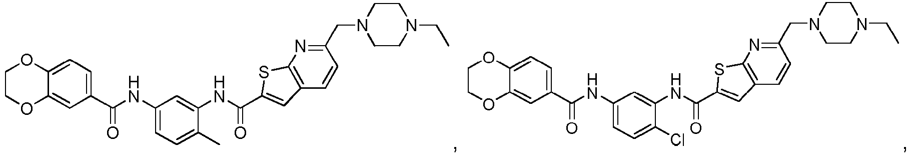 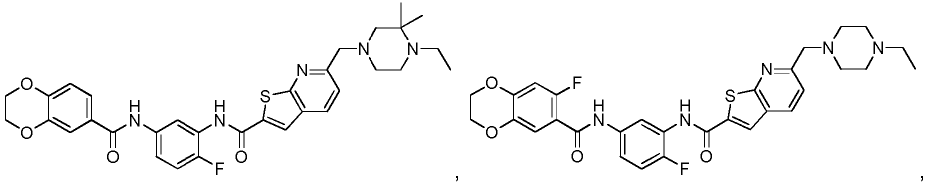 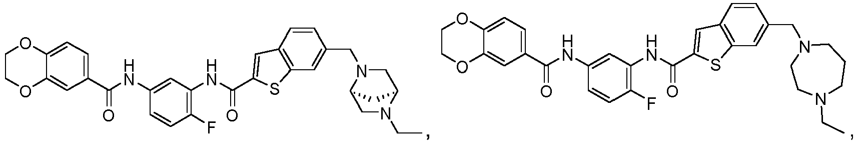 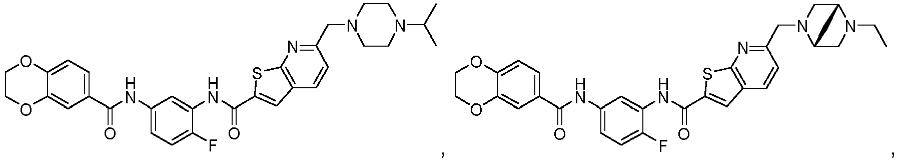 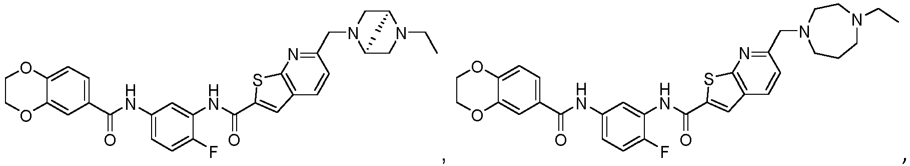 and the N-oxide, solvate or pharmaceutically acceptable salt thereof.

Compounds of the invention according to formula (I) exhibit a surprising biological activity.

Lactate lies at the crossroads between tumor metabolism and the late-stage cancer microenvironment, which resists the body's natural immune response. Specifically, lactate is the end product of the so-called Warburg metabolism, a metabolism prevalent in cancer cells which favors fermentative over oxidative pathways. Warburg metabolism converts glucose into lactate, diverting carbon flux into energy to support the growth of the ever-dividing cancer cells. At the same time, lactate and the associated acidic pH in the tumor environment also exert an immuno-suppressive effect which reduces the ability of immune cells to combat nascent or preexisting cancer cells. This "lactic acid immune shield" is permissive for only those immune cells that themselves use Warburg metabolism, and that are passive against the tumor. Such so-called myeloid-derived suppressor cells (MDSCs) support the cancer by signaling other active immune cells to stay out of the tumor, thus protecting the growing tumor by helping it to evade immune attack. In total, then, an increase in lactate and its associated acidity in the tumor microenvironment promotes tumor development both directly, by driving tumor growth *per se*, and indirectly, by inhibiting the body's ability to counter it.

It has been found that the compounds of the present invention inhibit lactate production. This activity reverses the tumor-promoting processes described above. Specifically, and without being bound by theory, reducing lactate concentration in cancer cells which depend on lactate as a source of energy has the effect of starving cancer cells of the energy they need for growth, thereby slowing or even halting cancer growth. Simultaneously, a reduction in lactate concentration in the tumor microenvironment weakens the "lactic acid immune shield" which otherwise normally protects cancer cells, thus advantageously exposing existing cancer cells to attack by cells of the body's own immune system. In this way, the tumor is impeded in its ability to spawn new cells and is rendered more vulnerable to immune attack and destruction.

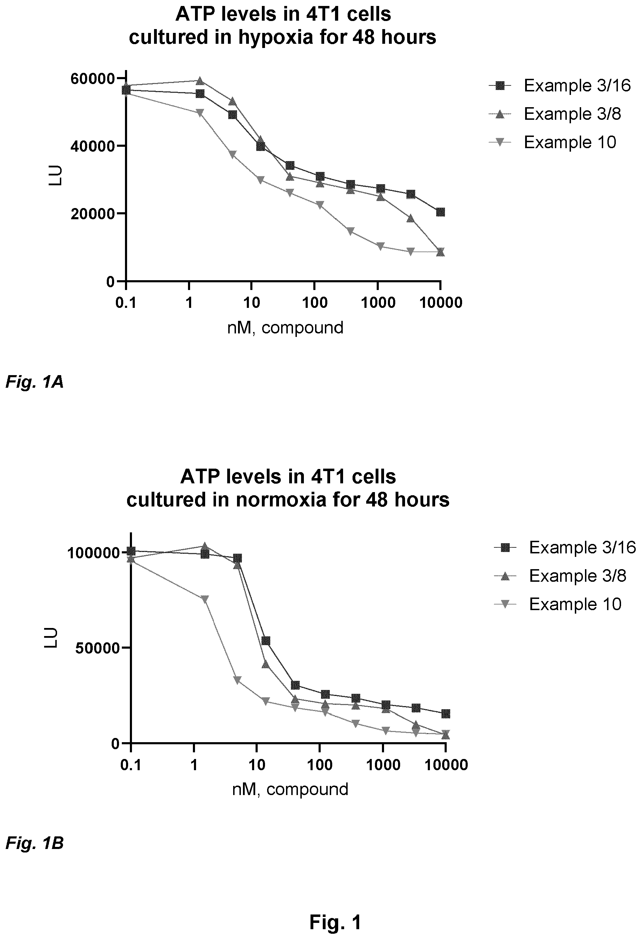

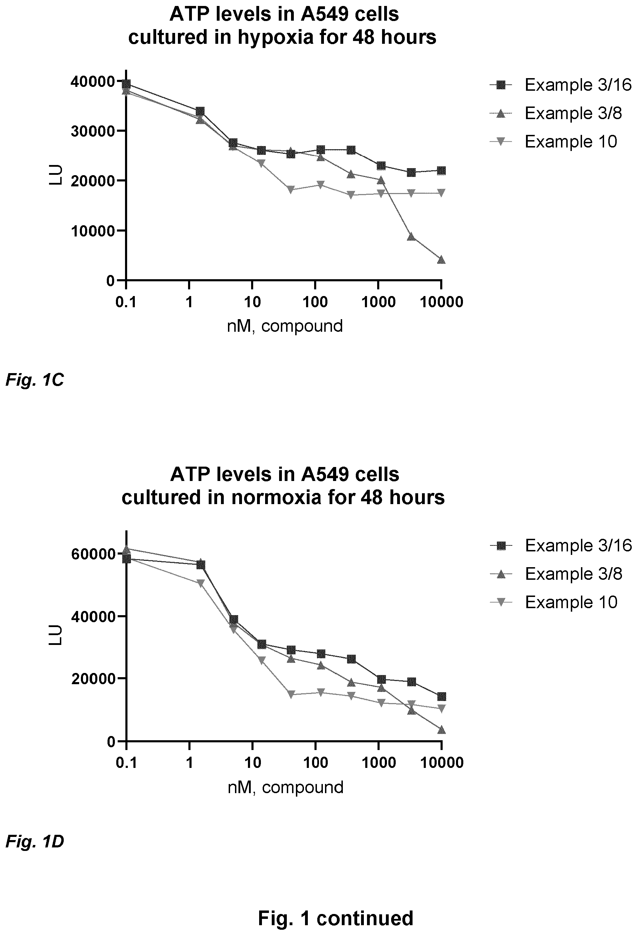

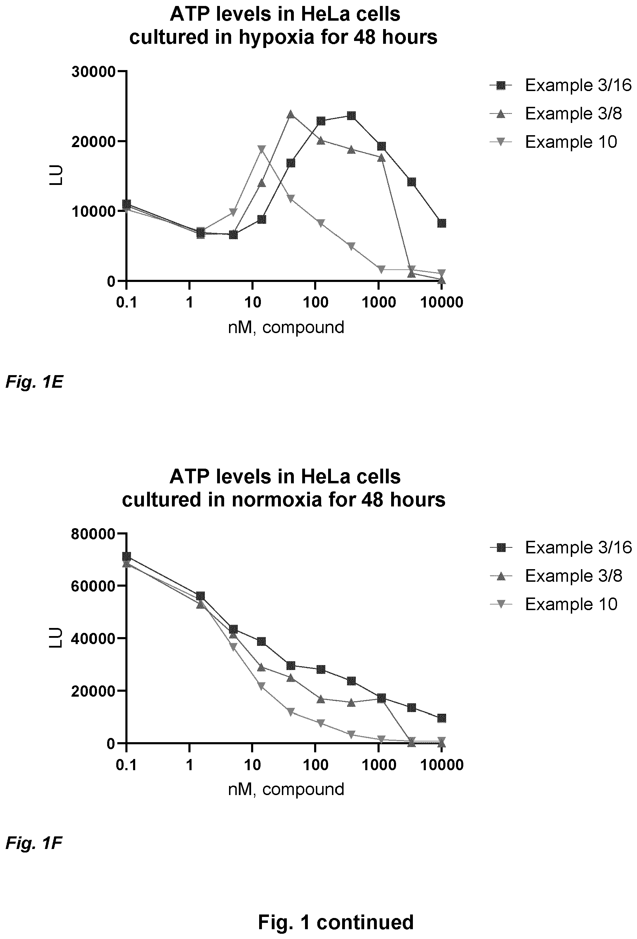

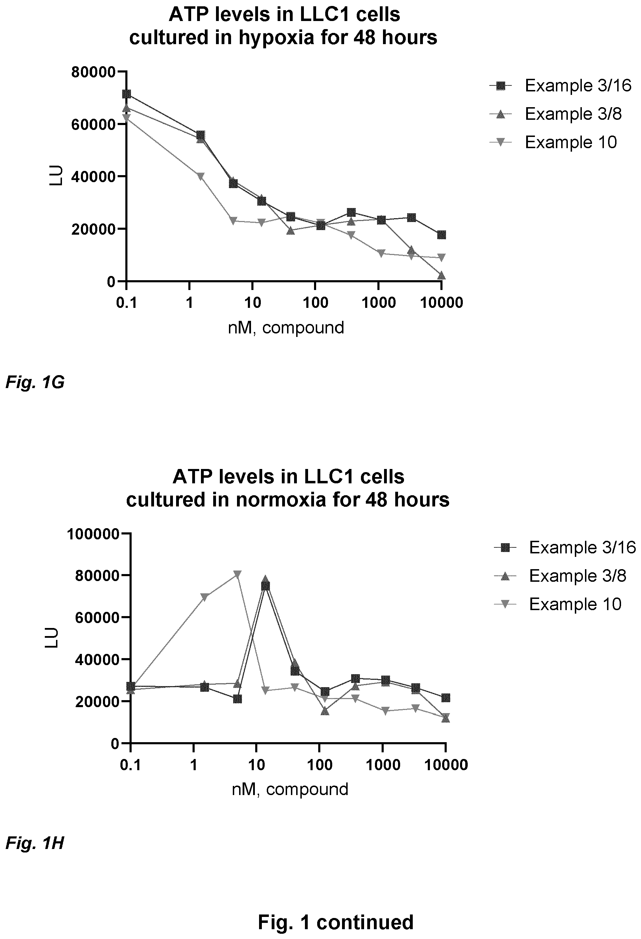

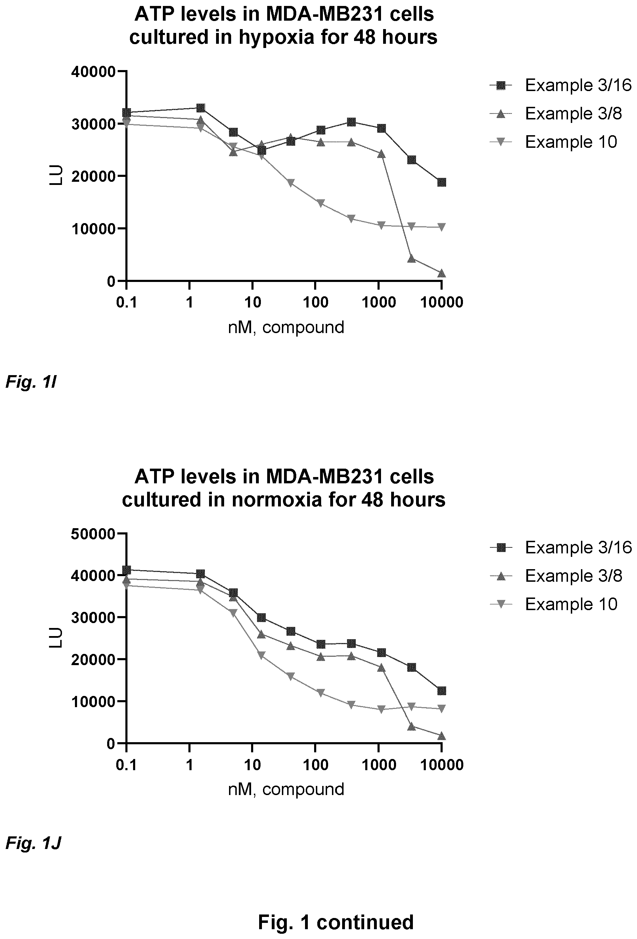

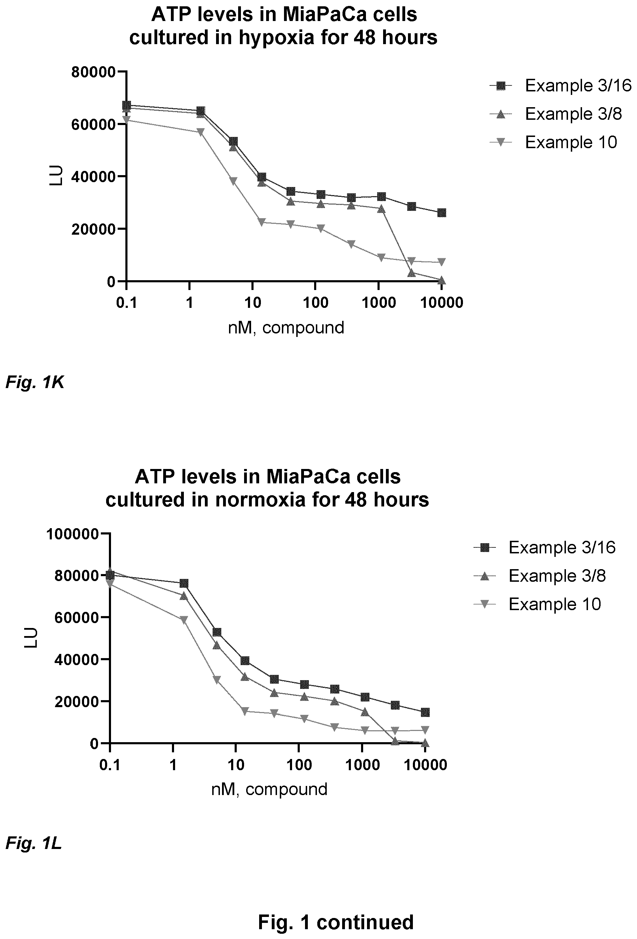

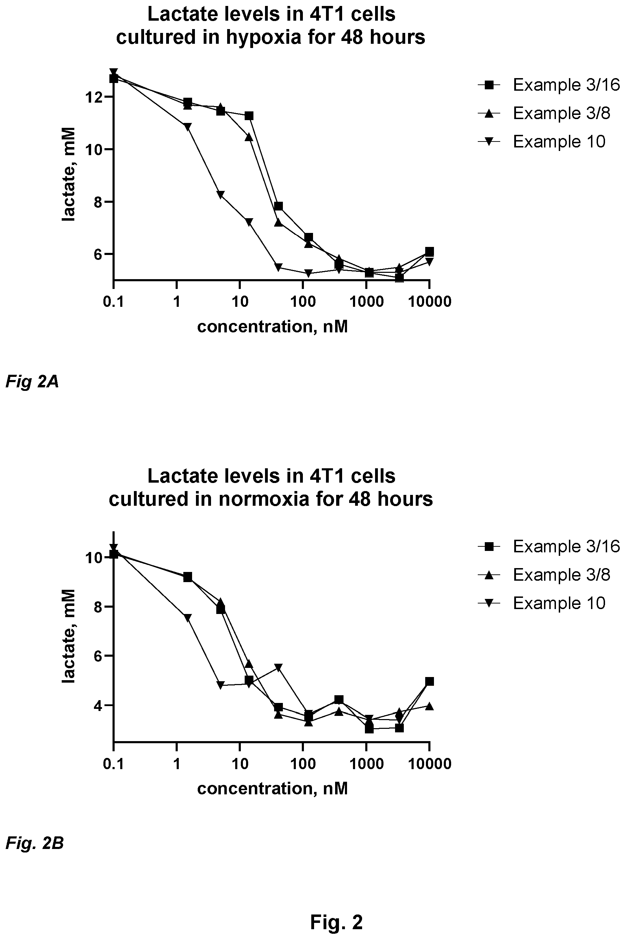

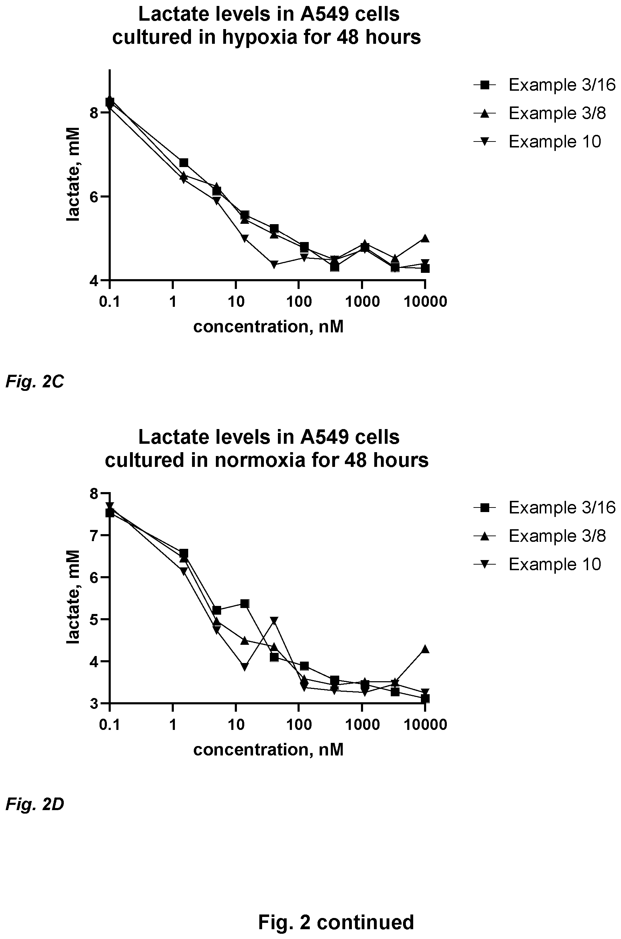

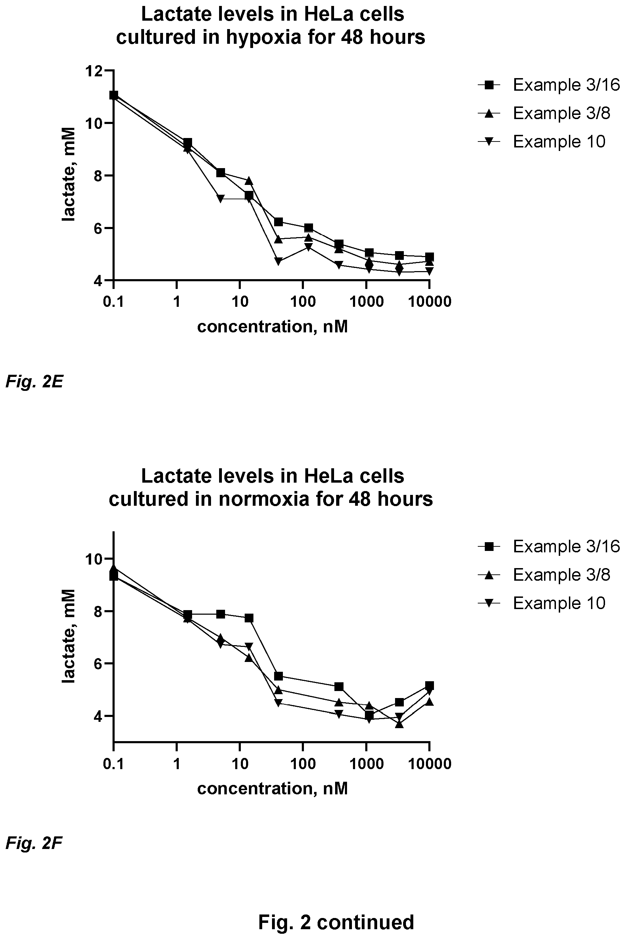

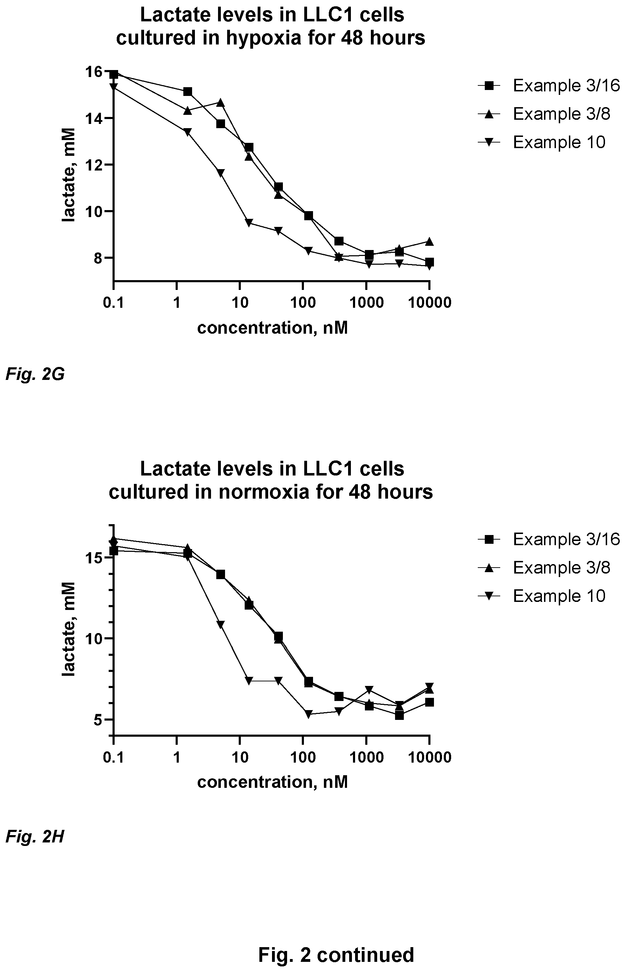

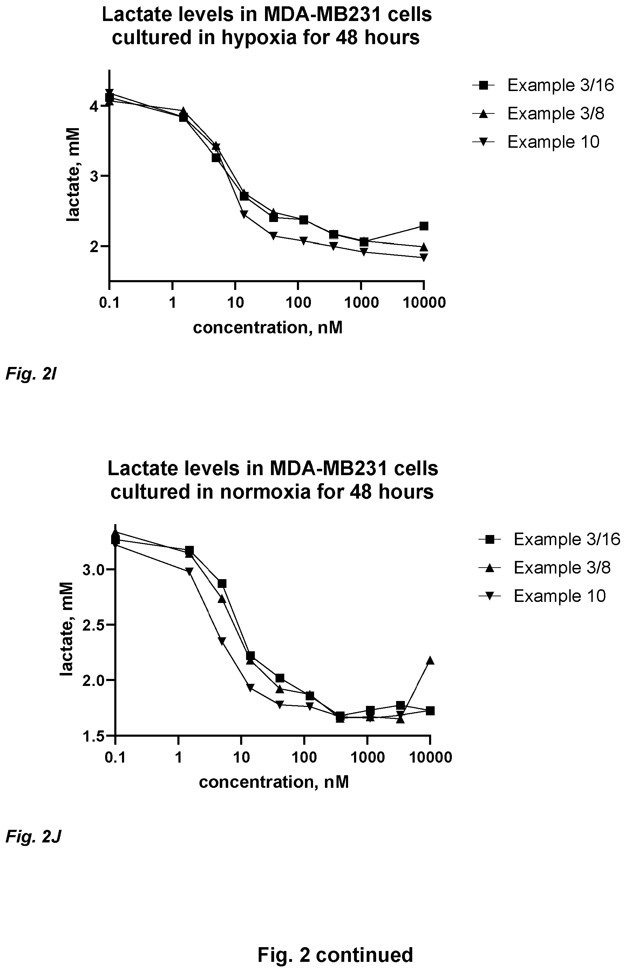

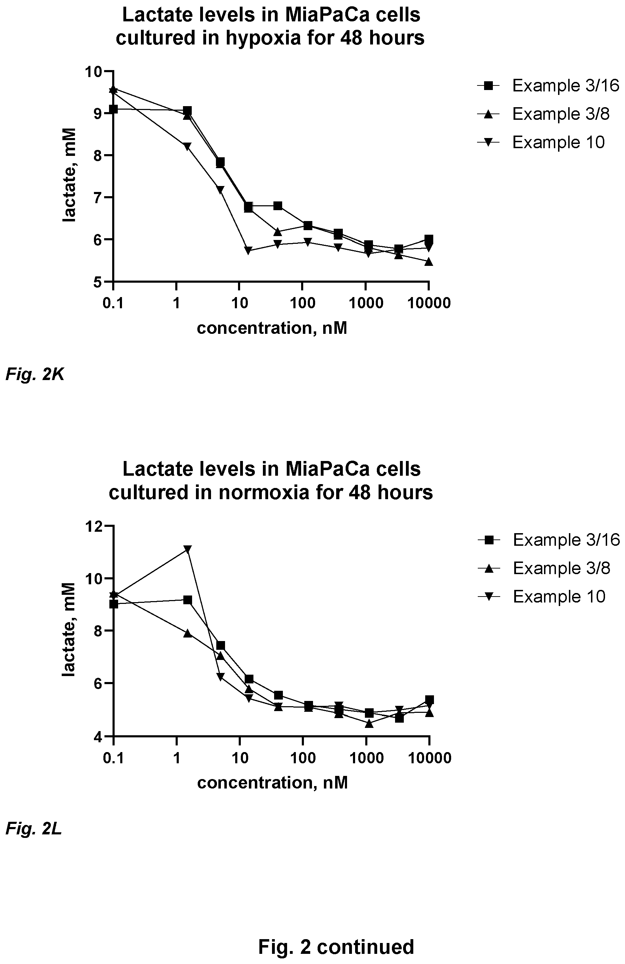

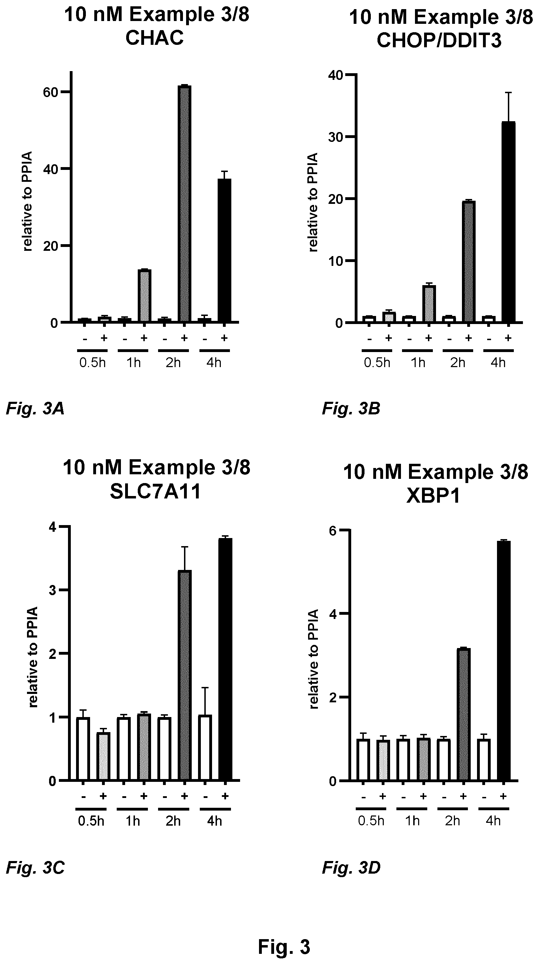

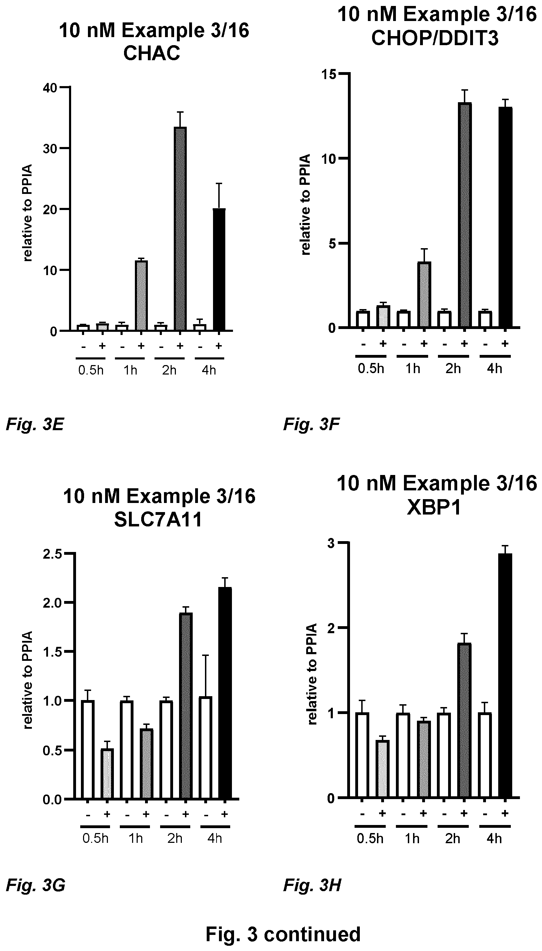

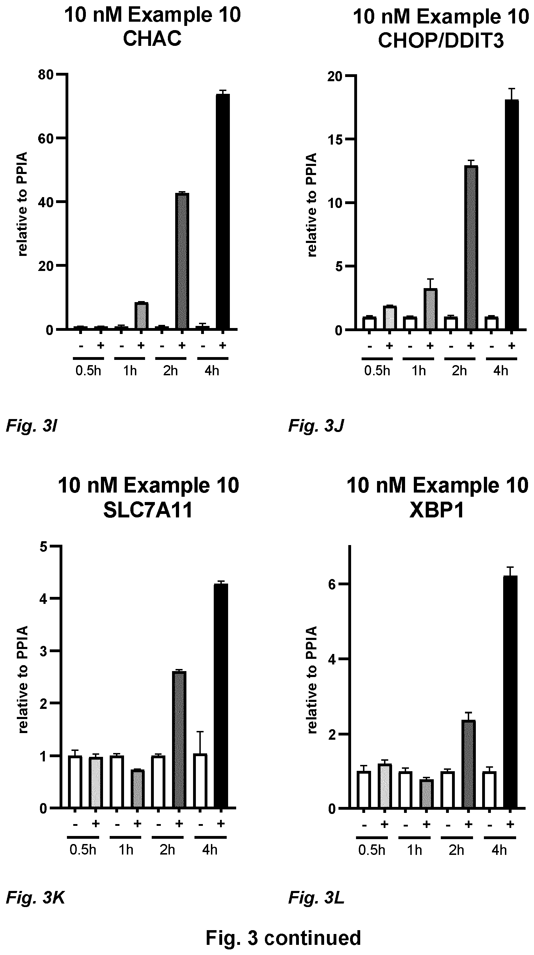

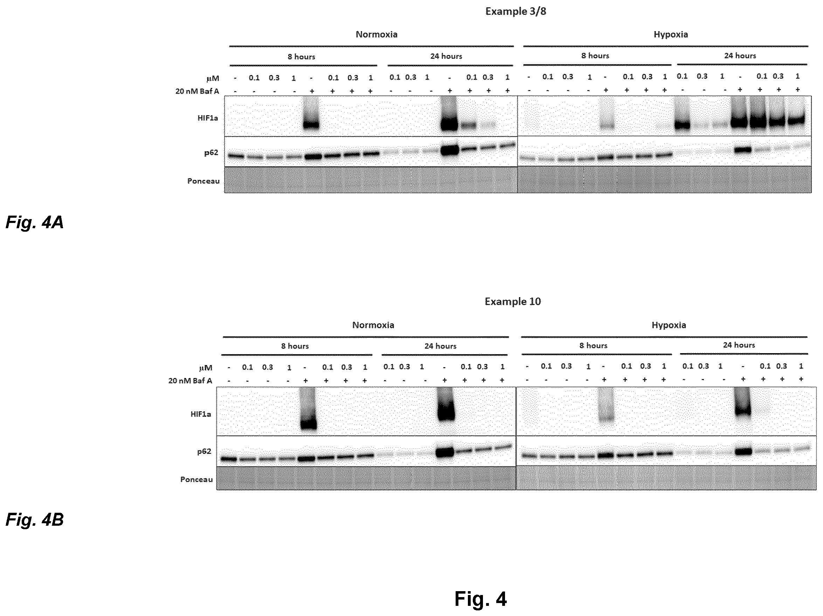

Specifically, a thorough investigation revealed that the compounds of the present invention not only inhibit cancerous proliferation but that this inhibition is mediated by an unprecedented lowering of ATP and lactate in cancer cell culture (Fig. 1, 2). A unique feature of this ATP-lowering mechanism is that it comes as a biphasic effect. In the first phase, ranging from single-digit nanomolar to low single-digit micromolar concentrations, depending on the actual compounds of invention, the heteroaryl bisamide compounds of the invention lower ATP and lactate and inhibit cellular proliferation but without inducing cell death, i.e. apoptosis or necrosis. Thus, the first phase can be called "cytostatic". A further analysis revealed that compounds of the present invention induced markers of endoplasmic reticulum stress such as CHAC-1, Chop, XBP-1 or SLC7A11 in the same concentration range where they displayed cytostatic effects (Fig. 3, H. Ren et al, Front. Aging Neurosci. (2021), 13: 691881). The induction of these ER stress markers as determined by quantitative real-time PCR preceded the concentration-dependent reduction in Hif1-alpha protein levels as determined from the same cancer cells (Fig. 4). Since Hif1-alpha plays a major role as master regulator of the transcriptional control of Warburg effect-related genes under hypoxic conditions in the tumor, this effect is likely one mechanism for the strong anti-proliferative effects observed for the compounds of the present invention.

Thus, the applicant herewith provides not only novel heteroaryl-core containing bisamide compounds with unprecedented activity against various cancer cell lines and with no predicted genotoxic potential, but also identifies novel biological mechanisms by which these compounds exert their anti-proliferative effects in such cancer cells.

A further aspect of the invention relates to a pharmaceutical composition comprising the compound according to any one of the aforementioned embodiments and a pharmaceutically acceptable excipient. In a preferred embodiment, the pharmaceutical composition may comprise about 5 wt% to about 98 wt% of one or more compounds of the invention, preferably from about 0.5 mg to about 0.5 g of active compound, even more preferably from about 1 mg to about 100 mg. In certain preferred embodiments, the pharmaceutical composition is formulated to allow administration to a subject enterally (for example orally, sublingually, and/or buccally), parenterally (e.g. intravenously, intramuscularly, subcutaneously), intranasally, topically, rectally, vaginally and/or by inhalation.

A further aspect of the invention relates to the compound according to any one of the aforementioned embodiments (i.e. compounds according to formula (I) and/or pharmaceutical compositions comprising the same), for use as a medicament.

A further aspect of the invention relates to the compound according to any one of the aforementioned embodiments or the pharmaceutical composition comprising the compound according to any one of the aforementioned embodiments for use in a method of preventing and/or treating a disease or condition mediated by the lactate/ATP mechanism, in particular cancer. In certain preferred embodiments, the method comprises administering the compound or the pharmaceutical composition to a subject enterally (for example orally, sublingually, and/or buccally), parenterally (for example intravenously, intramuscularly and/or subcutaneously), intranasally, topically, rectally, vaginally and/or by inhalation.

A further aspect of the invention relates to a method of treating a disease or condition mediated by the lactate/ATP mechanism, in particular cancer, in which an effective amount of a compound of the invention or of a pharmaceutical composition of the invention is administered to a subject in need thereof. In certain preferred embodiments, the method comprises administering the compound or the pharmaceutical composition to a subject enterally (for example orally, sublingually, and/or buccally), parenterally (for example intravenously, intramuscularly and/or subcutaneously), intranasally, topically, rectally, vaginally and/or by inhalation.

The present invention further relates to the use of a compound of the invention or a pharmaceutical composition of the invention in the manufacture of a medicament for treating a disease or condition mediated by the lactate/ATP mechanism, in particular cancer. In certain preferred embodiments, the medicament is formulated to allow administration of the compound or of the pharmaceutical composition to a subject enterally (for example orally, sublingually, and/or buccally), parenterally (for example intravenously, intramuscularly and/or subcutaneously), intranasally, topically, rectally, vaginally and/or by inhalation.

In a preferred embodiment in combination with any of the above or below embodiments, the disease or condition mediated by the lactate/ATP mechanism is cancer.

In a preferred embodiment in combination with any of the above or below embodiments, the cancer is selected from the group consisting of include cancers of the head and neck, eye, skin, mouth, throat, esophagus, chest, bone, lung, colon, sigmoid, rectum, stomach, prostate, uterine, breast, ovaries, kidney, liver, pancreas, brain, intestine, heart, or adrenal. More particularly, cancers include solid tumor, sarcoma, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendothelio sarcoma, synovioma, mesothelioma, Ewing's tumor, leiomyosarcoma, rhabdomyosarcoma, colon carcinoma, pancreatic cancer, breast cancer, ovarian cancer, uterine carcinoma, adenoic cystic carcinoma, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, non-small-cell lung carcinoma, bladder carcinoma, epithelial carcinoma, glioma, astrocytoma, medulloblastoma, craniopharyngioma, ependymoma, Kaposi's sarcoma, pinealoma, hemangioblastoma, acoustic neuroma, oligodendroglioma, menangioma, melanoma, neuroblastoma, retinoblastoma, a blood-borne tumor, acute lymphoblastic leukemia, acute lymphoblastic B-cell leukemia, acute lymphoblastic T-cell leukemia, acute myeloblastic leukemia, acute promyelocytic leukemia, acute monoblastic leukemia, acute erythroleukemic leukemia, acute megakaryoblastic leukemia, acute myelomonocytic leukemia, acutenonlymphocyctic leukemia, acute undifferentiated leukemia, chronic myelocytic leukemia, chronic lymphocytic leukemia, hairy cell leukemia, or multiple myeloma (see, e.g., Encyclopedia of Cancer, 3rd Edition (2018) Editors Paolo Boffetta, Pierre Hainaut). In a preferred embodiment in combination with any of the above or below embodiments, the cancer is a solid tumor. In accordance with a preferred embodiment, the cancer is selected from leukemia, melanoma, liver cancer, pancreatic cancer, lung cancer, colon cancer, brain cancer, ovarian cancer, breast cancer, prostate cancer, and renal cancer. In another embodiment, the cancer is liver cancer, pancreatic cancer, non-small cell lung cancer, breast cancer, or renal cancer. In a preferred embodiment in combination with any of the above or below embodiments, the cancer is selected from non-small-cell lung cancer, sarcoma, gynecological cancers such as cervix, ovarian cell or uterine carcinoma, adenoic cystic carcinoma, pancreatic cancer and other gastrointestinal cancers such as stomach or esophagus cancers, preferably in cases wherein the compound of the invention or a pharmaceutical composition of the invention is administered in combination with a biguanide.

The type of cancer is not particularly limited, but can be characterized as hypoxic and/or highly glycolytic relative to normal tissue of the same type. "Hypoxic" cells or tissue areas as used herein relates to one or more cells that are exposed, transiently or permanently, to an oxygen partial pressure (p0₂) that is lower than the typical p0₂ in cells in tissue that is considered as normal or healthy. Hypoxic cells or tissue areas can include, for example, cells or tissue areas with reduced or no access to vasculature, such as in a solid tumor.

The compounds of the present invention exert their anticancer and antiproliferative effects through a novel mechanism which induces cytostatic effects at lower and cytotoxic or cell killing effects at concentrations typically 100 -1000-fold higher than the cytostatic concentrations. The compounds of the present invention induce endoplasmatic reticulum stress in the first cytostatic phase which coincides with the onset of ATP and lactate lowering in cancer cell culture. This unique mechanism shown by the compounds of the invention allows for a certain therapeutic window, i.e. an effective dose of such a compound that can be used to stop cancerous growth without exerting cytotoxic side effects in the same patient.

In a preferred embodiment in combination with any of the above or below embodiments, the compound or the pharmaceutical composition is administered
- together with one or more therapeutic agents for cancer selected from the group consisting of a PD-1 agent, a PD-L1 agent, a CTLA-4 agent, an IDO1 inhibitor, and an anticancer vaccine, or
- together with a cytokine therapy or known chemo- or pharmacotherapy, or during irradiation therapy.

Preferred agents, inhibitors, vaccines and therapies according to this embodiment are as set out hereinbelow.

A further aspect of the invention relates to the compound according to a compound according to any one of the aforementioned embodiments (i.e. according to formula (I)) or a pharmaceutical composition comprising a compound according to any one of the aforementioned embodiments (i.e. according to formula (I)), for use in a method of preventing and/or treating an autophagy-related disease.

A further aspect of the invention relates to a method of preventing and/or treating an autophagy-related disease, the method comprising administering an effective amount of a compound according to any one of the aforementioned embodiments (i.e. according to formula (I)) or a pharmaceutical composition comprising a compound according to any one of the aforementioned embodiments (i.e. according to formula (I)) to a subject in need thereof.

The present invention further relates to the use of a compound according to any one of the aforementioned embodiments (i.e. according to formula (I)) or a pharmaceutical composition comprising a compound according to any one of the aforementioned embodiments (i.e. according to formula (I)) in the manufacture of a medicament for preventing and/or treating an autophagy-related disease.

In an embodiment in combination with any of the above or below embodiments, the autophagy-related disease is preferably selected from the group consisting of neuro-degenerative diseases such as Parkinson's disease, Alzheimer's disease and Huntington's disease; amyotrophic lateral sclerosis; metabolic disorders such as obesity and diabetes type I and II; viral infections and diseases which lead to accumulation of certain debris particles, such as fibrotic lung diseases.

### Combination of the compounds of the present invention with biguanides

As set out herein, compounds according to formula (I) exhibit advantageous utility in the treatment of certain diseases, in particular cancer. In addition, it has surprisingly been found that the compounds according to formula (I) also exhibit unexpectedly advantageous utility in combination with biguanide compounds in the treatment of certain diseases, in particular cancer.

As explained herein, biguanide compounds such as metformin, phenformin and buformin are known for their activity in treating diabetes as well as cancer, however certain of these compounds (in particular buformin and the potent phenformin) lead to toxic lactic acidosis at higher doses, effectively limiting the dose at which biguanides can be administered to below the doses needed for therapeutic efficacy, especially in treating cancer.

At the same time, as described above, the inventive compounds according to formula (I) themselves exhibit anticancer activity which, without being bound by theory, appears to be at least partly associated with these compounds' ability to reduce lactate levels.

It has now surprisingly been found that combination therapy involving administration of a compound according to formula (I) of the present invention together with a biguanide compound allows one to tap the therapeutic potential of each of these compounds, while at the same time attenuating or eliminating the disadvantageous side effect of lactic acidosis which has previously restricted the application of biguanides at the higher doses typically of interest for treatment of diseases, in particular for treatment of cancer. Such combination of compounds according to the present invention (i.e. according to formula (I)) with biguanide compounds thus widens the therapeutic window for the safe administration of biguanide compounds, synergistically leveraging the activities of each of these compounds in the prevention and/or treatment of disease, in particular during the prevention and/or treatment of cancer.

Accordingly, a further aspect of the invention relates to a composition comprising a compound according to formula (I) and a biguanide. The biguanide may preferably be metformin, phenformin and/or buformin. The composition may preferably be a pharmaceutical composition comprising, in addition to the inventive compound according to formula (I) and a biguanide, a pharmaceutically acceptable excipient as specified herein. The composition may be in any form, but a preferred form is a form suitable for oral administration during prevention and/or treatment, as specified elsewhere herein.

A further aspect of the invention relates to a kit comprising a compound according formula (I) and a biguanide. The kit may comprise the compound according to formula (I) and the biguanide in separate containers. If separate containers, these containers may be of the same or different types, as most suitable for substances' intended routes of delivery. The compound according to formula (I) and the biguanide as comprised in the kit may be in the same or different delivery forms, i.e. may be present in the kit in the same or different formulations. For example, the compound according to formula (I) comprised within the kit may be in a formulation suitable for intravenous delivery as set out herein (for example a solution in a septum-fitted receptacle), while the biguanide comprised within the same kit may be in a formulation suitable for oral delivery as set out herein (for example a tablet or capsule comprised, for example, within a blister package).

A further aspect of the invention relates to the composition comprising a compound according to formula (I) and a biguanide, or the kit comprising a compound according to formula (I) and a biguanide, for use as a medicament. A related aspect of the invention relates to a method of preventing and/or treating a disease, said method comprising administering a combination of a compound according to formula (I), or a pharmaceutical composition comprising the compound according to formula (I), and a biguanide, to a subject. A further related aspect of the invention relates to a combination of a compound according to formula (I) and a biguanide, for use in therapy. The combination of these substances during prevention and/or treatment of disease is associated with the advantages specified herein.

As further elaborated herein, while the combination therapy with a compound according to formula (I) and a biguanide is especially well-suited to treatment of cancer, such combination therapy may also be of similar utility in preventing and/or treating other diseases. For instance, this combination therapy may also be applied to the prevention and/or treatment of such diseases as type II diabetes mellitus (T2DM); neurodegenerative diseases such as for instance dementia, in particular Alzheimer's dementia, Parkinson's disease, Huntington's disease and Amyotrophic Lateral Sclerosis (ALS). The combination of these substances during prevention and/or treatment of disease is associated with the advantages specified herein. Due to the therapeutic potential of this combination therapy in the treatment of cancer, its application to cancer is discussed in further detail below.

Accordingly, a further aspect of the invention relates to a combination of a compound according to formula (I) and a biguanide, or a kit comprising a compound according to formula (I) and a biguanide, for use in a method of preventing and/or treating cancer. The combination of these substances, i.e. of a compound according to formula (I) and a biguanide, during prevention and/or treatment of cancer is associated with the advantages specified herein. Preferably, the cancer which is prevented and/or treated is an advanced stage cancer, in particular an advanced stage glycolytic cancer. Suitable doses of a compound according to formula (I) of the present invention in this combination therapy are as indicated elsewhere herein. In cases of combination therapy with a compound according to formula (I) of the present invention together with metformin as the biguanide, the metformin is advantageously provided at a dose of about 500 mg to about 2000 mg twice, thrice or four times daily. In such a case, administration of metformin is preferably via the oral route. In cases of combination therapy with a compound according to formula (I) of the present invention together with phenformin as the biguanide, the phenformin is advantageously provided at a dose of about 25 mg to about 100 mg twice or thrice daily. In such a case, administration of phenformin is preferably via the oral route. In cases of combination therapy with a compound according to formula (I) of the present invention together with buformin as the biguanide, the buformin is advantageously provided at a dose of about 50 mg to about 1000 mg twice or thrice daily. In such a case, administration of buformin is preferably via the oral route.

In a further aspect, the present invention relates to a method of preventing and/or treating cancer, said method comprising administering a combination of a compound according to formula (I), or a pharmaceutical composition comprising the compound according to formula (I), and a biguanide, to a subject. The combination of these substances, i.e. of a compound according to formula (I) and a biguanide, during prevention and/or treatment of cancer is associated with the advantages specified herein. Preferably, the cancer which is prevented and/or treated is an advanced stage cancer, in particular an advanced stage glycolytic cancer. Suitable doses of a compound according to formula (I) of the present invention in this combination therapy are as indicated elsewhere herein. In cases of combination therapy with a compound according to formula (I) of the present invention together with metformin as the biguanide, the metformin is advantageously provided at a dose of about 500 mg to about 2000 mg twice, thrice or four times daily. In such a case, administration of metformin is preferably via the oral route. In cases of combination therapy with a compound according to formula (I) of the present invention together with phenformin as the biguanide, the phenformin is advantageously provided at a dose of about 25 mg to about 100 mg twice or thrice daily. In such a case, administration of phenformin is preferably via the oral route. In cases of combination therapy with a compound according to formula (I) of the present invention together with buformin as the biguanide, the buformin is advantageously provided at a dose of about 50 mg to about 1000 mg twice or thrice daily. In such a case, administration of buformin is preferably via the oral route.

In a further aspect, the present invention relates to the use of a combination of a compound according to formula (I), or a pharmaceutical composition comprising the compound according to formula (I), and a biguanide, in the manufacture of a medicament for preventing and/or treating cancer. The combination of these substances, i.e. a compound according to formula (I) and a biguanide, during prevention and/or treatment of cancer is associated with the advantages specified herein. Preferably, the cancer which is prevented and/or treated is an advanced stage cancer, in particular an advanced stage glycolytic cancer. Suitable doses of a compound according to formula (I) of the present invention in this combination therapy are as indicated elsewhere herein. In cases of combination therapy with a compound according to formula (I) of the present invention together with metformin as the biguanide, the metformin is advantageously provided at a dose of about 500 mg to about 2000 mg twice, thrice or four times daily. In such a case, administration of metformin is preferably oral. In cases of combination therapy with a compound according to formula (I) of the present invention together with phenformin as the biguanide, the phenformin is advantageously provided at a dose of about 25 mg to about 100 mg twice or thrice daily. In such a case, administration of phenformin is preferably oral. In cases of combination therapy with a compound according to formula (I) of the present invention together with buformin as the biguanide, the buformin is advantageously provided at a dose of about 50 mg to about 1000 mg twice or thrice daily. In such a case, administration of buformin is preferably oral.

In a preferred embodiment of the above combination prevention and/or therapy entailing a compound according to formula (I) of the invention together with a biguanide, when the prevented and/or treated disease is cancer, the cancer may preferably be an LKB-1-deficient cancer. Herein, an LKB-1-deficient cancer is understood as a human cancer that harbors a mutation, deletion or translocation of the skt11 gene encoding LKB-1 (**l**iver **k**inase **B1**, see e.g. UNIPROT accession number Q15831, and/or NCBI accession number NP_000446) which attenuates or eliminates its expression. In one preferred embodiment, the LKB-1-deficient cancer may be one or more subforms of skt11/LKB-1 mutated non-small-cell lung cancer (NSCLC, see Facchinetti et al, Lung Cancer.112:62-68. (2017)). Other cancer types that frequently show mutations, deletions or translocations of LKB-1 functions are Sarcoma, Cholangiocarcinoma, Adenoid Cystic Carcinoma or certain gynecological cancers such as Cervical, Ovarian or Uterine Carcinomas (see Li et al., Biomed Pharmacother. 132:110872. (2020)).

In a further preferred embodiment of the above combination prevention and/or therapy entailing a compound according to formula (I) of the invention together with a biguanide, when the prevented and/or treated disease is cancer, the cancer may preferably be a cancer that harbors a mutation, deletion or translocation of the arid1a gene encoding Arid1a. ARID1a is the gene which in humans encodes the **A**T-rich **i**nteractive **d**omain-containing protein 1A (see e.g. UNIPROT accession number 014497 and/or NCBI accession numbers NP_006006 and NP_624361). Elevated proteotoxic stress through the genetic impairment of Arid1a together with a pharmacological treatment that results in further increased proteotoxic stress response provides a kind of "synthetic lethality" situation which has turned out to be an excellent prerequisite, in general, for preferred pharmacological treatments (Huang et al.,Nat Rev Drug Discov 19(1):23-38 (2020).

In an especially preferred embodiment of the above combination prevention and/or therapy entailing a compound according to formula (I) of the invention together with a biguanide, when the prevented and/or treated disease is cancer, the cancer may preferably be a cancer which is simultaneously LKB-1-deficient and ARID1a-mutated. Without being bound by theory, such doubly deficient/mutated cancers are especially amenable to treatment by combination therapy with a compound according to formula (I) of the invention together with a biguanide, because an additional mutation in ARID1a in a cancer which is already deficient in LKB-1 renders the LKB-1-deficient cancer more prone to proteotoxic stress. In an especially preferred embodiment, one such cancer harboring mutations in both LKB-1 and ARID1a is non-small-cell lung cancer.

### Definitions

All technical and scientific terms used hereafter carry the commonly understood meaning of the word unless defined otherwise.

It is to be understood that section headings as used throughout the present application are merely for organizational purposes, and do not confer exclusive character to the disclosure pertaining to aspects of the invention mentioned in a section headings. Section headings thus do not exclude the combination of the disclosure within any one section heading to a corresponding or analogous context provided under another section heading. Such intra-section disclosures are explicitly within the disclosure of the present application.

As used herein, each of the terms "comprising', "having" and "containing", including grammatical variants thereof, are meant in a non-exhaustive sense to mean "including", but not necessarily "composed of", and does not exclude elements in addition to those explicitly recited as being present. As used herein, then, the terms "comprising", "having" and "containing", and grammatical variants thereof, indicate that components other than those explicitly recited may, but need not, be present. As such these terms include as a limiting case embodiments in which no other elements than those recited are present, e.g. in the commonly accepted sense of "consisting of".

As used herein, the phrase "consisting of", and grammatically related variants thereof, means that no other elements are present in those recited. In standing with the above definition of "comprising" (and grammatically and semantically related terms), the term "consisting of" therefore denotes a limiting scenario within the meaning of "comprising".

Unless defined otherwise, any feature within any aspect or embodiment of the invention may be combined with any feature within any other aspect or embodiment of the invention, and the skilled person understands such combination as being encompassed in the original disclosure of the present application. This applies in particular to all embodiments described within the section relating to compounds *per se*, in respect of other aspects, e.g. methods and uses, of those compositions. This also applies in particular, but not exclusively, to endpoints of ranges disclosed herein. For instance, if a given substance is disclosed as existing in a composition in a concentration range of about X-Y% or about A-B%, the present application is to be understood as explicitly disclosing not only the ranges about X-Y% and about A-B%, but also the ranges about X-B%, about A-Y% and, in as far as numerically possible, about Y-A% and about B-X%. Each of these ranges, and range combinations, are contemplated, and are to be understood as being directly and unambiguously disclosed in the present application.

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from about X to about Y". Accordingly, the expressions of ranges as "X -Y", "of X to Y", "from X to Y", "of X - Y" and "from X - Y" are to be understood equivalently as meaning and disclosing a range encompassing the end value X, all values between X and Y, as well as the end value Y. In the event that the range in question specifies the number of atoms in an entity where only integral values would be technically meaningful, for instance a chemical substituent such as the term "C₁-C₆ alkyl" or, equivalently, "C₁₋₆ alkyl", the specified range is understood as meaning and disclosing each integral value within that range. For instance, in the example of "C₁-C₆ alkyl" (or equivalently "C₁₋₆ alkyl"), this term is to be understood as meaning and directly and unambiguously disclosing each of the separate integral options "C₁ alkyl", "C₂ alkyl", "C₃ alkyl", "C₄ alkyl", "C₅ alkyl" and "C₆ alkyl".

The designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included. As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below an indicated specific value, said percentage values being relative to the specific recited value itself, as follows. The term "about" may denote variability of ± 5.0%. The term "about" may denote variability of ± 4.9%. The term "about" may denote variability of ± 4.8%. The term "about" may denote variability of ± 4.7%. The term "about" may denote variability of ± 4.6%. The term "about" may denote variability of ± 4.5%. The term "about" may denote variability of ± 4.4%. The term "about" may denote variability of ± 4.3%. The term "about" may denote variability of ± 4.2%. The term "about" may denote variability of ± 4.1%. The term "about" may denote variability of ± 4.0%. The term "about" may denote variability of ± 3.9%. The term "about" may denote variability of ± 3.8%. The term "about" may denote variability of ± 3.7%. The term "about" may denote variability of ± 3.6%. The term "about" may denote variability of ± 3.5%. The term "about" may denote variability of ± 3.4%. The term "about" may denote variability of ± 3.3%. The term "about" may denote variability of ± 3.2%. The term "about" may denote variability of ± 3.1%. The term "about" may denote variability of ± 3.0%. The term "about" may denote variability of ± 2.9%. The term "about" may denote variability of ± 2.8%. The term "about" may denote variability of ± 2.7%. The term "about" may denote variability of ± 2.6%. The term "about" may denote variability of ± 2.5%. The term "about" may denote variability of ± 2.4%. The term "about" may denote variability of ± 2.3%. The term "about" may denote variability of ± 2.2%. The term "about" may denote variability of ± 2.1%. The term "about" may denote variability of ± 2.0%. The term "about" may denote variability of ± 1.9%. The term "about" may denote variability of ± 1.8%. The term "about" may denote variability of ± 1.7%. The term "about" may denote variability of ± 1.6%. The term "about" may denote variability of ± 1.5%. The term "about" may denote variability of ± 1.4%. The term "about" may denote variability of ± 1.3%. The term "about" may denote variability of ± 1.2%. The term "about" may denote variability of ± 1.1%. The term "about" may denote variability of ± 1.0%. The term "about" may denote variability of ± 0.9%. The term "about" may denote variability of ± 0.8%. The term "about" may denote variability of ± 0.7%. The term "about" may denote variability of ± 0.6%. The term "about" may denote variability of ± 0.5%. The term "about" may denote variability of ± 0.4%. The term "about" may denote variability of ± 0.3%. The term "about" may denote variability of ± 0.2%. The term "about" may denote variability of 0.1%. The term "about", in reference to the particular recited value, may denote that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y".

As used herein, "C₁₋₆-alkyl" means a saturated alkyl chain having 1, 2, 3, 4, 5 or 6 carbon atoms which may be straight chained or branched. In the context of the present invention, any subgroup falling within the term "C₁₋₆-alkyl" is also encompassed, such as a C₁₋₃-alkyl, C₂₋₅-alkyl or C₃₋₆-alkyl group. Examples of the C₁₋₆-alkyl group include methyl, ethyl, propyl, isopropyl, *n*-butyl, isobutyl, *tert*-butyl, *n*-pentyl, isopentyl, neopentyl, and hexyl.

A "C₁₋₆-alkylene" means that the respective group is divalent and connects an attached residue with the remaining part of the molecule. Moreover, in the context of the present invention, C₁-alkylene means a methylene linker, C₂-alkylene means an ethylene linker or a methyl-substituted methylene linker and so on. In the context of the present invention, a C₁₋₆-alkylene preferably represents a methylene or ethylene group.

The term "OC₁₋₆-alkyl" means that the alkyl chain is connected via an oxygen atom with the remainder of the molecule.

The term "halo-C₁₋₆-alkyl" means that one or more hydrogen atoms in the alkyl chain are replaced by a halogen. A preferred example thereof is CF₃. O-halo-C₁₋₆-alkyl means an alkoxy group with one or more hydrogen atoms being replaced by a halogen.

A C₃₋₆-cycloalkyl group means a saturated or partially unsaturated mono- or bicyclic ring system comprising 3, 4, 5 or 6 carbon atoms. Examples include cyclopropyl, cyclobutyl, cyclopentyl, cyclohexyl and cyclohexenyl.

A 4-8 membered mono or bicyclic heterocycloalkyl group means a saturated or partially unsaturated 4-8 membered mono or bicyclic carbon ring wherein up to 4 carbon atoms may be replaced by up to 4 heteroatoms, and wherein the heteroatoms are independently selected from N, O, S, SO and SO₂. Examples thereof include epoxidyl, oxetanyl, pyrrolidinyl, tetrahydrofuranyl, piperidinyl, piperazinyl, tetrahydropyranyl, 1,4-dioxanyl, morpholinyl, 4-quinuclidinyl, 1,4-dihydropyridinyl and 6-azabicyclo[3.2.1]octanyl. The heterocycloalkyl group can be connected with the remaining part of the molecule via a carbon, nitrogen (e.g. in morpholine or piperidine) or sulfur atom.

A cycloalkyl or heterocycloalkyl substituent may be spirocyclicly fused to the remainder of the molecule. In the context of the present invention, "spirocyclicly fused" means that the cycloalkyl or hetercycloalkyl group is connected through a single common atom with the remainder of the molecule, i.e. the ring shares a common atom with another moiety. An example of a spiro connection is shown below:

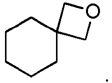

A 5- or 6-membered heteroaromatic ring system (also referred to herein as heteroaryl) containing up to 4 heteroatoms means a monocyclic heteroaromatic ring such as pyrrolyl, imidazolyl, furanyl, thiophenyl, pyridinyl, pyrimidinyl, pyrazinyl, pyrazolyl, oxazolyl, isoxazolyl, triazolyl, oxadiazolyl and thiadiazolyl.

A nitrogen atom of a heteroaryl system may also be optionally oxidized to the corresponding N-oxide. Further, the nitrogen atom of a heteroaryl system may also be optionally quaternized. If not stated otherwise, the heteroaryl system can be connected via a carbon or nitrogen atom.

Moreover, where not explicitly defined, the term "heteroaryl" contains 1 to 4 heteroatoms independently selected from the group consisting of N, O and S.

A 6-membered aromatic ring system (within the application also referred to as aryl) means an aromatic carbon cycle such as phenyl.

The structural moiety 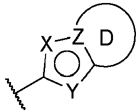 is to be understood to represent a 8- or 9-membered bicyclic heteroaryl ring exemplified by but not limited to:

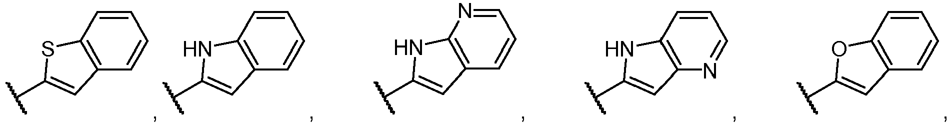

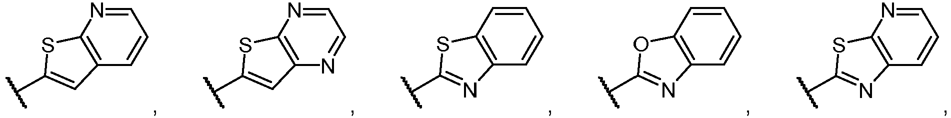

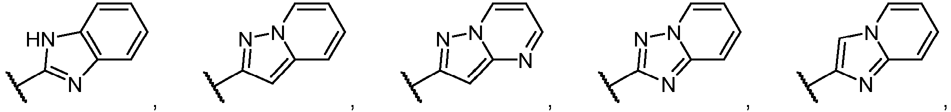

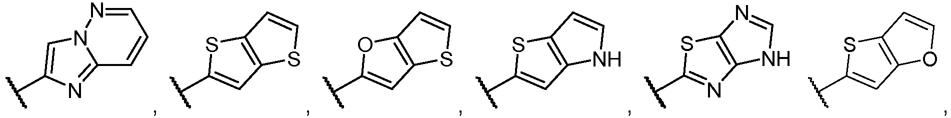

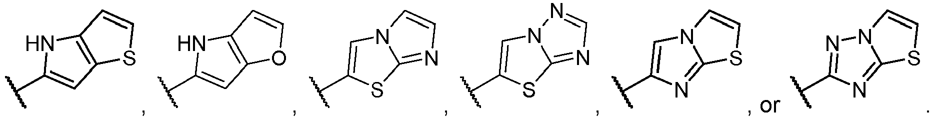

The structural moiety 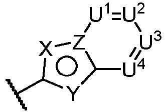 in formula (II) is to be understood to represent a 9-membered bicyclic heteroaryl ring exemplified by but not limited to: 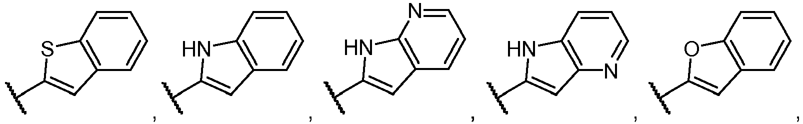 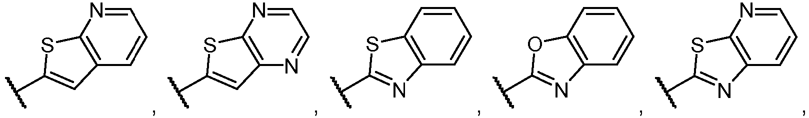 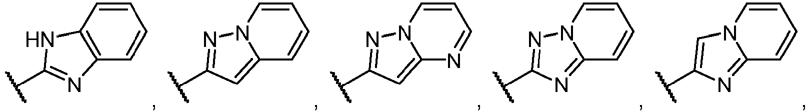 or

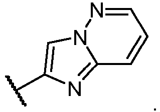

The term "halogen" comprises the specific halogen atoms fluorine, bromine, chlorine and iodine.

It will be appreciated by the skilled person that when lists of alternative substituents include members which, because of their valency requirements or other reasons, cannot be used to substitute a particular group, the list is intended to be read with the knowledge of the skilled person to include only those members of the list which are suitable for substituting the particular group without contravening valency rules.

Any formula or structure given herein, is intended to represent unlabelled forms as well as isotopically labelled forms of the compounds. Isotopically labelled compounds have structures depicted by the formulas given herein, except that one or more atoms is/are replaced by an atom having a selected atomic mass or mass number. Examples of isotopes that can be incorporated into compounds of the disclosure include isotopes of hydrogen, carbon, nitrogen, oxygen, fluorine and chlorine, such as, but not limited to ²H (deuterium, D), ³H (tritium), ¹¹C, ¹³C, ¹⁴C, ¹⁵N, ¹⁸F, ³⁵S, ³⁶Cl and ¹²⁵I. The present invention therefore also encompasses such isotopically labelled compounds, for example those containing radioactive isotopes such as ³H, ¹³C and ¹⁴C. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of patients. Isotopically labelled compounds of the invention can generally be prepared by carrying out the procedures disclosed in the schemes or in the examples and preparations described below by substituting a readily available isotopically labelled reagent for a non-isotopically labelled reagent.

The present invention also includes "deuterated analogs" of compounds of Formula (I) in which from 1 to n hydrogens attached to a carbon atom is/are replaced by deuterium, in which n is the number of hydrogens in the molecule. Such compounds may exhibit increased resistance to metabolism and thus be useful for increasing the half-life of any compound of Formula (I) when administered to a mammal, e.g. a human. See, for example, Foster in Trends Pharmacol. Sci. 1984:5;524. Such compounds are synthesized by means well known in the art, for example by employing starting materials in which one or more hydrogens have been replaced by deuterium, or are enriched in deuterium content at the position(s) in question.

Deuterium labelled or substituted therapeutic compounds of the invention may have improved DMPK (drug metabolism and pharmacokinetics) properties, relating to distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index. An ¹⁸F labelled compound may be useful for PET or SPECT studies.

The concentration of such a heavier isotope, specifically deuterium, may be defined by an isotopic enrichment factor. In the compounds of the invention, any atom not specifically designated as a particular isotope is meant to represent any stable isotope of that atom. Unless otherwise stated, when a position is designated specifically as "H" or "hydrogen", the position is understood to have hydrogen at its natural abundance isotopic composition.

Where tautomerism, like e.g. keto-enol tautomerism, of compounds of the present invention may occur, the individual forms, like e.g. the keto and enol form, are each within the scope of the invention as well as their mixtures in any ratio. The same applies for stereoisomers, like e.g. enantiomers, cis/trans isomers, conformers and the like.

If desired, isomers can be separated by methods well known in the art, e.g. by liquid chromatography. The same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of the present invention may be obtained from stereoselective synthesis using optically pure starting materials. Another way to obtain pure enantiomers from racemic mixtures would use enantioselective crystallization with chiral counterions, by methods known in the art.

As used herein, "metformin" refers to the compound having the following chemical structure:

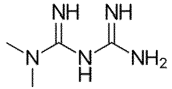

As used herein, "phenformin" refers to the compound having the following chemical structure:

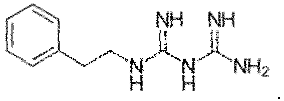

As used herein, "buformin" refers to the compound having the following chemical structure:

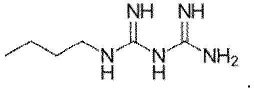

Administration of one or more compounds of the invention "together with" or "in combination with" another substance or substances or type of therapy, as used herein, need not denote simultaneity of administration. Rather, a "combination" of 2 or more compounds as used herein, such as administration of one compound "together with" or "in combination with" another or others or a type of therapy encompasses and discloses multiple possibilities regarding the route and timing of administration of the respective substances and/or therapies. Encompassed and disclosed in this sense is for example the administration of two substances simultaneously by the same route, simultaneous administration by different routes, chronologically staggered administration by the same route, or chronologically staggered administration by different routes. Any of the administration routes described herein may be combined in any way. Further, the order of administration is not important. For instance, applying the generic example of two substances A and B, the chronologically staggered administration of substance A and substance B "together with" or "in combination with" one another, or as a "combination", might encompass prior administration of substance A and subsequent administration of substance B, or prior administration of substance B and subsequent administration of substance A. For example, staggered administration of substance A and substance B by the same route may take the form of initial oral administration of substance A, followed by oral administration of substance B; administration of substances A and B in this way falls within the meaning of, and is disclosed by, administration of the two substances "together with" or "in combination with" one another. Similarly, staggered administration of substance A and substance B by different routes might take the form of initial oral administration of substance A, followed by administration of substance B intravenously; administration of substances A and B in this way also falls within the meaning of, and is disclosed by, administration of the two substances "together with" or "in combination with" one another. Administration of substance A and substance B in one and the same formulation is also envisaged by the present invention. In some embodiments, in particular relating to a combination of a compound of the invention according to formula (I) and a biguanide, the administration route is preferably oral, and the timing of administration of the combined substances is preferably simultaneous. In other embodiments, in particular relating to a combination of a compound of the invention according to formula (I) and a biguanide, the administration route is preferably intravenous for the compound according to formula (I) and oral for the biguanide. In this case, the administration of each of the combined substances may be timed so as to occur simultaneously or chronologically staggered.

There are no particular restrictions on the duration of time between administration of two substances "together with" or "in combination with" one another as meant herein, as long as the two substances in question are administered as part of the same overall prophylactic and/or therapeutic regimen relating to the disease or condition in question.

Similarly, administration of one substance "during" a specified type of therapy, as used herein, is not to be understood as necessitating chronological simultaneity. Rather, administration of a substance or substances "during" a therapy encompasses and includes any timing and route of administration as part of the broader prophylactic and/or therapeutic regimen. For example, administration of one or more compounds according to the present invention "during" irradiation therapy of a subject does not require that such compound(s) be administered to the subject at the same moment as radiation is being applied to that subject, although it includes that possibility. For instance, administration "during" irradiation therapy might include administration of one or more compounds of the present invention before or after such application of radiation, or before and after such application of radiation, or before, simultaneously with and after such application of radiation.

As used here, the term "advanced stage cancer" should be understood as meaning a cancer which is classified as a cancer of Stage III or beyond according to the TMN staging system (see American Society for Clinical Oncology (ASCO) definition as accessible under https://www.cancer.net/navigating-cancer-care/diagnosing-cancer/stages-cancer).

As used here, the term "glycolytic cancer" should be understood as meaning a cancer which presents with an over average glucose consumption, i.e. evidenced by ¹⁹F-Deoxyglucose uptake in positron emission tomography and an over average lactate production as evidenced by ¹H-NMR or by determining lactate levels in blood by enzymatic analysis.

As used here, the term "endoplasmic reticulum stress" should be understood as meaning a response coming from the endoplasmic reticulum that is characterized by certain markers such as upregulation of Chac and Chop on the transcriptional level. It can be understood as having the same meaning as the term unfolded protein response (see Thangaraj et al. International Review of Cell and Molecular Biology. Vol 350, 285-325 (2020)).

As used here, the term "reduction of hypoxic areas" and "significant reduction of hypoxic areas" should be understood as meaning a reduction in pimonidazole positive stained areas in histological sections as evidenced by immunofluorescence staining and subsequent morphometric analysis. Significant reduction is present when the quantitative image analysis of at least ten individual sections and stains shows a statistically significant reduction in pimonidazole-positively stained compared to a respective control group analyzed by an appropriate statistical method (see Zaidi et al., Front Bioeng Biotechnol. 5;7:397.(2019)).

The compounds of the present invention can be in the form of a pharmaceutically acceptable salt. The term "pharmaceutically acceptable salts" refers to salts prepared from pharmaceutically acceptable non-toxic bases or acids, including inorganic bases or acids and organic bases or acids. In case the compounds of the present invention contain one or more acidic or basic groups, the invention also comprises their corresponding pharmaceutically or toxicologically acceptable salts, in particular their pharmaceutically utilizable salts. Thus, the compounds of the present invention which contain acidic groups can be present on these groups and can be used according to the invention, for example, as alkali metal salts, alkaline earth metal salts or ammonium salts. More specific examples of such salts include sodium salts, potassium salts, calcium salts, magnesium salts or salts with ammonia or organic amines such as, for example, ethylamine, ethanolamine, triethanolamine or amino acids. The compounds of the present invention which contain one or more basic groups, i.e. groups which can be protonated, can be present and can be used according to the invention in the form of their addition salts with inorganic or organic acids. Examples of suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the present invention simultaneously contain acidic and basic groups in the molecule, the invention also includes, in addition to the salt forms mentioned, inner salts or betaines (zwitterions). The respective salts can be obtained by customary methods which are known to the person skilled in the art such as, for example, by contacting these with an organic or inorganic acid or base in a solvent or dispersant, or by anion exchange or cation exchange with other salts. The present invention also includes all salts of the compounds of the present invention which, owing to low physiological compatibility, are not directly suitable for use in pharmaceuticals but which can be used, for example, as intermediates for chemical reactions or for the preparation of pharmaceutically acceptable salts.

Further the compounds of the present invention may be present in the form of solvates, such as those which include as solvate water, or pharmaceutically acceptable solvates, such as alcohols, in particular ethanol.

The following now sets out exemplary, non-limiting ways a compound according to formula (I) may be formulated for administration to a subject. Throughout the following, it is to be understood that the potential formulations are intended to apply to formulations of a compound of the invention, i.e. a compound according to formula (I), either alone or in combination with another substance, such as for instance a biguanide as set out elsewhere herein, even when the presence such other substance is not mentioned.

Further, when in the following "compounds" (plural) of the present invention are mentioned, this is to be understood as including the limiting case specifying a (singular) compound of the present invention, i.e. a compound according to formula (I) herein. Similarly, mention of an "active compound" or "active compounds" also refers to a compound of the present invention, i.e. a compound according to formula (I) herein. Similarly, mention of an "inventive compound" or "inventive compounds" also refers to a compound of the present invention, i.e. a compound according to formula (I) herein.

Furthermore, the present invention provides pharmaceutical compositions comprising at least one compound of the present invention, or a pharmaceutically acceptable salt or solvate thereof as active ingredient together with a pharmaceutically acceptable carrier. This applies equally in the event that the at least one compound of the present invention according to formula (I) is to be administered in combination with another compound, such as, preferably, a biguanide.

"Pharmaceutical composition" means one or more compounds of the present invention, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the compositions and pharmaceutical compositions of the present invention encompass any composition made by admixing at least one compound of the present invention and a pharmaceutically acceptable carrier.

In practical use, the compounds of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavouring agents, preservatives, colouring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least about 0.1 percent of active compound. The percentage of active compound in these compositions may, of course, be varied and may conveniently be between about 5 percent to about 98 percent of the weight of the unit, more preferably about 10 to about 90 percent of the weight of the unit. The amount of active compound in such therapeutically useful compositions is such that an effective dosage will be obtained. The active compounds can also be administered intranasally as, for example, liquid drops or spray. The tablets, pills, capsules, and the like may also contain a binder such as gum tragacanth, acacia, corn starch or gelatine; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavouring such as cherry or orange flavour.

The compounds used in the present invention may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

Further specific details regarding the above and further modes of formulation are set out below.

Formulation of the inventive compound for suitability for oral administration: A compound of the invention suitable for oral administration may be prepared, packaged, or solid in the form of a discrete solid dose unit including a sachet (e.g. a powder in a sachet), a tablet, a hard or soft capsule, a cachet, a troche, or a lozenge, each containing a predetermined amount of the compound(s) of the invention. Other formulations suitable for oral administration include a powdered or granular formulation, an aqueous or oily suspension, an aqueous or oily solution, or an emulsion. As used herein, an "oily" liquid comprises a carbon-containing liquid molecule that exhibits a less polar character than water.

A tablet comprising a compound of the invention may, for example, be made by compressing or molding an inventive compound, optionally with one or more additional ingredients. Compressed tablets may be prepared by compressing, in a suitable device, an inventive compound in a free flowing form such as a powder or granular preparation, optionally mixed with one or more of a binder, a lubricant, an excipient, a surface active agent, and a dispersing agent. Molded tablets may for example be made by molding, in a suitable device, an inventive compound, a pharmaceutically acceptable carrier, and at least sufficient liquid to moisten the mixture.

Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface active agents include, but are not limited to, sodium lauryl sulfate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the inventive compound. By way of example, a material such as glyceryl monostearate or glyceryl distearate may be used to coat tablets. Tablets may further comprise a sweetening agent, a flavoring agent, a coloring agent, a preservative, or some combination of these in order to provide pharmaceutically attractive and palatable preparation.

Hard capsules comprising an inventive compound may be made using a physiologically degradable composition, such as gelatin or cellulose derivatives. Such hard capsules comprise the inventive compound, and may further comprise additional ingredients including, for example, an inert solid diluent such as calcium carbonate, calcium phosphate, or kaolin.

Soft gelatin capsules comprising the inventive compound may be made using a physiologically degradable composition, such as gelatin combined with a plasticizer (i.e. glycerol) as basic component of the soft gelatin shell. Soft gelatin capsules may contain a liquid or semisolid solution, suspension, or microemulsion preconcentrate. The soft capsule filling comprises the inventive compound, which may be mixed with water or an oil medium such as peanut oil, liquid paraffin, olive oil, soybean oil, sunflower oil, a lecithin such as for example soy lecithin or sunflower lecithin, medium chain triglycerides, polyglycerol oleate, beeswax, mono- and diglycerides of fatty acids, or combinations of any of the above.

Liquid formulations of the inventive compound that are especially suitable for oral administration may be prepared, packaged, and sold either in liquid form or in the form of a dry product intended for reconstitution with water or another suitable vehicle prior to ingestion.

Liquid suspensions may be prepared using conventional methods to achieve suspension of the active ingredient in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropyl methylcellulose. Known dispersing or wetting agents include, but are not limited to, naturally occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Powdered and granular formulations of an inventive compound, e.g. a pharmaceutical composition of the invention may be prepared using known methods. Such formulations may be administered directly to a subject, used, for example, to form sachet or tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

The inventive compound may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material (e.g. such as with a physiologically degradable material), and methods of absorbing an aqueous or oily solution or suspension into an absorbent material, with or without subsequent drying.

Formulation of the inventive compound for suitability for parenteral administration: For parenteral administration, the inventive compound may be formulated for injection or infusion, for example, intravenous, intramuscular or subcutaneous injection or infusion, or for administration in a bolus dose and/or continuous infusion. Suspensions, solutions or emulsions in an oily or aqueous vehicle, optionally containing other agents such as suspending, stabilizing and/or dispersing agents such as those mentioned above, may be used.

The inventive compound may be rendered especially suitable for parenteral administration by formulation with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules, crushable or otherwise, or in multidose containers containing a preservative. Compositions especially suitable for parenteral administration include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of the inventive composition which is especially suitable for parenteral administration, the active ingredient is provided in dry (e.g. powder or granular) form for reconstitution with a suitable vehicle (e.g. sterile pyrogen free water) prior to parenteral administration of the reconstituted composition.

The inventive compound may be prepared in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable compositions may be prepared using a non toxic, parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or diglycerides. Other usual parentally-administrable formulations include those that comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

Formulation of the inventive compound for suitability for transmucosal administration: The inventive compound may be formulated to be suitable for transmucosal administration. The formulation may include any substances or dosage unit suitable for application to mucosal tissue. For example, the selected active agent may be administered to the buccal mucosa in an adhesive tablet or patch, sublingually administered by placing a solid dosage form under the tongue, lingually administered by placing a solid dosage form on the tongue, administered nasally as droplets or a nasal spray, a non-aerosol liquid formulation, or a dry powder, placed within or near the rectum ("transrectal" formulations), or administered to the urethra as a suppository, ointment, or the like.

Formulation of the inventive compound for suitability for transurethal administration: The inventive compound may also be formulated to be suitable for transurethal administration. In this case, the inventive composition may comprise a urethral dosage form containing the active agent and one or more selected carriers or excipients, such as water, silicone, waxes, petroleum jelly, polyethylene glycol ("PEG"), propylene glycol ("PG"), liposomes, sugars such as mannitol and lactose, and/or a variety of other materials. A transurethral permeation enhancer may be included in the dosage from. Examples of suitable permeation enhancers include dimethylsulfoxide ("DMSO"), dimethyl formamide ("DMF"), N,N-dimethylacetamide ("DMA"), decylmethylsulfoxide ("C10 MSO"), polyethylene glycol monolaurate ("PEGML"), glycerol monolaurate, lecithin, the 1-substituted azacycloheptan-2-ones, particularly 1-n-dodecyl-cyclazacycloheptan-2-one, surfactants as discussed above, including, for example TWEEN-80^{™}, and lower alkanols such as ethanol.

Formulation of the inventive compound for suitability for transrectal administration: The inventive compound may also be formulated to be suitable for transrectal administration. Transrectal dosage forms may include rectal suppositories, creams, ointments, and liquid formulations (enemas). The suppository, cream, ointment or liquid formulation for transrectal delivery comprises an inventive compound and one or more conventional nontoxic carriers suitable for transrectal drug administration. The transrectal dosage forms of the inventive composition may be manufactured using conventional processes. The transrectal dosage unit may be fabricated to disintegrate rapidly or over a period of several hours. The time period for complete disintegration may be in the range of from about 10 minutes to about 6 hours, e.g., less than about 3 hours.

Formulation of the inventive compound for suitability for vaginal or perivaginal administration: The inventive compound may also be formulated to be suitable for vaginal or perivaginal administration. Suitable dosage forms to this end may include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent and one or more conventional nontoxic carriers suitable for vaginal or perivaginal drug administration. The vaginal or perivaginal forms of the present invention may be manufactured using conventional processes as for example disclosed in Remington: The Science and Practice of Pharmacy, supra. The vaginal or perivaginal dosage unit may be fabricated to disintegrate rapidly or over a period of several hours. The time period for complete disintegration may be in the range of from about 10 minutes to about 6 hours, e.g., less than about 3 hours.

Formulation of the inventive compound for suitability for topical formulations: The inventive compound may also be formulated to be suitable for topical administration. Suitable dosage forms to this end may include any form suitable for application to the body surface, and may comprise, for example, an ointment, cream, gel, lotion, solution, paste or the like, and/or may be prepared so as to contain liposomes, micelles, and/or microspheres. In certain embodiments, topical formulations herein are ointments, creams and gels.

Formulation of the inventive compound for suitability for transdermal administration: The inventive compound may also be formulated to be especially suitable for transdermal administration. As known to one skilled in the art, transdermal administration involves the delivery of pharmaceutical compounds via percutaneous passage of the compound into the systemic circulation of the patient. This can be effected e.g. by transdermal patches or iontophoresis devices. Other components besides the inventive compound may be incorporated into the transdermal patches as well. For example, compositions and/or transdermal patches may be formulated with one or more preservatives or bacteriostatic agents including, but not limited to, methyl hydroxybenzoate, propyl hydroxybenzoate, chlorocresol, benzalkonium chloride, and the like. Dosage forms of the inventive compound may include creams, sprays, lotions, gels, ointments, eye drops, nose drops, ear drops, and the like. In such dosage forms, the ingredients of the inventive composition may be mixed to form a white, smooth, homogeneous, opaque cream or lotion with, for example, benzyl alcohol 1% or 2% (wt/wt) as a preservative, emulsifying wax, glycerin, isopropyl palmitate, lactic acid, purified water and sorbitol solution. In addition, the compositions may contain polyethylene glycol 400. They may be mixed to form ointments with, for example, benzyl alcohol about 2% (wt/wt) as preservative, white petrolatum, emulsifying wax, and tenox II (butylated hydroxyanisole, propyl gallate, citric acid, propylene glycol). Woven pads or rolls of bandaging material, e.g., gauze, may be impregnated with the compositions in solution, lotion, cream, ointment or other such form may also be used for topical application. The compositions may also be applied topically using a transdermal system, such as one of an acrylic-based polymer adhesive with a resinous crosslinking agent impregnated with the composition and laminated to an impermeable backing.

Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are separate and distinct layers, with the adhesive underlying the reservoir that, in this case, may be either a polymeric matrix as described above, or be a liquid or hydrogel reservoir, or take some other form. Any suitable route of administration may be employed for providing a mammal, especially a human, with an effective dose of a compound of the present invention. For example, oral, rectal, topical, parenteral (including intravenous, intramuscular and subcutaneous), ocular (ophthalmic), pulmonary (nasal or buccal inhalation), nasal and the like may be employed. As set out above, dosage forms include tablets, troches, dispersions, suspensions, solutions, capsules, creams, ointments, aerosols, and the like. Preferably, compounds of the present invention are administered orally or intraveneously.

The effective dosage of active ingredient employed may vary depending on the particular compound employed, the mode of administration, the condition being treated and the severity of the condition being treated. Such dosage may be ascertained readily by a person skilled in the art.

When treating or preventing lactate/ATP-mediated diseases for which compounds of formula (I) are indicated, generally satisfactory results are obtained when the compounds are administered at a daily dosage of from about 0.1 mg to about 100 mg per kilogram of mammal body weight, preferably given as a single daily dose or in divided doses two to six times a day, or in sustained release form. For most large mammals, the total daily dosage is from about 1 mg to about 1000 mg, preferably from about 1 mg to about 50 mg. In the case of a 70 kg adult human, the total daily dose will generally be from about 7 mg to about 350 mg. This dosage regimen may be adjusted to provide the optimal therapeutic response.

The compounds of the present invention are effective for treating cancer. Without being bound by theory, this is due to the compounds' ability to modulate lactate production pathways.

The compound of the present invention can be administered together with one or more therapeutic agents for cancer selected from the group consisting of a PD-1 agent, a PD-L1 agent, a CTLA-4 agent, an IDO1 inhibitor and an anticancer vaccine. Alternatively, the compound may be administered together with a cytokine therapy, a known chemo- or pharmacotherapy, or during irradiation therapy. Such combination therapy may be alternative to, or supplementary to, the combination therapy involving biguanides discussed above.

Examples of PD-1 agents include, but are not limited to, pembrolizumab and nivolumab.

Examples of PD-L1 agents include, but are not limited to, atezolizumab, avelumab and durvalumab.

Examples of CTLA-4 agents include, but are not limited to, ipilimumab.

Examples of IDO1 inhibitors include, but are not limited to, epacadostat, navoximod and BMS-986205.

Examples of anticancer vaccines include, but are not limited to, Hepa-VAC-101 and Sipuleucel-T.

Examples of cytokine therapy include, but are not limited to therapy involving the administration of IL-2, GM-CSF, IL-12 and/or IL-10.

Examples of drugs which may be used in chemo- or pharmacotherapy herein include: platinum compounds (e.g., cisplatin, carboplatin, oxaliplatin), alkylating agents (e.g., cyclophosphamide, ifosfamide, chlorambucil, nitrogen mustard, thiotepa, melphalan, busulfan, procarbazine, streptozocin, temozolomide, dacarbazine, bendamustine), antitumor antibiotics (e.g., daunorubicin, doxorubicin, idarubicin, epirubicin, mitoxantrone, bleomycin, mytomycin C, plicamycin, dactinomycin), taxanes (e.g., paclitaxel, cabazitaxel and docetaxel), antimetabolites (e.g., 5-fluorouracil, cytarabine, premetrexed, thioguanine, floxuridine, capecitabine, gemcitabine, 6-mercatopurine and methotrexate), nucleoside analogues (e.g., fludarabine, clofarabine, cladribine, pentostatin, nelarabine), topoisomerase inhibitors (e.g., topotecan and irinotecan), hypomethylating agents (e.g., azacitidine and decitabine), proteosome inhibitors (e.g., bortezomib), epipodophyllotoxins (e.g., etoposide and teniposide), DNA synthesis inhibitors (e.g., hydroxyurea), vinca alkaloids (e.g., vicristine, vindesine, vinorelbine, and vinblastine), tyrosine kinase inhibitors (e.g., imatinib, dasatinib, nilotinib, sorafenib, sunitinib, bosutinib, ponatinib, erlotinib, gefitinib, afatinib osimertinib, lapatinib, crizotinib, ceritinib, axitinib, cabozantinib and lanvatinib), monoclonal antibodies (e.g., pertuzumab, rituximab, cetuximab, panetumumab, tositumomab, trastuzumab, alemtuzumab, gemtuzumab ozogamicin, bevacizumab, nivolumab, pembrolizumab or other immune checkpoint inhibitor monoclonal antibodies), nitrosoureas (e.g., carmustine, fotemustine, and lomustine), enzymes (e.g., L-asparaginase), biological agents (e.g., interferons and interleukins), hexamethylmelamine, mitotane, angiogenesis inhibitors (e.g., thalidomide, lenalidomide), steroids (e.g., prednisone, dexamethasone, and prednisolone), hormonal agents (e.g., tamoxifen, raloxifene, leuprolide, bicaluatmide, granisetron, flutamide), aromatase inhibitors (e.g., letrozole, exemestane and anastrozole), arsenic trioxide, tretinoin, nonselective cyclooxygenase inhibitors (e.g., nonsteroidal anti-inflammatory agents, salicylates, aspirin, piroxicam, ibuprofen, indomethacin, naprosyn, diclofenac, tolmetin, ketoprofen, nabumetone, oxaprozin), selective cyclooxygenase-2 (COX-2) inhibitors, or any combination thereof.

### Abbreviations

Herein and throughout the application, the following abbreviations are used.
- Ac: acetyl
- 9-BBN: 9-borabicyclo[3.3.1]nonane
- CDCl₃: deuterated chloroform
- d: doublet
- dba: dibenzylideneacetone
- dd: doublet of doublets
- DCE: 1,2-dichloroethane
- DIBAL: diisobutylaluminiumhydrid
- DCM: dichloromethane
- DIAD: diisopropyl azodicarboxylate
- DIPEA: *N,N*-diisopropylethylamine
- DMAP: 4-(dimethylamino)pyridine
- DMF: *N,N*-dimethylformamide
- DMP: Dess-Martin periodinane
- DMSO: dimethyl sulfoxide
- DMSO-*d*₆: deuterated dimethyl sulfoxide
- dppf: 1,1'-bis(diphenylphosphino)ferrocene
- Et: ethyl
- EtOAc: ethyl acetate
- EtOH: ethanol
- ESI: electrospray ionization
- HATU: *O*-(7-azabenzotriazol-1-yl)-*N*,*N*,*N',N*'-tetramethyluronium hexafluorophosphate
- HPLC: high performance liquid chromatography
- m: multiplet
- m/z: mass-to-charge ratio
- M: mass or molar
- Me: methyl
- MeCN: acetonitrile
- MeOH: methanol
- MeOD-*d*₃: deuterated methanol
- MS: mass spectrometry
- NBS: *N*-bromosuccinimide
- NMI: *N*-methylimidazole
- NMR: nuclear magnetic resonance
- PyBroP: bromotripyrrolidinophosphonium hexafluorophosphate
- PE: petroleum ether
- *p*-TsCl: *p* toluenesulfonyl chloride
- rt: room temperature
- s: singlet
- t: triplet
- TEA: triethylamine
- TCFH: chloro-*N*,*N*,*N'*,*N'*-tetramethylformamidinium hexafluorophosphate
- TFA: trifluoroacetic acid
- TFAA: trifluoroacetic anhydride
- THF: tetrahydrofuran
- Ts: tosyl
- XPhos: 2-dicyclohexylphosphino-2',4',6'-triisopropylbiphenyl

### General Schemes

The compounds of the present invention can be prepared by a combination of methods known in the art including the procedures described in schemes **1** and **2** below. The following reaction schemes are meant to exemplify the invention.

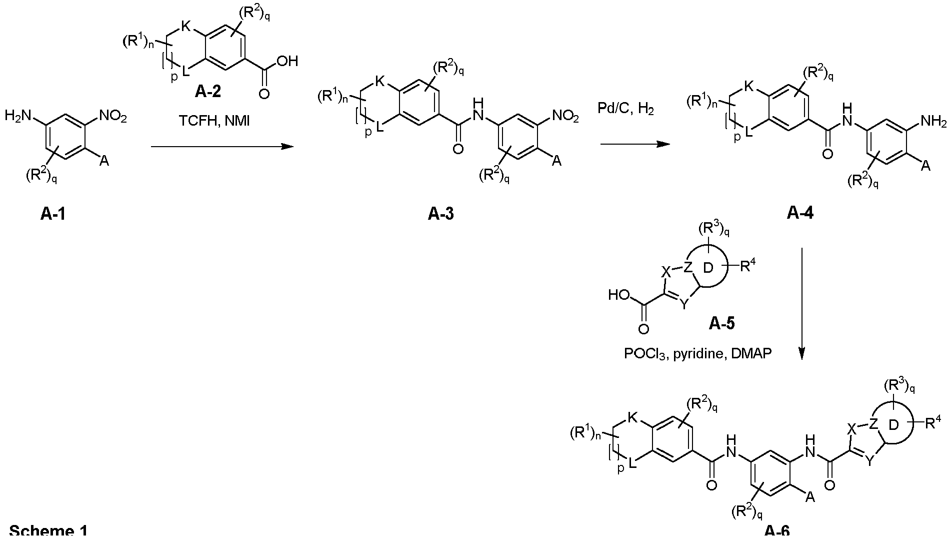

Scheme **1** describes one route of preparation for the compounds of the present invention. Amide coupling of a substituted nitroaniline starting material **(A-1)** with an appropriate carboxylic acid **A-2** with, for example, TFCH/NMI as coupling reagent affords monoamide intermediates of structure **A-3.** Reduction of the nitro group in **A-3** with, for example, hydrogen and Pd catalysis yields the amino intermediates of structure **A-4.** A second amide coupling in the sequence of reactions with appropriate carboxylic acids **A-5** converts **A-4** into compounds of structure **A-6.**

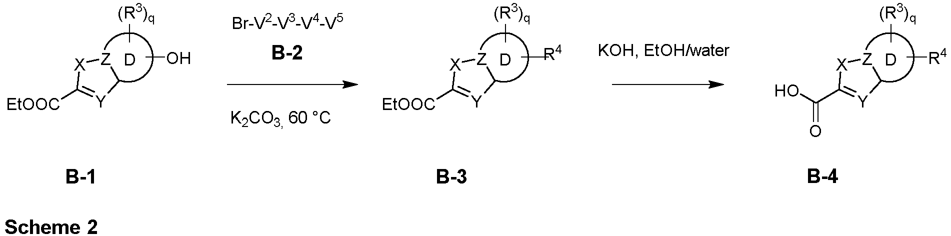

Scheme **2** describes the preparation for some of the carboxylic acid intermediates **A-5** bearing specific R⁴ groups. A hydroxy substituted bicyclic heteroaryl starting material **(B-1)** is converted to **B-3** through alkylation with an appropriate bromide **B-2** and, for example, K₂CO₃ at elevated temperatures. Ester saponification affords the corresponding carboxylic acid intermediate **B-4.**

### Intermediate 1: N-(3-Amino-4-methylphenyl)-2,3-dihydrobenzo[b][1,4]dioxine-6-carboxamide (Int 1)

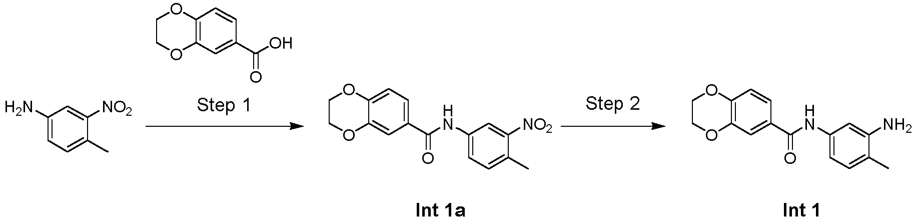

### Step 1: N-(4-Methyl-3-nitrophenyl)-2,3-dihydrobenzo[b][1,4]dioxine-6-carboxamide (Int 1a)

To a solution of 2,3-dihydrobenzo[*b*][1,4]dioxine-6-carboxylic acid (7.04 g, 39.1 mmol) in MeCN (120 mL) were added 4-methyl-3-nitroaniline (6.00 g, 39.4 mmol), NMI (11.2 g, 137 mmol) and TCFH (19.8 g, 70.4 mmol). The mixture was stirred at rt for 16 h. Then, water (120 mL) was added to the mixture. The mixture was filtered, and the filter cake was triturated with MeCN/water (1:1) to afford the title compound as a yellow solid.

### Step 2: N-(3-Amino-4-methylphenyl)-2,3-dihydrobenzo[b][1,4]dioxine-6-carboxamide (Int 1)

To a solution of *N*-(4-methyl-3-nitrophenyl)-2,3-dihydrobenzo[*b*][1,4]dioxine-6-carboxamide **(Int 1a)** (11.3 g, 36.0 mmol) in MeOH (260 mL) was added Pd/C (1.20 g, 11.3 mmol), and the mixture was stirred at rt overnight under a H₂ atmosphere. Then the mixture was filtered, and the filtrate was concentrated to dryness. The residue was triturated with MeOH to afford the title compound as a white solid.

### Intermediate 2: N-(3-Amino-4-fluorophenyl)-2,3-dihydrobenzo[b][1,4]dioxine-6-carboxamide (Int 2)

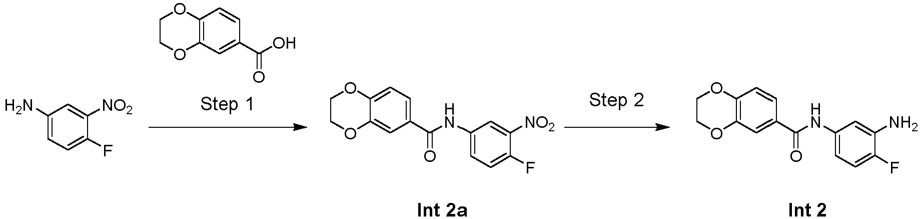

### Step 1: N-(4-Fluoro-3-nitrophenyl)-2,3-dihydrobenzo[b][1,4]dioxine-6-carboxamide (Int 2a)

To a solution of 2,3-dihydrobenzo[*b*][1,4]dioxine-6-carboxylic acid (6.35 g, 35.3 mmol) and NMI (10.1 g, 123 mmol) in MeCN (150 mL) was added TCFH (17.8 g, 63.5 mmol), and the mixture was stirred at rt for 1 h. Then 4-fluoro-3-nitroaniline (5.50 g, 35.3 mmol) was carefully added upon cooling with an ice-water bath and the mixture stirred for 3 h. The mixture was concentrated to dryness and the residue was triturated with MeCN/water (1:1) to afford the title compound as a light yellow solid.

### Step 2: N-(3-Amino-4-fluorophenyl)-2,3-dihydrobenzo[b][1,4]dioxine-6-carboxamide (Int 2)

To a mixture of *N*-(3-amino-4-fluorophenyl)-2,3-dihydrobenzo[*b*][1,4]dioxine-6-carboxamide **(Int 2a)** (7.80 g, 24.5 mmol) and NH₄Cl (7.92 g, 147 mmol) in EtOH (120 mL) and water (40.0 mL) was added iron powder (8.22 g, 147 mmol) under N₂ atmosphere, and the mixture was allowed to stir at 80 °C for 1 h. Then, the mixture was cooled to rt and filtered. The filtrate was concentrated to dryness and the residue was triturated with MeCN/water (1:1) to afford the title compound as a yellow solid.

### Intermediates 2/1: N-(3-Amino-4-chlorophenyl)-2,3-dihydrobenzo[b][1,4]dioxine-6-carboxamide (Int 2/1)

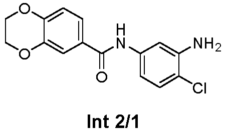

The title compound was prepared similar as described for **Int 2,** using in step 1, 4-chloro-3-nitroaniline in place of 4-fluoro-3-nitroaniline.

### Intermediate 3: 5-(3-(Pyrrolidin-1-yl)propoxy)benzo[b]thiophene-2-carboxylic acid (Int 3)

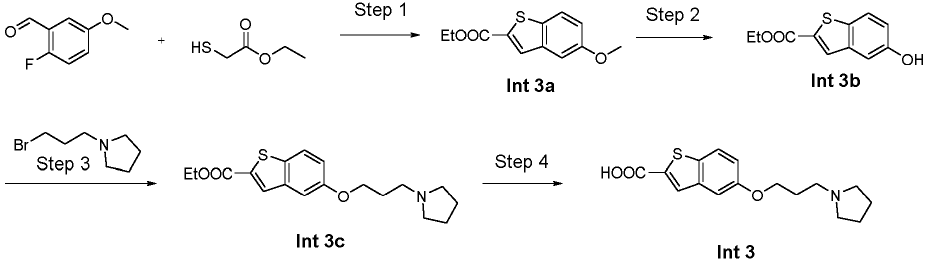

### Step 1: Ethyl 5-methoxybenzo[b]thiophene-2-carboxylate (Int 3a)

To a solution of ethyl 2-mercaptoacetate (4.30 mL, 38.9 mmol) in DMSO (20.0 mL) was added K₂CO₃ (9.00 g, 64.9 mmol) at 0 °C, and the mixture was stirred at 0 °C for 20 min. A solution of 2-fluoro-5-methoxybenzaldehyde (5.00 g, 32.4 mmol) in DMSO (10.0 mL) was added and the mixture was stirred at 70 °C for 9 h. The mixture was cooled to rt and poured into water (100 mL). The resulting mixture was extracted with DCM (3 x 100 mL). The combined organic layers were washed with brine, dried over Na₂SO₄, filtered, and concentrated to dryness. The residue was purified by column chromatography on silica gel (DCM/PE = 2:3) to afford the title compound as a green solid.

### Step 2: Ethyl 5-hydroxybenzo[b]thiophene-2-carboxylate (Int 3b)

To a solution of ethyl 5-methoxybenzo[*b*]thiophene-2-carboxylate **(Int 3a)** (1.00 g, 4.40 mmol) in DCM (20.0 mL) was added BBr₃ (1 M in DCM, 22.0 mL, 21.9 mmol) dropwise at -20 °C. After addition, the mixture was slowly warmed to rt and stirred for 2 h. Water (20 mL) was added to the mixture. The resulting mixture was extracted with DCM (3 x 50 mL). The combined organic layers were washed with brine, dried over Na₂SO₄, filtered, and concentrated to dryness. The residue was purified by column chromatography on silica gel (EtOAc/PE = 1:2) to afford the title compound as a pale yellow solid.

### Step 3: Ethyl 5-(3-(pyrrolidin-1-yl)propoxy)benzo[b]thiophene-2-carboxylate (Int 3c)

To a solution of ethyl 5-hydroxybenzo[*b*]thiophene-2-carboxylate **(Int 3b)** (640 mg, 2.90 mmol) and 1-(3-bromopropyl)pyrrolidine hydrobromide salt (500 mg, 1.80 mmol) in DMSO (10.0 mL) was added K₂CO₃ (460 mg, 3.40 mmol), and the mixture was stirred at 60 °C overnight. Then, the mixture was quenched with water (20 mL). The resulting mixture was extracted with DCM (3 x 10 mL). The combined organic layers were washed with brine, dried over Na₂SO₄, filtered, and concentrated to dryness. The residue was purified by column chromatography on silica gel (MeOH/DCM = 1:10) to afford the title compound as a pale yellow solid.

### Step 4: 5-(3-(Pyrrolidin-1-yl)propoxy)benzo[b]thiophene-2-carboxylic acid (Int 3)

To a mixture of ethyl 5-(3-(pyrrolidin-1-yl)propoxy)benzo[*b*]thiophene-2-carboxylate **(Int 3c)** (790 mg, 2.40 mmol) in EtOH (15.0 mL) and water (7.50 mL) was added KOH (324 mg, 5.60 mmol), and the mixture was refluxed for 1 h. Then, the mixture was cooled to rt and adjusted to pH = 5 ~ 6 by 1 M HCl. The resulting mixture was filtered, and the filter cake was triturated with MeCN to afford the title compound as an off-white solid.

### Intermediate 4: 6-(Pyridin-2-ylmethoxy)benzo[b]thiophene-2-carboxylic acid (Int 4)

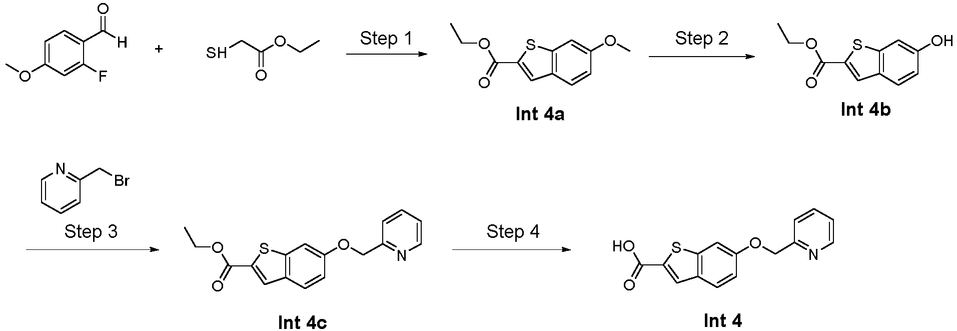

### Step 1: Ethyl 6-methoxybenzo[b]thiophene-2-carboxylate (Int 4a)

To a mixture of 2-fluoro-4-methoxybenzaldehyde (10.0 g, 64.9 mmol) and ethyl-2-mercaptoacetate (9.36 g, 77.9 mmol) in DMF (50.0 mL) was added K₂CO₃ (22.4 g, 162 mmol), and the mixture was stirred at rt overnight. Then, the mixture was diluted with water (150 mL) and extracted with EtOAc (3 x 100 mL). The combined organic layers were washed with brine, dried over Na₂SO₄, filtered, and concentrated to dryness. The residue was purified by column chromatography on silica gel (EtOAc/PE = 1:10) to afford the title compounds as a white solid.

### Step 2: Ethyl 6-hydroxybenzo[b]thiophene-2-carboxylate (Int 4b)

To a solution of ethyl 6-methoxybenzo[*b*]thiophene-2-carboxylate **(Int 4b)** (10.0 g, 42.4 mmol) in dry DCM (200 mL) was added BBr₃ (1.00 M in DCM, 106 mL, 106 mmol) dropwise at -75 °C under N₂ atmosphere. After addition, the mixture was stirred at -75 °C for 2 h and stirred at rt for another 16 h. EtOH (30 mL) and sat. aq. NaHCO₃ (30 mL) were added to the mixture. The resulting mixture was extracted with DCM (3 x 100 mL), and the combined organic layers were washed by brine, dried over NaSO₄, filtered, and concentrated to dryness. The residue was purified by column chromatography on silica gel (EtOAc/PE = 1:6) to afford the title compound as a light yellow solid.

### Step 3: Ethyl 6-(pyridin-2-ylmethoxy)benzo[b]thiophene-2-carboxylate (Int 4c)

To a mixture of ethyl 6-hydroxybenzo[*b*]thiophene-2-carboxylate **(Int 4b)** (1.20 g, 5.40 mmol) and K₂CO₃ (2.23 g, 16.2 mmol) in MeCN (10.0 mL) was added 2-(bromomethyl)pyridine (1.11 g, 6.50 mmol), and the mixture was stirred at 85 °C overnight. The mixture was cooled to rt and filtered. The filtrate was concentrated to dryness, and the residue was purified by column chromatography on silica gel (EtOAc/PE = 1:6) to afford the title compound as a light yellow solid.

### Step 4: 6-(Pyridin-2-ylmethoxy)benzo[b]thiophene-2-carboxylic acid (Int 4)

To a solution of ethyl 6-(pyridin-2-ylmethoxy)benzo[*b*]thiophene-2-carboxylate **(Int 4c)** (1.20 g, 3.80 mmol) in EtOH (10.0 mL) and water (2.00 mL) was added LiOH (275 mg, 11.5 mmol), and the mixture was stirred at rt for 6 h. The mixture was diluted with water (10.0 mL) and the aqueous layer was extracted with DCM (3 x 30 mL). The water layer was adjusted to pH = 5 by 1 M HCl and solid was precipitated. The resulting mixture was filtered, and the filter cake was triturated with MeCN to afford the title compound as a light yellow solid.

### Intermediate 5: 6-(3-(Pyrrolidin-1-yl)propoxy)benzo[b]thiophene-2-carboxylic acid (Int 5)

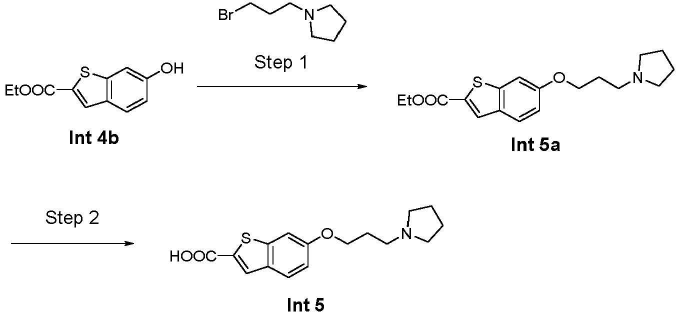

### Step 1: Ethyl 6-(3-(pyrrolidin-1-yl)propoxy)benzo[b]thiophene-2-carboxylate (Int 5a)

To a mixture of ethyl 6-hydroxybenzo[*b*]thiophene-2-carboxylate **(Int 4b)** (700 mg, 3.20 mmol) and 1-(3-bromopropyl)pyrrolidine hydrobromide salt (1.03 g, 3.80 mmol) in DMF (10.0 mL) was added K₂CO₃ (1.31 g , 9.50 mmol), and the mixture was stirred at 75 °C overnight. Then, the mixture was cooled to rt, diluted with water (20 mL) and extracted with DCM (3 x 10 mL). The combined organic layers were washed with water and brine, dried over Na₂SO₄, filtered, and concentrated to dryness. The residue was purified by column chromatography on silica gel (MeOH/DCM = 1:10) to afford the title compound as a pale yellow solid.

### Step 2: 6-(3-(Pyrrolidin-1-yl)propoxy)benzo[b]thiophene-2-carboxylic acid (Int 5)

To a mixture of ethyl 6-(3-(pyrrolidin-1-yl)propoxy)benzo[*b*]thiophene-2-carboxylate (**Int 5a**) (880 mg, 2.64 mmol) in EtOH (15.0 mL) and water (7.50 mL) was added LiOH (254 mg, 10.6 mmol), and the mixture was stirred at rt overnight. Then, the mixture was adjusted to pH = 5 ~ 6 by 1 M HCl. The resulting mixture was filtered, and the filter cake was triturated with MeCN to afford the title compound as a light yellow solid.

### Intermediate 6: 6-(2-(Pyrrolidin-1-yl)ethoxy)benzo[b]thiophene-2-carboxylic acid (Int 6)

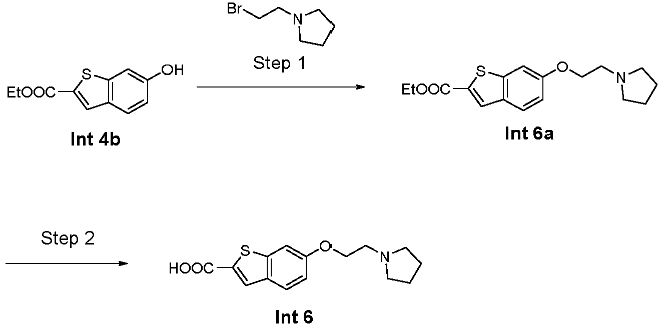

### Step 1: Ethyl 6-(2-(pyrrolidin-1-yl)ethoxy)benzo[b]thiophene-2-carboxylate (Int 6a)

To a mixture of ethyl 6-hydroxybenzo[*b*]thiophene-2-carboxylate **(Int 4b)** (2.50 g, 11.3 mmol) and 1-(2-chloroethyl)pyrrolidine (1.80 g, 13.1 mmol) in DMF (25.0 mL) was added K₂CO₃ (4.65 g , 33.8 mmol), and the mixture was stirred at 75 °C overnight. Then, the mixture was cooled to rt, diluted with water (100 mL), and extracted with DCM (3 x 50 mL). The combined organic layers were washed with water and brine, dried over anhydrous Na₂SO₄, filtered, and concentrated to dryness. The residue was purified by column chromatography on silica gel (MeOH/DCM = 1:10) to afford the title compound as a pale yellow solid.

### Step 2: 6-(2-(Pyrrolidin-1-yl)ethoxy)benzo[b]thiophene-2-carboxylic acid (Int 6)

To a mixture of ethyl 6-(2-(pyrrolidin-1-yl)ethoxy)benzo[*b*]thiophene-2-carboxylate **(Int 6a)** (1.40 g, 4.40 mmol) in EtOH (25.0 mL) and water (5.00 mL) was added LiOH (315 mg, 3.10 mmol), and the mixture was stirred at rt overnight. Then, EtOH was removed under reduced pressure. The mixture was adjusted to pH = 5 ~ 6 by 1 M HCl. The resulting mixture was filtered, and the filter cake was triturated with MeCN to afford the title compound as a pale yellow solid.

### Intermediate 7: 6-(2-(Pyridin-2-yl)ethoxy)benzo[b]thiophene-2-carboxylic acid (Int 7)

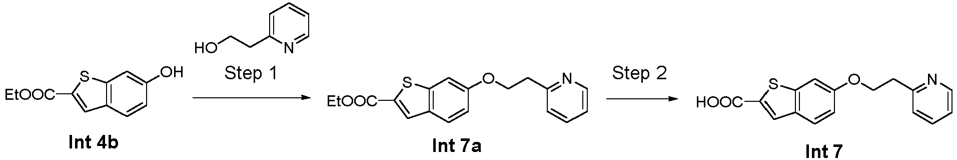

### Step 1: Ethyl 6-(2-(pyridin-2-yl)ethoxy)benzo[b]thiophene-2-carboxylate (Int 7a)

To a mixture of ethyl 6-hydroxybenzo[*b*]thiophene-2-carboxylate **(Int 4b)** (2.00 g, 9.00 mmol) and 2-(pyridin-2-yl)ethan-1-ol (1.66 g, 13.5 mmol) in THF (40.0 mL) were added PPh₃ (7.08 g, 27.0 mmol) and DIAD (5.46 g, 27.0 mmol), the mixture was stirred at 50 °C overnight. The mixture was concentrated to dryness and the residue was purified by column chromatography on silica gel (EtOAc/PE = 1:5) to afford the title compound as a light yellow solid.

### Step 2: 6-(2-(Pyridin-1-yl)ethoxy)benzo[b]thiophene-2-carboxylic acid (Int 7)

To a solution of ethyl 6-(2-(pyridin-2-yl)ethoxy)benzo[*b*]thiophene-2-carboxylate **(Int 7a)** (1.10 g, 3.40 mmol) in EtOH (10.0 mL) and water (2.00 mL) was added LiOH (241 mg, 10.1 mmol), and the mixture was stirred at rt for 6 h. The mixture was diluted with water (10.0 mL) and the aqueous layer was extracted with DCM (3 x 30 mL). The water layer was adjusted to pH = 5 by 1 M HCl and a solid was precipitated. The resulting mixture was filtered, and the filter cake was triturated with MeCN to afford the title compound as a light yellow solid.

### Intermediate 8: 6-Methoxybenzo[d]thiazole-2-carboxylic acid (Int 8)

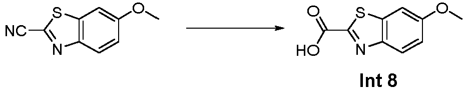

To a solution of 6-methoxybenzo[*d*]thiazole-2-carbonitrile (30.0 mg, 0.158 mmol) in THF (1.60 mL) was added 2 M NaOH (0.118 mL, 0.237 mmol). The mixture was stirred at rt for 48 h. The reaction mixture was diluted with EtOAc (20 mL) and water (10 mL). The organic layer was separated. The aqueous layer was acidified to pH ~ 1 with 1 M HCl and then extracted with EtOAc (2 x 15 mL). The organic layer was dried over MgSO₄, filtered, and concentrated to afford the title compound as a yellow solid.

### Intermediate 9: 6-((4-Ethylpiperazin-1-yl)methyl)thieno[2,3-b]pyridine-2-carboxylic acid (Int 9)

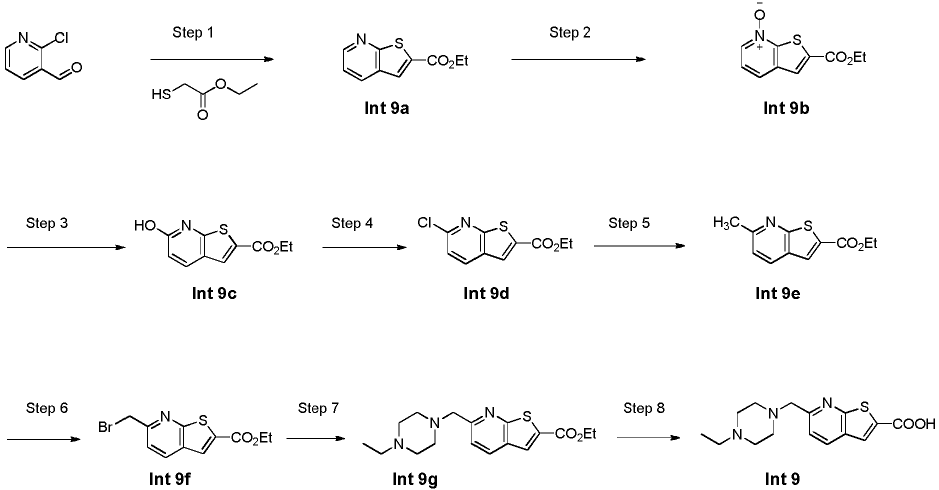

### Step 1: Ethyl thieno[2,3-b]pyridine-2-carboxylate (Int 9a)

To a solution of 2-chloronicotinaldehyde (50.0 g, 354 mmol) in DMF (400 mL) was added TEA (74.1 g, 732 mmol) and ethyl 2-mercaptoacetate (42.5 g, 354 mmol). The mixture was stirred at 100 °C for 4 h under nitrogen atmosphere. The mixture was diluted with water (1.5 L) and extracted with EtOAc (3 x 1000 mL). The combined organic layers were washed with water and brine, dried over anhydrous Na₂SO₄, filtered and concentrated to dryness. The residue was purified by flash chromatography on silica (PE/EtOAc = 8:1) to give the title compound.

### Step 2: 2-(Ethoxycarbonyl)thieno[2,3-b]pyridine 7-oxide (Int 9b)

To a solution of ethyl thieno[2,3-b]pyridine-2-carboxylate **(Int 9a)** (52.8 g, 255.0 mmol) in acetic acid (400 mL) was added H₂O₂ (100 mL, 30% in water) in portions. The resulting mixture was stirred at 60 °C overnight. The mixture was stirred at 0 °C for 1 h. A precipitate was formed during the reaction. The solid was collected by filtration and dried to give the title compound.

### Step 3: Ethyl 6-hydroxythieno[2,3-b]pyridine-2-carboxylate (Int 9c)

To a solution of 2-(ethoxycarbonyl)thieno[2,3-b]pyridine 7-oxide **(Int 9b)** (15.0 g, 67.2 mmol) in DMF (150 mL) was added TFAA (28.0 g, 134 mmol) dropwise at 0 °C. The mixture was stirred at 0 °C for 3 h. The mixture was poured into water (300 mL) and stirred for 5 min. A precipitate was formed. The solid was collected by filtration and dried to give the title compound as a yellow solid, which was used in the next step without further purification.

### Step 4: Ethyl 6-chlorothieno[2,3-b]pyridine-2-carboxylate (Int 9d)

To a solution of ethyl 6-hydroxythieno[2,3-b]pyridine-2-carboxylate **(Int 9c)** (14.8 g, 66.3 mmol) in DMF (150 mL) was added POCI₃ (50.7 g, 332 mmol), and the mixture was stirred at 100 °C overnight. The mixture was cooled to rt and the solvent was removed under reduced pressure. The residue was diluted with water (200 mL) and adjusted to pH = 8 with saturated aqueous NaHCO₃. The mixture was extracted with EtOAc (3 x 200 mL). The combined organic layers were washed with brine (200 mL), dried over anhydrous Na₂SO₄, filtered and concentrated to dryness. The crude product was purified by column chromatography (PE/EtOAc = 8:1) to give the title compound.

### Step 5: Ethyl 6-methylthieno[2,3-b]pyridine-2-carboxylate (Int 9e)

A solution of ethyl 6-chlorothieno [2,3-b]pyridine-2-carboxylate **(Int 9d)** (5.0 g, 20.7 mmol), 2,4,6-trimethyl-1,3,5,2,4,6-trioxatriborinane (25 g, 200 mmol), Pd(PPh₃)₄ (2.3 g, 2.0 mmol), K₂CO₃ (8.4 g, 62.1 mmol) in dioxane (100 mL) was stirred at 100 °C for 14 h under N₂ atmosphere. The mixture was cooled to rt and filtered. The filtrate was concentrated to dryness. The residue was purified by column chromatography (PE/EtOAc = 8:1) to give the title compound.

### Step 6: Ethyl 6-(bromomethyl)thieno[2,3-b]pyridine-2-carboxylate (Int 9f)

A solution of ethyl 6-methylthieno[2,3-b]pyridine-2-carboxylate **(Int 9e)** (3.8 g,17.2 mmol), AIBN (281 mg, 1.72 mmol) and NBS (3.04 g, 17.2 mmol) in CCl₄ (50 mL) was stirred at 80 °C for 14 h. The mixture was cooled to rt and concentrated to dryness. The residue was purified by column chromatography (PE/EtOAc = 6:1) to give the title compound.

### Step 7: Ethyl 6-((4-ethylpiperazin-1-yl)methyl)thieno[2,3-b]pyridine-2-carboxylate (Int 9g)

To a solution of ethyl 6-(bromomethyl)thieno[2,3-b]pyridine-2-carboxylate **(Int 9f)** (4.6 g, 15.3 mmol) in acetonitrile (100 mL) was added 1-ethylpiperazine (2.6 g, 23 mmol) and K₂CO₃ (6.3 g, 45.9 mmol). The mixture was stirred at 70 °C overnight. The mixture was diluted with water (500 mL) and extracted with EtOAc (3 x 200 mL). The combined organic layers were washed with brine (200 mL), dried over anhydrous Na₂SO₄, filtered and concentrated to dryness to give the title compound which was used in the next step without further purification.

### Step 8: 6-((4-Ethylpiperazin-1-yl)methyl)thieno[2,3-b]pyridine-2-carboxylic acid (Int 9)

To a solution of ethyl 6-((4-ethylpiperazin-1-yl)methyl)thieno[2,3-b]pyridine-2-carboxylate **(Int 9g)** (4.1 g, 12.3 mmol) in MeOH/H₂O (5:1, 50 mL) was added NaOH (984 mg, 24.6 mmol) and the mixture was stirred at rt overnight. The mixture was diluted with water and adjusted to pH = 5 using 1M HCl. The mixture was extracted with EtOAc (3 x 200 mL). The aqueous phase was concentrated to dryness and the residue was purified by reverse phase column chromatography (gradient acetonitrile / H₂O = 5% - 80%) to give the title compound.

### Intermediate 10: 6-((4-Ethyl-3,3-dimethylpiperazin-1-yl)methyl)thieno[2,3-b]pyridine-2-carboxylic acid (Int 10)

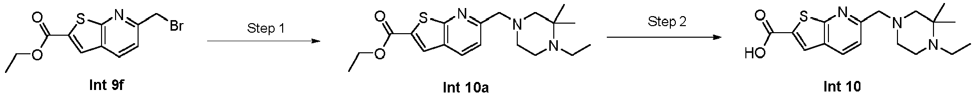

### Step 1: Ethyl 6-((4-ethyl-3,3-dimethylpiperazin-1-yl)methyl)thieno[2,3-b]pyridine-2-carboxylate (Int 10a)

To a mixture of ethyl 6-(bromomethyl)thieno[2,3-b]pyridine-2-carboxylate **(Int 9f)** (50.0 mg, 0.167 mmol) and cesium carbonate (162.8 mg, 0.500 mmol) in dry THF (2 mL) was added 1-ethyl-2,2-dimethylpiperazine (23.7 mg, 0.167 mmol) and the mixture was stirred at 50 °C overnight. The mixture was concentrated to dryness to afford the title compound which was used in the next step without further purification.

### Step 2: 6-((4-Ethyl-3,3-dimethylpiperazin-1-yl)methyl)thieno[2,3-b]pyridine-2-carboxylic acid (Int 10)

To ethyl 6-((4-ethyl-3,3-dimethylpiperazin-1-yl)methyl)thieno[2,3-b]pyridine-2-carboxylate **(Int 10a)** (55.1 mg, 0.152 mmol) dissolved in a mixture of MeOH and H₂O (3:1, 4 mL) sodium hydroxide (2M aqueous solution) (1 mL) was added and the mixture was stirred at 50 °C for 1 h. The mixture was concentrated to dryness and the residue was purified by preparative HPLC to afford the title compound as a white powder.

### Intermediate 10/1: 6-((3,3-Difluoroazetidin-1-yl)methyl)thieno[2,3-b]pyridine-2-carboxylic acid (Int 10/1)

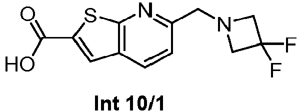

The title compound was prepared similar as described for **Int 10,** using in step 1 3,3-difluoroazetidine hydrochloride in place of 1-ethyl-2,2-dimethylpiperazine.

### Intermediate 10/2: 6-(((1-Ethylpiperidin-4-yl)amino)methyl)thieno[2,3-b]pyridine-2-carboxylic acid (Int 10/2)

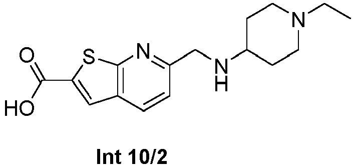

The title compound was prepared similar as described for **Int 10,** using in step 1 1-ethylpiperidin-4-amine in place of 1-ethyl-2,2-dimethylpiperazine.

### Intermediate 10/3: 6-((Diethylamino)methyl)thieno[2,3-b]pyridine-2-carboxylic acid (Int 10/3)

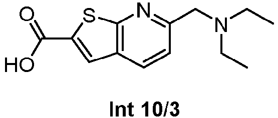

The title compound was prepared similar as described for **Int 10,** using in step 1 diethylamine in place of 1-ethyl-2,2-dimethylpiperazine.

### Intermediate 10/4: 6-(Piperazin-1-ylmethyl)thieno[2,3-b]pyridine-2-carboxylic acid (Int 10/4)

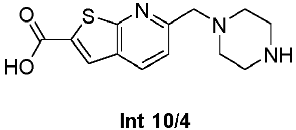

The title compound was prepared similar as described for Intermediate **10** using in step 1 1-(piperazin-1-yl)ethan-1-one in place of 1-ethyl-2,2-dimethylpiperazine.

### Intermediate 11: 6-((4-Ethylpiperazin-1-yl)methyl)benzo[b]thiophene-2-carboxylic acid (Int 11)

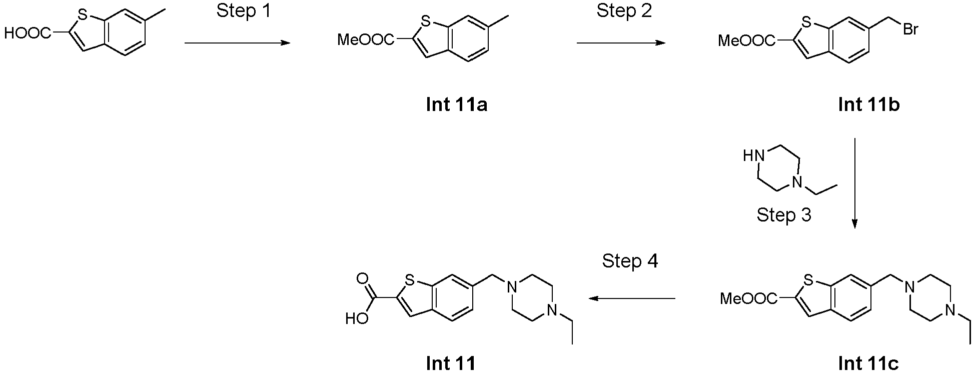

### Step 1: Methyl 6-methylbenzo[b]thiophene-2-carboxylate (Int 11a)

To a solution of 6-methylbenzo[b]thiophene-2-carboxylic acid (600.0 mg, 3.121 mmol) in dry MeOH (20.0 mL) was added SOCl₂ (0.250 mL, 3.433 mmol) dropwise at 0 °C . The mixture was warmed up to rt and then refluxed for 18 h. The mixture was concentrated to dryness and the crude product was used in the next step without further purification.

### Step 2: Methyl 6-(bromomethyl)benzo[b]thiophene-2-carboxylate (Int 11b)

To a solution of methyl 6-methylbenzo[b]thiophene-2-carboxylate **(Int 11a)** (644 mg, 3.121 mmol) in dry MeCN (15.6 mL) was added NBS (611 mg, 3.433 mmol) and benzoyl peroxide (151 mg, 0.468 mmol) at rt. The mixture was refluxed for 18 h. The mixture was cooled to rt and diluted with EtOAc (50 mL). The organic layer was washed with NaHCO₃ (20 mL), water (20 mL) and brine (20 mL). The organic layer was dried over MgSO₄, filtered and concentrated to dryness. The residue was purified by column chromatography on silica gel using gradient 0-4% EtOAc in cyclohexane to afford the title compound.

**Step 3:** Methyl 6-((4-ethylpiperazin-1-yl)methyl)benzo[b]thiophene-2-carboxylate **(Int 11c)** To a solution of methyl 6-(bromomethyl)benzo[b]thiophene-2-carboxylate **(Int 11b)** (522 mg, 0.915 mmol) in dry THF (4.6 mL) was added Cs₂CO₃ (328 mg, 1.007 mmol) and 1-ethylpiperazine (0.116 mL, 0.915 mmol). The mixture was stirred at 60 °C for 3 h. The mixture was taken directly for the next step.

### Step 4: 6-((4-Ethylpiperazin-1-yl)methyl)benzo[b]thiophene-2-carboxylic acid (Int 11)

To methyl 6-((4-ethylpiperazin-1-yl)methyl)benzo[b]thiophene-2-carboxylate **(Int 11c)** (mixture from step 3) was added water (1 mL) and LiOH·H₂O (115 mg, 2.746 mmol). The mixture was stirred at rt for 18 h. The mixture was concentrated to dryness and the residue was purified by preparative HPLC to afford the title compound as a white solid.

### Intermediate 11/1: 6-((4-(2,2,2-Trifluoroethyl)piperazin-1-yl)methyl)benzo[b]thiophene-2-carboxylic acid (Int 11/1)

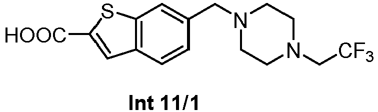

The title compound was prepared similar as described for intermediate **11** using in step 3 1-(2,2,2-trifluoroethyl)piperazine in place of 1-ethylpiperazine.

### Intermediate 11/2: 6-((4-Methylpiperazin-1-yl)methyl)benzo[b]thiophene-2-carboxylic acid (Int 11/2)

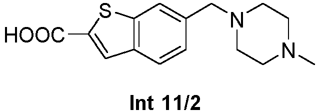

The title compound was prepared similar as described for intermediate **11** using in step 3 1-methylpiperazine in place of 1-ethylpiperazine.

### Intermediate 11/3: 6-(Morpholinomethyl)benzo[b]thiophene-2-carboxylic acid (Int 11/3)

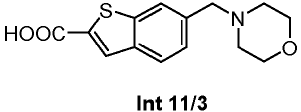

The title compound was prepared similar as described for intermediate **11** using in step 3 morpholine in place of 1-ethylpiperazine.

### Intermediate 11/4: 6-((4-(tert-Butoxycarbonyl)-1,4-diazepan-1-yl)methyl)benzo[b]thiophene-2-carboxylic acid (Int 11/4)

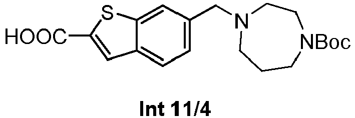

The title compound was prepared similar as described for intermediate **11** using in step 3 *tert-*butyl 1,4-diazepane-1-carboxylate in place of 1-ethylpiperazine.

### Intermediate 12: 6-(3-(3-Methoxyazetidin-1-yl)propoxy)benzo[b]thiophene-2-carboxylic acid (Int 12)

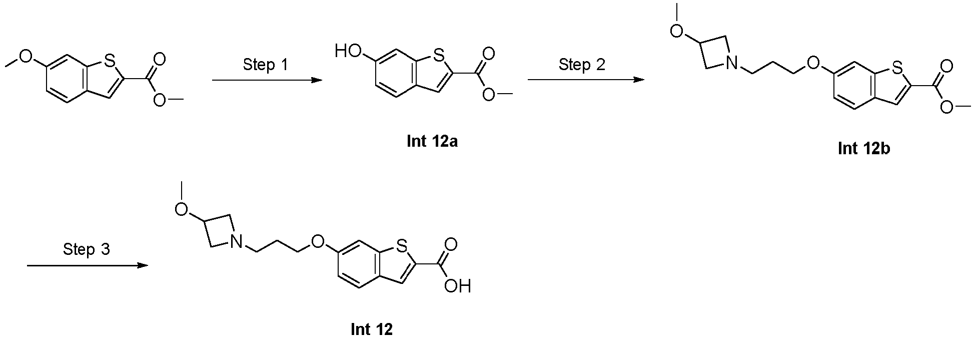

### Step 1: Methyl 6-hydroxybenzo[b]thiophene-2-carboxylate (Int 12a)

To a mixture of methyl 6-methoxybenzo[b]thiophene-2-carboxylate (200 mg, 0.90 mmol) in dry DCM (5 mL) was added boron tribromide (1.00 M in DCM, 1.8 mL, 1.80 mmol) dropwise at - 75 °C under N₂ atmosphere. After addition, the mixture was stirred at - 75 °C for 2 h and stirred at rt for another 16 h. EtOH (20 mL) and saturated aqueous NaHCO₃ (20 mL) were added to the mixture. The mixture was extracted with DCM (3 x 50 mL), and the combined organic layers were washed with brine, dried over Na₂SO₄, filtered, and concentrated to dryness. The residue was purified by column chromatography on silica gel (EtOAc/PE = 1:6) to afford the title compound as a light-yellow solid.

### Step 2: Methyl 6-(3-(3-methoxyazetidin-1-yl)propoxy)benzo[b]thiophene-2-carboxylate (Int 12b)

Methyl 6-hydroxybenzo[b]thiophene-2-carboxylate **(Int 12a)** (50 mg, 0.24 mmol), 1-bromo-3-chloropropane (28 µL, 0.29 mmol) and cesium carbonate (172 mg, 0.53 mmol) were dissolved in dry DMF (2 mL) and the mixture was stirred at 50 °C overnight. 3-Methoxyazetidine hydrochloride (35.6 mg, 0.29 mmol) was added and the mixture was stirred for 2 h at 50 °C. The mixture was concentrated to dryness to afford the title compound which was used in the next step without further purification.

### Step 3: 6-(3-(3-Methoxyazetidin-1-yl)propoxy)benzo[b]thiophene-2-carboxylic acid (Int 12)

To a mixture of methyl 6-(3-(3-methoxyazetidin-1-yl)propoxy)benzo[*b*]thiophene-2-carboxylate **(Int 12b)** (80 mg, 0.24 mmol) in THF/H₂O (3:1) was added lithium hydroxide monohydrate (50 mg, 1.20 mmol) and the mixture was stirred at rt overnight. The mixture was concentrated to dryness and the residue was purified by preparative HPLC to afford the title compound as a white powder.

### Intermediate 13: 6-(2-Acetamidoethoxy)benzo[b]thiophene-2-carboxylic acid (Int 13)

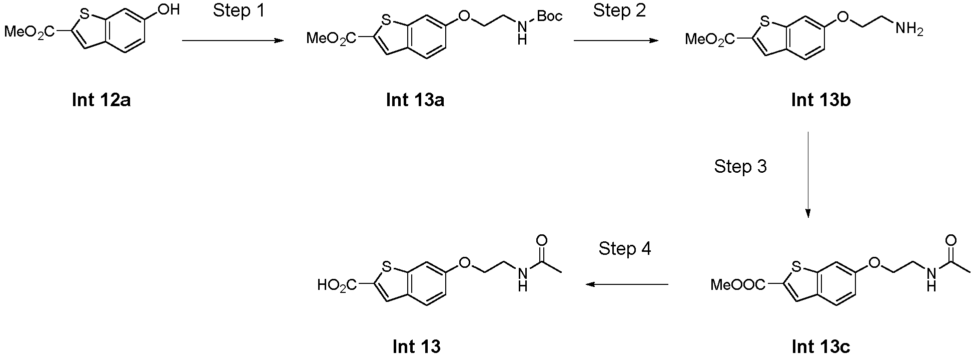

### Step 1: Methyl 6-(2-((tert-butoxycarbonyl)amino)ethoxy)benzo[b]thiophene-2-carboxylate (Int 13a)

To a solution of methyl 6-hydroxybenzo[b]thiophene-2-carboxylate **(Int 12a)** (50.0 mg, 0.240 mmol) and Cs₂CO₃ (93.9 mg, 0.288 mmol) in DMF (2.4 mL) was added *tert-*butyl (2-bromoethyl)carbamate (83.9 mg, 0.288 mmol). The mixture was stirred at 60 °C overnight. Water was added (10 mL) and the mixture was extracted with EtOAc (3 x 10 mL). The combined organic layers were dried over MgSO₄, filtered and concentrated to dryness to afford the title compound as a yellow oil which was used in the next step without further purification.

### Step 2: Methyl 6-(2-aminoethoxy)benzo[b]thiophene-2-carboxylate (Int 13b)

TFA (1.4 mL) was added to a solution of methyl 6-(2-((tert-butoxycarbonyl)amino)ethoxy)benzo[b]thiophene-2-carboxylate **(Int 13a)** (100 mg, 0.285 mmol) in DCM (2.8 mL) at 0 °C. The mixture was stirred at rt overnight. Saturated aqueous NaHCO₃ (2 mL) was added and the mixture was extracted with EtOAc (3 x 10 mL). The combined organic layers were dried over MgSO₄, filtered and concentrated to dryness to afford the title compound, which was used in the next step without further purification.

### Step 3: Methyl 6-(2-acetamidoethoxy)benzo[b]thiophene-2-carboxylate (Int 13c)

Methyl 6-(2-aminoethoxy)benzo[b]thiophene-2-carboxylate **(Int 13b)** (20.0 mg, 0.080 mmol) was dissolved in THF (1.7 mL). Triethylamine (0.022 mL, 0.159 mmol) was added, followed by dropwise addition of acetyl chloride (0.006 mL, 0.088 mmol). The mixture was stirred overnight at rt. Water (5 mL) was added and the mixture was extracted with EtOAc (3 x 10 mL). The combined organic layers were dried over MgSO₄, filtered and concentrated to dryness to afford the title compound which was used in the next step without further purification.

### Step 4: 6-(2-Acetamidoethoxy)benzo[b]thiophene-2-carboxylic acid (Int 13)

To a solution of methyl 6-(2-acetamidoethoxy)benzo[b]thiophene-2-carboxylate **(Int 13c)** (24.0 mg, 0.082 mmol) in THF (0.8 mL) and water (0.8 mL) was added LiOH·H₂O (17.5 mg, 0.417 mmol) and the mixture was stirred at rt overnight. 1M HCl was added to adjust the pH to 5. The mixture was extracted with EtOAc (3 x 10 mL). The combined organic layers were dried over MgSO₄, filtered and concentrated to dryness to afford the title compound which was used in the next step without further purification.

### Intermediate 14: Methyl 6-chlorothieno[2,3-b]pyridine-2-carboxylate (Int 14)

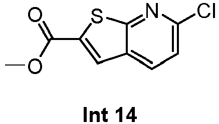

The title compound was prepared similar as described for intermediate **9d** steps 1 - 4 using in step 1 methyl 2-mercaptoacetate in place of ethyl 2-mercaptoacetate.

### Intermediate 15: 6-(2-(Pyrrolidin-1-yl)ethoxy)thieno[2,3-b]pyridine-2-carboxylic acid (Int 15)

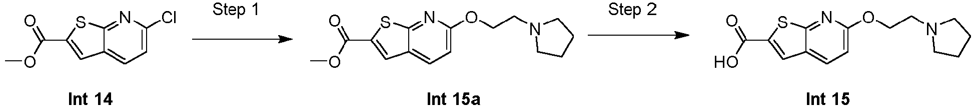

### Step 1: Methyl 6-(2-(pyrrolidin-1-yl)ethoxy)thieno[2,3-b]pyridine-2-carboxylate (Int 15a)

To a solution of 2-(pyrrolidin-1-yl)ethan-1-ol (0.54 g, 4.68 mmol) in THF (50mL) was added NaH (60% dispersed in mineral oil, 0.374 g, 15.6 mmol) in portions at 0 °C. The mixture was stirred at 0 °C for 5 min, then warmed to rt and stirred for 1 h at that temperature. Methyl 6-chlorothieno[2,3-b] pyridine-2-carboxylate **(Int 14)** (1.0 g, 4.4 mmol) was added and the mixture was stirred at 75 °C for 20 min. The mixture was cooled to 0 °C, quenched with H₂O (20 mL) and extracted with EtOAc (3 x 30 mL). The combined organic layers were washed with water and brine, dried over anhydrous Na₂SO₄, filtered and concentrated to dryness. The residue was purified by flash chromatography on silica (DCM/MeOH = 20:1) to give the title compound.

### Step 2: 6-(2-(Pyrrolidin-1-yl)ethoxy)thieno[2,3-b]pyridine-2-carboxylic acid (Int 15)

To a solution of methyl 6-(2-(pyrrolidin-1-yl)ethoxy)thieno[2,3-b]pyridine-2-carboxylate **(Int 15a)** (850 mg, 2.77 mmol) in EtOH (15 mL) was added a solution of NaOH (359 mg in 5 mL water, 5.97 mmol). The mixture was stirred at 70 °C for 1 h. The mixture was diluted with water and adjusted to pH = 6 using 1M HCl. The mixture was filtered and the filtrate was concentrated to dryness. The residue was purified by reverse-phase column chromatography (Solvent A: H₂O, Solvent B: MeCN) to give the title compound.

### Intermediate 15/1: 6-(3-(Pyrrolidin-1-yl)propoxy)thieno[2,3-b]pyridine-2-carboxylic acid (Int 15/1)

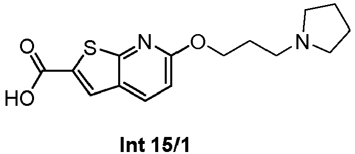

The title compound was prepared similar as described for intermediate **15** using in step 1 3-(pyrrolidin-1-yl)propan-1-ol in place of 2-(pyrrolidin-1-yl)ethan-1-ol.

### Intermediate 16: 6-((Pyridin-2-yloxy)methyl)benzo[b]thiophene-2-carboxylic acid (Int 16)

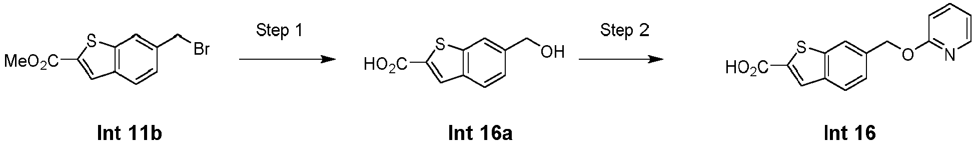

### Step1: 6-(Hydroxymethyl)benzo[b]thiophene-2-carboxylic acid (Int 16a)

To a mixture of methyl 6-(bromomethyl)benzo[b]thiophene-2-carboxylate **(Int 11b)** (3.0 g, 10.5 mmol) in dioxane (100 mL) was added K₂CO₃ (5.8 g, 42 mmol) in H₂O (100 mL). The mixture was stirred at 100 °C for 1 h. Aqueous HCl was added to the mixture to adjust to pH = 2. The aqueous layer was extracted with ethyl acetate (3 x 100 mL). The combined organic layers were washed with brine (100 mL), dried over Na₂SO₄, filtered and concentrated to dryness to give the title compound as a yellow solid.

### Step 2: 6-((Pyridin-2-yloxy)methyl)benzo[b]thiophene-2-carboxylic acid (Int 16)

To a mixture of 6-(hydroxymethyl)benzo[b]thiophene-2-carboxylic acid **(Int 16a)** (2.2 g, 10.56 mmol), 2-chloropyridine (3.56 g, 31.68 mmol) and 18-Crown-6 (0.44 g, 1.66 mmol) in toluene (150 mL) was added *t*-BuOK (11.86 g, 105.6 mmol). The mixture was stirred at 120 °C for 6 h. The mixture was washed with H₂O (3 x 100 mL). The aqueous layer was adjusted to pH = 2 with aqueous HCl and extracted with EtOAc (3 x 100 mL). The combined organic layers were dried over Na₂SO₄, filtered and concentrated to dryness to afford the title compound as a white solid, which was used in the next step without further purification.

### Intermediate 17: 6-(((3aR,6aS)-5-Ethylhexahydropyrrolo[3,4-c]pyrrol-2(1H)-yl)methyl)thieno[2,3-b]pyridine-2-carboxylic acid (Int 17)

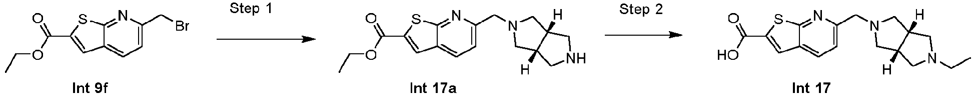

### Step 1: Ethyl 6-(((3aR,6aS)-hexahydropyrrolo[3,4-c]pyrrol-2(1H)-yl)methyl)thieno[2,3-b]pyridine-2-carboxylate (Int 17a)

A mixture of ethyl 6-(bromomethyl)thieno[2,3-b]pyridine-2-carboxylate **(Int 9f)** (30 mg, 0.10 mmol) in DCM (0.5 mL), triethylamine (11.1 mg, 0.11 mmol) and *tert-*butyl (3aR,6aS)-3a,6a-dimethylhexahydropyrrolo[3,4-c]pyrrole-2(1*H*)-carboxylate (22.3 mg, 0.11 mmol) was stirred at rt overnight. TFA (0.25 mL) was added and the mixture was stirred at rt overnight. The mixture was concentrated to dryness to afford the title compound which was used in the next step without further purification.

### Step 2: 6-(((3aR,6aS)-5-Ethylhexahydropyrrolo[3,4-c]pyrrol-2(1H)-yl)methyl)thieno[2,3-b]pyridine-2-carboxylic acid (Int 17)

To a solution of ethyl 6-(((3aR,6aS)-3a,6a-dimethylhexahydropyrrolo[3,4-c]pyrrol-2(1H)-yl)methyl)thieno[2,3-b]pyridine-2-carboxylate **(Int 17a)** (25 mg, 0.075 mmol) in dry THF were added Cs₂CO₃ (24 mg, 0.075 mmol) and iodoethane (12 mg, 0.075 mmol). The mixture was stirred at rt for 2 h. Aqueous NaOH was added (2M, 200 µL) and the mixture was stirred at rt for 2 h. The mixture was concentrated to dryness and the residue was purified by preparative HPLC to afford the title compound.

### Intermediate 18: Methyl 6-hydroxythieno[2,3-b]pyridine-2-carboxylate (Int 18)

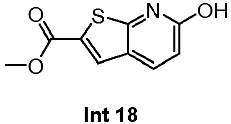

The title compound was prepared similar as described for intermediate **9c** steps 1 - 3 using in step 1 methyl 2-mercaptoacetate in place of ethyl 2-mercaptoacetate.

### Intermediate 19: 6-(2-(4-Ethylpiperazin-1-yl)ethyl)thieno[2,3-b]pyridine-2-carboxylic acid (Int 19)

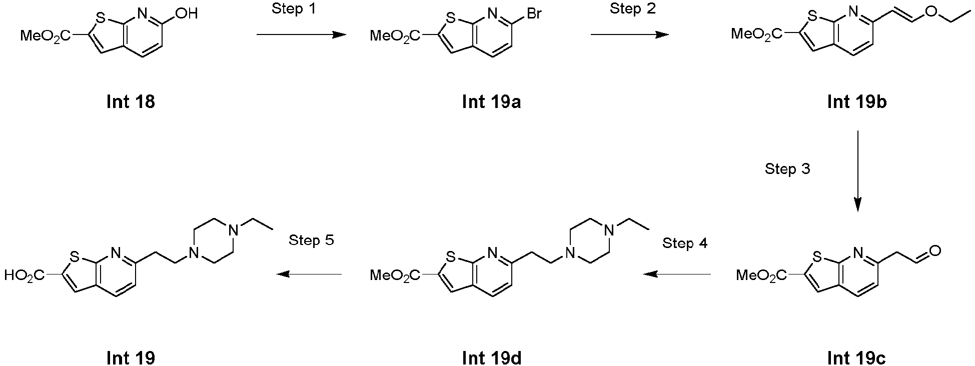

### Step 1: Methyl 6-bromothieno[2,3-b]pyridine-2-carboxylate (Int 19a)

A mixture of methyl 6-hydroxythieno [2, 3-b] pyridine-2-carboxylate **(Int 18)** (15.7 g, 75 mmol) and POBr₃ (96.2 mL, 0.96 mol) in DMF (150 mL) was stirred at 120°C for 16 h. The mixture was concentrated and diluted with DCM (300 mL). The organic layer was washed with saturated aqueous NaHCO₃ (2 x 80 mL) and brine (2 x 100 mL), dried over anhydrous Na₂SO₄, filtered and concentrated to dryness. The residue was purified by silica gel column chromatography (DCM/MeOH = 20:1) to afford the title compound.

### Step 2: Methyl (E)-6-(2-ethoxyvinyl)thieno[2,3-b]pyridine-2-carboxylate (Int 19b)

To a mixture of methyl 6-bromothieno[2,3-b]pyridine-2-carboxylate **(Int 19a)** (2.1 g, 7.7 mmol) and KF (893 mg, 15.37 mmol) in 1,4-dioxane (40 mL) and water (8 mL) was added tricyclohexylphosphine (418 mg, 1.49 mmol), (*E*)-2-(2-ethoxyvinyl)-4,4,5,5-tetramethyl-1,3,2-dioxaborolane (1.75 g, 8.8 mmol) and Pd₂(dba)₃ (673 mg, 0.74 mmol). The mixture was stirred at 100 °C for 16 h under nitrogen atmosphere. The mixture was cooled to rt, diluted with water and extracted with EtOAc (3 x 100 mL). The combined organic layers were washed with water and brine, dried over anhydrous sodium sulfate, filtered and concentrated to dryness. The residue was purified by silica gel chromatography (PE/EtOAc = 1:1) to afford the title compound.

### Step 3: Methyl 6-(2-oxoethyl)thieno[2,3-b]pyridine-2-carboxylate (Int 19c)

To a solution of methyl (*E*)-6-(2-ethoxyvinyl) thieno[2,3-b]pyridine-2-carboxylate **(Int 19b)** (0.6 g, 2.28 mmol) in DCM (10 mL) was added concentrated HCl (6 mL) and the mixture was stirred at rt for 16 h. The mixture was concentrated to dryness to afford the title compound which was used in the next step without further purification.

**Step 4:** Methyl 6-(2-(4-ethylpiperazin-1-yl)ethyl)thieno[2,3-b]pyridine-2-carboxylate **(Int 19d)** To a solution of methyl 6-(2-oxoethyl)thieno[2,3-b]pyridine-2-carboxylate (**Int 19c**) (400 mg, 1.7 mmol), 1-ethylpiperazine (213.5 mg, 1.86 mmol) and sodium triacetoxyborohydride (1.08 g, 5.1 mmol) in DCE (50 mL) was added AcOH (0.2 mL). The mixture was stirred at rt for 16 h. The mixture was diluted with water (80 mL) and extracted with DCM (3 x 100 mL). The combined organic layers were washed with water and brine, dried over anhydrous sodium sulfate, filtered and concentrated to dryness. The residue was purified by silica gel chromatography (EtOAc/PE = 1:5) to afford the title compound.

### Step 5: 6-(2-(4-Ethylpiperazin-1-yl)ethyl)thieno[2,3-b]pyridine-2-carboxylic acid (Int 19)

To a solution of methyl 6-(2-(4-ethylpiperazin-1-yl) ethyl)thieno[2,3-b]pyridine-2-carboxylate **(Int 19d)** (350 mg, 1.05 mmol) in MeOH (10 mL) and water (2 mL) was added LiOH (66 mg, 2.76 mmol). The mixture was stirred at 60 °C for 2 h. The mixture was adjusted to pH = 5 using 1M HCl and extracted with EtOAc (2 x 20 mL). The combined organic layers were washed with brine, dried over anhydrous sodium sulfate, filtered and concentrated to dryness to afford the title compound, which was used in the next step without further purification.

### Intermediate 20: 6-((1-Ethylpiperidin-4-yl)methyl)thieno[2,3-b]pyridine-2-carboxylic acid (Int 20)

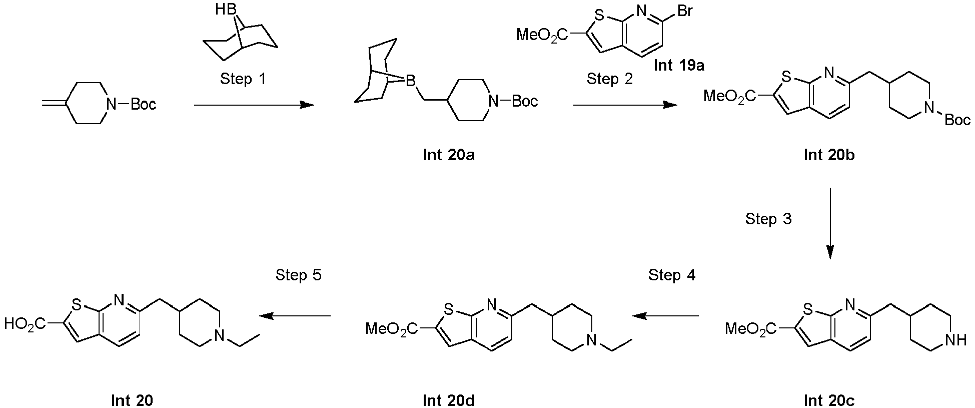

### Step 1: tert-Butyl 4-(((1s,5s)-9-borabicyclo[3.3.1]nonan-9-yl)methyl)piperidine-1-carboxylate (Int 20a)

To a solution of *tert-*butyl 4-methylenepiperidine-1-carboxylate (4.0 g, 0.020 mol) in THF (60 mL) was added 9-BBN (4.9 g, 40 mmol) and the mixture was refluxed for 1 h. The mixture was cooled to rt and concentrated to dryness to afford the title compound, which was used in the next step without further purification.

### Step 2: Methyl 6-((1-(tert-Butoxycarbonyl)piperidin-4-yl)methyl)thieno[2,3-b]pyridine-2-carboxylate (Int 20b)

To a solution of *tert-*Butyl 4-(((1s,5s)-9-borabicyclo[3.3.1]nonan-9-yl)methyl)piperidine-1-carboxylate **(Int 20a)** (5.0 g crude) in DMF (40 mL) and H₂O (4 mL) was added Pd(dppf)Cl₂ (1.6 g, 2.0 mmol), methyl 6-bromothieno[2,3-b]pyridine-2-carboxylate **(Int 19a)** (6.7 g, 0.025 mol) and K₂CO₃ (4.0 g, 0.029 mol). The mixture was stirred at 60 °C for 16 h under nitrogen atmosphere. The mixture was concentrated under reduced pressure, diluted with EtOAc, washed with H₂O and brine, dried over anhydrous Na₂SO₄, filtered and concentrated to dryness. The residue was purified by silica gel chromatography (PE/EtOAc = 1:1) to give the title compound.

### Step 3: Methyl 6-(piperidin-4-ylmethyl)thieno[2,3-b]pyridine-2-carboxylate (Int 20c)

To a solution of methyl 6-((1-(tert-butoxycarbonyl)piperidin-4-yl)methyl)thieno[2,3-b]pyridine-2-carboxylate **(Int 20b)** (2.6 g, 6.7 mmol) in DCM (20 mL) was added TFA (10 mL). The mixture was stirred at rt for 1 h. The mixture was concentrated to dryness to afford the title compound, which was used in the next step without further purification.

### Step 4: Methyl 6-((1-ethylpiperidin-4-yl)methyl)thieno[2,3-b]pyridine-2-carboxylate (Int 20d)

To a solution of methyl 6-(piperidin-4-ylmethyl)thieno[2,3-b]pyridine-2-carboxylate **(Int 20c)** (3 g crude) in DCE (50 mL) was added acetaldehyde (792 mg, 18 mmol), TEA (2.4 g, 24 mmol). Sodium triacetoxyborohydride (5.0 g, 24 mmol) was added and the mixture was stirred at rt for 16 h. Water (50 mL) was added and the mixture was extracted with DCM (2 x 200 mL). The combined organic layers were washed with brine, dried over anhydrous sodium sulfate, filtered and concentrated to dryness. The residue was purified by silica gel chromatography (DCM/MeOH = 20:1) to afford the title compound.

### Step 5: 6-((1-Ethylpiperidin-4-yl)methyl)thieno[2,3-b]pyridine-2-carboxylic acid (Int 20)

To a solution of methyl 6-((1-ethylpiperidin-4-yl)methyl)thieno[2,3-b]pyridine-2-carboxylate **(Int 20d)** (400 mg, 1.25 mmol) in MeOH (16 mL) and water (4 mL) was added LiOH (52 mg, 2.2 mmol). The mixture was stirred at 60 °C for 1 h. The pH was adjusted to pH = 5 with 1M HCl and the mixture was extracted with EtOAc (2 x 20 mL). The combined organic layers were washed with brine, dried over anhydrous sodium sulfate, filtered and concentrated to dryness to afford the title compound which was used in the next step without further purification.

### Intermediate 21: 6-((4-Ethylpiperazin-1-yl)methyl)imidazo[2,1-b]thiazole-2-carboxylic acid (Int 21)

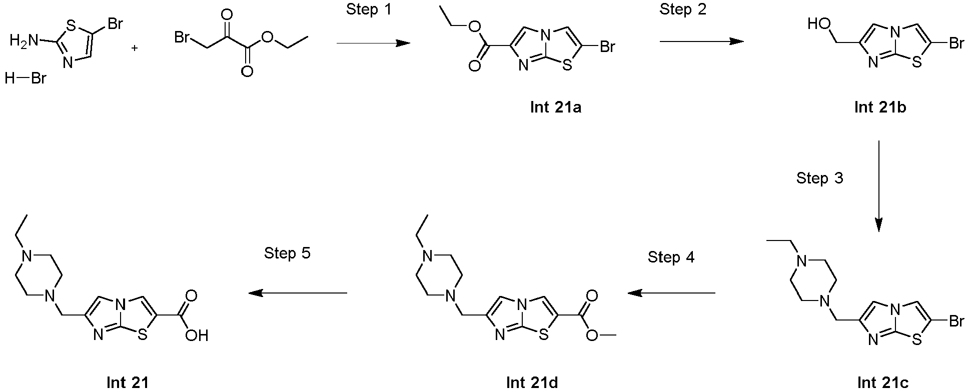

### Step 1: Ethyl 2-bromoimidazo[2,1-b]thiazole-6-carboxylate (Int 21a)

To a solution of 2-amino-5-bromothiazole monohydrobromide (20 g, 77 mmol) in ethanol (300 mL) was added ethyl bromopyruvate (19 mL, 154 mmol). The mixture was stirred at reflux overnight. The mixture was concentrated. Water (300 mL) was added to the residue and the aqueous layer was extracted with EtOAc (300 mL). The organic layer was washed with brine (200 mL), dried over Na₂SO₄, filtered and concentrated to dryness. The residue was purified by flash column chromatography on silica gel (PE/EtOAc = 1:1) to afford the title compound as a brown solid.

### Step 2: (2-Bromoimidazo[2,1-b]thiazol-6-yl)methanol (Int 21b)

To a solution of ethyl 2-bromoimidazo[2,1-b]thiazole-6-carboxylate **(Int 21a)** (11.5 g, 42.0 mmol) was added DIBAL-H (42 mL, 1M in hexane) dropwise at 0 °C over a period of 0.5 h under argon. The mixture was allowed to warm up to rt and stirred for 3 h. Sodium sulfate decahydrate (27 g, 83.8 mmol) was added in batches and under ice bath cooling. The suspension was filtered through Celite^{®} using MeOH to rinse the Celite^{®} pad. The filtrate was concentrated to dryness and the residue was purified by column chromatography on silica gel (DCM/MeOH =10:1) to afford the title compound as a pale yellow solid.

### Step 3: 2-Bromo-6-((4-ethylpiperazin-1-yl)methyl)imidazo[2,1-b]thiazole (Int 21c)

To a stirring solution of (2-bromoimidazo[2,1-b]thiazol-6-yl)methanol **(Int 21b)** (1.6 g, 6.9 mmol) and triethylamine (1.9 mL, 13.6 mmol) in dry DCM (20 mL) was added methanesulfonyl chloride (0.8 mL, 10.3 mmol). The mixture was stirred for 0.5 h. The mixture was concentrated and redissolved in dry DCM (20 mL). Triethylamine (1.9 mL, 13.6 mmol) was added, followed by the addition of 1-ethylpiperazine (0.96 mL, 7.6 mmol). The mixture was stirred for 2 h at rt. The mixture was washed with water (40 mL) and brine (40 mL). The organic layer was dried over Na₂SO₄, filtered and concentrated to dryness. The residue was purified by flash chromatography (DCM/MeOH = 10:1) to afford the title compound as pale yellow solid.

### Step 4: Methyl 6-((4-ethylpiperazin-1-yl)methyl)imidazo[2,1-b]thiazole-2-carboxylate (Int 21d)

To a solution of 2-bromo-6-((4-ethylpiperazin-1-yl)methyl)imidazo[2,1-b]thiazole **(Int 21c)** (1.1 g, 3.3 mmol) and triethylamine (1.9 mL, 13.6 mmol) in MeOH (20 mL) was added Pd(OAc)₂ (226 mg, 1.0 mmol) and XPhos (481 mg, 1.0 mmol) under argon protection. The mixture was saturated with CO gas and stirred at 60 °C overnight. The mixture was filtered through a pad of Celite^{®}. The filtrate was concentrated to dryness to afford the title compound as an orange solid.

### Step 5: 6-((4-Ethylpiperazin-1-yl)methyl)imidazo[2,1-b]thiazole-2-carboxylic acid (Int 21)

To a solution of methyl 6-((4-ethylpiperazin-1-yl)methyl)imidazo[2,1-b]thiazole-2-carboxylate **(Int 21d)** (1.0 g, 3.2 mmol) in dry THF (10 mL) was added aqueous LiOH (6.4 mL, 1M). The mixture was stirred at reflux for 3 h. The mixture was concentrated and aqueous HCl (1M) was added with stirring until pH = 2. The mixture was concentrated to dryness. DCM was added to the residue and the suspension was stirred for 1 h. The mixture was filtered and the filtrate was concentrated to dryness to afford the title compound as an orange solid.

### Intermediate 22: 5-(2-(Pyrrolidin-1-yl)ethoxy)-1H-indole-2-carboxylic acid (Int 22)

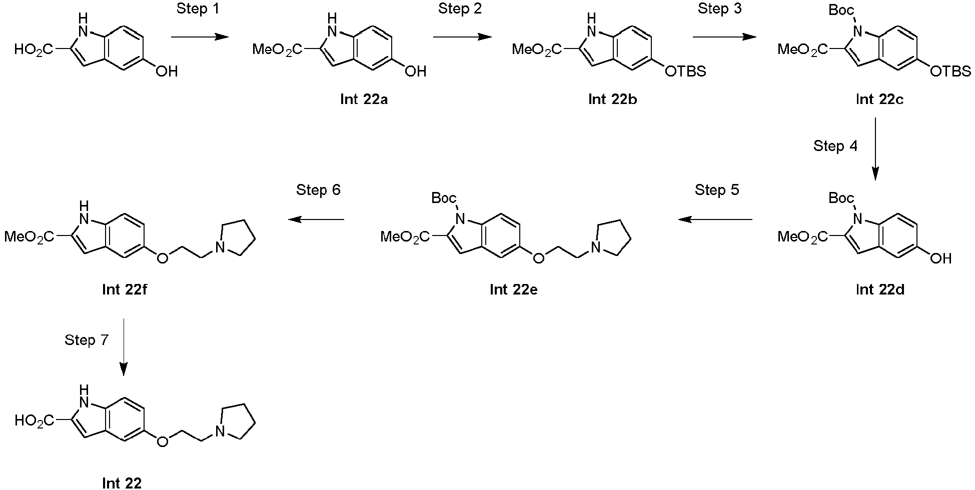

### Step 1: Methyl 5-hydroxy-1H-indole-2-carboxylate (Int 22a)

A mixture of 5-hydroxy-1*H*-indole-2-carboxylic acid (500 mg, 2.82 mmol) in methanol (28 mL) was cooled to 0 °C and thionyl chloride (369 mg, 3.11 mmol) was added. The mixture was stirred at 50 °C overnight. The mixture was concentrated to dryness to afford the title compound, which was used in the next step without further purification.

### Step2: Methyl 5-((tert-butyldimethylsilyl)oxy)-1H-indole-2-carboxylate (Int 22b)

To a of mixture of methyl 5-hydroxy-1*H*-indole-2-carboxylate **(Int 22a)** (540 mg, 2.82 mmol), imidazole (385 mg, 5.65 mmol) in DMF (28 mL) was added dropwise a solution of *tert-*butylchlorodimethylsilane (511 mg, 3.39 mmol) in DMF. The mixture was stirred at rt overnight. Imidazole (192 mg, 2.82 mmol) and tert-butylchlorodimethylsilane (255 mg, 1.69 mmol) were added and the mixture was stirred at rt for 1 h. The mixture was concentrated to dryness. The residue was purified by column chromatography (gradient EtOAc/cyclohexane) on silica gel to afford the title compound.

### Step 3: 1-(tert-Butyl) 2-methyl 5-((tert-butyldimethylsilyl)oxy)-1H-indole-1,2-dicarboxylate (Int 22c)

A mixture of methyl 5-((tert-butyldimethylsilyl)oxy)-1*H*-indole-2-carboxylate (**Int 22b** ) (561 mg, 1.837 mmol) and NaH (132 mg, 24 mmol) in DMF (18 mL) was stirred for 30 min at rt. Di-*tert-*butyl dicarbonate (521 mg, 2.39 mmol) was added and the mixture was stirred at rt overnight. The mixture was concentrated to dryness and the residue was purified by column chromatography on silica gel (gradient EtOAc/cyclohexane) to afford the title compound.

### Step 4: 1-(tert-Butyl) 2-methyl 5-hydroxy-1H-indole-1,2-dicarboxylate (Int 22d)

A mixture of 1-(*tert*-butyl) 2-methyl 5-((tert-butyldimethylsilyl)oxy)-1*H*-indole-1,2-dicarboxylate **(Int 22c)** (376 mg, 0.927 mmol) in dry THF (10 L) a solution of TBAF in THF (1 M, 0.927 mL, 0.927 mmol) dropwise at 0 °C. The mixture was stirred at rt for 20 min. The mixture was concentrated to dryness and the residue was purified by column chromatography on silica gel (gradient EtOAc/cyclohexane) to afford the title compound.

### Step 5: 1-(tert-Butyl) 2-methyl 5-(2-(pyrrolidin-1-yl)ethoxy)-1H-indole-1,2-dicarboxylate (Int 22e)

1-(*tert*-Butyl) 2-methyl 5-hydroxy-1*H*-indole-1,2-dicarboxylate **(Int 22d)** (50 mg, 0.172 mmol) was dissolved in dry DMF. NaH was added at 0 °C and the mixture was stirred at rt for 20 min. A solution of 1-(2-chloroethyl)pyrrolidine hydrochloride (29.2 mg, 0.172 mmol) in dry DMF (0.7 mL) was added dropwise and the mixture was stirred at 50 °C overnight. The mixture was concentrated and water was added to the residue (10 mL). The suspension was extracted with EtOAc (3 x 10 mL). The combined organic layers were dried over anhydrous MgSO₄, filtered and concentrated to dryness to afford the title compound, which was used in the next step without further purification.

### Step 6: Methyl 5-(2-(pyrrolidin-1-yl)ethoxy)-1H-indole-2-carboxylate (Int 22f)

TFA (600 µL) was added to a solution of 1-(*tert-*butyl) 2-methyl 5-(2-(pyrrolidin-1-yl)ethoxy)-1*H*-indole-1,2-dicarboxylate **(In 22e)** (47.0 mg, 0.121 mmol) in DCM (1.2 mL) at 0 °C. The mixture was stirred at rt for 1 h. Water was added and the mixture was extracted with DCM. The combined organic layers were dried over MgSO₄, filtered and concentrated to dryness to afford the title compound, which was used in the next step without further purification.

### Step 7: 5-(2-(Pyrrolidin-1-yl)ethoxy)-1H-indole-2-carboxylic acid (Int 22)

LiOH·H₂O (11.0 mg, 0.253 mmol) was added to a mixture of methyl 5-(2-(pyrrolidin-1-yl)ethoxy)-1*H*-indole-2-carboxylate **(Int 22f)** (11.0 mg, 0.038 mmol) in a mixture of water and THF (1:1, 0.8 mL). The mixture was stirred at rt overnight. The mixture was neutralized with 1M HCl and concentrated to dryness to afford the title compound, which was used in the next step without further purification.

### Intermediate 23: 6-((4-(tert-Butoxycarbonyl)piperazin-1-yl)methyl)thieno[2,3-b]pyridine-2-carboxylic acid (Int 23)

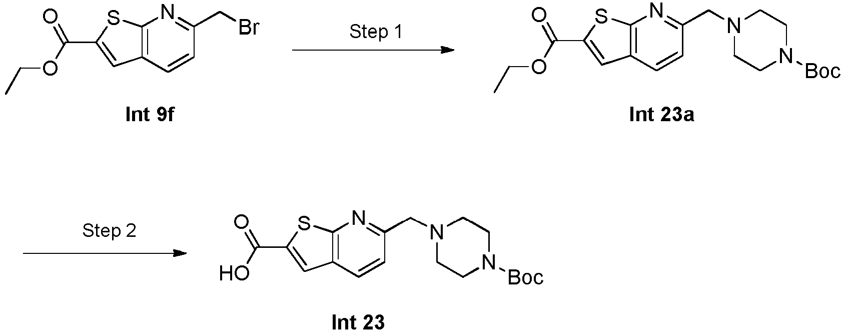

### Step 1: Ethyl 6-((4-(tert-butoxycarbonyl)piperazin-1-yl)methyl)thieno[2,3-b]pyridine-2-carboxylate (Int 23a)

To a mixture of ethyl 6-(bromomethyl)thieno[2,3-b]pyridine-2-carboxylate **(Int 9f)** (50.0 mg, 0.167 mmol) and cesium carbonate (162.8 mg, 0.500 mmol) in dry THF (2 mL) was added 1-*tert-*butyl piperazine-1-carboxylate (31.0 mg, 0.167 mmol) and the mixture was stirred at 50 °C overnight. The mixture was concentrated to dryness to afford the title compound, which was used in the next step without further purification.

### Step 2: 6-((4-(tert-Butoxycarbonyl)piperazin-1-yl)methyl)thieno[2,3-b]pyridine-2-carboxylic acid (Int 23)

To a mixture of ethyl 6-((4-(*tert*-butoxycarbonyl)piperazin-1-yl)methyl)thieno[2,3-b]pyridine-2-carboxylate **(Int 23a)** (60 mg, 0.148 mmol) in THF and H₂O (3:1, 4 mL) was added lithium hydroxide monohydrate (10.5 mg, 0.222 mmol) and the mixture was stirred at rt overnight. The mixture was concentrated to dryness and the residue was purified by preparative HPLC to afford the title compound as a white powder.

### Intermediate 23/1: 6-(((1R,4R)-5-(tert-Butoxycarbonyl)-2,5-diazabicyclo[2.2.1]heptan-2-yl)methyl)thieno[2,3-b]pyridine-2-carboxylic acid (Int 23/1)

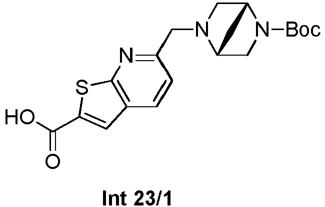

The title compound was prepared similar as described for **Int 23,** using in step 1 (1R,4*R*)-*tert-*butyl 2,5-diazabicyclo[2.2.1]heptane-2-carboxylate in place of 1-*tert-*butyl piperazine-1-carboxylate.

### Intermediate 23/2: 6-(((1S,4S)-5-(tert-Butoxycarbonyl)-2,5-diazabicyclo[2.2.1]heptan-2-yl)methyl)thieno[2,3-b]pyridine-2-carboxylic acid (Int 23/2)

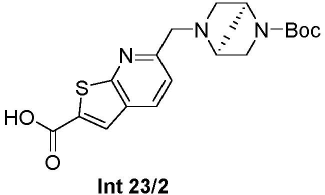

The title compound was prepared similar as described for **Int 23,** using in step 1 (1S,4S)-*tert-*butyl 2,5-diazabicyclo[2.2.1]heptane-2-carboxylate in place of 1*-tert-*butyl piperazine-1-carboxylate.

### Intermediate 23/3: 6-((4-(tert-Butoxycarbonyl)-1,4-diazepan-1-yl)methyl)thieno[2,3-b]pyridine-2-carboxylic acid (Int 23/3)

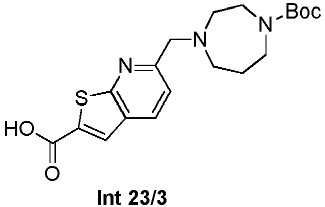

The title compound was prepared similar as described for **Int 23,** using in step 1 *tert*-butyl 1,4-diazepane-1-carboxylate in place of 1-*tert*-butyl piperazine-1-carboxylate.

### Intermediate 23/4: 6-(((3aR,6aS)-5-(tert-Butoxycarbonyl)hexahydropyrrolo[3,4-c]pyrrol-2(1H)-yl)methyl)benzo[b]thiophene-2-carboxylic acid (Int 23/4)

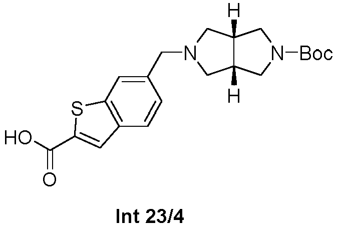

The title compound was prepared similar as described for **Int 23** using in step 1 methyl 6-(bromomethyl)benzo[b]thiophene-2-carboxylate **(Int 11b)** in place of ethyl 6-(bromomethyl)thieno[2,3-b]pyridine-2-carboxylate and *tert-*butyl (3aR,6aS)-hexahydropyrrolo[3,4-c]pyrrole-2(1*H*)-carboxylate in place of *1-tert-*butyl piperazine-1-carboxylate.

### Intermediate 24: 6-(Bromomethyl)benzo[b]thiophene-2-carboxylic acid (Int 24)

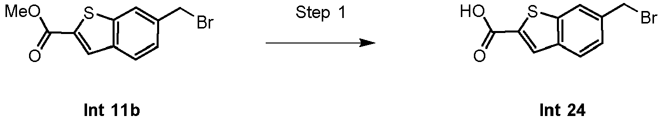

To a mixture of methyl 6-(bromomethyl)benzo[b]thiophene-2-carboxylate **(Int 11b)** (5.13 g, 14 mmol) in a mixture of THF and water (4:1, 100 mL) was added LiOH·H₂O (623 mg, 15 mmol). The mixture was stirred at rt for 4.5 h. EtOAc (100 mL) was added and the organic layer was extracted with 0.1 M NaOH (30 mL). The combined aqueous layers were adjusted to pH = 4 using 1M HCl and extracted with EtOAc (3 x 50 mL). The combined organic layers were dried over anhydrous MgSO₄, filtered and concentrated to dryness. The residue was purified by column chromatography on silica gel to afford the title compound.

### Intermediate 24/1: 6-(Bromomethyl)thieno[2,3-b]pyridine-2-carboxylic acid (Int 24/1)

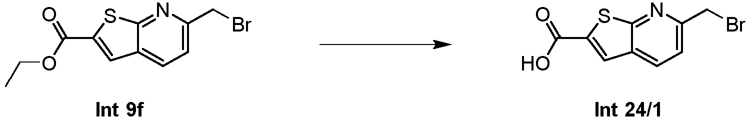

The title compound was prepared similar as described for **Int 24** using ethyl 6-(bromomethyl)thieno[2,3-b]pyridine-2-carboxylate **(Int 9f)** in place of methyl 6-(bromomethyl)benzo[b]thiophene-2-carboxylate.

### Intermediate 25: 7-Fluoro-2,3-dihydrobenzo[b][1,4]dioxine-6-carboxylic acid (Int 25)

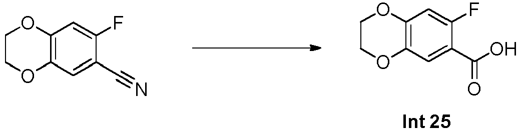

7-Fluoro-2,3-dihydrobenzo[b][1,4]dioxine-6-carbonitrile (30 mg, 0.167 mmol) was dissolved in MeOH (1 mL). To the mixture was added 2M NaOH (1.6 mL, 3.2 mmol). The mixture was stirred at 75 °C overnight. The mixture was concentrated to dryness and the residue was purified by preparative HPLC.

### Intermediate 26: 7-Fluoro-2,3-dihydrobenzo[b][1,4]dioxine-6-carboxylic acid (Int 26)

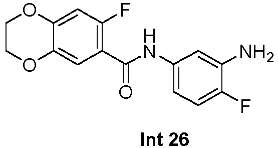

The title compound was prepared similar as described for **Int 2,** using in step 1 7-Fluoro-2,3-dihydrobenzo[b][1,4]dioxine-6-carboxylic acid **(Int 25)** in place of 2,3-dihydrobenzo[b][1,4]dioxine-6-carboxylic acid.

In the following the compounds of the present invention are named with their Example number.

### Example 1: N-(3-(Benzo[b]thiophene-2-carboxamido)-4-methylphenyl)-2,3-dihydrobenzo[b][1,4]dioxine-6-carboxamide (Example 1)

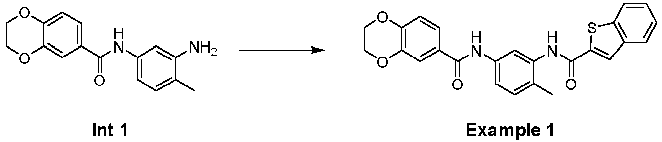

To a solution of *N*-(3-amino-4-methylphenyl)-2,3-dihydro-1,4-benzodioxine-6-carboxamide **(Int 1)** (100 mg, 0.352 mmol), benzo[b]thiophene-2-carboxylic acid (62.7 mg, 0.352 mmol), DIPEA (0.123, 0.703 mmol) and DMAP (6.40 mg, 0.053 mmol) in pyridine (0.980 mL) was added POCI₃ (0.035 mL, 0.387 mmol) at 0 °C. The mixture stirred for 15 min at 0 °C. Then, the mixture was warmed to rt and diluted by EtOAc (15 mL). The organic layer was washed with 0.2 M HCl (3 x 15 mL), then sat. NaHCO₃ (3 x 15 mL). The organic layer was dried over MgSO₄, filtered, and concentrated to dryness. The residue was purified by preparative HPLC to afford the title compound as a white solid.

¹H NMR (300 MHz, DMSO-*d*₆) δ ppm: δ 10.19 (s, 1H), 10.06 (s, 1H), 8.33 (s, 1H), 8.07 (d, *J* = 7.8 Hz, 1H), 8.01 (d, *J* = 7.2 Hz, 1H), 7.87 (s, 1H), 7.61 (d, *J* = 8.2 Hz, 1H), 7.57 - 7.45 (m, 4H), 7.25 (d, *J* = 8.4 Hz, 1H), 6.99 (d, *J* = 8.3 Hz, 1H), 4.41 - 4.22 (m, 4H), 2.24 (s, 3H). MS (ESI): m/z 445.4 [M+H]⁺.

### Examples 1/1 to 1/18

The following Examples were prepared similar as described for **Example 1** using the appropriate building blocks.

| # | Building blocks | Structure | Analytical Data |
|---|---|---|---|
| **1/1** | 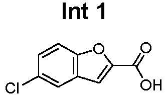 | 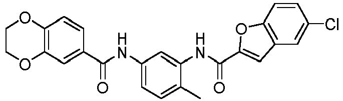 | ¹H NMR (300 MHz, DMSO-*d*₆) δ ppm: δ 10.28 (s, 1H), 10.09 (s, 1H), 7.94 (s, 1H), 7.84 (s, 1H), 7.77 (d, *J* = 9.0 Hz, 1H), 7.74 (s, 1H), 7.60 (d, *J* = 8.5 Hz, 1 H), 7.56 - 7.46 (m, 3H), 7.25 (d, *J* = 8.4 Hz, 1H), 6.99 (d, *J* = 8.4 Hz, 1H), 4.38 - 4.25 (m, 4H), 2.22 (s, 3H). MS (ESI): m/z 463.3 [M+H]⁺. |
| **1/2** | 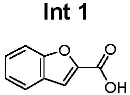 | 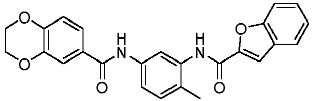 | ¹H NMR (300 MHz, DMSO-*d*₆) δ ppm: δ 10.20 (s, 1H), 10.09 (s, 1H), 7.89 - 7.79 (m, 2H), 7.77 - 7.70 (m, 2H), 7.60 (d, *J* = 8.1 Hz, 1H), 7.56 - 7.46 (m, 3H), 7.38 (t, *J* = 7.5 Hz, 1H), 7.24 (d, *J* = 8.3 Hz, 1H), 6.99 (d, *J* = 8.3 Hz, 1H), 4.38 - 4.24 (m, 4H), 2.23 (s, 3H). MS (ESI): m/z 429.4 [M+H]⁺. |
| **1/3** | 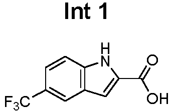 | 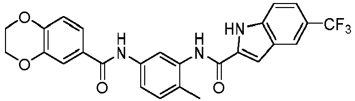 | ¹H NMR (300 MHz, DMSO-*d*₆) δ ppm: 12.20 (s, 1H), 10.14 (s, 1H), 10.09 (s, 1H), 8.14 (s, 1H), 7.85 (s, 1H), 7.63 (t, *J* = 9.9 Hz, 2H), 7.58 - 7.43 (m, 4H), 7.26 (d, *J* = 8.3 Hz, 1H), 6.99 (d, *J* = 8.3 Hz, 1H), 4.41 - 4.21 (m, 4H), 2.23 (s, 3H). MS (ESI): m/z 496.4 [M+H]⁺. |
| **1/4** | 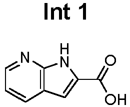 | 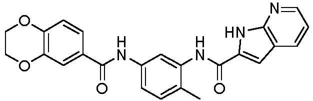 | ¹H NMR (300 MHz, MeOD-*d*₃) δ ppm: δ 8.37 (d, *J* = 4.9 Hz, 1H), 8.16 (d, *J* = 7.9 Hz, |
| | | | 1H), 7.79 (s, 1H), 7.54 (d, *J* = 8.2 Hz, 1H), 7.50 - 7.43 (m, 2H), 7.34 - 7.26 (m, 2H), 7.22 (dd, *J* = 8.0, 4.8 Hz, 1H), 6.96 (d, *J* = 8.2 Hz, 1H), 4.36 - 4.26 (m, 4H), 2.31 (s, 3H). MS (ESI): m/z 429.4 [M+H]⁺. |
| **1/5** | 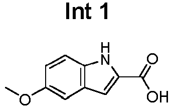 | 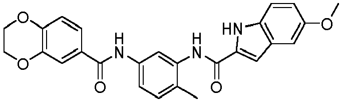 | ¹H NMR (300 MHz, MeOD-*d*₃) δ ppm: δ 7.76 (s, 1H), 7.54 (d, *J* = 8.3 Hz, 1H), 7.50 - 7.42 (m, 2H), 7.36 (d, *J* = 8.9 Hz, 1H), 7.27 (d, *J* = 8.3 Hz, 1H), 7.21 (s, 1H), 7.12 (s, 1H), 6.99 - 6.88 (m, 2H), 4.37 - 4.25 (m, 4H), 3.83 (s, 3H), 2.31 (s, 3H). MS (ESI): m/z 456.2 [M-H]⁻. |
| **1/6** | 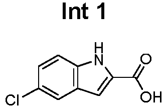 | 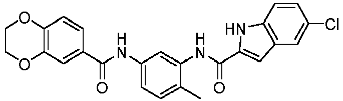 | ¹H NMR (300 MHz, MeOD-*d*₃) δ ppm: δ 7.78 (s, 1H), 7.65 (s, 1H), 7.53 (d, *J* = 8.6 Hz, 1H), 7.49 - 7.41 (m, 3H), 7.31 - 7.18 (m, 3H), 6.93 (d, *J* = 8.2 Hz, 1H), 4.34 - 4.25 (m, 4H), 2.30 (s, 3H). MS (ESI): m/z 460.2 [M-H]⁻. |
| **1/7** | 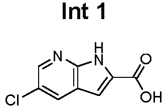 | 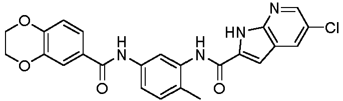 | ¹H NMR (300 MHz, DMSO-*d*₆) δ ppm: δ 12.52 (s, 1H), 10.05 (s, 1H), 10.03 (s, 1H), 8.36 (d, *J* = 2.2 Hz, 1H), 8.29 (d, *J* = 2.5 Hz, 1H), 7.88 (s, 1H), 7.59 (d, 8.1 Hz, 1H), 7.56 - 7.47 (m, 2H), 7.35 (s, 1H), 7.25 (d, *J* = 8.3 Hz, 1H), 6.99 |
| | | | (d, *J* = 8.3 Hz, 1H), 4.37 - 4.26 (m, 4H), 2.24 (s, 3H). MS (ESI): m/z 461.2 [M-H]⁻ |
| **1/8** | 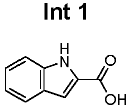 | 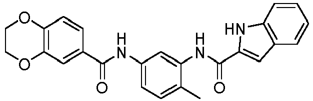 | ¹H NMR (300 MHz, DMSO-*d*₆) δ ppm: δ 11.69 (s, 1H), 10.06 (s, 1H), 9.89 (s, 1H), 7.85 (s, 1H), 7.67 (d, *J* = 8.0 Hz, 1H) , 7.60 (d, *J* = 8.2 Hz, 1H) 7.57 - 7.43 (m, 3H), 7.37 (s, 1H), 7.28 - 7.18 (m, 2H), 7.08 (t, *J* = 7.5 Hz, 1H), 6.99 (d, *J* = 8.2 Hz, 1H), 4.35 - 4.28 (m, 4H), 2.24 (s, 3H). MS (ESI): m/z 426.2 [M-H]⁻. |
| **1/9** | 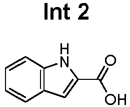 | 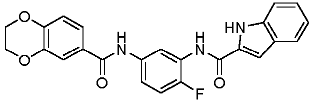 | ¹H NMR (300 MHz, DMSO-d₆) δ ppm: δ 11.74 (s, 1H), 10.16 (s, 1H), 10.11 (s, 1H), 8.11 (d, *J* = 6.7 Hz, 1H), 7.71 - 7.61 (m, 2H), 7.58 - 7.44 (m, 3H), 7.41 (s, 1H), 7.34 - 7.19 (m, 2H), 7.08 (t, *J* = 7.5 Hz, 1H), 7.00 (d, *J* = 8.3 Hz, 1H), 4.38 - 4.26 (m, 4H). MS (ESI): m/z 432.5 [M+H]⁺. |
| **1/10** | 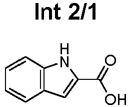 | 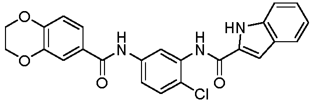 | ¹H NMR (300 MHz, DMSO-*d*₆) δ ppm: δ 11.76 (s, 1H), 10.25 (s, 1H), 10.02 (s, 1H), 8.13 (s, 1H), 7.75 (d, *J* = 8.8 Hz, 1H), 7.69 (d, *J* = 7.9 Hz, 1H), 7.58 - 7.53 (m, 2H), 7.53 - 7.45 (m, 2H), 7.40 (s, 1H) 7.24 (t, *J* = 7.7 Hz, 1H), 7.09 (t, *J* = 7.4 Hz, |
| | | | 1H), 7.00 (d, *J* = 8.3 Hz, 1H), 4.38 - 4.26 (m, 4H). MS (ESI): m/z 448.3 [M+H]⁺. |
| **1/11** | 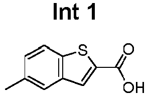 | 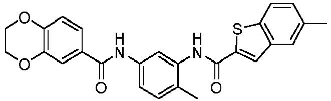 | ¹H NMR (300 MHz, DMSO-*d*₆) δ ppm: δ 10.17 (s, 1H), 10.05 (s, 1H), 8.24 (s, 1H), 7.93 (d, *J* = 8.4 Hz, 1H), 7.85 (s, 1H), 7.79 (s, 1H), 7.60 (d, *J* = 8.4 Hz, 1H), 7.56 - 7.46 (m, 2H), 7.34 (d, *J* = 8.3 Hz, 1H), 7.24 (d, *J* = 8.5 Hz, 1H), 6.98 (d, *J* = 8.3 Hz, 1H), 4.37 - 4.26 (m, 4H), 2.46 (s, 3H), 2.24 (s, 3H). MS (ESI): m/z 459.3 [M+H]⁺. |
| **1/12** | 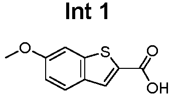 | 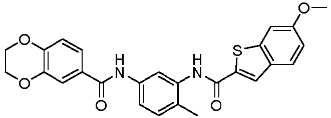 | ¹H NMR (300 MHz, DMSO-*d*₆) δ ppm: δ 10.07 (s, 1H), 10.05 (s, 1H), 8.23 (s, 1H), 7.93 - 7.82 (m, 2H), 7.63 (s, 1H), 7.59 (d, *J* = 8.3 Hz, 1H), 7.56 - 7.48 (m, 2H), 7.24 (d, *J* = 8.3 Hz, 1H), 7.09 (d, *J* = 8.8 Hz, 1H), 6.98 (d, *J* = 8.3 Hz, 1H), 4.38 - 4.26 (m, 4H), 3.87 (s, 3H), 2.23 (s, 3H). MS (ESI): m/z 475.3 [M+H]⁺. ¹H NMR (300 MHz, DMSO- |
| **1/13** | 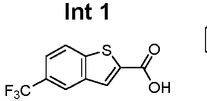 | 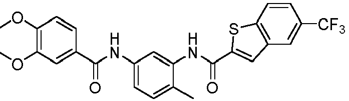 | *d*₆) δ ppm: δ 10.38 (s, 1H), 10.06 (s, 1H), 8.54 - 8.43 (m, 2H), 8.34 (d, *J* = 8.6 Hz, 1H), 7.87 (s, 1H), 7.80 (d, *J* = 8.7 Hz, 1H), 7.61 (d, *J* = 8.1 Hz, 1H) 7.57 - 7.47 (m, 2H), 7.26 (d, *J* = 8.4 Hz, |
| | | | 1H), 6.99 (d, J = 8.2 Hz, 1H), 4.36 - 4.26 (m, 4H), 2.24 (s, 3H). MS (ESI): m/z 511.1 [M-H]-. |
| **1/14** | 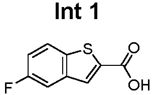 | 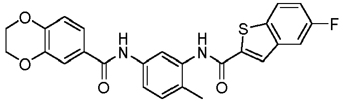 | ¹H NMR (300 MHz, MeOD-*d*₃) δ ppm: δ 8.13 (s, 1H), 7.98 (dd, *J* = 8.9, 4.8 Hz, 1H), 7.79 (s, 1H), 7.68 (d, *J* = 8.9 Hz, 1H), 7.56 (d, *J* = 8.3, 1H), 7.52 - 7.43 (m, 2H), 7.36 - 7.26 (m, 2H), 6.95 (d, *J* = 8.2 Hz, 1H), 4.37 - 4.28 (m, 4H), 2.33 (s, 3H). MS (ESI): m/z 463.4 [M+H]⁺. |
| **1/15** | 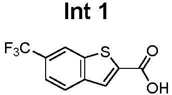 | 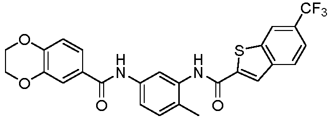 | ¹H NMR (300 MHz, DMSO-*d*₆) δ ppm: δ 10.35 (s, 1H), 10.07 (s, 1H), 8.61 (s, 1H), 8.43 (s, 1H), 8.27 - 8.17 (m, 1H), 7.88 (s, 1H), 7.77 (d, *J* = 8.4 Hz, 1H), 7.61 (d, *J* = 8.3 Hz, 1H), 7.57 - 7.47 (m, 2H), 7.26 (d, *J* = 8.2 Hz, 1H), 6.99 (d, *J* = 8.2 Hz, 1H), 4.26 - 4.39 (m, 4H), 2.25 (s, 3H). MS (ESI): m/z 511.2 [M-H]⁻. |
| **1/16** | 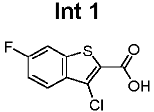 | 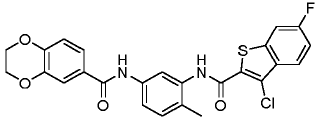 | ¹H NMR (300 MHz, DMSO-*d*₆) δ ppm: δ 10.08 (s, 1H), 9.94 (s, 1H), 8.13 (d, *J* = 9.2 Hz, 1H), 8.05 (s, 1H), 8.00 (dd, *J* = 9.1, 4.6 Hz, 1H), 7.63 (d, *J* = 8.3 Hz, 1H), 7.58 - 7.46 (m, 3H), 7.26 (d, *J* = 8.3 Hz, 1H), 6.99 (d, *J* = 8.3 Hz, 1H), 4.37 - 4.27 (m, 4H), 2.30 (s, 3H). |
| | | | MS (ESI): m/z 497.3 [M+H]⁺. |
| **1/17** | 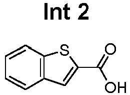 | 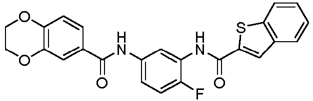 | ¹H NMR (300 MHz, DMSO-*d*₆) δ ppm: δ 10.45 (s, 1H), 10.16 (s, 1H), 8.37 (s, 1H), 8.16 - 7.98 (m, 3H), 7.71 - 7.63 (m, 1H), 7.57 - 7.45 (m, 4H), 7.30 (t, *J* = 9.2 Hz, 1H), 7.00 (d, *J* = 8.3 Hz, 1H), 4.37 - 4.27 (m, 4H). MS (ESI): m/z 449.4 [M+H]⁺. |
| **1/18** | 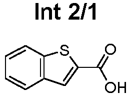 | 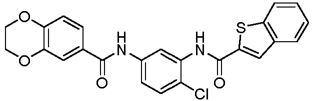 | ¹H NMR (300 MHz, DMSO-*d*₆) δ ppm: δ 10.37 (s, 1H), 10.24 (s, 1H), 8.37 (s, 1H), 8.12 (s, 1H), 8.08 (d, *J* = 7.9 Hz, 1H), 8.03 (d, *J* = 7.5 Hz, 1H), 7.76 (d, *J* = 8.8 Hz, 1H), 7.58 - 7.43 (m, 5H), 7.00 (d, *J =* 8.3 Hz, 1H), 4.36 - 4.28 (m, 4H). MS (ESI): m/z 465.3 [M+H]⁺. |
| **1/19** | 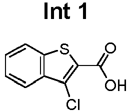 | 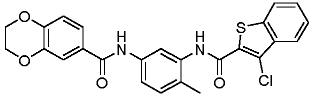 | ¹H NMR (300 MHz, DMSO-*d*₆) δ ppm : δ 10.08 (s, 1H), 9.96 (s, 1H), 8.21 - 8.12 (m, 1H), 8.04 (s, 1H), 8.00 - 7.89 (m, 1H), 7.68 - 7.59 (m, 3H), 7.59 - 7.50 (m, 2H), 7.26 (d, *J* = 8.4 Hz, 1H), 6.99 (d, *J* = 8.1 Hz, 1H), 4.38 - 3.25 (m, 4H), 2.31 (s, 3H). MS (ESI) : m/z 479.4 [M+H]⁺. |

### Example 2: N-(5-(2,3-Dihydrobenzo[b][1,4]dioxine-6-carboxamido)-2-methylphenyl)-6-hydroxy-1H-indole-2-carboxamide (Example 2)

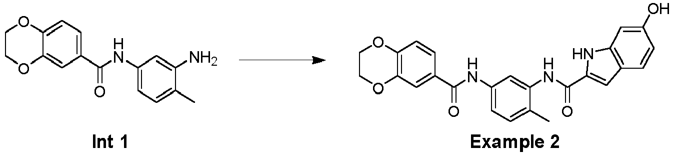

To a solution of *N*-(3-amino-4-methylphenyl)-2,3-dihydrobenzo[*b*][1,4]dioxine-6-carboxamide **(Int 1)** (30.0 mg, 0.106 mmol) and 6-hydroxyindole-2-carboxylic acid (18.7 mg, 0.106 mmol) in DMF (0.500 mL) was added NMI (0.025 mL, 0.317 mmol). The mixture was stirred at rt for 10 min. PyBroP (54.1 mg, 0.116 mmol) was added, and the mixture was heated at 75 °C for 18 h. The mixture was concentrated to dryness and the residue was dissolved in EtOAc (30 mL). The organic layer was washed with 0.1 M HCl (3 x 15 mL) and sat. aq. NaHCO₃ (3 x 15 mL). The organic layer was dried over MgSO₄, filtered, and concentrated to dryness. The residue was purified by preparative HPLC to afford the title compound as a white solid.

¹H NMR (300 MHz, MeOD-*d*₃) δ ppm: δ 7.77 (s, 1H), 7.53 (d, *J* = 8.1 Hz, 1H), 7.50 - 7.43 (m, 3H), 7.27 (d, *J* = 8.3 Hz, 1H), 7.21 (s, 1H), 6.94 (d, *J* = 8.3 Hz, 1H), 6.86 (s, 1H), 6.70 (d, *J* = 8.7 Hz, 1H), 4.36 - 4.25 (m, 4H), 2.31 (s, 3H). MS (ESI): m/z 442.2 [M-H]⁻.

### Example 2/1

The following Example was prepared similar as described for **Example 2** using the appropriate building blocks.

| **#** | Building blocks | Structure | Analytical Data |
|---|---|---|---|
| **2/1** | 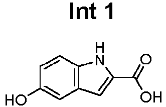 | 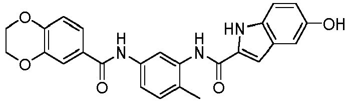 | ¹H NMR (300 MHz, MeOD-*d*₃) δ ppm: δ 7.78 (d, *J* = 2.2 Hz, 1H), 7.54 (d, *J* = 8.3 Hz, 1H), 7.51 - 7.44 (m, 2H), 7.32 (d, *J* = 8.9 Hz, 1H), 7.28 (d, *J* = 8.3 Hz, 1H), 7.13 (s, 1H), 7.01 (s, 1H), 6.94 (d, *J* = 8.2 Hz, 1H), 6.86 (d, *J* = 8.8 Hz, 1H), 4.37 - 4.24 (m, 4H), 2.32 (s, 3H). MS (ESI): m/z 444.4 [M+H]⁺. |

### Example 3: N-(4-Methyl-3-(6-(pyridin-2-ylmethoxy)benzo[b]thiophene-2-carboxamido)phenyl)-2,3-dihydrobenzo[b][1,4]dioxine-6-carboxamide (Example 3)

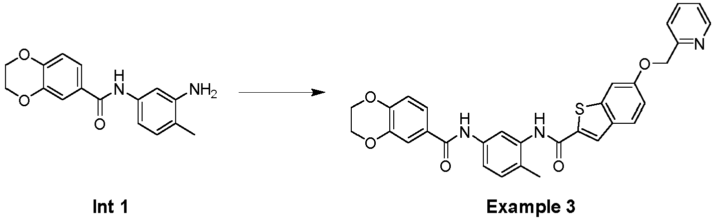

To a mixture of *N*-(3-amino-4-methylphenyl)-2,3-dihydrobenzo[*b*][1,4]dioxine-6-carboxamide **(Int 1)** (15.0 mg, 0.053 mmol), 6-(pyridin-2-ylmethoxy)benzo[*b*]thiophene-2-carboxylic acid **(Int 4)** (15.1 mg, 0.053 mmol) in DMF (530 µL) was added NMI (13.0 µL, 0.158 mmol). After 5 min, TCFH (16.3 mg, 0.058 mmol) was added. The mixture was stirred for 1 h at rt. Then, the residue was concentrated to dryness and purified by preparative HPLC to afford the title compound as a white solid.

¹H NMR (300 MHz, MeOD-*d*₃) δ ppm: δ 8.58 (s, 1H), 8.56 (s, 1H), 8.06 (s, 1H), 7.95 - 7.80 (m, 2H), 7.74 (s, 1H), 7.65 (d, *J* = 7.9 Hz, 1H), 7.57 (s, 1H), 7.53 (d, *J* = 8.4 Hz, 1 H), 7.57 (s, 1H), 7.49 - 7.43 (m, 2H), 7.39 (t, *J* = 6.2 Hz, 1H), 7.27 (d, J = 8.3 Hz, 1 H), 7.19 (d, *J* = 8.8, 1H), 6.93 (d, *J* = 8.0 Hz, 1H), 5.28 (s, 2H), 4.36 - 4.24 (m, 4H), 2.30 (s, 3H). MS (ESI): m/z 552.4 [M+H]⁺.

### Examples 3/1 to 3/16

The following Examples were prepared similar as described for **Example 3** using the appropriate building blocks.

| **#** | Building blocks | Structure | Analytical Data |
|---|---|---|---|
| **3/1** | **Int 2, Int 4** | 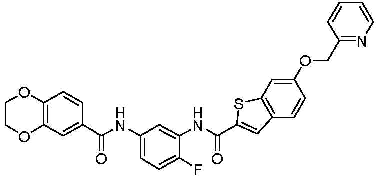 | ¹H NMR (300 MHz, MeOD-*d*₃) δ ppm: δ 8.11 (s, 1H), 7.97 - 7.83 (m, 2H), 7.67 (d, *J* = 7.6 Hz, 1H) 7.63 - 7.54 (m, 2H), 7.53 - 7.45 (m, 2H), 7.41 (t, *J* = 6.0 Hz, 1H), 7.26 - 7.17 (m, 2H), 6.96 (d, *J* = 8.3 |
| | | | Hz, 1H), 5.31 (s, 2H), 4.36 - 4.28 (m, 4H), 3.33 (s, 2H). MS (ESI): m/z 556.4 [M+H]⁺. |
| **3/2** | **Int 2/1, Int 4** | 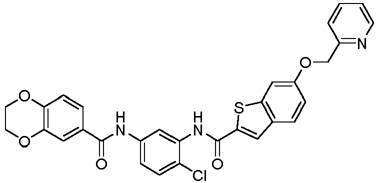 | ¹H NMR (300 MHz, DMSO-*d*₆) δ ppm: δ 10.24 (s, 1H), 10.22 (s, 1H), 8.60 (d, *J* = 4.7 Hz, 1H), 8.27 (s, 1H), 8.10 (s, 1H), 7.92 (d, *J* = 8.7, 1H), 7.86 (t, *J* = 7.7 Hz, 1H), 7.78 - 7.70 (m, 2H), 7.58 (d, *J* = 7.8 Hz, 1H), 7.55 - 7.48 (m, 2H), 7.37 (d, *J* = 12.6 Hz, 1H), 7.20 (d, *J* = 8.8, 1H), 6.99 (d, *J* = 8.4 Hz, 1H), 5.29 (s, 2H), 4.36 - 4.28 (m, 4H). MS (ESI): m/z 572.4 [M+H]⁺. |
| **3/3** | **Int 1**, **Int 7** | 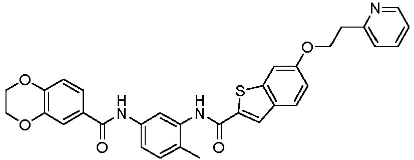 | ¹H NMR (300 MHz, MeOD-*d*₃) δ ppm: δ 8.51 (d, *J* = 5.1 Hz, 1H), 8.04 (s, 1H), 7.89 - 7.70 (m, 3H), 7.55 - 7.41 (m, 5H), 7.33 (t, *J* = 6.2 Hz, 1H), 7.27 (d, *J* = 8.3 Hz, 1H), 7.03 (d, *J* = 8.8 Hz, 1H), 6.93 (d, *J* = 8.3 Hz, 1H), 4.46 (t, *J* = 6.5 Hz, 2H), 4.36 - 4.26 (m, 4H), |
| | | | 3.37 - 3.32 (m, 2H) 2.30 (s, 3H). MS (ESI): m/z 566.5 [M+H]⁺. |
| **3/4** | **Int 2, Int 7** | 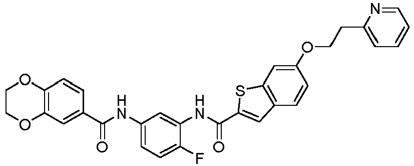 | ¹H NMR (300 MHz, DMSO-*d*₆) δ ppm: δ 10.31 (s, 1H), 10.15 (s, 1H), 8.53 (s, 1H), 8.26 (s, 1H), 8.10 (d, *J* = 6.6 Hz, 1H), 7.87 (d, *J* = 8.8 Hz, 1H), 7.75 (t, *J* = 7.8 Hz, 1H), 7.69 - 7.59 (m, 2H), 7.57 - 7.48 (m, 2H), 7.40 (d, *J* = 7.9 Hz, 1H), 7.34 - 7.21 (m, 2H), 7.07 - 6.98 (m, 2H), 4.48 (t, *J* = 6.7 Hz, 2H), 4.39 - 4.23 (m, 4H), 3.26 - 3.18 (m, 2H). MS (ESI): 570.4 m/z [M+H]⁺. |
| **3/5** | **Int 1, Int 3** | 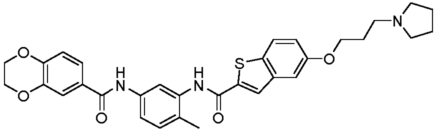 | ¹H NMR (300 MHz, MeOD-*d*₃) δ ppm: δ 8.03 (s, 1H), 7.87 - 7.74 (m, 2H), 7.60 - 7.35 (m, 4H), 7.29 (d, *J* = 8.3 Hz, 1H), 7.16 (d, *J* = 8.9 Hz, 1H), 6.94 (d, *J* = 8.2 Hz, 1H), 4.36 - 4.25 (m, 4H), 4.20 (t, *J* = 5.7 Hz, 2H), 3.51 - 3.39 (m, 6H), 2.31 (s, 3H), 2.30 - 2.19 (m, 2H), 2.17 - 2.07 |
| | | | (m, 4H). MS (ESI): 572.5 m/z [M+H]⁺. |
| **3/6** | **Int 2, Int 3** | 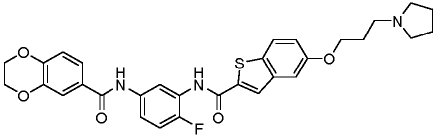 | ¹H NMR (300 MHz, MeOD-*d*₃) δ ppm: δ 8.16 (dd, *J* = 7.0, 2.6 Hz, 1H), 8.08 (s, 1H), 7.85 (d, *J* = 8.9 Hz, 1H), 7.62 - 7.52 (m, 1H), 7.57 - 7.43 (m, 3H), 7.29 - 7.13 (m, 2H), 6.96 (d, *J =* 8.2 Hz, 1H), 4.38 - 4.27 (m, 4H), 4.22 (t, *J =* 5.7 Hz, 2H), 3.56 - 3.38 (m, 6H), 2.37 - 2.22 (m, 2H), 2.19 - 2.06 (m, 4H). MS (ESI): 576.5 m/z [M+H]⁺. |
| **3/7** | **Int 1, Int 5** | 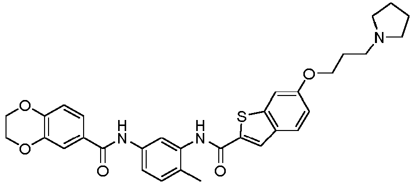 | ¹H NMR (300 MHz, DMSO-*d*₆) δ ppm: δ 10.06 (s, 1H), 10.05 (s, 1H), 8.22 (s, 1H), 7.90 - 7.82 (m, 2H), 7.64 - 7.56 (m, 2H), 7.55 - 7.47 (m, 2H), 7.23 (d, *J* = 8.3 Hz, 1H), 7.07 (d, *J = 9.0* Hz, 1H), 6.98 (d, *J =* 8.4 Hz, 1H), 4.36 - 4.27 (m, 4H), 4.11 (t, *J* = 7.3 Hz, 2H), 2.60 - 2.55 (m, 2H), 2.48 - 2.40 (m, 4H), 2.23 (s, 3H), 1.99 - 1.86 (m, 2H), 1.77 - 1.65 (m, 4H). MS |
| | | | (ESI): 572.5 m/z [M+H]⁺. |
| **3/8** | **Int 2, Int 5** | 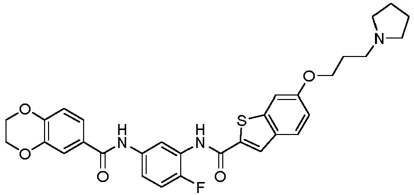 | ¹H NMR (300 MHz, DMSO-*d*₆) δ ppm: δ 10.31 (s, 1H), 10.15 (s, 1H), 8.27 (s, 1H), 8.10 (d, *J* = Hz, 1H), 7.88 (d, *J =* 8.9 Hz, 1H), 7.70 - 7.59 (m, 2H), 7.57 - 7.49 (m, 2H), 7.28 (t, *J* = 9.6 Hz, 1H), 7.08 (d, *J =* 9.0 Hz, 1H), 7.00 (d, *J =* 8.3 Hz, 1H), 4.37 - 4.27 (m, 4H), 4.13 (t, *J =* 6.4 Hz, 2H), 2.60 - 2.55 (m, 2H), 2.48 - 2.42 (m, 4H), 2.06 - 1.88 (m, 2H), 1.74 - 1.65 (m, 4H). MS (ESI): 576.5 m/z [M+H]⁺. |
| **3/9** | **Int 2/1, Int 5** | 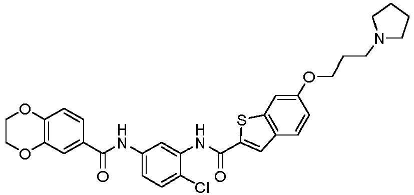 | ¹H NMR (300 MHz, MeOD-*d*₃) δ ppm: δ 8.20 (s, 1H), 8.10 (s, 1H), 7.86 (d, *J* = 8.8 Hz, 1H), 7.62 (d, *J =* 8.8 Hz, 1H), 7.55 - 7.42 (m, 4H), 7.11 (d, *J* = 8.9 Hz, 1H), 6.95 (d, *J* = 8.3 Hz, 1H), 4.36 - 4.26 (m, 4H), 4.22 (t, *J = 5.6* Hz, 2H) 3.48 - 3.36 (m, 6H), 2.35 - 2.21 (m, 2H), 2.18 - 2.05 |
| | | | (m, 4H). MS (ESI): m/z 592.5 [M+H]⁺. |
| **3/10** | **Int 2, Int 6** | 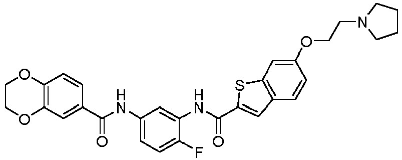 | ¹H NMR (300 MHz, DMSO-*d*₆) δ ppm: δ 10.31 (s, 1H), 10.15 (s, 1H), 8.27 (s, 1H), 8.10 (d, *J* = 7.0 Hz, 1H), 7.88 (d, *J* = 8.7 Hz, 1H), 7.67 - 7.61 (m, 2H), 7.56 - 7.48 (m, 2H), 7.28 (t, *J =* 9.6 Hz, 1H), 7.09 (d, *J =* 9.0 Hz, 1H), 6.99 (d, *J =* 8.3 Hz, 1H), 4.37 - 4.27 (m, 4H), 4.18 (t, *J* = 5.9 Hz, 2H), 2.85 (t, *J =* 5.8 Hz, 2H), 2.54 - 2.64 (m, 4H), 1.71 (d, *J =* 5.8 Hz, 4H). MS (ESI): m/z 562.5 [M+H]⁺. |
| **3/11** | 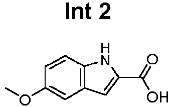 | 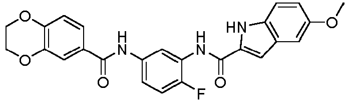 | ¹H NMR (300 MHz, MeOD-*d*₃) δ ppm: δ 8.20 - 8.14 (m, 1H), 7.60 - 7.52 (m, 1H), 7.51 - 7.44 (m, 2H) 7.37 (d, *J* = 9.1 Hz, 1H), 7.25 - 7.15 (m, 2H), 7.13 (s, 1H) 6.98 - 6.89 (m, 2H), 4.36 - 4.25 (m, 4H), 3.84 (s, 3H). MS (ESI): m/z 462.4 [M+H]⁺. |
| **3/12** | 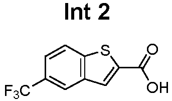 | 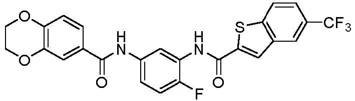 | ¹H NMR (300 MHz, MeOD-*d*₃) δ ppm: δ 8.30 (d, *J* = 5.1 Hz, 2H), 8.21 - 8.09 (m, 2H), 7.72 (d, *J* = 8.7 Hz, 1H), 7.63 - 7.54 (m, 1H), 7.51 - 7.43 (m, 2H), 7.23 (t, *J =* 9.5 Hz, 1H), 6.94 (d, *J* = 8.1 Hz, 1H), 4.36 - 4.25 (m, 4H). MS (ESI): m/z 515.2 [M-H]⁻. |
| **3/13** | 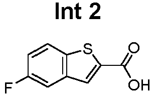 | 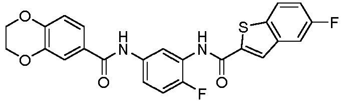 | ¹H NMR (300 MHz, MeOD-*d*₃) δ ppm: δ 8.17 - 8.07 (m, 2H), 7.96 (dd, *J* = 8.6, 3.9 Hz, 1H), 7.67 (d, *J =* 7.6 Hz, 1H), 7.63 - 7.53 (m, 1H), 7.52 - 7.42 (m, 2H), 7.29 (t, *J = 8.5,* 1H), 7.21 (t, *J* = 9.5 Hz, 1H), 6.94 (d, *J* = 8.2 Hz, 1H), 4.30 (m, 4H). MS (ESI): m/z 467.3 [M+H]⁺. |
| **3/14** | 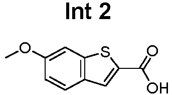 | 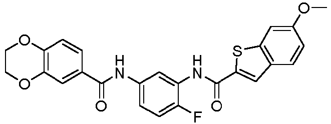 | ¹H NMR (300 MHz, DMSO-*d*₆) δ ppm: δ 10.32 (s, 1H), 10.15 (s, 1H), 8.28 (s, 1H), 8.11 (d, *J =* 7.1 Hz, 1H), 7.89 (d, *J = 8.8* Hz, 1H), 7.70 - 7.60 (m, 2H), 7.58 - 7.47 (m, 2H), 7.29 (t, *J =* 9.6 Hz, 1H), 7.09 |
| | | | (d, *J =* 8.7, 1H), 7.00 (d, *J =* 8.2 Hz, 1H), 4.37 - 4.26 (m, 4H), 3.87 (s, 3H). MS (ESI): m/z 479.4 [M+H]⁺. |
| **3/15** | 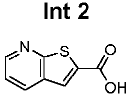 | 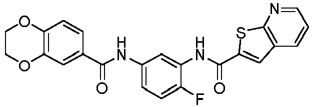 | ¹H NMR (300 MHz, DMSO-*d*₆) δ ppm: δ 10.56 (s, 1H), 10.17 (s, 1H), 8.71 (s, 1H), 8.46 (d, *J* = 8.0 Hz, 1H), 8.36 (s, 1H), 8.12 (d, *J* = 7.0 Hz, 1H), 7.76 - 7.61 (m, 1H), 7.60 - 7.43 (m, 3H), 7.31 (t, *J =* 9.7 Hz, 1H), 7.00 (d, *J =* 8.3 Hz, 1H), 4.42 - 4.22 (m, 4H). MS (ESI): m/z 450.4 [M+H]⁺. |
| **3/16** | 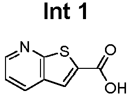 | 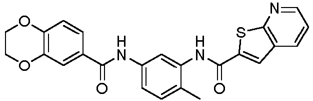 | ¹H NMR (300 MHz, MeOD-*d*₃) δ ppm: δ 8.65 (d, *J* = 4.7 Hz, 1H), 8.37 (d, *J =* 7.9 Hz, 1H), 8.14 (s, 1H), 7.78 (s, 1H), 7.58 - 7.42 (m, 4H), 7.30 (d, *J* = 8.5 Hz, 1H), 6.94 (d, *J* = 8.2 Hz, 1H), 4.32 - 4.26 (m, 4H), 2.32 (s, 3H). MS (ESI): m/z 446.4 [M+H]⁺. |
| **3/17** | **Int 1, Int 8** | 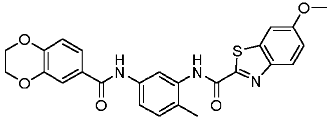 | ¹H NMR (300 MHz, DMSO-*d*₆) δ ppm : δ 10.43 (s, 1H), 10.07 |
| | | | (s, 1H), 8.09 (d, *J =* 9.1 Hz, 1H), 7.98 (s, 1H), 7.83 (s, 1H), 7.61 (d, *J* = 8.3 Hz, 1H), 7.55 - 7.47 (m, 2H), 7.29 - 7.20 (m, 2H), 6.99 (d, *J* = 8.2 Hz, 1H), 4.35 - 4.26 (m, 4H), 3.90 (s, 3H), 2.26 (s, 3H). MS (ESI): m/z 476.4 [M+H]⁺. |
| **3/18** | **Int 2, Int 11** | 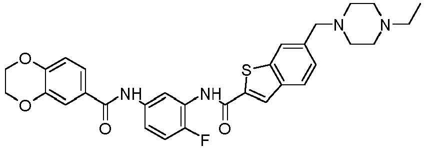 | ¹H NMR (300 MHz, CDCl₃): δ ppm 8.39 (d, *J* = 6 Hz, 1H), 8.05 (d, *J* = 2.4 Hz, 1H), 7.91 - 7.78 (m, 5H), 7.46 - 7.32 (m, 3H), 7.16 (t, *J =* 9.8 Hz, 1H), 6.95 (d, *J* = 8.4 Hz, 1H), 4.36 - 4.28 (m, 4H), 3.68 (s, 2H), 2.66-2.54 (m, 10H), 1.17 (t, *J =* 7.2 Hz, 3H). MS (ESI): m/z 575.6 [M+H]⁺. |
| **3/19** | **Int 2, Int 9** | 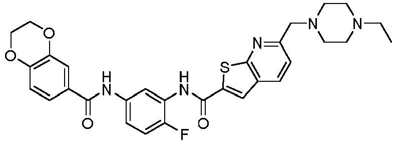 | ¹H NMR (300 MHz, DMSO-d₆): δ ppm 10.52 (s, 1H), 10.16 (s, 1H), 8.39 (d, *J =* 8.3 Hz, 1H), 8.32 (s, 1H), 8.10 (d *J* = 7,2 Hz, 1H), 7.70 - 7.62 (m, 1H), 7.59 (d, *J* = 8,4Hz, 1H) 7.56 - 7.48 (m, 2H), 7.30 |
| | | | (t, *J* = 9.6 Hz, 1H), 7.00 (d, *J* = 8.3 Hz, 1H), 4.36-4.28 (m, 4H), 3.73 (s, 2H), 2.48 - 2.25 (m, 10H), 0.99 (t, *J =* 7.1 Hz, 3H). MS (ESI): m/z 576.5 [M+H]⁺. |
| **3/20** | **Int 2, Int 11/3** | 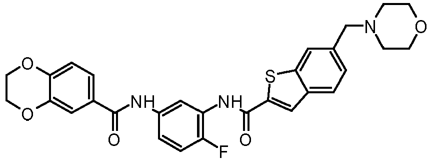 | ¹H NMR (300 MHz, CD₃OD): δ ppm 8.22 (s, 1H) 8.16 (d, *J* = 6,3 Hz, 1H), 8.10 - 7.99 (m, 2H), 7.62 - 7.45 (m, 4H), 7.24 (t, *J* = 9.6 Hz, 1H), 6.96 (d, *J* = 8.4 Hz, 1H), 4.36 - 4.28 (m, 4H), 4.14 (s, 2H), 3.89 - 3.77 (m, 4H), 3.05 - 2.84 (m, 4H). MS (ESI): m/z 548.5 [M+H]⁺. |
| **3/21** | **Int 1, Int 13** | 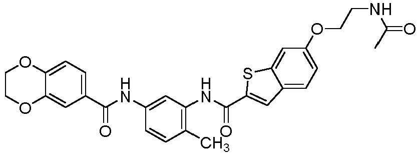 | ¹H NMR (300 MHz, DMSO-d₆): δ ppm 10.06 - 10.05 (m, 2H), 8.22 (s, 1H), 8.12 - 8.08 (m, 1H), 7.89 - 7.87 (m, 1H), 7.85 (s, 1H), 7.63 (s, 1H), 7.58 (d, *J* = 8.0 Hz, 1H), 7.52 - 7.49 (m, 2H), 7.23 (d, *J* = 8.4 Hz, 1H), 7.08 (d, *J* = 8.6 Hz, 1H), 6.97 (d, *J* = 8.2 Hz, 1H), 4.36 - |
| | | | 4.28 (m, 4H), 4.10 - 4.07 (m, 2H), 3.47 - 3.42 (m, 2H), 2.22 (s, 3H), 1.83 (s, 3H). MS (ESI): m/z 546.5 [M+H]⁺. |
| **3/22** | **Int 2, Int 17** | 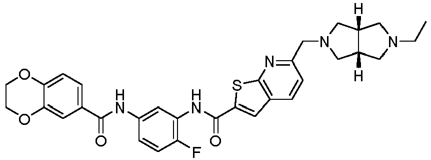 | ¹H NMR (300 MHz, DMSO-d₆): δ ppm 10.52 (s, 1H), 10.17 (s, 1H), 8.41 (d, *J =* 8.1 Hz, 1H), 8.34 (s, 1H), 8.16 - 8.10 (m, 1H), 7.68 - 7.61 (m, 2H), 7.54 (s, 1H), 7.50 (s, 1H), 7.31 (t, *J* = 9.6 Hz, 1H), 6.99 (d, *J* = 7.9 Hz, 1H), 4.36-4.38 (m, 4H), 3.87 (s, 2H), 3.12 - 2.54 (m, 12H), 1.12 (t, *J =* 6.8 Hz, 3H). MS (ESI): m/z 602.6 [M+H]⁺. |
| **3/23** | **Int 1, Int 12** | 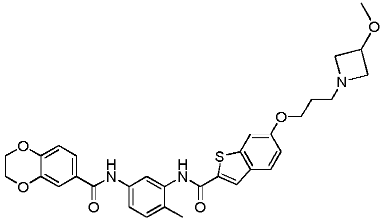 | ¹H NMR (400 MHz, DMSO-*d*₆) *δ* 10.08 (s, 1H), 10.06 (s, 1H), 8.22 (s, 1H), 7.87 - 7.85 (m, 2H), 7.60 - 7.58 (m, 2H), 7.54 - 7.50 (m, 2H), 7.24 (d, *J* = 8.4 Hz, 1H), 7.06 (dd, J *=* 8.8, 2.4 Hz, 1H), 6.97 (d, *J =* 8.4 Hz, 1H), 4.32 - 4.29 (m, 4H), 4.07 (t, *J =* 6.4 |
| | | | Hz, 2H), 3.95 - 3.93 (m, 1H), 3.55 - 3.52 (m, 2H), 3.15 (s, 3H), 2.84 - 2.80 (m, 2H), 2.59 - 2.56 (m, 2H ), 2.22 (s, 3H), 1.78 - 1.75 (m, 2H). MS (ESI): m/z 588.3 [M+H]⁺. |
| **3/24** | 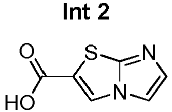 | 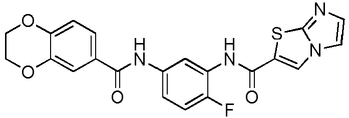 | ¹H NMR (300 MHz, DMSO-d₆): δ ppm 10.45 (s, 1H), 10.14 (s, 1H), 8.82 (s, 1H), 8.10 (d, *J* = 6.7 Hz, 1H), 7.94 (s, 1H), 7.67 - 7.60 (m, 1H), 7.53 (s, 1H), 7.49 (s, 1H), 7.36 (s, 1H), 7.28 (t, *J* = 9.7 Hz, 1H), 6.99 (d, *J =* 8.5 Hz, 1H), 4.35-4.37 (m, 4H). MS (ESI): m/z 439.4 [M+H]⁺. |
| **3/25** | 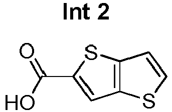 | 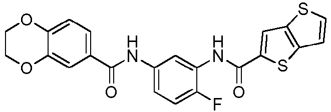 | ¹H NMR (300 MHz, DMSO-d₆): δ ppm 10.32 (s, 1H), 10.13 (s, 1H), 8.33 (s, 1H), 8.07 (d, *J* = 5.8 Hz, 1H), 7.88 (s, 1H), 7.65 - 7.48 (m, 4H), 7.24 (t, *J* = 9.4 Hz, 1H), 6.98 (d, *J =* 7.8 Hz, 1H), 4.37-4.24 (s, 4H). MS (ESI): m/z 455.4 [M+H]⁺. |
| **3/26** | 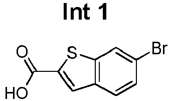 | 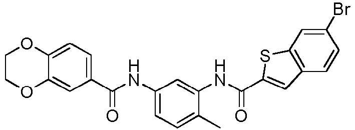 | ¹H NMR (300 MHz, DMSO-d₆): δ ppm 10.24 (s, 1H), 10.06 (s, 1H), 8.39 (s, 1H), 8.31 (s, 1H), 7.96 (d, *J* = 8.5 Hz, 1H), 7.86 (s, 1H), 7.62 (t, *J* = 8.8 Hz, 2H), 7.54 (s, 1H), 7.50 (s, 1H), 7.25 (d, *J =* 8.3 Hz, 1H), 6.98 (d, *J* = 8.2 Hz, 1H), 4.35 - 4.27 (m, 4H), 2.23 (s, 3H). MS (ESI): m/z 523.1 [M+H]⁺. |
| **3/27** | **Int 2, Int 11/2** | 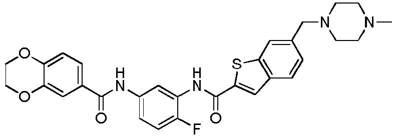 | ¹H NMR (300 MHz, DMSO-d₆): δ ppm 10.42 (s, 1H), 10.16 (s, 1H), 8.34 (s, 1H), 8.13 (m, 1H), 7.97 (m, 2H), 7.67 - 7.61 (m, 1H), 7.54 (s, 1H), 7.50 (s, 1H), 7.43 (d, *J* = 8.7 Hz, 1H), 7.29 (t, *J =* 9.7 Hz, 1H), 6.99 (d, *J* = 8.1 Hz, 1H), 4.35 - 4.27 (m, 4H), 3.64 (s, 2H), 2.75 - 2.54 (m, 5H), 2.45 - 2.22 (m, 6H). MS (ESI): m/z 561.6 [M+H]⁺. |
| **3/28** | **Int 2, Int 11/1** | 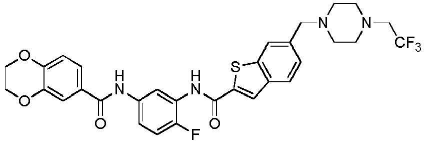 | ¹H NMR (300 MHz, DMSO-d₆): δ ppm 10.41 (s, 1H), 10.16 (s, 1H), 8.34 (s, 1H), 8.09 (d, *J* = 6.7 Hz, 1H), 7.94 (s, 2H), 7.68-7.61 (m, 1H), 7.52 (s, 1H), 7.49 (s, 1H), 7.41 (d, *J =* 8.5 Hz, 1H), 7.28 (t, *J* = 9.5 Hz, 1H), 6.98 (d, *J* = 8.2 Hz, 1H), 4.35 - 4.27 (m, 4H), 3.61 (s, 2H), 3.19 - 3.09 (m, 2H), 2.68 - 2.58 (m, 4H), 2.46 - 2.36 (m, 4H). MS (ESI): m/z 629.7 [M+H]⁺ |
| **3/29** | **Int 1, Int 9** | 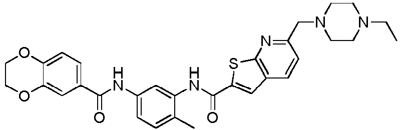 | ¹H NMR (300 MHz, DMSO-d₆): δ ppm 10.28 (s, 1H), 10.06 (s, 1H), 8.38 (d, *J =* 8.3 Hz, 1H), 8.29 (s, 1H), 7.86 (s, 1H), 7.62 - 7.57 (m, 2H), 7.54 (s, 1H), 7.46 (s, 1H), 7.25 (d, *J =* 8.3 Hz, 1H), 6.98 (d, *J* = 8.4 Hz, 1H), 4.35-4.27 (m, 4H), 3.73 (s, 2H), 2.48-2.29 (m, 10H), 2.23 (s, 3H), 0.99 (t, *J =* 7.1 Hz, 3H). MS (ESI): m/z 572.5 [M+H]⁺ |
| **3/30** | **Int 2/1, Int 9** | 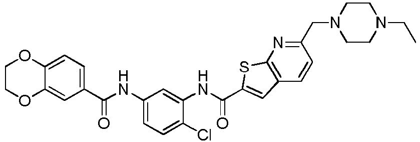 | ¹H NMR (300 MHz, DMSO-d₆): δ ppm 10.48 (s, 1H), 10.25 (s, 1H), 8.44 (d, *J =* 8.4 Hz, 1H), 8.34 (s, 1H), 8.13 (s, 1H), 7.73 (d, *J* = 8.8 Hz, 1H), 7.60 (d, *J =* 8.3 Hz, 1H), 7.56-7.53 (m, 2H), 7.50 (s, 1H), 7.00 (d, *J =* 8.4 Hz, 1H), 4.36-4.28 (m, 4H), 3.84 (s, 2H), 3.14 - 2.56 (m, 10H), 1.14 (t, *J* = 7.1 Hz, 3H). MS (ESI): m/z 592.5 [M+H]⁺ |
| **3/31** | **Int 2, Int 10/2** | 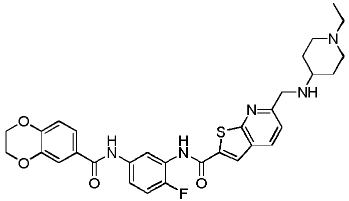 | ¹H NMR (300 MHz, DMSO-d₆): δ ppm 10.61 (s, 1H), 10.19 (s, 1H), 8.49 (d, *J =* 7.8 Hz, 1H), 8.39 (s, 1H), 8.14 (s, 1H), 7.67-7.62 (m, 2H), 7.54 (s, 1H), 7.51 (s, 1H), 7.31 (t, *J =* 9.5 Hz, 1H), 6.99 (d, *J* = 8.2 Hz, 1H), ), 4.36 - 4.27 (m, 6H), 3.14 - 2.60 (m, 5H), 2.28 - 1.64 (m, 6H), 1.25 (s, 1H), 1.19 (t, *J* = 7.0 Hz, 3H). MS (ESI): m/z 590.6 [M+H]⁺ |
| **3/32** | **Int 2, Int 10** | 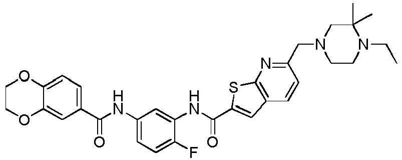 | ¹H NMR (300 MHz, DMSO-d₆): δ ppm 10.52 (s, 1H), 10.17 (s, 1H), 8.41 (d, *J =* 8.2 Hz, 1H), 8.33 (s, 1H), 8.15-8.11 (m, 1H), 7.69 - 7.64 (m, 1H), 7.61 (s, 1H), 7.54 (s, 1H), 7.50 (s, 1H), 7.30 (t, *J =* 9.5 Hz, 1H), 6.99 (d, *J* = 8.2 Hz, 1H), 4.35- 4.28 (m, 4H), 3.71 (s, 2H), 2.66 (s, 2H), 2.49 - 2.40 (m, 4H), 2.23 (s, 2H), 1.09-0.96 (m, 9H). MS (ESI): m/z 604.6 [M+H]⁺ |
| **3/33** | **Int 2, Int 10/1** | 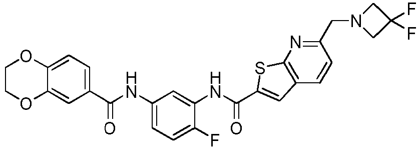 | ¹H NMR (300 MHz, DMSO-d₆): δ ppm 10.54 (s, 1H), 10.17 (s, 1H), 8.42 (d, *J =* 8.4 Hz, 1H), 8.34 (s, 1H), 8.11 (d, *J* = 7.3 Hz, 1H), 7.70 - 7.64 (m, 1H), 7.57 - 7.49 (m, 3H), 7.31 (t, *J* = 9.5 Hz, 1H), 6.99 (d, *J* = 8.3 Hz, 1H), 4.35 - 4.28 (m, 4H), 4.07 (s, 2H), 3.82 (t, *J= 11.6* Hz, 4H). MS (ESI): m/z 555.3 [M+H]⁺ |
| **3/34** | **Int 2, Int 10/3** | 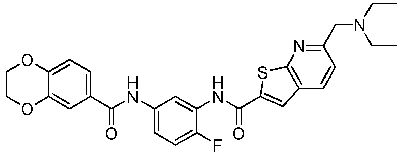 | ¹H NMR (300 MHz, DMSO-d₆): δ ppm 10.63 (s, 1H), 10.18 (s, 1H), 8.58 (d, *J =* 8.2 Hz, 1H), 8.42 (s, 1H), 8.15 (d, *J =* 7.0 Hz, 1H), 7.70-7.61 (m, 2H), 7.54 (s, 1H), 7.50 (s, 1H), 7.32 (t, *J* = 9.5 Hz, 1H), 7.00 (d, *J =* 8.3 Hz, 1H), 4.63 (s, 2H), 4.35 - 4.28 (m, 4H), 3.27 - 3.09 (m, 4H), 1.26 (t, *J =* 6.9 Hz, 6H). MS (ESI): m/z 535.4 [M+H]⁺ |
| **3/35** | 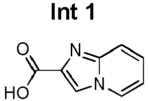 | 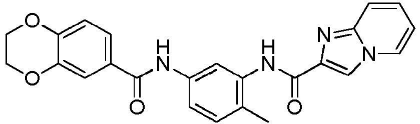 | ¹H NMR (300 MHz, CD₃OD): δ ppm 8.49 (d, *J* = 6.9 Hz, 1H), 8.40 (s, 1H), 8.12 (s, 1H), 7.64 (d, *J* = 9.2 Hz, 1H), 7.53 - 7.38 (m, 4H), 7.26 (d, *J* = 8.5 Hz, 1H), 6.99 (t, *J =* 6.9 Hz, 1H), 6.93 (d, *J* = 8.3 Hz, 1H), 4.35-4.28 (m, 4H), 2.37 (s, 3H). MS (ESI): m/z 429.4 [M+H]⁺ |
| **3/36** | 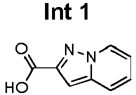 | 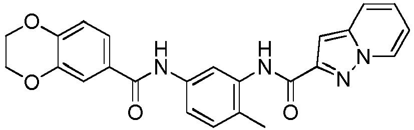 | ¹H NMR (300 MHz, DMSO-d₆): δ ppm 10.05 (s, 1H), 9.77 (s, 1H), 8.76 (d, *J =* |
| | | | 7.2 Hz, 1H), 8.04 (s, 1H), 7.82 (d, *J =* 8.9 Hz, 1H), 7.59 (s, 1H), 7.57 - 7.50 (m, 2H), 7.34 (t, *J =* 7.7 Hz, 1H), 7.22 (d, *J* = 8.4 Hz, 1H), 7.13 (s, 1H), 7.08 (t, *J* = 6.8 Hz, 1H), 6.98 (d, *J* = 8.0 Hz, 1H), 44.34 - 4.27 (m, 4H), 2.26 (s, 3H). MS (ESI): m/z 429.4 [M+H]⁺ |
| **3/37** | 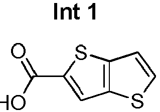 | 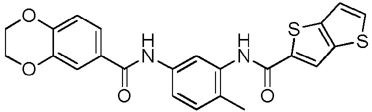 | ¹H NMR (300 MHz, DMSO-d₆): δ ppm 10.04 (s, 1H), 10.03 (s, 1H), 8.32 (s, 1H), 7.89 (d, *J* = 5.2 Hz, 1H), 7.48 (s, 1H), 7.59 (d, *J* = 8.4 Hz, 1H), 7.55-7.48 (m, 3H), 7.23 (d, *J =* 8.2 Hz, 1H), 6.98 (d, *J* = 8.0 Hz, 1H), 4.34 - 4.27 (m, 4H), 2.22 (s, 3H). MS (ESI): m/z 451.6 [M+H]⁺ |
| **3/38** | 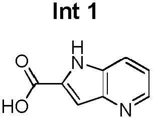 | 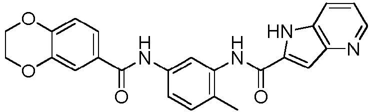 | ¹H NMR (300 MHz, DMSO-d₆): δ ppm 13.02 (s, 1H), 10.44 (s, 1H), 10.08 (s, 1H), 8.74 (d, *J =* 5.4 Hz, 1H), 8.40 (d, *J* = 7.9 Hz, 1H), 7.89 (s, 1H), 7.70 - |
| | | | 7.63 (m, 2H), 7.58 (d, *J* = 7.8 Hz, 1H), 7.52 (s, 1H), 7.49 (s, 1H), 7.29 - 7.23 (m,1H), 7.06 - 6.89 (m, 1H), 4.34 - 4.26 (m, 4H), 2.23 (s, 3H). MS (ESI): m/z 429.5 [M+H]⁺ |
| **3/39** | 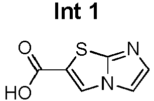 | 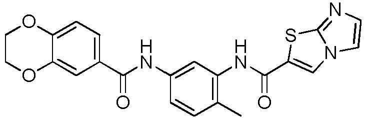 | ¹H NMR (300 MHz, DMSO-d₆): δ ppm 10.19 (s, 1H), 10.06 (s, 1H), 8.79 (s, 1H), 7.94 (s, 1H), 7.84 (s, 1H), 7.59 (d, *J* = 8.3 Hz, 1H), 7.54 - 7.49 (m, 1H), 7.36 (s, 1H), 7.24 (d, *J =* 8.3 Hz, 1H), 6.98 (d, *J* = 8.4 Hz, 1H), 4.34 - 4.27 (m, 4H), 2.21 (s, 3H). MS (ESI): m/z 435.4 [M+H]⁺ |
| **3/40** | 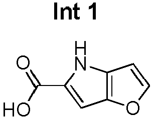 | 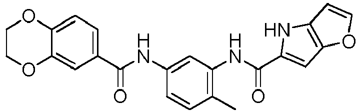 | ¹H NMR (300 MHz, CD₃OD): δ ppm 7.71 (s, 1H), 7.57 (s, 1H), 7.52-7.44 (m, 3H), 7.25 (d, *J =* 8.2 Hz, 1H), 6.94 (s, 1H), 6.91 (s, 1H), 6.54 (s, 1H), 4.34 - 4.26 (m, 4H), 2.28 (s, 3H). MS (ESI): m/z 418.4 [M+H]⁺ |
| **3/41** | 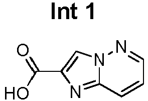 | 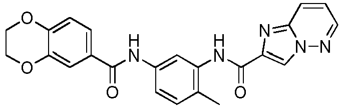 | ¹H NMR (300 MHz, DMSO-d₆): δ ppm 10.06 (s, 1H), 9.79 (s, 1H), 8.80 (s, 1H), 8.66 (s, 1H), 8.25 (d, *J =* 9.1 Hz, 1H), 8.15 (s, 1H), 7.59 - 7.50 (m, 3H), 7.40 - 7.35 (m, 1H), 7.22 (d, *J* = 8.2 Hz, 1H), 6.97 (d, *J* = 8.6 Hz, 1H), 4.34 - 28 (m, 4H), 2.27 (s, 3H). MS (ESI): m/z 430.5 [M+H]⁺ |
| **3/42** | 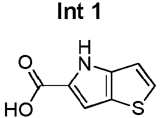 | 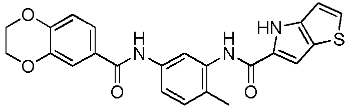 | ¹H NMR (300 MHz, DMSO-d₆): δ ppm 11.82 (s, 1H), 10.04 (s, 1H), 9.62 (s, 1H), 7.82 (s, 1H), 7.59-7.49 (m, 3H), 7.43 (d, *J* = 5.0 Hz, 1H), 7.33 (s, 1H), 7.22 (d, *J* = 7.8 Hz, 1H), 7.00 - 6.97 (m, 2H), 4.35 - 4.27 (m, 4H), 2.21 (s, 3H). MS (ESI): m/z 434.4 [M+H]⁺ |
| **3/43** | 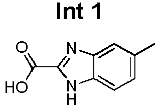 | 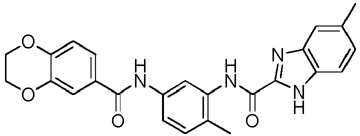 | 1H NMR (300 MHz, CDCl₃): δ ppm 9.29 (s, 1H), 8.26 (s, 1H), 7.78 - 7.63 (m, 3H), 7.45 - 7.34 (m, 3H), 7.18 (s, 1H), 6.93 (d, *J* = 8.1 Hz, 1H), 4.34 - 4.26 (m, 4H), |
| | | | 2.50 (s, 3H), 2.43 (s, 3H). MS (ESI): m/z 443.5 [M+H]⁺ |
| **3/44** | **Int 2, Int 22** | 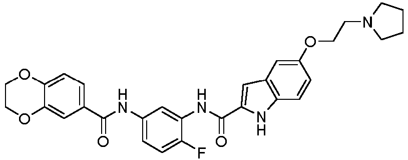 | ¹H NMR (300 MHz, DMSO-d₆): δ ppm 11.67 (s, 1H), 10.15 (s, 1H), 10.07 (s, 1H), 8.11 (d, *J =* 6.8 Hz, 1H), 7.65-7.58 (m ,1H), 7.53-7.49 (m, 2H), 7.41 (d, *J* = 8.9 Hz, 1H), 7.34 - 7.31 (m, 1H), 7.29 (s, 1H), 7.25 (s, 1H), 7.00 - 6.95 (m, 2H), 4.35 - 4.26 (m, 4H), 3.65 - 3.52 (m, 2H), 3.23 - 3.00 (m, 4H), 2.09 - 1.83 (m, 4H). MS (ESI): m/z 545.5 [M+H]⁺ |
| **3/45** | **Int 2, Int 26** | 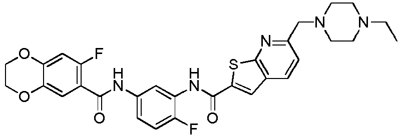 | ¹H NMR (300 MHz, DMSO-d₆): δ ppm 10.53 (s, 1H), 10.29 (s, 1H), 8.39 (d, *J =* 7.9 Hz, 1H), 8.32 (s, 1H), 8.05 (d, *J =* 7.8 Hz, 1H), 7.61-7.57 (m, 2H), 7.31 (t, *J* = 9.3 Hz, 1H), 7.18 (d, *J* = 6.8 Hz, 1H), 6.93 (d, *J =* 10.8 Hz, 1H), 4.32 (s, 2H), 4.29 (s, 1H), 3.73 (s, 2H), 2.47-2.27 (m, 10H), 0.99 (t, *J* = 6.9 Hz, 3H). MS (ESI): m/z 594.6 [M+H]⁺. |

### Example 4: N-(5-(2,3-Dihydrobenzo[b][1,4]dioxine-6-carboxamido)-2-methylphenyl)thieno[2,3-b]pyrazine-6-carboxamide (Example 4)

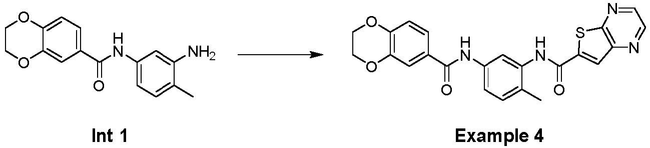

Thionyl chloride (5.00 mL) was added to thieno[2,3-b]pyrazine-6-carboxylic acid (15.2 mg, 0.059 mmol). The mixture was stirred at 50 °C for 1 h. After the mixture was complete, the mixture was concentrated to dryness. Toluene (2 mL) was added to the concentrated mixture, and this was concentrated to dryness. The co-evaporation was repeated two more times and then the residue was dissolved in THF (2 mL). A solution of *N*-(3-amino-4-methylphenyl)-2,3-dihydrobenzo[*b*][1,4]dioxine-6-carboxamide **(Int 1)** (20.0 mg, 0.070 mmol) and DIPEA (74.0 µL, 0.352 mmol) in THF (2.00 mL) was prepared and brought to 0 °C. The thieno[2,3-*b*]pyrazine-6-carboxylic acid mixture in THF was then added to the cooled **Int 1** mixture. This was brought to rt and stirred for 18 h. Then, the mixture was concentrated to dryness and then redissolved in EtOAc (20 mL). The resulting mixture was washed with 2 M HCI (15.0 mL). The combined organic layers were dried over MgSO₄, filtered, and concentrated to dryness to afford the crude product. The residue was purified by preparative HPLC to afford the title compound as a white solid.

¹H NMR (300 MHz, MeOD-*d*₃) δ ppm: δ 8.81 (s, 1H), 8.69 (s, 1H), 8.33 (s, 1H), 7.82 (s, 1H), 7.56 (d, *J =* 8.4, 1H), 7.52 - 7.43 (m, 2H), 7.32 (d, *J =* 8.4 Hz, 1H), 6.96 (d, *J =* 8.2 Hz, 1H), 4.37 - 4.28 (m, 4H), 2.34 (s, 3H). MS (ESI): m/z 447.2 [M+H]⁺.

### Example 5: N-(3-(5-(2-Hydroxy-2-methylpropoxy)benzofuran-2-carboxamido)-4-methylphenyl)-2,3-dihydrobenzo[b][1,4]dioxine-6-carboxamide (Example 5)

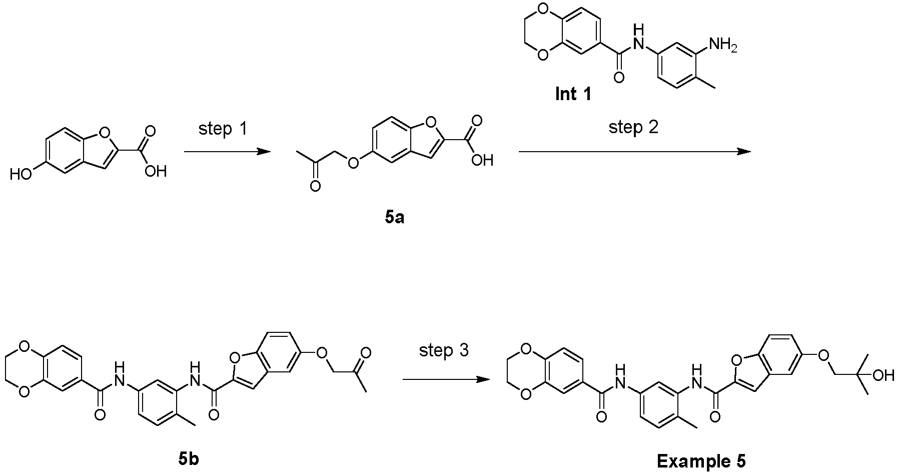

### Step 1: 5-(2-Oxopropoxy)benzofuran-2-carboxylic acid (5a)

To a solution of 5-hydroxybenzofuran-2-carboxylic acid (25.0 mg, 0.140 mmol) in acetone (2.00 mL) was added K₂CO₃ (120 mg, 0.870 mmol). After 30 min, 1-chloropropan-2-one (69.0 µL, 0.870 mmol) and Nal (25.2 mg, 0.170 mmol) were added. The mixture was stirred at 65 °C for 72 h. K₂CO₃ (41.9 mg, 0.300 mmol) and water (0.500 mL) were added to the mixture. The mixture was stirred at 45 °C for 4.5 h. Then, the mixture was cooled to rt and 1 M HCI (10 mL) was added. The resulting mixture was extracted with EtOAc (3 x 15 mL). The combined organic layers were dried over MgSO₄, filtered, and concentrated to dryness to afford the crude title compound.

### Step 2: N-(4-Methyl-3-(5-(2-oxopropoxy)benzofuran-2-carboxamido)phenyl)-2,3-dihydrobenzo[b][1,4]dioxine-6-carboxamide (5b)

Thionyl chloride (2 mL) was added to 5-(2-oxopropoxy)benzofuran-2-carboxylic acid **(5a)** (28.0 mg, 0.120 mmol). The mixture was stirred at 53 °C for 1 h. After the mixture was complete, the mixture was concentrated to dryness. Toluene (2 mL) was added to the concentrated mixture, and this was concentrated to dryness. The co-evaporation was repeated two more times and then the crude was dissolved in THF (2 mL). A solution of *N*-(3-amino-4-methylphenyl)-2,3-dihydrobenzo[b][1,4]dioxine-6-carboxamide **(Int 1)** (34.0 mg, 0.120 mmol) and DIPEA (42.0 µL, 0.240 mmol) in THF (2.00 mL) was prepared and brought to 0 °C. The crude **5a** mixture in THF was then added to the cooled **Int 1** mixture. The mixture was brought to rt and stirred for 18 h. Then, the mixture was concentrated to dryness in vacuo and then redissolved in EtOAc (20 mL). The resulting mixture was washed with 2 M HCI (15 mL). The combined organic layers were dried over MgSO₄, filtered, and concentrated to dryness to afford the title compound.

### Step 3: N-(3-(5-(2-Hydroxy-2-methylpropoxy)benzofuran-2-carboxamido)-4-methylphenyl)-2,3-dihydrobenzo[b][1,4]dioxine-6-carboxamide (Example 5)

To a solution of *N*-(4-methyl-3-(5-(2-oxopropoxy)benzofuran-2-carboxamido)phenyl)-2,3-dihydrobenzo[*b*][1,4]dioxine-6-carboxamide **(5b)** (65.0 mg, 0.112 mmol) in THF (1.00 mL) at 0 °C was added methylmagnesium bromide (1.0 M in THF, 0.503 mL, 0.503 mmol). Then, water (2 mL) was added. The resulting mixture was extracted with EtOAc (3 x 4 mL). The combined organic layers were dried over MgSO₄, filtered, and concentrated to dryness. The residue was purified by preparative HPLC to afford the title compound as a white solid.

¹H NMR (300 MHz, CDCl₃) δ ppm: δ 8.26 (s, 1H), 8.17 (s, 1H), 7.84 (s, 1H), 7.77 (d, *J* = 8.3 Hz, 1H), 7.52 (s, 1H), 7.48 (d, *J* = 8.7 Hz, 1H), 7.44 (s, 1H), 7.37 (d, *J* = 8.5 Hz, 1H), 7.22 (s, 1H), 7.13 - 7.10 (m, 2H), 6.93 (d, *J* = 8.4, 1H), 4.34 - 4.26 (m, 4H), 3.85 (s, 2H), 2.39 (s, 3H), 1.38 (s, 6H). MS (ESI): m/z 517.5 [M+H]⁺.

### Example 6: N-(3-(6-Hydroxybenzo[b]thiophene-2-carboxamido)-4-methylphenyl)-2,3-dihydrobenzo[b][1,4]dioxine-6-carboxamide (Example 6)

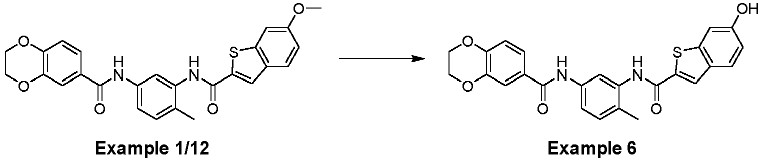

BBr₃ (1.0 M solution in DCM, 7.9 mL, 0.800 mmol) was added to a solution of **Example 1/12** (0.750 mg, 0.200 mmol) in DCM (15.8 mL) dropwise at -78 °C. The mixture was stirred at -78 °C for 1 h and then slowly warmed to rt for 18 h. EtOH (1.00 mL) was added dropwise at 0 °C and the mixture was brought to room temperature. This was washed with 1 M HCI (3 x 30 mL), sat. aq. NaHCO₃ (3 x 30 mL), and then brine (30 mL). The organic layer was dried over MgSO₄ and concentrated to dryness. The mixture was then purified by preparative HPLC to afford the title compound as a white solid.

¹H NMR (300 MHz, DMSO-*d*₆) δ ppm: δ 10.04 (s, 1H), 10.0 (s, 1H), 9.94 (s, 1H), 8.17 (s, 1H), 7.84 (s, 1H), 7.79 (d, *J* = 8.7 Hz, 1H), 7.59 (d, *J* = 8.3 Hz, 1H), 7.55 - 7.47 (m, 2H), 7.32 (s, 1H), 7.23 (d, *J* = 8.3 Hz, 1H), 7.02 - 6.93 (m, 2H), 4.37 - 4.27 (m, 4H), 2.23 (s, 3H). MS (ESI): m/z 461.4 [M+H]⁺.

### Example 7: N-(3-(6-(2-Methoxyethoxy)benzo[b]thiophene-2-carboxamido)-4-methylphenyl)-2,3-dihydrobenzo[b][1,4]dioxine-6-carboxamide (Example 7)

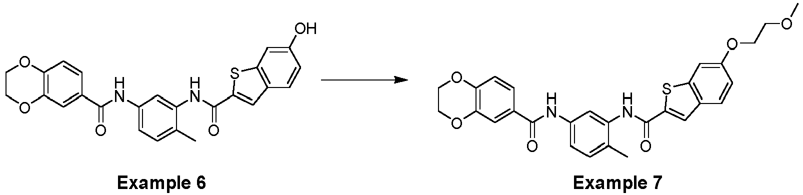

To a solution of *N*-(3-(6-hydroxybenzo[b]thiophene-2-carboxamido)-4-methylphenyl)-2,3-dihydrobenzo[b][1,4]dioxine-6-carboxamide **(Example 6)** (17.5 mg, 0.038 mmol) and K₂CO₃ (10.5 mg, 0.076 mmol) in DMF (0.380 mL) was added 2-bromoethyl methyl ether (5.80 mg, 0.042 mmol). The mixture was stirred at 120 °C for 1 h. The mixture was purified by preparative HPLC to afford the title compound as a white solid.

¹H NMR (300 MHz, DMSO-*d*₆) δ ppm: δ 10.06 (s, 1H), 10.05 (s, 1H), 8.23 (s, 1H), 7.92 - 7.82 (m, 2H), 7. 63 (s, 1H), 7.59 (d, *J =* 8.6 Hz, 1H), 7.55 - 7.48 (m, 2H), 7.24 (d, *J =* 8.3 Hz, 1H), 7.10 (d, *J* = 8.8, 1H), 6.98 (d, *J* = 8.3 Hz, 1H), 4.37 - 4.27 (m, 4H), 4.25 - 4.16 (m, 2H), 3.71 (t, *J =* 4.4 Hz, 2H), 3.34 (s, 3H), 2.23 (s, 3H). MS (ESI): m/z 519.5 [M+H]⁺.

### Example 8: N-(4-Methyl-3-(6-(2-oxopropoxy)benzo[b]thiophene-2-carboxamido)phenyl)-2,3-dihydrobenzo[b][1,4]dioxine-6-carboxamide (Example 8)

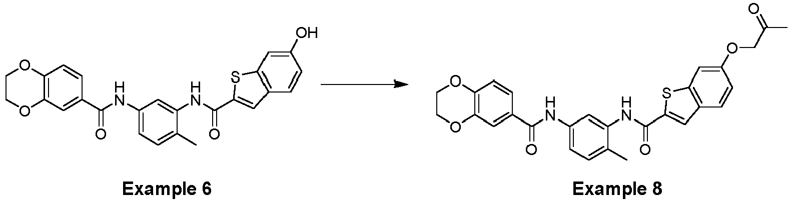

To a solution of *N*-(3-(6-hydroxybenzo[b]thiophene-2-carboxamido)-4-methylphenyl)-2,3-dihydrobenzo[b][1,4]dioxine-6-carboxamide **(Example 6)** (15.0 mg, 0.033 mmol) and K₂CO₃ (3.00 µL, 0.039 mmol) in DMF (0.326 mL) was added chloroacetone (3.00 µL, 0.039 mmol). The mixture was stirred at 100 °C for 1 h. The mixture was then purified by preparative HPLC to afford the title compound as a white solid.

¹H NMR (300 MHz, DMSO-*d*₆) δ ppm: δ 10.08 (s, 1H), 10.04 (s, 1H), 8.23 (s, 1H), 7.93 - 7.82 (m, 2H), 7.66 - 7.48 (m, 4H), 7.24 (d, *J* = 8.4 Hz, 1H), 7.11 (d, *J* = 8.8 Hz, 1H), 6.98 (d, *J* = 8.2 Hz, 1H), 4.94 (s, 2H), 4.38 - 4.26 (m, 4H), 2.23 (s, 3H), 2.20 (s, 3H). MS (ESI): m/z 515.2 [M-H]⁺.

### Example 9: N-(3-(6-(2-Hydroxy-2-methylpropoxy)benzo[b]thiophene-2-carboxamido)-4-methylphenyl)-2,3-dihydrobenzo[b][1,4]dioxine-6-carboxamide (Example 9)

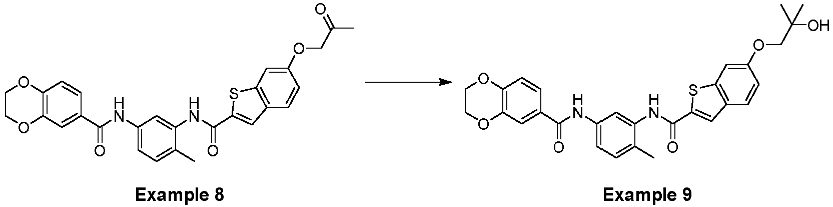

To a solution of *N*-(4-methyl-3-(6-(2-oxopropoxy)benzo[b]thiophene-2-carboxamido)phenyl)-2,3-dihydrobenzo[*b*][1,4]dioxine-6-carboxamide **(Example 8)** (10.0 mg, 0.019 mmol) in THF (0.190 mL) was added MeMgBr (2 M, 0.015 mL, 0.031 mmol) at 0 °C, then warmed to rt. After 1h, sat. aq. NH₄CI (1 mL) was added to the mixture. This was purified by preparative HPLC to the title compound as a white solid.

¹H NMR (300 MHz, CDCl₃) δ ppm: δ 8.03 (s, 1H), 7.86 (s, 1H), 7.80 (s, 1H), 7.77 - 7.62 (m, 3H), 7.42 (s, 1H), 7.40 - 7.29 (m, 2H), 7.19 (d, *J* = 8.3 Hz, 1H), 7.08 (d, *J* = 8.9 Hz, 1H), 6.92 (d, *J =* 8.3 Hz, 1H), 4.35 - 4.24 (m, 4H), 3.89 (s, 2H), 2.32 (s, 3H), 1.39 (s, 6H). MS (ESI): m/z 533.4 [M+H]⁺.

### Example 10: N-(3-(6-(2-Hydroxyethoxy)benzo[b]thiophene-2-carboxamido)-4-methylphenyl)-2,3-diydrobenzo[b][1,4]dioxine-6-carboxamide (Example 10)

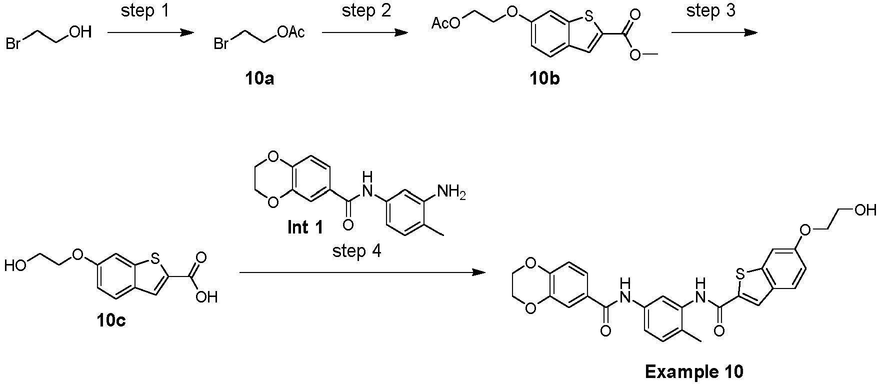

### Step 1: 2-Bromoethyl acetate (10a)

To a solution of 2-bromoethanol in DCM (16.0 mL) was added acetyl chloride (0.683 mL, 9.67 mmol) and then TEA (0.728 mL, 10.6 mmol). The mixture was stirred for 72 h at room temperature. The mixture was diluted with DCM (50 mL) and washed with sat. aq. NH₄Cl (20 mL) and then sat. aq. NaHCO₃ (20 mL). The organic layer was dried over MgSO₄, filtered, and concentrated to dryness to afford the crude title compound as a yellow liquid.

### Step 2: Methyl 6-(2-acetoxyethoxy)benzo[b]thiophene-2-carboxylate (10b)

To a solution of ethyl 6-methoxybenzo[b]thiophene-2-carboxylate **(Int 4b)** (200 mg, 0.960 mmol) and Cs₂CO₃ (250 mg, 1.15 mmol) in DMF (3.20 mL) was added crude 2-bromoethyl acetate **(10a)** (250 mg, 1.15 mmol). The mixture was stirred at 60 °C for 1h. The solvent was removed under reduced pressure to afford the crude title compound.

### Step 3: 6-(2-Hydroxyethoxy)benzo[b]thiophene-2-carboxylic acid (10c)

To a solution of methyl 6-(2-acetoxyethoxy)benzo[*b*]thiophene-2-carboxylate **(10b)** (283 mg, 0.960 mmol) in MeOH (4.00 mL) was added 2 M aq. NaOH (1.00 mL). This was brought to reflux and stirred for 1 h. The mixture was allowed to cool to rt and the pH was adjusted to pH ~ 2 by 1 M HCl. The mixture was then extracted by EtOAc (3 x 30 mL). The organic layers were combined, washed with brine, dried over MgSO₄, filtered, and concentrated to dryness to afford the title compound as a light brown solid.

### Step 4: N-(3-(6-(2-Hydroxyethoxy)benzo[b]thiophene-2-carboxamido)-4-methylphenyl)-2,3-diydrobenzo[b][1,4]dioxine-6-carboxamide (Example 10)

To a mixture of *N*-(3-amino-4-methylphenyl)-2,3-dihydrobenzo[b][1,4]dioxine-6-carboxamide **(Int 1)** (298 mg, 1.05 mmol), 6-(2-hydroxyethoxy)benzo[b]thiophene-2-carboxylic acid **(10c)** (100 mg, 0.420 mmol) in DMF (4.20 mL) was added NMI (0.117 mL, 1.47 mmol). After 5 min, TCFH (130 mg, 1.47 mmol) was added. The mixture stirred for 15 min at rt. Then, the residue was diluted with EtOAc (200 mL) and washed with 1 M HCI (3 x 30 mL) and sat. aq. NaHCO₃ (30 mL). The organic layer was concentrated to dryness and purified by column chromatography on silica gel (Acetone/Cyclohexane = 1:10). The resulting solid was triturated with MeCN (2 x 10 mL) and pentane (7 x 5 mL). This was then suspended in MeCN/water (1:1), frozen, and placed on the freeze drier to afford the title compound as a white solid.

¹H NMR (300 MHz, DMSO-*d*₆) δ ppm: 10.06 (s, 1H), 10.04 (s, 1H), 8.22 (s, 1H), 7.90 - 7.81 (m, 2H), 7.64 - 7.56 (m, 2H), 7.55 - 7.49 (m, 2H), 7.23 (d, *J =* 8.2 Hz, 1H), 7.09 (d, *J =* 8.2 Hz, 1H), 6.98 (d, *J* = 8.3 Hz, 1H), 4.89 (t, *J* = 5.6 Hz, 1H), 4.35 - 4.27 (m, 4H), 4.10 (t, *J* = 4.8 Hz, 2H), 3.80 - 3.72 (m, 2H), 2.23 (s, 3H). MS (ESI): m/z 503.2 [M-H]⁻.

### Example 11: N-(4-Methyl-3-(6-(2-(2-oxopropoxy)ethoxy)benzo[b]thiophene-2-carboxamido)phenyl)-2,3-dihydrobenzo[b][1,4]dioxine-6-carboxamide (Example 11)

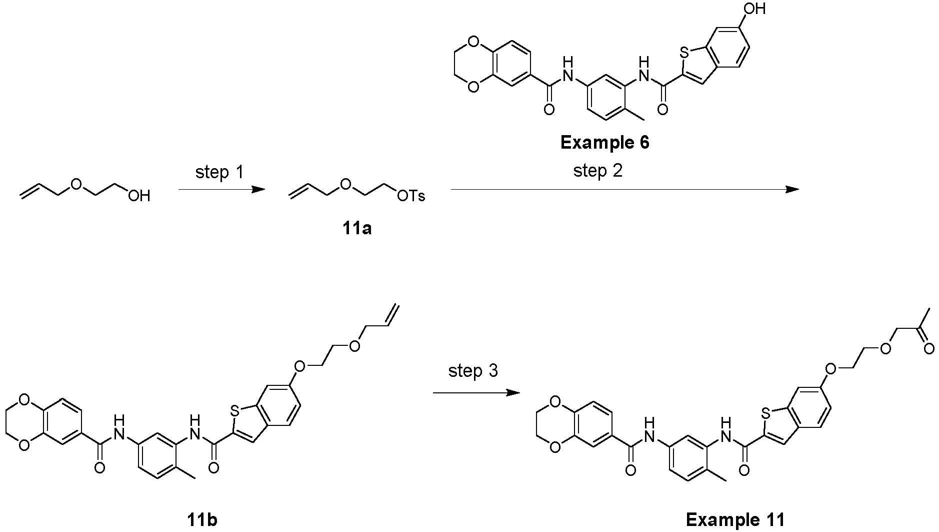

### Step 1: 2-(Allyloxy)ethyl 4-methylbenzenesulfonate (11a)

To a mixture of 2-allyloxyethanol (200 mg, 1.96 mmol) in DCM (20.0 mL) were added sodium hydroxide (411 mg, 2.15 mmol) and 4-toluene sulfonyl chloride (313 mg, 7.84 mmol) at 0 °C, and then the solution was stirred for 0.5 h. The solution was stirred at 25 °C for 4 h. The mixture was washed water (3 x 20 mL). The organic layer was dried over Na₂SO₄, and filtered, concentrated to dryness to afford the crude title compound.

### Step 2: N-(3-(6-(2-(Allyloxy)ethoxy)benzo[b]thiophene-2-carboxamido)-4-methylphenyl)-2,3-dihydrobenzo[b][1,4]dioxine-6-carboxamide (11b)

To a solution of *N*-(3-(6-hydroxybenzo[b]thiophene-2-carboxamido)-4-methylphenyl)-2,3-dihydrobenzo[*b*][1,4]dioxine-6-carboxamide **(Example 6)** (45.0 mg, 0.097 mmol) and K₂CO₃ (27.0 mg, 0.195 mmol) in DMF (0.977 mL) was added 2-(allyloxy)ethyl 4-methylbenzenesulfonate **(11a)** (27.6 mg, 0.107 mmol). The mixture stirred at 120 °C for 18 h. The solvent was removed under reduced pressure to afford the crude title compound.

### Step 3: N-(4-Methyl-3-(6-(2-(2-oxopropoxy)ethoxy)benzo[b]thiophene-2-carboxamido)phenyl)-2,3-dihydrobenzo[b][1,4]dioxine-6-carboxamide (Example 11)

To a stirred solution of *N*-(3-(6-(2-(allyloxy)ethoxy)benzo[*b*]thiophene-2-carboxamido)-4-methylphenyl)-2,3-dihydrobenzo[*b*][1,4]dioxine-6-carboxamide **(11b)** (30.0 mg, 0.055 mmol) in MeCN (1.00 mL) and water (0.150 mL) were added Pd(OAc)₂ (0.600 mg, 0.003 mmol) and DMP (28.0 mg, 0.066 mmol) at rt. The mixture was warmed to 50 °C and stirred for 18 h. This was purified by preparative HPLC to afford the title compound as a white solid.

¹H NMR (300 MHz, CDCl₃) δ ppm: δ 8.09 (s, 1H), 7.84 - 7.69 (m, 4H), 7.64 (s, 1H), 7.43 (s, 1H), 7.40 - 7.31 (m, 2H), 7.22 (s, 1H), 7.09 (d, *J* = 8.9 Hz, 1H), 6.94 (d, *J* = 8.5 Hz, 1H), 4.37 - 4.24 (m, 6H), 4.21 (s, 2H), 3.95 (t, *J =* 4.8 Hz, 2H), 2.35 (s, 3H), 2.19 (s, 3H). MS (ESI): m/z 561.5 [M+H]⁺.

### Example 12: N-(4-Methyl-3-(6-(2-(pyrrolidin-1-yl)ethoxy)benzo[b]thiophene-2-carboxamido)phenyl)-2,3-dihydrobenzo[b][1,4]dioxine-6-carboxamide (Example 12)

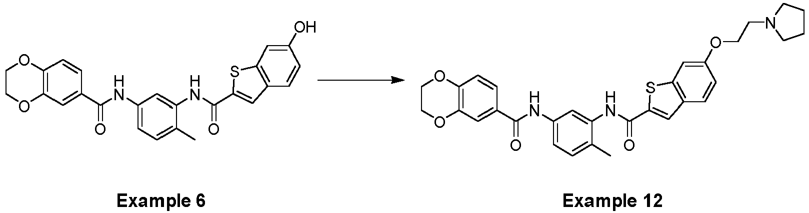

To a solution of *N*-(3-(6-hydroxybenzo[*b*]thiophene-2-carboxamido)-4-methylphenyl)-2,3-dihydrobenzo[*b*][1,4]dioxine-6-carboxamide **(Example 6)** (20.0 mg, 0.043 mmol) and NaH (1.90 mg, 0.086 mmol) in DMF (0.391 mL) was added 1-(2-chloroethyl)pyrrolidine hydrochloride (7.3 mg, 0.047 mmol). The mixture was stirred at 50 °C for 18 h. This was purified by preparative HPLC to afford the title compound as a white solid.

¹H NMR (300 MHz, DMSO-*d*₆) δ ppm: δ 10.06 (s, 1H), 10.05 (s, 1H), 8.22 (s, 1H), 7.92 - 7.81 (m, 2H), 7.63 (s, 1H), 7.59 (d, *J* = 8.0, 1H), 7.56 - 7.47 (m, 2H), 7.24 (d, *J* = 8.4 Hz, 1H), 7.09 (d, *J =* 8.8 Hz, 1H), 6.98 (d, *J =* 8.3 Hz, 1H), 4.36 - 4.27 (m, 4H), 4.18 (t, *J* = 5.8 Hz, 2H), 2.84 (t, *J* = 6.0 Hz, 2H), 2.23 (s, 3H), 1.77 - 1.65 (m, 4H). MS (ESI): m/z 558.6 [M+H]⁺.

### Example 13: (S)-N-(3-(6-(3-(3-Fluoropyrrolidin-1-yl)propoxy)benzo[b]thiophene-2-carboxamido)-4-methylphenyl)-2,3-dihydrobenzo[b][1,4]dioxine-6-carboxamide (Example 13)

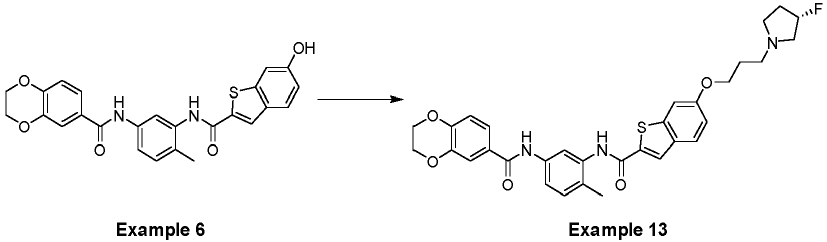

To a solution of *N*-(3-(6-hydroxybenzo[*b*]thiophene-2-carboxamido)-4-methylphenyl)-2,3-dihydrobenzo[*b*][1,4]dioxine-6-carboxamide **(Example 6)** (50.0 mg, 0.108 mmol) in DMF (1.10 mL) was added K₂CO₃ (33.0 mg, 0.238 mmol) and 1-bromo-3-chloropropane (0.013 mL, 0.130 mmol). This was brought to 65 °C and stirred for 18 h. Thereafter, (S)-3-fluoropyrrolidine hydrochloride (16.4 mg, 0.130 mmol) and K₂CO₃ (11.5 mg, 0.116 mmol) were added and stirred at 65°C for 24 h. This was purified by preparative HPLC to afford the title compound as a white solid.

¹H NMR (300 MHz, DMSO-*d*₆) δ ppm: δ 10.06 (s, 1H), 10.05 (s, 1H), 8.22 (s, 1H), 7.92 - 7.81 (m, 2H), 7.65 - 7.47 (m, 4H), 7.24 (d, *J* = 8.4 Hz, 1H), 7.08 (d, *J =* 9.1 Hz, 1H), 6.98 (d, *J* = 8.5 Hz, 1H), 5.20 (dt, *J* = 13.5, 5.4 Hz, 1H), 4.38 - 4.25 (m, 4H), 4.13 (t, *J =* 6.3 Hz, 2H), 2.92 - 2.74 (m, 2H), 2.73 - 2.74 (m, 1H), 2.63 - 2.54 (m, 2H), 2.39 - 2.28 (m, 1H), 2.23 (s, 3H), 2.20 - 2.01 (m, 1H), 1.99 - 1.89 (m, 2H), 1.88 - 1.74 (m, 1H). MS (ESI): m/z 590.6 [M+H]⁺.

### Examples 13/1 to 13/3

The following Examples were prepared similar as described for **Example 13** using the appropriate building blocks.

| **#** | Building blocks | Structure | Analytical Data |
|---|---|---|---|
| **13/1** | **Example 6,** 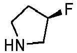 | 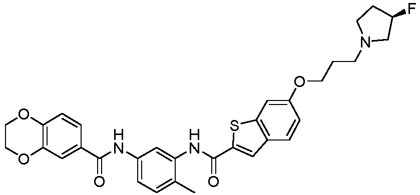 | ¹H NMR (300 MHz, DMSO-*d*₆) δ ppm: δ 10.06 (s, 1H), 10.05 (s, 1H), 8.24 - 8.20 (m, 1H), 7.91 - 7.83 (m, 2H), 7.65 - 7.56 (m, 2H), 7.55 - 7.48 (m, 2H), 7.24 (d, *J =* 8.3 Hz, 1H), 7.08 (d, *J* = 8.9, 1H), 6.98 (d, *J =* 8.3 Hz, 1H), 5.20 (dt, *J* = 13.2, 5.4 Hz, 1H), 4.37 - 4.25 (m, 4H), 4.13 (t, *J =* 6.5 Hz, 2H), 2.92 - 2.74 (m, 2H), 2.73 - 2.64 (m, 1H), 2.63 - 2.52 (m, 2H), 2.39 - 2.28 (m, 1H), 2.23 (s, 3H), 2.21 - 2.01 (m, 1H), 1.99 - 1.89 (m, 2H), 1.89 - 1.76 (m, 1H). MS (ESI): m/z 590.6 [M+H]⁺. |
| **13/2** | **Example 6,** 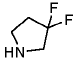 | 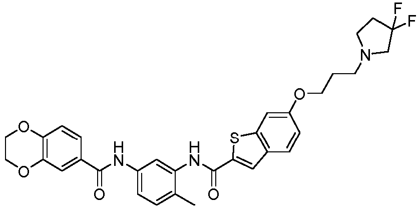 | ¹H NMR (300 MHz, MeOD-*d*₃) δ ppm: δ 8.07 (s, 1H), 7.83 (d, *J =* 9.0 Hz, 1H), 7.76 (s, 1H), 7.55 (d, *J* = 8.2 Hz, 1H), 7.51 - 7.42 (m, 3H), 7.29 |
| | | | (d, *J* = 8.3 Hz, 1H), 7.09 (s, J = 8.6 Hz, 1H), 6.95 (d, *J* = 8.3 Hz, 1H), 4.37 - 4.27 (m, 4H), 4.17 (t, *J =* 6.1 Hz, 2H), 3.04 - 2.91 (m, 2H), 2.83 (t, *J* = 6.9 Hz, 2H), 2.73 (t, *J* = 7.3 Hz, 2H), 2.41 - 2.20 (m, 5H), 2.10 - 1.98 (m, 2H). MS (ESI): m/z 608.6 [M+H]⁺. |
| **13/3** | **Example 6,** 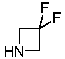 | 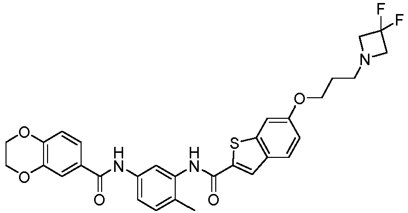 | ¹H NMR (300 MHz, MeOD-*d*₃) δ ppm: 8.05 (s, 1H), 7.81 (d, *J* = 8.6 Hz, 1H), 7.74 (s, 1H), 7.53 (d, *J* = 8.4 Hz, 1H), 7.49 - 7.43 (m, 3H), 7.28 (d, *J =* 8.5 Hz, 1H), 7.07 (d, *J =* 8.8 Hz, 1H), 6.93 (d, *J* = 7.8 Hz, 1H), 4.34 - 4.26 (m, 4H), 4.13 (t, *J* = 5.9 Hz, 2H), 3.65 (t, *J =* 12.1 Hz, 4H), 2.82 (t, J = 6.9 Hz, 2H), 2.31 (s, 3H), 1.92 (t, *J =* 7.2 Hz, 2H). MS (ESI): m/z 594.5 [M+H]⁺. |

### Example 14: N-(5-(2,3-Dihydrobenzo[b][1,4]dioxine-6-carboxamido)-2-methylphenyl)-1-methyl-1H-indole-2-carboxamide (Example 14)

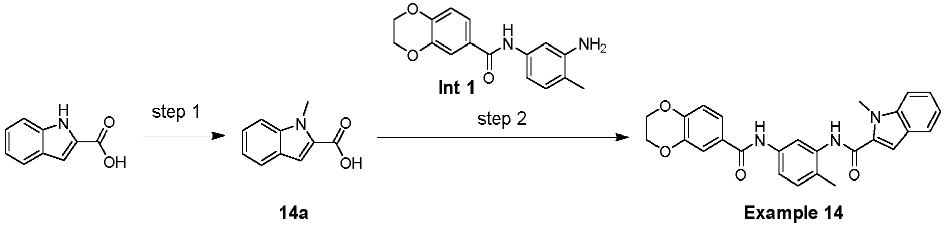

### Step 1: 1-Methyl-1H-indole-2-carboxylic acid (14a)

To a mixture of 1*H*-indole-2-carboxylic acid (10.0 mg, 0.062 mmol) in anhydrous DMF (0.621 mL) was added NaH (4.80 mg, 0.200 mmol) at 0 °C. The mixture was stirred at 0 °C for 15 min, then Mel (12.0 µL, 0.200 mol) was added. The mixture was brought to rt and stirred for 18 h. Then, the solvent was removed in vacuo. Water (10 mL) was added to the residue and the pH was adjusted to pH = 3. The resulting mixture was extracted with EtOAc (10 mL x 3). The combined organic layers were dried over MgSO₄, filtered, and concentrated to dryness to afford the crude 1-methyl-1*H*-indole-2-carboxylic acid.

### Step 2: N-(5-(2,3-Dihydrobenzo[b][1,4]dioxine-6-carboxamido)-2-methylphenyl)-1-methyl-1H-indole-2-carboxamide (Example 14)

To a mixture of *N*-(3-amino-4-methylphenyl)-2,3-dihydrobenzo[b][1,4]dioxine-6-carboxamide **(Int 1)** (10.0 mg, 0.035 mmol), 1-methyl-1*H*-indole-2-carboxylic acid **(14a)** (7.40 mg, 0.042 mmol) in DMF (0.350 mL) was added NMI (8.00 µL, 0.106 mmol). After 5 min, TCFH (10.9 mg, 0.039 mmol) was added. The mixture stirred for 1 h at rt. Then, the residue was concentrated to dryness and purified by preparative HPLC to afford the title compound as a white solid.

¹H NMR (300 MHz, MeOD-*d*₃) δ ppm: δ 7.80 (s, 1H), 7.67 (d, *J* = 8.1 Hz, 1H), 7.56 - 7.43 (m, 4H), 7.37 - 7.24 (m, 3H), 7.14 (t, *J =* 7.5 Hz, 1H), 6.94 (d, *J =* 8.1 Hz, 1H), 4.36 - 4.24 (m, 4H), 4.05 (s, 3H), 2.33 (s, 3H). MS (ESI): m/z 442.4 [M+H]⁺.

### Example 15: N-(3-(Benzo[b]thiophene-2-carboxamido)-4-methylphenyl)chromane-7-carboxamide (Example 15)

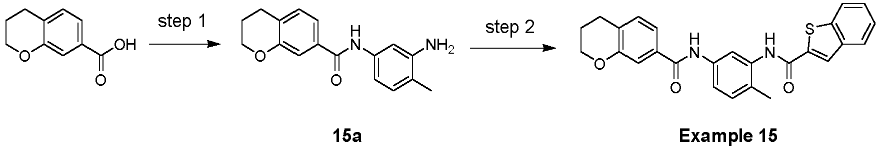

### Step 1: N-(3-Amino-4-methylphenyl)chromane-7-carboxamide (15a)

To a mixture of chromane-7-carboxylic acid (50.0 mg, 0.409 mmol), 2,4-diaminotoluene (72.9 mg, 0.409 mmol) in DMF (0.949 mL) was added NMI (98.0 µL, 1.23 mmol). After 5 min, TCFH (149 mg, 0.532 mmol) was added. The mixture was stirred for 1 h at rt. The reaction was diluted with 1 M HCl (40 mL) and washed with EtOAc (3 x 30 mL). The aqueous layer was adjusted to pH = 3 and extracted with EtOAc (3 x 50 mL), dried over MgSO₄, filtered, and concentrated to dryness to afford the title compound.

### Step 2: N-(3-(Benzo[b]thiophene-2-carboxamido)-4-methylphenyl)chromane-7-carboxamide (Example 15)

To a mixture of *N*-(3-amino-4-methylphenyl)chromane-7-carboxamide (15a) (104 mg, 0.368 mmol), 1-benzothiophene-2-carboxylic acid (65.6 mg, 0.368 mmol) in DMF (1.50 mL) was added NMI (88.0 µL, 1.105 mmol). After 5 min, TCFH (134 mg, 0.479 mmol) was added. The mixture was stirred for 1 h at rt. Then, MeCN/water (5 mL) was added to the mixture and the precipitated solid was triturated with MeCN/water (1:1) to afford the title compound as a white solid.

¹H NMR (300 MHz, DMSO-*d*₆) δ ppm: δ 10.20 (s, 1H), 10.02 (s, 1H), 8.33 (s, 1H), 8.07 (d, *J*= 7.6, 1H), 9.01 (d, *J* = 7.4 Hz, 1H) 7.86 (s, 1H), 7.78 - 7.67 (m, 2H), 7.61 (d, *J* = 8.4 Hz, 1H), 7.54 - 7.42 (m, 2H), 7.25 (d, *J =* 8.4 Hz, 1H), 6.84 (d, *J =* 8.4 Hz, 1H), 4.21 (t, *J =* 5.1 Hz, 2H), 2.83 (t, J = 6.4 Hz, 2H), 2.24 (s, 3H), 2.01 - 1.91 (m, 2H). MS (ESI): m/z 443.4 [M+H]⁺.

### Example 16: N-(3-(6-(((1S,4S)-5-Ethyl-2,5-diazabicyclo[2.2.1]heptan-2-yl)methyl)benzo[b]thiophene-2-carboxamido)-4-fluorophenyl)-2,3-dihydrobenzo[b][1,4]dioxine-6-carboxamide (Example 16)

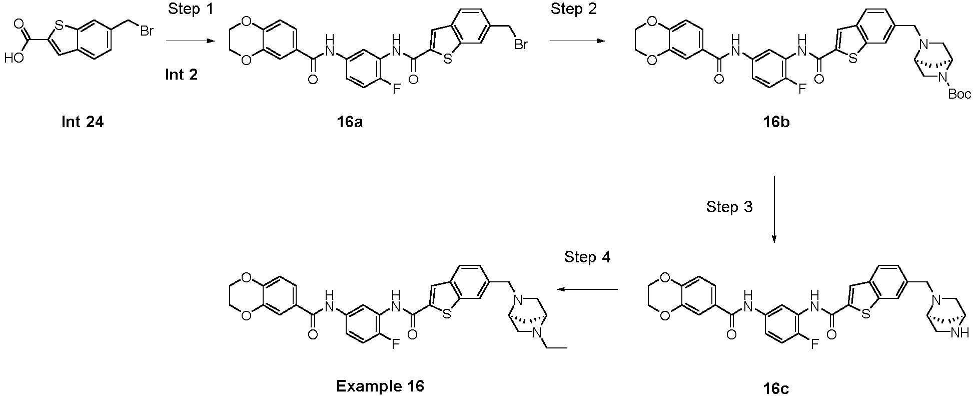

### Step 1: N-(3-(6-(bromomethyl)benzo[b]thiophene-2-carboxamido)-4-fluorophenyl)-2,3-dihydrobenzo[b][1,4]dioxine-6-carboxamide (16a)

The title compound was prepared similar as described for Example 3 using 6-(bromomethyl)benzo[b]thiophene-2-carboxylic acid **(Int 24)** in place of 6-(pyridin-2-ylmethoxy)benzo[b]thiophene-2-carboxylic acid and *N*-(3-amino-4-fluorophenyl)-2,3-dihydrobenzo[b][1,4]dioxine-6-carboxamide **(Int 2)** in place of *N*-(3-amino-4-methylphenyl)-2,3-dihydrobenzo[b][1,4]dioxine-6-carboxamide.

### Step 2: tert-Butyl (1S,4S)-5-((2-((5-(2,3-dihydrobenzo[b][1,4]dioxine-6-carboxamido)-2-fluorophenyl)carbamoyl)benzo[b]thiophen-6-yl)methyl)-2,5-diazabicyclo[2.2.1]heptane-2-carboxylate (16b)

To a solution of *N*-(3-(6-(bromomethyl)benzo[b]thiophene-2-carboxamido)-4-fluorophenyl)-2,3-dihydrobenzo[b][1,4]dioxine-6-carboxamide **(16a)** (17.0 mg, 0.031 mmol) in dry DMF (0.300 mL) was added (1S,4S)-tert-butyl 2,5-diazabicyclo[2.2.1]heptane-2-carboxylate (6.2 mg, 0.031 mmol) and Cs₂CO₃ (11.3 mg, 0.035 mmol). The mixture was stirred at 60 °C for 3 h. The mixture was diluted with EtOAc (20 mL) and washed with 1M HCI (10 mL) and water (10 mL). The organic layer was dried over MgSO₄, filtered, and concentrated to dryness to afford the title compound, which was used in next step without further purification

### Step 3: N-(3-(6-(((1S,4S)-2,5-Diazabicyclo[2.2.1]heptan-2-yl)methyl)benzo[b]thiophene-2-carboxamido)-4-fluorophenyl)-2,3-dihydrobenzo[b][1,4]dioxine-6-carboxamide (16c)

To a solution of *tert*-butyl (1S,4S)-5-((2-((5-(2,3-dihydrobenzo[b][1,4]dioxine-6-carboxamido)-2-fluorophenyl)carbamoyl)benzo[b]thiophen-6-yl)methyl)-2,5-diazabicyclo[2.2.1]heptane-2-carboxylate **(16b)** (20 mg, 0.030 mmol) in dry DCM (0.300 mL) was added TFA (0.100 mL) at 0 °C dropwise. The mixture was allowed to warm to rt and stirred for 2 h. Saturated NaHCO₃ was added and the aqueous layer was extracted with DCM (3 x 15 mL). The combined organic layers were dried over MgSO₄, filtered, and concentrated to dryness to afford the title compound, which was used in the next step without further purification.

### Step 4: N-(3-(6-(((1S,4S)-5-Ethyl-2,5-diazabicyclo[2.2.1]heptan-2-yl)methyl)benzo[b] thiophene-2-carboxamido)-4-fluorophenyl)-2,3-dihydrobenzo[b][1,4]dioxine-6-carboxamide (Example 16)

To a solution of *N*-(3-(6-((1S,4S)-2,5-diazabicyclo[2.2.1]heptan-2-ylmethyl)benzo[b]thiophene-2-carboxamido)-4-fluorophenyl)-2,3-dihydrobenzo[b][1,4]dioxine-6-carboxamide (16c) (8.0 mg, 0.014 mmol) in dry DMF (0.143 mL) was added NaBH₃CN (0.9 mg, 0.014 mmol) and the mixture was cooled to 0 °C. Acetaldehyde (0.001 mL, 0.014 mmol) as a solution in 0.5 mL DMF was added. The mixture was stirred for 5 h. Saturated NaHCO₃ (10 mL) was added and the aqueous layer was extracted with EtOAc (3 x 15 mL). The combined organic layers were dried over MgSO₄, filtered and concentrated to dryness. The residue was purified by preparative HPLC to afford the title compound as a white solid. ¹H NMR (300 MHz, CD₃OD): δ ppm 8.19 - 8.12 (m, 2H), 7.98 (s, 1H), 7.94 (d, *J* = 9 Hz, 1H), 7.62 - 7.54 (m, 1H), 7.54 - 7.46 (m, 3H), 7.23 (t, *J =* 9.6 Hz, 1H), 6.96 (d, *J =* 8.2 Hz, 1H), 4.36-4.29 (m, 4H), 4.03 - 3.80 (m, 3H), 3.55 (s, 2H), 3.12 - 2.76 (m, 5H), 2.82 (d, *J* = 11.5 Hz, 1H), 2.11 - 1.89 (m, 2H), 1.24 (t, *J* = 7.1 Hz, 3H). MS (ESI): m/z 587.6 [M+H]⁺.

### Example 17: N-(3-(6-((4-Ethyl-1,4-diazepan-1-yl)methyl)benzo[b]thiophene-2-carboxamido)-4-fluorophenyl)-2,3-dihydrobenzo[b][1,4]dioxine-6-carboxamide (Example 17)

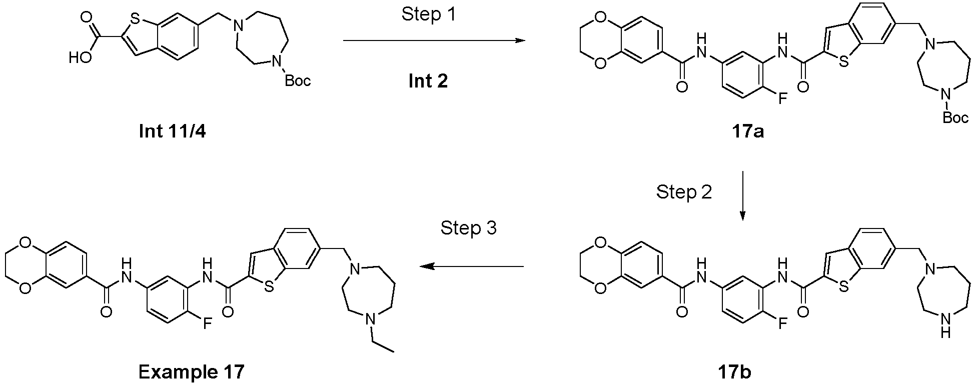

### Step 1: tert-Butyl 4-((2-((5-(2,3-dihydrobenzo[b][1,4]dioxine-6-carboxamido)-2-fluorophenyl)carbamoyl)benzo[b]thiophen-6-yl)methyl)-1,4-diazepane-1-carboxylate (17a)

The title compound was prepared similar as described for Example 3 using 6-((4-(*tert-*butoxycarbonyl)-1,4-diazepan-1-yl)methyl)benzo[b]thiophene-2-carboxylic acid **(Int 11/4)** in place of 6-((pyridin-2-yloxy)methyl)benzo[b]thiophene-2-carboxylic acid and *N-*(3-amino-4-fluorophenyl)-2,3-dihydrobenzo[b][1,4]dioxine-6-carboxamide **(Int 2)** in place of *N*-(3-amino-4-methylphenyl)-2,3-dihydrobenzo[b][1,4]dioxine-6-carboxamide.

### Step 2: N-(3-(6-((1,4-Diazepan-1-yl)methyl)benzo[b]thiophene-2-carboxamido)-4-fluorophenyl)-2,3-dihydrobenzo[b][1,4]dioxine-6-carboxamide (17b)

To a solution of *tert*-butyl 4-((2-((5-(2,3-dihydrobenzo[b][1,4]dioxine-6-carboxamido)-2-fluorophenyl)carbamoyl)benzo[b]thiophen-6-yl)methyl)-1,4-diazepane-1-carboxylate **(17a)** (45.6 mg, 0.069 mmol) in DCM (0.690 mL) was added TFA (0.200 mL) at 0 °C dropwise. The mixture was allowed to warm to rt. After 2 h the reaction was quenched with saturated NaHCO₃. The aqueous layer was extracted with DCM (3 x 20 mL). The combined organic layers were dried over anhydrous MgSO₄, filtered, and concentrated to dryness to afford the title compound.

### Step 3: N-(3-(6-((4-Ethyl-1,4-diazepan-1-yl)methyl)benzo[b]thiophene-2-carboxamido)-4-fluorophenyl)-2,3-dihydrobenzo[b][1,4]dioxine-6-carboxamide (Example 17)

To a solution of *N*-(3-(6-((1,4-diazepan-1-yl)methyl)benzo[b]thiophene-2-carboxamido)-4-fluorophenyl)-2,3-dihydrobenzo[b][1,4]dioxine-6-carboxamide **(17b)** (24.0 mg, 0.043 mmol) in dry DMF (0.214 mL) was added Cs₂CO₃ (13.9 mg, 0.043 mmol), then iodoethane (0.003 mL, 0.043 mmol) at rt. The mixture was stirred for 18 h. The mixture was concentrated to dryness and the residue was purified by preparative HPLC to afford the title compound as a white solid. ¹H NMR (300 MHz, CD₃OD): δ ppm 11.22 (s, 1H), 10.98 (s, 1H), 9.15 (s, 1H), 8.92 (d, *J =* 9 Hz, 1H), 8.80 - 8.72 (m, 2H), 8.50 - 8.42 (m, 1H), 8.38 - 8.22 (m, 3H), 8.10 (t, *J* = 9.3 Hz, 1H), 7.80 (d, *J* = 8.1 Hz, 1H), 5.13 (m, 4H), 4.56 (s, 2H), 4.05 - 3.90 (m, 4H), 3.55 - 3.40 (m, 6H) 2.60 - 2.50 (m, 2H), 1.80 (t, *J* = 6.9 Hz, 3H) MS (ESI): m/z 589.6 [M+H]⁺.

### Example 18: (R)-N-(5-(2,3-Dihydrobenzo[b][1,4]dioxine-6-carboxamido)-2-fluorophenyl)-6-((3-hydroxypyrrolidin-1-yl)methyl)thieno[2,3-b]pyridine-2-carboxamide (Example 18)

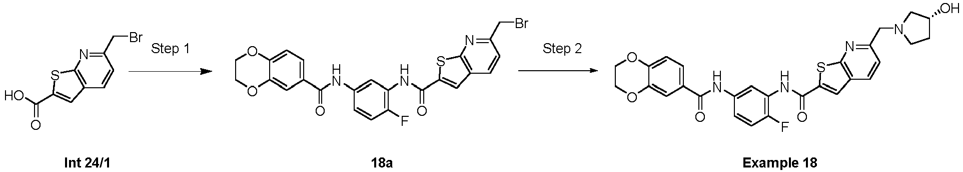

### Step 1: 6-(Bromomethyl)-N-(5-(2,3-dihydrobenzo[b][1,4]dioxine-6-carboxamido)-2-fluorophenyl)thieno[2,3-b]pyridine-2-carboxamide (18a)

The title compound was prepared similar as described for Example 3 using 6-(bromomethyl)thieno[2,3-b]pyridine-2-carboxylic acid **(Int 24/1)** in place of 6-((pyridin-2-yloxy)methyl)benzo[b]thiophene-2-carboxylic acid and N-(3-amino-4-fluorophenyl)-2,3-dihydrobenzo[b][1,4]dioxine-6-carboxamide **(Int 2)** in place of *N*-(3-amino-4-methylphenyl)-2,3-dihydrobenzo[b][1,4]dioxine-6-carboxamide.

### Step 2: (R)-N-(5-(2,3-dihydrobenzo[b][1,4]dioxine-6-carboxamido)-2-fluorophenyl)-6-((3-hydroxypyrrolidin-1-yl)methyl)thieno[2,3-b]pyridine-2-carboxamide (Example 18)

To a solution of 6-(bromomethyl)-N-(5-(2,3-dihydrobenzo[b][1,4]dioxine-6-carboxamido)-2-fluorophenyl)thieno[2,3-b]pyridine-2-carboxamide **(18a)** (10.0 mg, 0.018 mmol) in dry DMF (0.184 mL) was added Et₃N (0.003 mL, 0.020 mmol) and (R)-pyrrolidin-3-ol (1.7 mg, 0.019 mmol). The mixture was stirred at 60 °C for 18 h. The mixture was concentrated to dryness and the residue was purified by preparative HPLC to afford the title compound as a white solid. ¹H NMR (300 MHz, CD₃OD): δ ppm 8.35 (d, *J* = 8.2 Hz, 1H), 8.18 - 8.10 (m, 2H), 7.61 - 7.51 (m, 2H), 7.49 - 7.41 (m, 2H), 7.21 (t, *J =* 9.5 Hz, 1H), 6.93 (d, *J* = 8.2 Hz, 1H), 4.48 (s, 1H), 4.35 (s, 2H), 4.32 - 4.25 (m, 4H), 3.32 - 3.17 (m, 2H), 3.16 - 2.96 (m, 2H), 2.32 - 2.11 (m, 1H), 1.99 - 1.86 (m, 1H). MS (ESI): m/z 549.5 [M+H]⁺.

### Example 18/1: (S)-N-(5-(2,3-Dihydrobenzo[b][1,4]dioxine-6-carboxamido)-2-fluorophenyl)-6-((3-hydroxypyrrolidin-1-yl)methyl)thieno[2,3-b]pyridine-2-carboxamide (Example 18/1)

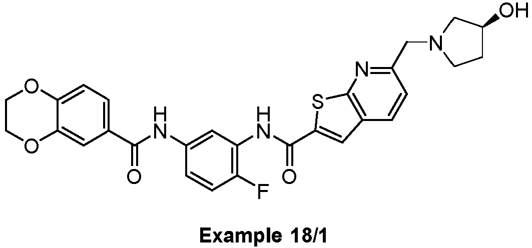

The title compound was prepared similar as described for Example **18** using in step 2 (*S*)-pyrrolidin-3-ol in place of (*R*)-pyrrolidin-3-ol. ¹H NMR (300 MHz, CD₃OD): δ ppm: 8.38 (d, *J =* 8.2 Hz, 1H), 8.20 - 8.10 (m, 2H), 7.62 - 7.52 (m, 2H), 7.50 - 7.43 (m, 2H), 7.22 (t, *J =* 9.5 Hz, 1H), 6.94 (d, *J =* 8.2 Hz, 1H), 4.52 (s, 1H), 4.45 (s, 2H), 4.34 - 4.27 (m, 4H), 3.48 - 3.34 (m, 2H), 3.30 - 3.08 (m, 2H), 2.34 - 2.18 (s, 1H), 2.02 - 1.93 (m, 1H). MS (ESI): m/z 549.5 [M+H]⁺.

### Example 18/2: N-(5-(2,3-Dihydrobenzo[b][1,4]dioxine-6-carboxamido)-2-fluorophenyl)-6-((4-hydroxypiperidin-1-yl)methyl)thieno[2,3-b]pyridine-2-carboxamide (Example 18/2)

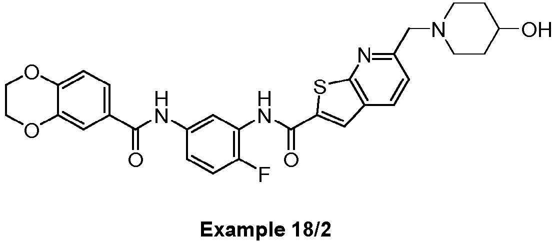

The title compound was prepared similar as described for Example **18** using in step 2 piperidin-4-ol in place of (3*R*)-3-pyrrolidinol. ¹H NMR (300 MHz, CD₃OD): δ ppm 8.37 (d, *J =* 7.5 Hz, 1H), 8.19 - 8.10 (m, 2H), 7.63 - 7.51 (m, 2H), 7.50 - 7.43 (m, 2H), 7.21 (t, *J* = 9.6 Hz, 1H), 6.94 (d, *J* = 8.3 Hz, 1H), 4.34 - 4.25 (m, 4H), 4.21 (s, 2H), 3.88 - 3.77 (m, 1H), 3.26 - 3.15 (m, 2H), 2.88 - 2.76 (m, 2H), 2.06 - 1.93 (m, 2H), 1.83 - 1.67 (m, 2H). MS (ESI): m/z 563.5 [M+H]⁺.

### Example 19: N-(5-(2,3-Dihydrobenzo[b][1,4]dioxine-6-carboxamido)-2-fluorophenyl)-6-(2-(pyrrolidin-1-yl)ethoxy)thieno[2,3-b]pyridine-2-carboxamide (Example 19)

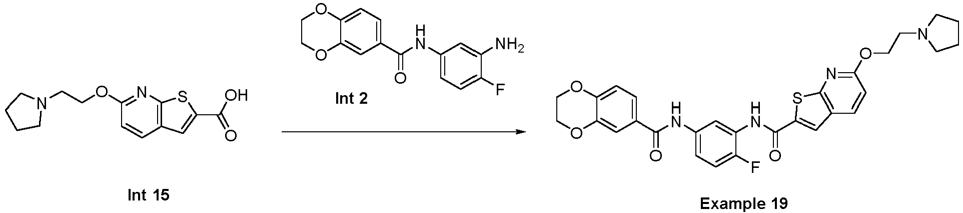

To a solution of 6-(2-(pyrrolidin-1-yl)ethoxy)thieno[2,3-b]pyridine-2-carboxylic acid **(Int 15)** (260 mg, 0.89 mmol) and *N*-(3-amino-4-fluorophenyl)-2,3-dihydrobenzo[b][1,4]dioxine-6-carboxamide (Int 2) (257 mg, 0.89 mmol) in acetonitrile (10 mL) were added NMI (255 mg, 3.11 mmol) and TCFH (448 mg, 1.6 mmol), and the mixture was stirred at 25 °C for 3 h. The mixture was poured into water. The mixture was extracted with EtOAc (3 x 10 ml ). The combined organic layers were washed with brine, dried over anhydrous Na₂SO₄, filtered and concentrated to dryness. The residue was purified by reverse-phase column chromatography (Solvent A: H₂O, Solvent B: MeCN) to afford the title compound. ¹H NMR (400 MHz, CD₃OD): δ ppm 8.23 (d, *J* = 8.8 Hz, 1H), 8.14 (dd, *J* = 6.8, 2.4 Hz, 1H), 8.08 (s, 1H), 7.56 - 7.52 (m, 1H), 7.49 - 7.45 (m, 2H), 7.25 - 7.17 (m, 1H), 7.00 (d, *J* = 8.8 Hz, 1H), 6.94 (d, *J* = 8.0 Hz, 1H), 4.78 - 4.71 (m, 2H), 4.35 - 4.27 (m, 4H), 3.63 - 3.56 (m, 2H), 3.38 - 3.34 (m, 4H), 2.10 - 2.06 (m, 4H). MS (ESI): m/z 563.7 [M+H]⁺.

### Example 19/1: N-(5-(2,3-Dihydrobenzo[b][1,4]dioxine-6-carboxamido)-2-fluorophenyl)-6-(3-(pyrrolidin-1-yl)propoxy)thieno[2,3-b]pyridine-2-carboxamide (Example 19/1)

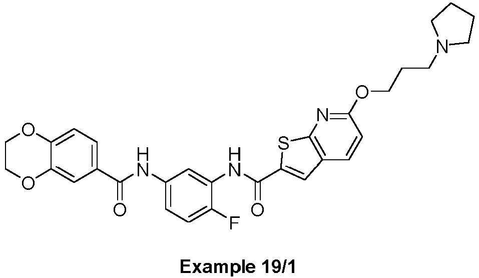

The title compound was prepared similar as described for Example **19** using 6-(3-(pyrrolidin-1-yl)propoxy)thieno[2,3-b]pyridine-2-carboxylic acid **(Int 15/1)** in place of 6-(2-(pyrrolidin-1-yl)ethoxy)thieno[2,3-b]pyridine-2-carboxylic acid **(Int 15).** ¹H NMR (400 MHz, DMSO-*d*₆): δ ppm 10.45 (s, 1H), 10.18 (s, 1H), 8.35 - 8.29 (m, 2H), 8.13-8.11 (m, 1H), 7.63 - 7.50 (m, 3H), 7.32 - 7.28 (m, 1H), 7.01 - 6.97 (m, 2H), 4.46 (t, *J* = 4.0 Hz, 2H), 4.33 -4.30 (m, 4H), 3.70 - 3.40 (m, 2H), 3.33-3.30 (m, 2H), 3.20 - 2.98 (m, 2H), 2.20 - 2.15 (m, 2 H), 2.08 - 2.95 (m, 4H). MS (ESI): m/z 577.6 [M+H]⁺.

### Example 19/2: N-(5-(2,3-Dihydrobenzo[b][1,4]dioxine-6-carboxamido)-2-fluorophenyl)-6-(2-(4-ethylpiperazin-1-yl)ethyl)thieno[2,3-b]pyridine-2-carboxamide (Example 19/2)

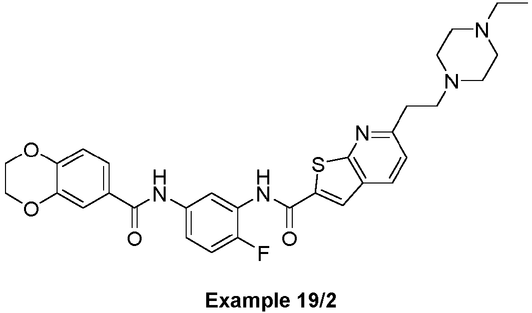

The title compound was prepared similar as described for Example **19** using 6-(2-(4-ethylpiperazin-1-yl)ethyl)thieno[2,3-b]pyridine-2-carboxylic acid **(Int 19)** in place of 6-(2-(pyrrolidin-1-yl)ethoxy)thieno[2,3-b]pyridine-2-carboxylic acid **(Int 15).** ¹H NMR (400 MHz, DMSO-*d*₆): δ ppm 10.52 (s, 1H), 10.18 (s, 1H), 8.34 - 8.31 (m, 2H), 8.11-8.09 (m, 1H), 7.54 - 7.53 (m, 1H), 7.47 - 7.45 (m, 3H), 7.30 (t, *J* = 8 Hz, 1H), 7.01 (d, *J* = 8 Hz, 1H), 4.32 - 4.31 (m, 4H), 3.06 - 3.02 (m, 2H), 2.73-2.70 (m, 2H), 2.34 - 2.26 (m, 10H), 0.98 (t, *J* = 8 Hz, 3H). MS (ESI): m/z 590.1 [M+H]⁺.

### Example 19/3: N-(5-(2,3-Dihydrobenzo[b][1,4]dioxine-6-carboxamido)-2-fluorophenyl)-6-((1-ethylpiperidin-4-yl)methyl)thieno[2,3-b]pyridine-2-carboxamide (Example 19/3)

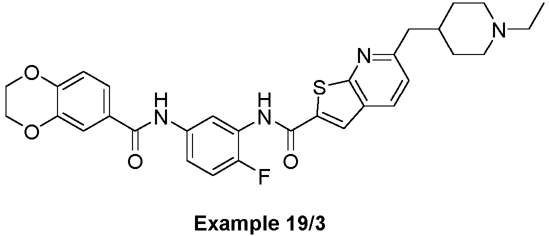

The title compound was prepared similar as described for Example **19** using 6-((1-ethylpiperidin-4-yl)methyl)thieno[2,3-b]pyridine-2-carboxylic acid **(Int 20)** in place of 6-(2-(pyrrolidin-1-yl)ethoxy)thieno[2,3-b]pyridine-2-carboxylic acid **(Int 15).** ¹H NMR (400 MHz, DMSO-*d*₆): δ ppm 10.52 (s, 1H), 10.19 (s, 1H), 8.33 - 8.31 (m, 2H), 8.12-8.11 (m, 1H), 7.69 - 7.67 (m, 1H), 7.66 - 7.53 (m, 2H), 7.51-7.31 (m, 2H), 7.28 - 6.99 (m, 1H), 4.33 - 4.30 (m, 4H), 2.83 - 2.79 (m, 4H), 2.27 - 2.24 (m, 2H), 1.82 - 1.76 (m, 3H), 1.57 - 1.54 (m, 2H), 1.29 - 1.24 (m, 2H), 0.96 (t, *J =* 8 Hz, 3H). MS (ESI): m/z 575.2 [M+H]⁺.

### Example 20: N-(5-(2,3-Dihydrobenzo[b][1,4]dioxine-6-carboxamido)-2-fluorophenyl)-6-((4-ethylpiperazin-1-yl)methyl)imidazo[2,1-b]thiazole-2-carboxamide (Example 20)

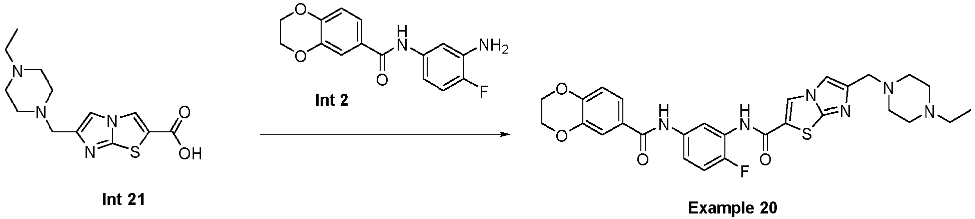

To a solution of 6-((4-ethylpiperazin-1-yl)methyl)imidazo[2,1-b]thiazole-2-carboxylic acid **(Int 21)** (0.7 g, 2.4 mmol) and N-(3-amino-4-fluorophenyl)-2,3-dihydrobenzo[b][1,4]dioxine-6-carboxamide **(Int 2)** (490 mg, 1.7 mmol) in DMF (10 mL) was added triethylamine (0.3 ml, 2.1 mmol) and HATU (323 mg, 0.85 mmol). The mixture was stirred at 50 °C for 6 h. The mixture was concentrated to dryness and the residue was purified by reverse phase column chromatography (H₂O/MeOH = 40:60) to afford a crude product that was further purified by preparative HPLC to afford the title compound as white solid. ¹H NMR (400 MHz, CD₃OD): δ ppm 8.57 (s, 1H), 8.11 (dd, *J* = 6.9, 2.6 Hz, 1H), 7.72 (s, 1H), 7.60 - 7.45 (m, 3H), 7.24 - 7.17 (m, 1H), 6.94 (d, *J =* 8.3 Hz, 1H), 4.36 - 4.24 (m, 4H), 3.64 (s, 2H), 2.80 - 2.43 (m, 10H), 1.10 (t, *J* = 7.2 Hz, 3H). MS (ESI): m/z 565.2 [M+H]⁺.

### Example 20/1: N-(4-Fluoro-3-(6-((pyridin-2-yloxy)methyl)benzo[b]thiophene-2-carboxamido) phenyl)-2,3-dihydrobenzo[b][1,4]dioxine-6-carboxamide (Example 20/1)

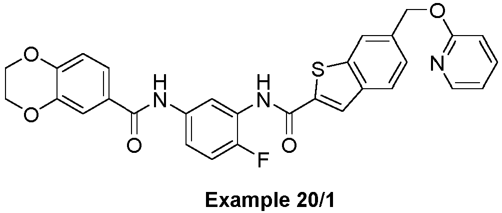

The title compound was prepared similar as described for Example **20** using 6-((pyridin-2-yloxy)methyl)benzo[b]thiophene-2-carboxylic acid **(Int 16)** in place of 6-((4-ethylpiperazin-1-yl)methyl)imidazo[2,1-b]thiazole-2-carboxylic acid **(Int 21).** ¹H NMR (400 MHz, DMSO-d₆): δ ppm 10.47 (s, 1H), 10.17 (s, 1H), 8.36 (s, 1H), 8.20 (dd, *J* = 5.0, 1.3 Hz, 1H), 8.14 (s, 1H), 8.11 - 8.08 (m, 1H), 8.02 (d, *J* = 8.3 Hz, 1H), 7.78 - 7.72 (m, 1H), 7.69 - 7.63 (m, 1H), 7.57 - 7.50 (m, 3H), 7.35 - 7.27 (m, 1H), 7.04 - 6.98 (m, 2H), 6.93 (d, *J =* 8.3 Hz, 1H), 5.50 (s, 2H), 4.33 - 4.30 (m, 4H). MS (ESI): m/z 556.2 [M+H]⁺.

### Example 21: N-(3-(6-(1-(4-Ethylpiperazin-1-yl)ethyl)benzo[b]thiophene-2-carboxamido)-4-fluorophenyl)-2,3-dihydrobenzo[b][1,4]dioxine-6-carboxamide (Example 21)

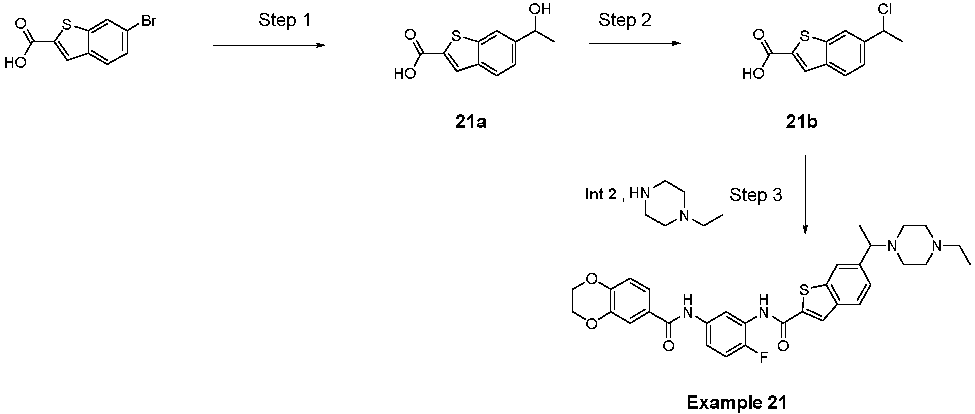

### Step 1: 6-(1-Hydroxyethyl)benzo[b]thiophene-2-carboxylic acid (21a)

To a solution of 6-bromobenzo[b]thiophene-2-carboxylic acid (50.0 mg, 0.194 mmol) in dry THF was added n-BuLi 2.5 M in hexane (0.163 mL, 0.408 mmol) at -78°C. The mixture was stirred at that temperature for 30 min. Acetaldehyde (0.009 mL, 0.214 mmol) as solution in 0.214 mL of dry THF was added dropwise. The mixture was allowed to slowly warm to 0 °C on ice bath and stirred for 1 h. Saturated aqueous NH₄Cl (1 mL) was added to the mixture. The mixture was diluted with H₂O (10 mL) and the pH was adjusted to ~2 with 1M HCl. The aqueous layer was extracted with EtOAc (2 x 20 mL). The combined organic layers were washed with brine, dried over MgSO₄, filtered and concentrated to dryness. The residue was purified by preparative HPLC to afford the title compound as a white solid.

### Step 2: 6-(1-Chloroethyl)benzo[b]thiophene-2-carboxylic acid (21b)

To a solution of 6-(1-hydroxyethyl)benzo[b]thiophene-2-carboxylic acid **(21a)** (4.5 mg, 0.020 mmol) in dry DCM (0.20 mL) was added SOCl₂ (3 µL, 0.040 mmol) and the mixture was refluxed for 4 h. The mixture was concentrated to dryness and the residue was used for the next step without further purification.

### Step 3: N-(3-(6-(1-(4-Ethylpiperazin-1-yl)ethyl)benzo[b]thiophene-2-carboxamido)-4-fluorophenyl)-2,3-dihydrobenzo[b][1,4]dioxine-6-carboxamide (Example 21)

To a solution of 6-(1-chloroethyl)benzo[b]thiophene-2-carboxylic acid **(21b)** (4.8 mg, 0.020 mmol) and *N*-(3-amino-4-fluorophenyl)-2,3-dihydrobenzo[b][1,4]dioxine-6-carboxamide **(Int 2)** (5.8 mg, 0.020 mmol) in dry DMF (0.1mL) was added TCFH (5.6 mg, 0.020 mmol) and NMI (0.006 mL, 0.070 mmol). The mixture was stirred for 30 min at rt. To the mixture was added 1-ethylpiperazine (0.005 mL, 0.040 mmol) and the mixture was stirred at 60 °C for 18 h. The mixture was concentrated to dryness and the residue was purified by preparative HPLC to afford the title compound as a white solid. ¹H NMR (300 MHz, CD₃OD): δ ppm: 8.18 - 8.07 (m, 2H), 7.95 - 7.89 (m, 2H), 7.60 - 7.53 (m, 1H) , 7.51 - 7.42 (m, 3H), 7.21 (t, *J* = 9.5 Hz, 1H), 6.94 (d, *J* = 8.2 Hz, 1H), 4.34-4.26 (m, 4H), 3.61 (q, *J* = 6.6 Hz, 1H), 2.84 - 2.47 (m, 10H), 2.17 (d, *J* = 7.3 Hz, 1H), 1.47 (d, *J* = 6.5 Hz, 3H),1.14 (t, *J* = 7.2 Hz, 3H). MS (ESI): m/z 589.6 [M+H]⁺.

### Example 22: N-(3-(6-(2-Aminoethoxy)benzo[b]thiophene-2-carboxamido)-4-methylphenyl)-2,3-dihydrobenzo[b][1,4]dioxine-6-carboxamide (Example 22)

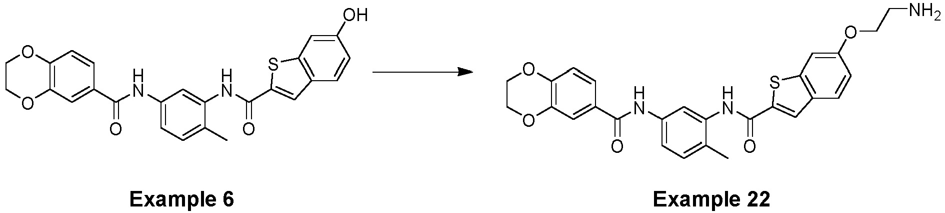

To a solution of *N*-(3-(6-hydroxybenzo[b]thiophene-2-carboxamido)-4-methylphenyl)-2,3-dihydrobenzo[b][1,4]dioxine-6-carboxamide **(Example 6)** (10.0 mg, 0.022 mmol) and K₂CO₃ (8.5 mg, 0.026 mmol) in DMF (0.217 mL) was added 2-(Boc-amino)ethyl bromide (5.4 mg, 0.024 mmol). The mixture was stirred at 60 °C for 18 h. The mixture was concentrated to dryness in vacuo and the residue was redissolved in EtOAc (20 mL). The resulting mixture was washed with saturated NH₄Cl (15 mL). The combined organic layers were dried over MgSO₄, filtered, and concentrated to dryness. The residue was dissolved in dry DCM (0.083 mL) and TFA (0.038 mL, 0.497 mmol) was added dropwise at 0 °C. The mixture was brought to rt and stirred for 3 h. The mixture was purified by preparative HPLC to afford the title compound as a white solid. ¹H NMR (300 MHz, CDCl₃): δ ppm 8.08 (s, 1H), 7.84 - 7.60 (m, 5H), 7.43 (s, 1H), 7.40 - 7.30 (m, 2H), 7.07 (d, *J* = 9.2 Hz, 1), 6.94 (d, *J* = 8.6 Hz, 1H), 4.37 - 4.24 (m, 4H), 4.13 - 4.02 (m, 2H), 3.15 (m, 2H), 2.35 (s, 3H). MS (ESI): m/z 504.5 [M+H]⁺.

### Example 23: 6-((4-Acetylpiperazin-1-yl)methyl)-N-(5-(2,3-dihydrobenzo[b][1,4]dioxine-6-carboxamido)-2-fluorophenyl)thieno[2,3-b]pyridine-2-carboxamide (Example 23)

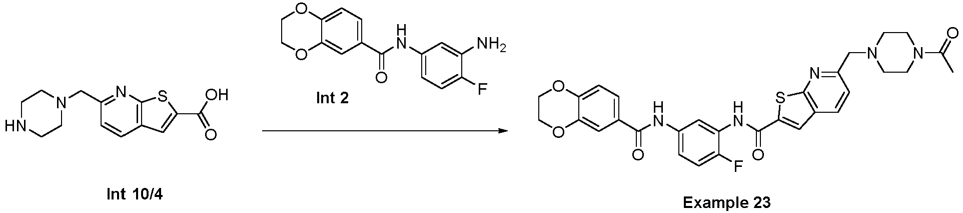

To a mixture of 6-(piperazin-1-ylmethyl)thieno[2,3-b]pyridine-2-carboxylic acid **(Int 10/4)** (30.0 mg, 0.108 mmol) in dry DMF (2mL) were added TEA (15 µL, 0.108 mmol) and acetyl chloride (8 µL, 0.108 mmol) and the mixture was stirred at rt for 2 h. N-(3-Amino-4-fluorophenyl)-2,3-dihydrobenzo[b][1,4]dioxine-6-carboxamide **(Int 2)** (31.2 mg, 0.108 mmol) and NMI (26 µL, 0.325 mmol) were added and the mixture was stirred at rt for 5 min. TCFH (30.4 mg, 0.108 mmol) was added portion-wise and the mixture was stirred at rt overnight. The mixture was concentrated to dryness and the residue was purified by preparative HPLC to afford the title compound. ¹H NMR (300 MHz, DMSO-d₆): δ ppm 10.55 (s, 1H), 10.17 (s, 1H), 8.48 - 8.31 (m, 2H), 8.15 - 8.10 (m, 1H), 7.68 - 7.61 (m, 2H), 7.55-7.50 (m, 2H), 7.30 (t, *J =* 9.7 Hz, 1H), 6.99 (d, *J* = 8.4 Hz, 1H), 4.35-4.27 (m, 4H), 3.86 - 3.70 (m, 2H), 3.46 (s, 2H), 3.25 - 3.02 (m, 2H), 2.47 - 2.28 (m, 4H), 2.00 (s, 3H). MS (ESI): m/z 590.5 [M+H]⁺.

### Example 24: N-(3-(6-(2-(4-Ethylpiperazin-1-yl)ethoxy)benzo[b]thiophene-2-carboxamido)-4-methylphenyl)-2,3-dihydrobenzo[b][1,4]dioxine-6-carboxamide (Example 24)

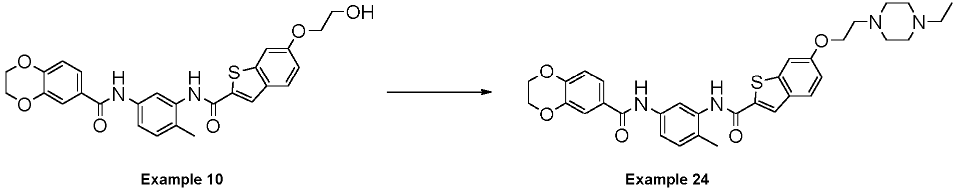

To a mixture of N-(3-(6-(2-hydroxyethoxy)benzo[b]thiophene-2-carboxamido)-4-methylphenyl)-2,3-dihydrobenzo[b][1,4]dioxine-6-carboxamide (30 mg, 0.059 mmol) **(Example 10)** in dry DMF (2 mL) was added sodium hydride (2.9 mg, 0.071 mmol) (60% suspension in mineral oil) and the mixture was stirred at rt for 30 min. Then *p*-TsCl (12.5 mg, 0.065 mmol) was added, and the mixture was stirred at rt for 30 min. 1-Ethylpiperazine (8 µL, 0.060 mmol) was added and the mixture was stirred at rt overnight. The mixture was concentrated to dryness and the residue was purified by preparative HPLC (with a gradient of 40-65% MeCN in water) to afford the title compound as a white solid. ¹H NMR (300 MHz, DMSO-d₆): δ ppm 10.06 (s, 1H), 10.03 (s, 1H), 8.22 (s, 1H), 7.86 (s, 1H), 7.63 (s, 1H), 7.55 - 7.51 (m, 1H), 7.48 - 7.45 (m, 2H), 7.23 (d, *J* = 8.1 Hz, 1H), 7.11 - 7.08 (m, 2H), 6.97 (d, *J =* 8.5 Hz, 1H), 4.34 - 4.27 (m, 4H), 4.23-4.17 (m, 2H), 3.18 - 2.76 (m, 8H), 2.74 - 2.70 (m, 2H), 2.29 - 2.27 (m, 2H), 2.21 (s, 3H), 1.21 - 1.10 (m, 3H). MS (ESI): m/z 601.7 [M+H]⁺.

### Example 25: N-(3-(Benzo[b]thiophene-2-carboxamido)-4-methylphenyl)-3,4-dihydro-2H-benzo[b][1,4]dioxepine-7-carboxamide (Example 25)

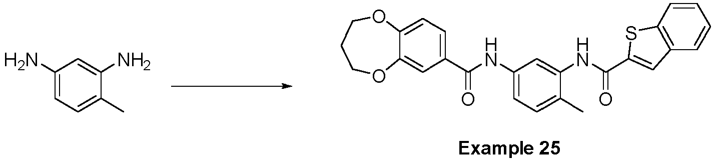

4-Methylbenzene-1,3-diamine (30 mg, 0.246 mmol), 3,4-dihydro-2*H*-benzo[*b*][1,4]dioxepine-7-carboxylic acid (47.7 mg, 0.246 mmol) and NMI (59 µL, 0.737 mmol) were dissolved in dry DMF (2 mL). TCFH (82.7 mg, 0.295 mmol) was added in portions, and the mixture was stirred at rt for 1 h. Benzo[b]thiophene-2-carboxylic acid (43.8 mg, 0.246 mmol) along with more TCHF (82.7 mg, 0.295 mmol) and NMI (82.7 mg, 0.295 mmol) were added and the mixture was stirred at rt for 2 h. The mixture was concentrated to dryness and the residue was purified by flash column chromatography (gradient cyclohexane/EtOAc 3:1) to afford the title compound as a white powder. ¹H NMR (300 MHz, DMSO-d₆): δ ppm 10.20 (s, 1H), 10.11 (s, 1H), 8.32 (s, 1H), 8.07 (d, *J* = 7.9 Hz, 1H), 8.01 (d, *J* = 7.6 Hz, 1H), 7.87 (s, 1H), 7.62 (d, *J =* 6.6 Hz, 2H), 7.58 (s, 1H), 7.50 -7.48 (m, 2H), 7.25 (d, *J* = 8.2*,* 1H), 7.07 (d, *J* = 8.2 Hz, 1H), 4.24 - 4.19 (m, 4H), 2.24 (s, 3H), 2.20 - 2.15 (m, 2H). MS (ESI): m/z 459.5 [M+H]⁺.

### Example 26: N-(3-(6-(3-(3-Hydroxyazetidin-1-yl)propoxy)benzo[b]thiophene-2-carboxamido)-4-methylphenyl)-2,3-dihydrobenzo[b][1,4]dioxine-6-carboxamide (Example 26)

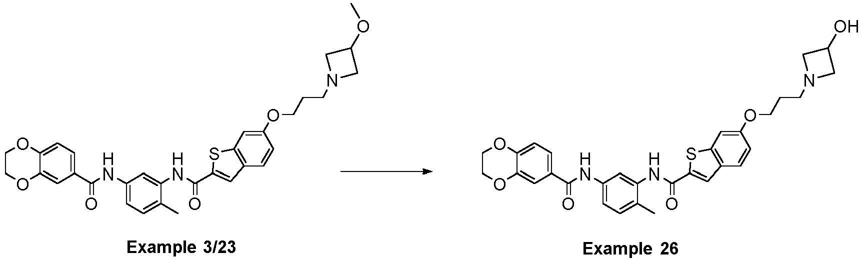

*N*-(3-(6-(3-(3-Methoxyazetidin-1-yl)propoxy)benzo[*b*]thiophene-2-carboxamido)-4-methylphenyl)-2,3-dihydrobenzo[*b*][1,4]dioxine-6-carboxamide **(Example 3/23)** (30.0 mg, 0.05 mmol) was dissolved in dry DCM (2 mL) and the mixture was stirred at -78 °C. Boron tribromide (204 µL, 0.20 mmol) was added dropwise. The dry ice/acetone bath was removed and the mixture was stirred at rt for 3 h. The reaction was quenched with water. The mixture was extracted with EtOAc (3 x 25 mL). The combined organic layers were concentrated to dryness and the residue was purified by preparative HPLC (20-40% MeCN in water) to afford the title compound. ¹H NMR (300 MHz, DMSO-d₆): δ ppm 10.04 (s, 2H), 8.21 (s, 1H), 7.86 (s, 1H), 7.83 (s, 1H), 7.59 (s, 1H), 7.56 (s, 1H), 7.52 (s, 1H), 7.49 (s, 1H), 7.22 (d, *J* = 8.3 Hz, 1H), 7.05 (d, *J* = 8.8 Hz, 1H), 6.97 (d, *J =* 8.3 Hz, 1H), 4.34-4.27 (m, 4H), 4.20 - 4.12 (m, 1H), 4.07 (t, *J =* 6.2 Hz, 2H), 3.54 (t, *J* = 6.3 Hz, 2H), 2.71 (t, *J* = 6.2 Hz, 2H), 2.57 - 2.53 (m, 2H), 2.22 (s, 3H), 1.80 - 1.71 (m, 2H). MS (ESI): m/z 574.7 [M+H]⁺.

### Example 27: N-(5-(2,3-Dihydrobenzo[b][1,4]dioxine-6-carboxamido)-2-fluorophenyl)-6-((4-isopropylpiperazin-1-yl)methyl)thieno[2,3-b]pyridine-2-carboxamide (Example 27)

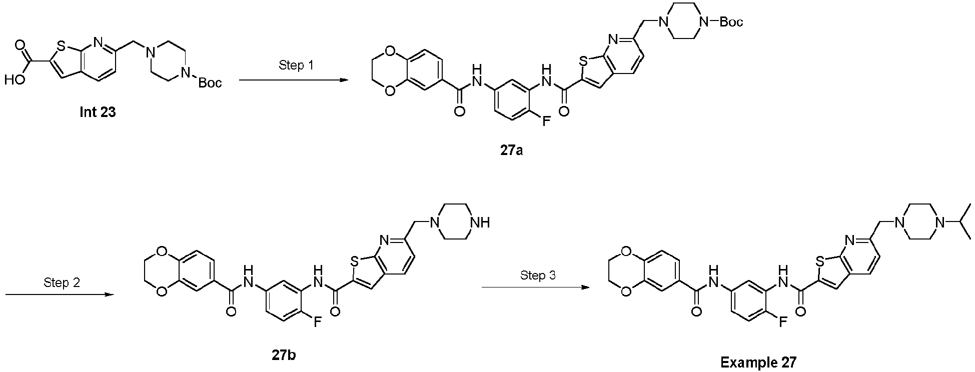

### Step 1: tert-Butyl-4-((2-((5-(2,3-dihydrobenzo[b][1,4]dioxine-6-carboxamido)-2-fluorophenyl)carbamoyl)thieno[2,3-b]pyridin-6-yl)methyl)piperazine-1-carboxylate (27a)

6-((4-(*tert*-Butoxycarbonyl)piperazin-1-yl)methyl)thieno[2,3-b]pyridine-2-carboxylic acid **(Int 23)** (21 mg, 0.056 mmol), *N*-(3-Amino-4-fluorophenyl)-2,3-dihydrobenzo[b][1,4]dioxine-6-carboxamide **(Int 2)** (16 mg, 0.056 mmol) and NMI (13 µL, 0.167 mmol) were dissolved in dry DMF (2 mL). TCFH (18.7 mg, 0.067 mmol) was added in portions, and the mixture was stirred at rt for 1 h. The mixture was concentrated to dryness and the residue was purified by preparative HPLC to afford the title compound.

### Step 2: N-(5-(2,3-Dihydrobenzo[b][1,4]dioxine-6-carboxamido)-2-fluorophenyl)-6-(piperazin-1-ylmethyl)thieno[2,3-b]pyridine-2-carboxamide (27b)

*tert*-Butyl-4-((2-((5-(2,3-dihydrobenzo[*b*][1,4]dioxine-6-carboxamido)-2-fluorophenyl) carbamoyl)thieno[2,3-b]pyridin-6-yl)methyl)piperazine-1-carboxylate **(27a)** (36 mg, 0.056 mmol) was dissolved in DCM (2 mL) and stirred at 0 °C. TFA was added to the mixture until the ratio was DCM/TFA = 2:1. The mixture was stirred at rt for 2 h. The mixture was concentrated to dryness to afford the title compound.

### Step 3: N-(5-(2,3-Dihydrobenzo[b][1,4]dioxine-6-carboxamido)-2-fluorophenyl)-6-((4-isopropylpiperazin-1-yl)methyl)thieno[2,3-b]pyridine-2-carboxamide (Example 27)

*N*-(5-(2,3-Dihydrobenzo[*b*][1,4]dioxine-6-carboxamido)-2-fluorophenyl)-6-(piperazin-1-ylmethyl)thieno[2,3-b]pyridine-2-carboxamide **(27b)** (30 mg, 0.055 mmol), cesium carbonate (35.7 mg, 0.110 mmol) and 2-bromopropane (6 µL, 0.06 mmol) were dissolved in dry DMF (2 mL) and the mixture was stirred at rt overnight. The mixture was concentrated to dryness and the residue was purified by preparative HPLC to afford the title compound. ¹H NMR (300 MHz, DMSO-d₆): δ ppm 10.51 (s, 1H), 10.16 (s, 1H), 8.38 (d, *J* = 8.4 Hz, 1H), 8.32 (s, 1H), 8.10 (d, *J* = 3.5 Hz, 1H), 7.68 - 7.63 (m, 1H), 7.59 (d, *J* = 8.4 Hz, 1H), 7.53 (s, 1H), 7.50 (s, 1H), 7.31 (t, *J =* 9.2 Hz, 1H), 6.99 (d, *J* = 8.4 Hz, 1H), 4.34 - 4.28 (m, 4H), 3.71 (s, 2H), 2.66 - 2.56 (m, 1H), 2.46 (s, 8H), 0.96 (d, *J* = 6.5 Hz, 6H). MS (ESI): m/z 590.6 [M+H]⁺.

### Example 27/1: N-(5-(2,3-Dihydrobenzo[b][1,4]dioxine-6-carboxamido)-2-fluorophenyl)-6-((1R,4R)-5-ethyl-2,5-diazabicyclo[2.2.1]heptan-2-yl)methyl)thieno[2,3-b]pyridine-2-carboxamide (Example 27/1)

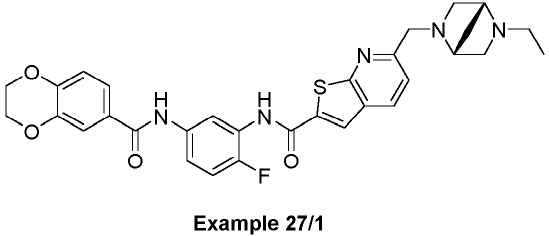

The title compound was prepared similar as described for Example **27,** using in step 1 6-(((1*R*,4*R*)-5-(*tert*-butoxycarbonyl)-2,5-diazabicyclo[2.2.1]heptan-2-yl)methyl)thieno[2,3-b]pyridine-2-carboxylic acid **(Int 23/1)** instead of 6-((4-(*tert*-butoxycarbonyl)piperazin-1-yl)methyl)thieno[2,3-b]pyridine-2-carboxylic acid **(Int 23)** and in step 3 ethyl iodide in place of 2-bromopropane. ¹H NMR (300 MHz, DMSO-d₆): δ ppm 10.51 (s, 1H), 10.16 (s, 1H), 8.38 (d, *J =* 8.3 Hz, 1H), 8.32 (s, 1H), 8.21 (s, 1H), 8.10 (d, *J* = 6.2 Hz, 1H), 7.68-7.61 (m, 2H), 7.53 (s, 1H), 7.50 (s, 1H), 7.29 (t, *J =* 9.4 Hz, 1H), 6.99 (d, *J =* 8.3 Hz, 1H), 4.34 - 4.28 (m, 4H), 3.99 - 3.83 (m, 2H), 3.42 (s, 1H), 2.81 - 2.61 (m, 6H), 1.74 - 1.64 (m, 2H) 1.01 (t, *J* = 7.1 Hz, 3H). MS (ESI): m/z 588.6 [M+H]⁺.

### Example 27/2: N-(5-(2,3-Dihydrobenzo[b][1,4]dioxine-6-carboxamido)-2-fluorophenyl)-6-(((1S,4S)-5-ethyl-2,5-diazabicyclo[2.2.1]heptan-2-yl)methyl)thieno[2,3-b]pyridine-2-carboxamide (Example 27/2)

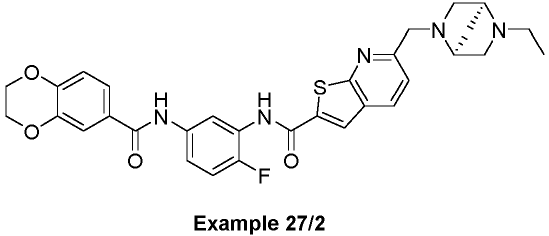

The title compound was prepared similar as described for Example **27,** using in step 1 6-(((1S,4S)-5-(*tert*-butoxycarbonyl)-2,5-diazabicyclo[2.2.1]heptan-2-yl)methyl)thieno[2,3-b]pyridine-2-carboxylic acid **(Int 23/2)** instead of **Int 23** and in step 3 ethyl iodide in place of 2-bromopropane. ¹H NMR (300 MHz, DMSO-d₆): δ ppm 10.51 (s, 1H), 10.16 (s, 1H), 8.38 (d, *J* = 8.3 Hz, 1H), 8.32 (s, 1H), 8.21 (s, 1H), 8.10 (d, *J* = 6.2 Hz, 1H), 7.68 - 7.61 (m, 1H), 7.53 (s, 1H), 7.50 (s, 1H), 7.29 (t, *J* = 9.4 Hz, 1H), 6.99 (d, *J* = 8.3 Hz, 1H), 4.34 -4.27 (m, 4H), 3.99-3.83 (m, 2H), 3.42 (s, 1H), 2.81-2.61 (m, 6H), 1.74 - 1.64 (m, 2H), 1.01 (t, *J* = 7.1 Hz, 3H). MS (ESI): m/z 588.5 [M+H]⁺.

### Example 27/3: N-(5-(2,3-Dihydrobenzo[b][1,4]dioxine-6-carboxamido)-2-fluorophenyl)-6-((4-ethyl-1,4-diazepan-1-yl)methyl)thieno[2,3-b]pyridine-2-carboxamide (Example 27/3)

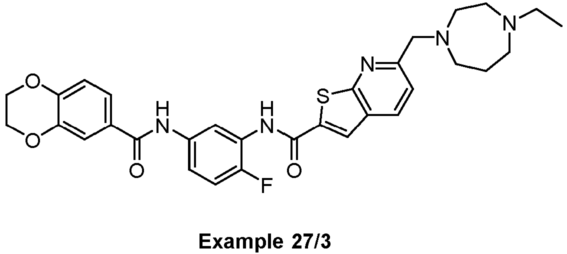

The title compound was prepared similar as described for Example **27,** using in step 1 6-((4-(*tert*-butoxycarbonyl)-1,4-diazepan-1-yl)methyl)thieno[2,3-b]pyridine-2-carboxylic acid **(Int 23/3)** in place of **Int 23** and in step 3 ethyl iodide in place of 2-bromopropane. ¹H NMR (300 MHz, DMSO-d₆): δ ppm 10.51 (s, 1H), 10.16 (s, 1H), 8.39 (d, *J* = 8.2 Hz, 1H), 8.32 (s, 1H), 8.17 (s, 1H), 8.11 (d, *J =* 6.5 Hz, 1H), 7.68 - 7.60 (m, 1H), ), 7.53 (s, 1H), 7.49 (s, 1H), 7.29 (t, *J* = 9.6 Hz, 1H), 6.99 (d, *J* = 8.2 Hz, 1H), 4.35 - 4.27 (m, 4H), 3.89 (s, 2H), 2.83-2.60 (m, 10 H), 1.83 -1.76 (m, 2H), 1.03 (t, *J* = 7.1 Hz, 3H). MS (ESI): m/z 590.6 [M+H]⁺.

### Example 28: N-(4-Fluoro-3-(6-(((3aR,6aS)-hexahydropyrrolo[3,4-c]pyrrol-2(1H)-yl)methyl) benzo[b]thiophene-2-carboxamido)phenyl)-2,3-dihydrobenzo[b][1,4]dioxine-6-carboxamide (Example 28)

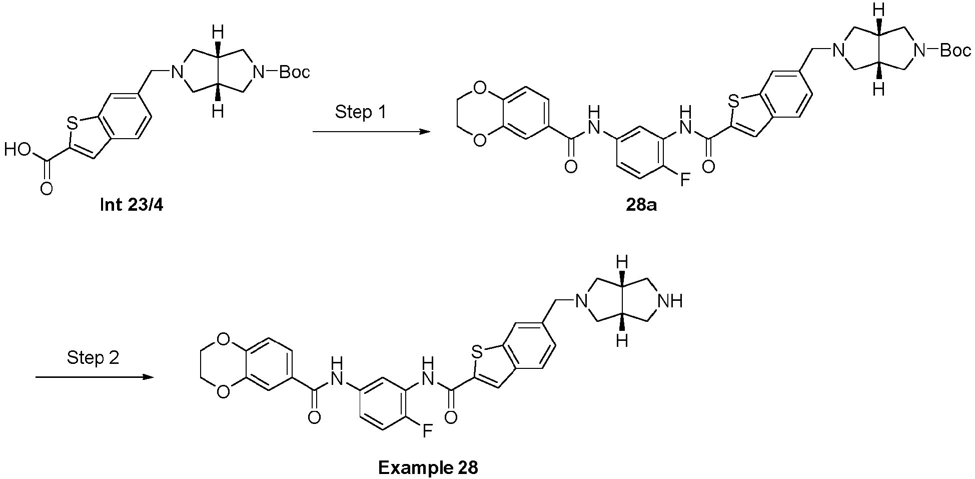

### Step 1: (3aR,6aS)-tert-Butyl 5-((2-((5-(2,3-dihydrobenzo[b][1,4]dioxine-6-carboxamido)-2-fluorophenyl)carbamoyl)benzo[b]thiophen-6-yl)methyl)hexahydropyrrolo[3,4-c]pyrrole-2(1H)-carboxylate (28a)

6-(((3aR,6aS)-5-(*tert*-butoxycarbonyl)hexahydropyrrolo[3,4-c]pyrrol-2(1*H*)-yl)methyl)benzo[b]thiophene-2-carboxylic acid **(Int 23/4)** (22.5 mg, 0.056 mmol), *N*-(3-Amino-4-fluorophenyl)-2,3-dihydrobenzo[b][1,4]dioxine-6-carboxamide **(Int 2)** (16.1 mg, 0.056 mmol) and NMI (13 µL, 0.168 mmol) were dissolved in dry DMF (2 mL). TCFH (18.7 mg, 0.067 mmol) was added portion-wise, and the mixture was stirred at rt for 1 h. The mixture was concentrated to dryness and the residue was purified by preparative HPLC to afford the title compound.

### Step 2: N-(4-fluoro-3-(6-(((3aR,6aS)-hexahydropyrrolo[3,4-c]pyrrol-2(1H)-yl)methyl) benzo[b]thiophene-2-carboxamido)phenyl)-2,3-dihydrobenzo[b][1,4]dioxine-6-carboxamide (Example 28)

(3aR,6aS)-*tert*-Butyl5-((2-((5-(2,3-dihydrobenzo[b][1,4]dioxine-6-carboxamido)-2-fluorophenyl)carbamoyl)benzo[b]thiophen-6-yl)methyl)hexahydropyrrolo[3,4-c]pyrrole-2(1H)-carboxylate (28a) (37.5 mg, 0.056 mmol) was dissolved in DCM and stirred at 0 °C. To the mixture was added TFA until the ratio is DCM:TFA = 2:1. The mixture was then stirred at rt for 2 h. The mixture was concentrated to dryness and the residue was purified by preparative HPLC to afford the title compound. ¹H NMR (300 MHz, DMSO-d₆): δ ppm 10.39 (s, 1H), 10.15 (s, 1H), 8.33 (s, 1H), 8.10 (d, *J =* 7.1 Hz, 1H), 7.94 (s ,1H), 7.92 (s, 1H), 7.69 - 7.62 (m, 1H), 7.53 (s, 1H), 7.49 (s, 1H), 7.42 (d, *J* = 8.0 Hz, 1H), 7.29 (t, *J* = 9.5 Hz, 1H), 6.98 (d, *J* = 8.4 Hz, 1H), 4.35 - 4.27 (m, 4H), 3.64 (s, 2H), 2.80 - 2.71 (m, 2H), 2.60 - 2.51 (m, 6H), 2.32 - 2.21 (m, 2H). MS (ESI): m/z 573.5 [M+H]⁺.

### Example 29: N-(3-(6-((1-Ethylpiperidin-4-yl)(hydroxy)methyl)benzo[b]thiophene-2-carboxamido)-4-fluorophenyl)-2,3-dihydrobenzo[b][1,4]dioxine-6-carboxamide (Example 29)

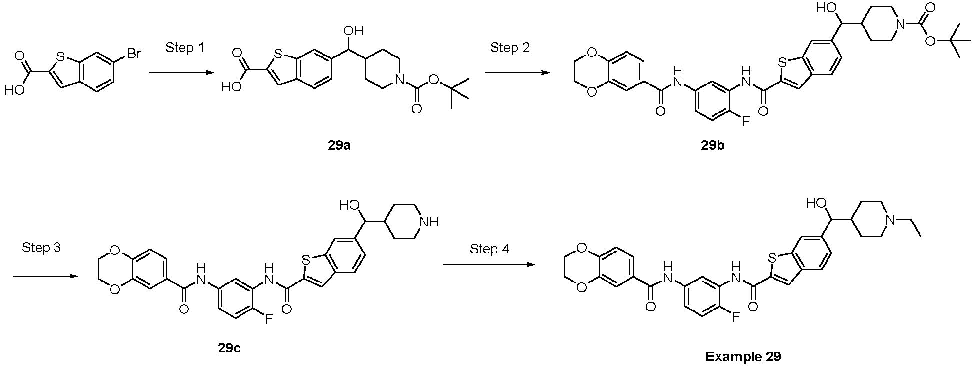

### Step 1: 6-((1-(tert-Butoxycarbonyl)piperidin-4-yl)(hydroxy)methyl)benzo[b]thiophene-2-carboxylic acid (29a)

To a mixture of 6-bromobenzo[b]thiophene-2-carboxylic acid (100 mg, 0.389 mmol) in dry THF (2 mL) methyl magnesium bromide (3M solution in Et₂O, 0.130 mL, 0.39 mmol) was added. The mixture was stirred for 30 min at rt and then cooled to -78 °C. To the mixture was added n-BuLi (2.5 M solution in hexane (0.31 mL, 0.78 mmol) and the mixture was stirred at -78 °C for 1 h. Then *tert*-butyl 4-formylpiperidine-1-carboxylate (91.3 mg, 0.428 mmol) was added and the mixture was stirred at 0 °C overnight. The mixture was concentrated to dryness and the residue was purified by preparative HPLC to afford the title compound.

### Step 2: tert-Butyl 4-((2-((5-(2,3-Dihydrobenzo[b][1,4]dioxine-6-carboxamido)-2-fluorophenyl) carbamoyl)benzo[b]thiophen-6-yl)(hydroxy)methyl)piperidine-1-carboxylate (29b)

**Int 2** (14.7 mg, 0.051 mmol), 6-((1-(tert-butoxycarbonyl)piperidin-4-yl)(hydroxy)methyl) benzo[b]thiophene-2-carboxylic acid (29a) (20.0 mg, 0.051 mmol) and NMI (14 µL, 0.179 mmol) were dissolved in dry DMF (2 mL). Then TCFH (14.3 mg, 0.051 mmol) was added in portions, and the mixture was stirred at rt for 1 h. The mixture was concentrated to dryness and the residue was purified by preparative HPLC to afford the title compound as a white powder.

### Step 3: N-(4-Fluoro-3-(6-(hydroxy(piperidin-4-yl)methyl)benzo[b]thiophene-2-carboxamido) phenyl)-2,3-dihydrobenzo[b][1,4]dioxine-6-carboxamide (29c)

To a mixture of *tert*-butyl 4-((2-((5-(2,3-dihydrobenzo[b][1,4]dioxine-6-carboxamido)-2-fluorophenyl)carbamoyl)benzo[b]thiophen-6-yl)(hydroxy)methyl)piperidine-1-carboxylate **(29b)** (33.7 mg, 0.051 mmol) in dry DCM (510 µL) was added TFA (100 µL) at 0 °C dropwise. The mixture was allowed to warm to rt. Saturated NaHCO₃ (10 mL) was added to the mixture. The aqueous layer was extracted with DCM (3 x 15 mL). The combined organic layers were dried over MgSO₄, filtered, and concentrated to dryness to afford the title compound.

### Step 4: N-(3-(6-((1-Ethylpiperidin-4-yl)(hydroxy)methyl)benzo[b]thiophene-2-carboxamido)-4-fluorophenyl)-2,3-dihydrobenzo[b][1,4]dioxine-6-carboxamide (Example 29)

To a mixture of *N*-(4-fluoro-3-(6-(hydroxy(piperidin-4-yl)methyl)benzo[b]thiophene-2-carboxamido)phenyl)-2,3-dihydrobenzo[b][1,4]dioxine-6-carboxamide (29c) (10.0 mg, 0.018 mmol) in dry DMF (1 mL) was added sodium cyanoborohydride (1.1 mg, 0.018 mmol). The mixture was cooled to 0 °C. Acetaldehyde (1 µL, 0.018 mmol) was added and the mixture was stirred at 0 °C for 15 min. NaHCO₃ (2 mL) was added and the mixture was extracted with EtOAc (10 mL). The organic layer was concentrated to dryness and the residue was purified by preparative HPLC to afford the title compound. ¹H NMR (300 MHz, CD₃OD): δ ppm 8.17 - 8.13 (m, 2H), 7.97 - 7.94 (m, 2H), 7.61-7.55 (m, 1H), 7.50 - 7.45 (m, 3H), 7.23 (t, *J* = 9.6 Hz, 1H), 6.96 (d, *J* = 8.3 Hz, 1H), 4.58 - 4.54 (m, 2H), 4.36 - 3.29 (m, 4H), 3.22-3.14 (m, 1H), 2.76 - 2.66 (m, 2H), 2.41 - 2.25 (m, 2H), 2.11 - 2.06 (m, 1H), 1.91 - 1.79 (m, 1H), 1.62 - 1.44 (m, 3H), 1.19 (t, *J =* 7.1 Hz, 3H). MS (ESI): m/z 590.6 [M+H]⁺

### Example 30: N-(4-Fluoro-3-(6-(2-hydroxy-3-(pyrrolidin-1-yl)propoxy)benzo[b]thiophene-2-carboxamido)phenyl)-2,3-dihydrobenzo[b][1,4]dioxine-6-carboxamide (racemate) (Example 30)

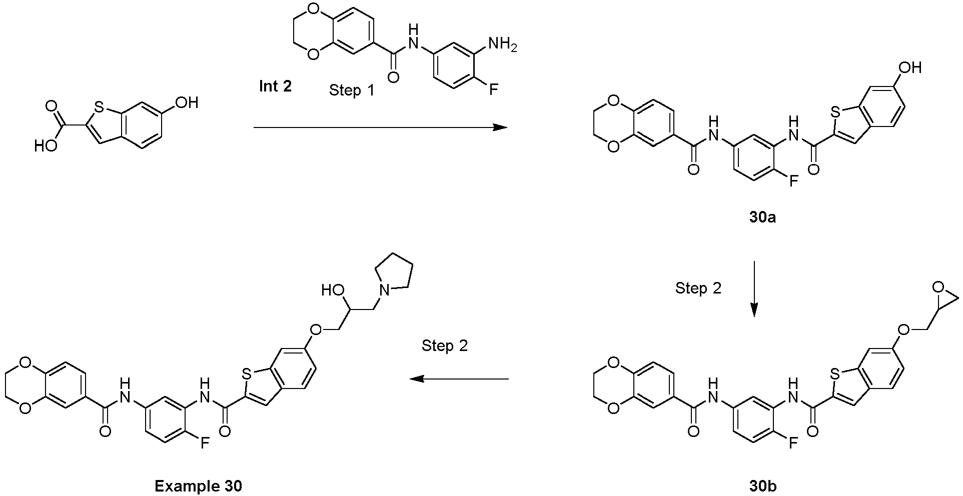

### Step 1: N-(4-Fluoro-3-(6-hydroxybenzo[b]thiophene-2-carboxamido)phenyl)-2,3-dihydrobenzo[b][1,4]dioxine-6-carboxamide (30a)

To a mixture of 6-hydroxybenzo[b]thiophene-2-carboxylic acid (2.0 g, 10.3 mmol), *N*-(3-amino-4-fluorophenyl)-2,3-dihydrobenzo[b][1,4]dioxine-6-carboxamide **(Int 2)** (800 mg, 2.8 mmol) and TEA (28 mg, 0.28 mmol) in DMF (50 mL) was added HATU (3.0 g, 7.9 mmol) and the mixture was stirred at 50 °C for 2 h. To the mixture was added KOH (313 mg, 5.6 mmol) as an aqueous solution. The mixture was extracted with ethyl acetate (3 x 100 mL). The combined organic layers were washed with brine (3 x 100 mL), dried over Na₂SO₄, filtered and concentrated to dryness. The residue was purified by column chromatography on silica gel (PE/EtOAc = 10:1) to afford the title compound as a brown solid.

### Step 2: N-(4-Fluoro-3-(6-(oxiran-2-ylmethoxy)benzo[b]thiophene-2-carboxamido)phenyl)-2,3-dihydrobenzo[b][1,4]dioxine-6-carboxamide (30b)

A mixture of *N*-(4-fluoro-3-(6-hydroxybenzo[b]thiophene-2-carboxamido)phenyl)-2,3-dihydrobenzo[b][1,4]dioxine-6-carboxamide **(30a)** (210 mg, 0.45 mmol) in DMF (10 mL) was cooled to 0 °C. NaH (22 mg, 0.91 mmol) was added and the mixture was stirred at 0 °C for 0.5 h. 2-(Chloromethyl)oxirane (54 mg, 0.58 mmol) was added and the mixture was stirred at 60 °C for 4 h. Water was added (100 mL) and the mixture was extracted with ethyl acetate (3 x 100 mL). The combined organic layers were washed with brine (3 x 100 mL), dried over Na₂SO₄, filtered and concentrated to dryness to afford the title compound which was used in the next step without further purification.

### Step 3: N-(4-Fluoro-3-(6-(2-hydroxy-3-(pyrrolidin-1-yl)propoxy)benzo[b]thiophene-2-carboxamido)phenyl)-2,3-dihydrobenzo[b][1,4]dioxine-6-carboxamide (racemate) (Example 30)

To a mixture of *N*-(4-fluoro-3-(6-(oxiran-2-ylmethoxy)benzo[b]thiophene-2-carboxamido)ph enyl)-2,3-dihydrobenzo[b][1,4]dioxine-6-carboxamide **(30b)** (210 mg, 0.40 mmol), TEA (40 mg, 40 mmol) in ethanol (5 mL) was added pyrrolidine (142 mg, 2.00 mmol). The mixture was stirred under microwave irradiation at 80 °C for 2 h. The mixture was concentrated to dryness and the residue was purified by column chromatography on silica gel (DCM/MeOH = 15:1) to afford a crude product as a white solid. The crude product was purified by preparative HPLC to give the title compound as a white solid. ¹H NMR (400 MHz, DMSO-d₆ ): δ ppm 10.34 (s, 1H), 10.17 (s, 1H), 8.27 (s, 1H), 8.10 - 8.08 (m, 1H), 7.88 (d, *J* = 8.9 Hz, 1H), 7.68 - 7.61 (m, 2H), 7.55 - 7.48 (m, 2H), 7.31 - 7.26 (m, 1H), 7.11 - 7.08 (m, 1H), 6.99 (d, *J* = 8.4 Hz, 1H), 4.95 (broad s, 1H), 4.34 - 4.29 (m, 4H), 4.11 - 4.07 (m, 1H), 4.00 - 3.94 (m, 2H), 2.70-2.63 (m, 1H), 2.53 - 2.46 (m, 5H), 1.70 - 1.67 (m, 4H). MS (ESI): m/z 592.2 [M+H]⁺.

### Example 30/1: N-(4-Fluoro-3-(6-(2-hydroxy-3-(pyrrolidin-1-yl)propoxy)benzo[b]thiophene-2-carboxamido)phenyl)-2,3-dihydrobenzo[b][1,4]dioxine-6-carboxamide (single enantiomer) (Example 30/1)

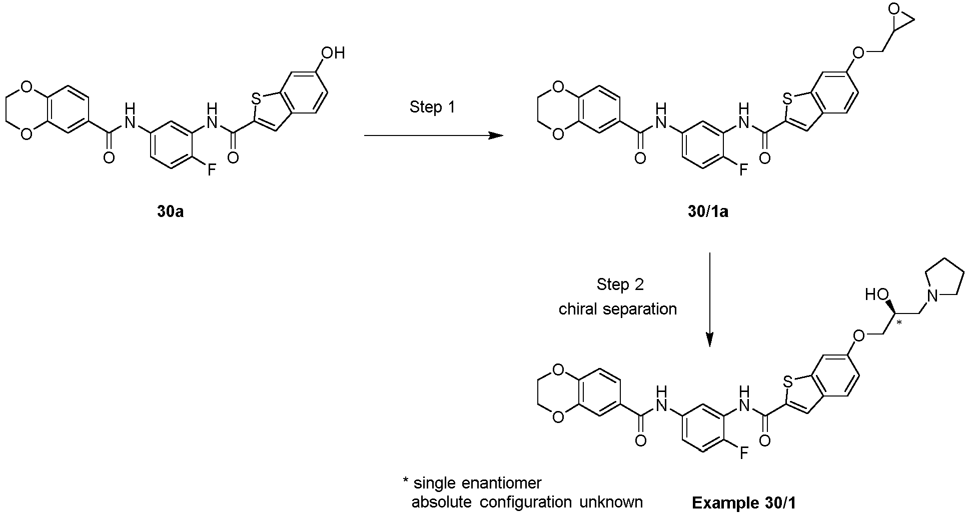

### Step 1: N-(4-Fluoro-3-(6-(oxiran-2-ylmethoxy)benzo[b]thiophene-2-carboxamido)phenyl)-2,3-dihydrobenzo[b][1,4]dioxine-6-carboxamide (30/1a)

To a solution of *N*-(4-fluoro-3-(6-hydroxybenzo[b]thiophene-2-carboxamido)phenyl)-2,3-dihydrobenzo[b][1,4]dioxine-6-carboxamide **(30a)** (1.0 g, 2.16 mmol) in DMF (10 mL) was added NaH (172 mg, 60% dispersion in mineral oil, 4.3 mmol) under nitrogen atmosphere at 0 °C. (S)-2-(chloromethyl)oxirane (297 mg, 3.21 mmol) was added to the mixture. The mixture was stirred at 60 °C for 1.5 h. Water was added (100 mL) and the mixture was extracted with DCM (2 x 100 mL). The combined organic layers were dried over Na₂SO₄, filtered and concentrated to dryness to afford the title compound which was used in the next step without further purification.

### Step 2: N-(4-Fluoro-3-(6-(2-hydroxy-3-(pyrrolidin-1-yl)propoxy)benzo[b]thiophene-2-carboxamido)phenyl)-2,3-dihydrobenzo[b][1,4]dioxine-6-carboxamide (single enantiomer) (30/1)

A solution of *N*-(4-fluoro-3-(6-(oxiran-2-ylmethoxy)benzo[b]thiophene-2-carboxamido)phenyl)-2,3-dihydrobenzo[b][1,4]dioxine-6-carboxamide **(30/1a)** (400 mg, 0.77 mmol) and pyrrolidine (56 mg, 0.79 mmol) in EtOH (10 mL) was stirred at 70 °C overnight. The mixture was concentrated to dryness and the residue was purified by silica gel chromatography (DCM/MeOH = 20:1) to give a crude product, which was further purified by reversed-phase column (CH₃CN/ H₂O = 5%-95%), followed by chiral SFC (column: CHIRALCEL OD-3, 4.6*100 mm, co-solvent: MeOH/acetonitrile (0.2% 7M NH₃)) to give the title compound as an off white solid (second eluting enantiomer, retention time = 1.9 min). ¹H NMR (400 MHz, DMSO-d₆): δ ppm 10.36 (s, 1H), 10.18 (s, 1H), 8.27 (s, 1H), 8.11-8.09 (m, 1H), 7.88 (d, *J* = 8 Hz, 1H), 7.67-7.63 (m, 2H), 7.54 - 7.50 (m, 2H), 7.31 - 7.27(m, 1H), 7.11 - 7.08 (m, 1H), 7.00 (d, *J =* 8 Hz, 1H), 4.96 - 4.95 (m, 1H), 4.33 - 4.30 (m, 4H), 4.11 - 4.08 (m, 1H), 3.99 - 3.95 (m, 2H), 2.66 - 2.62 (m, 1H), 2.50 - 2.43 (m, 5H), 1.71 - 1.65 (m, 4H). MS (ESI): m/z 592.3 [M+H]⁺.

### Example 30/2: N-(4-Fluoro-3-(6-(2-hydroxy-3-(pyrrolidin-1-yl)propoxy)benzo[b]thiophene-2-carboxamido)phenyl)-2,3-dihydrobenzo[b][1,4]dioxine-6-carboxamide (single enantiomer) (Example 30/2)

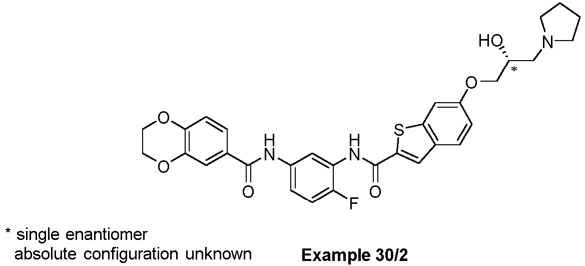

The title compound was prepared similar as described for Example **30/1,** using in step 1 (*R*)-2-(chloromethyl)oxirane in place of (*S*)-2-(chloromethyl)oxirane. Final purification by chiral SFC (column: CHIRALCEL OD-3 (4.6*100 mm), co-solvent: MeOH/acetonitrile (0.2% 7M NH3)) to give the title compound as an off white solid (first eluting enantiomer, retention time = 1.5 min). ¹H NMR (400 MHz, DMSO-d*₆*): δ ppm 10.35 (s, 1H), 10.18 (s, 1H), 8.27 (s, 1H), 8.11 - 8.09 (m, 1H), 7.88 (d, *J* = 8 Hz, 1H), 7.67 - 7.63 (m, 2H), 7.54 - 7.50 (m, 2H), 7.31 - 7.26 (m, 1H), 7.11 - 7.08 (m, 1H), 7.00 (d, *J* = 8 Hz, 1H), 4.95 - 4.94 (m, 1H), 4.33 - 4.30 (m, 4H), 4.12 - 4.08 (m, 1H), 3.99 - 3.95 (m, 2H), 2.67 - 2.62 (m, 1H), 2.48 - 2.43 (m, 5H), 1.71 - 1.64 (m, 4H). MS (ESI): m/z 592.3 [M+H]⁺.

### Biological Assays

### Assay for inhibition of ATP production

The assay determines the amount of ATP in cells in culture.

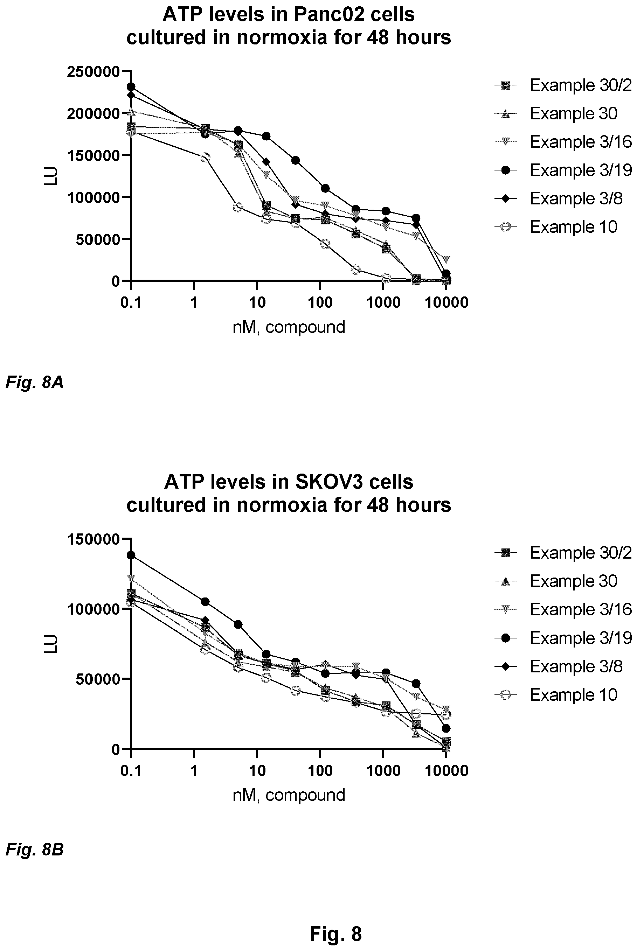

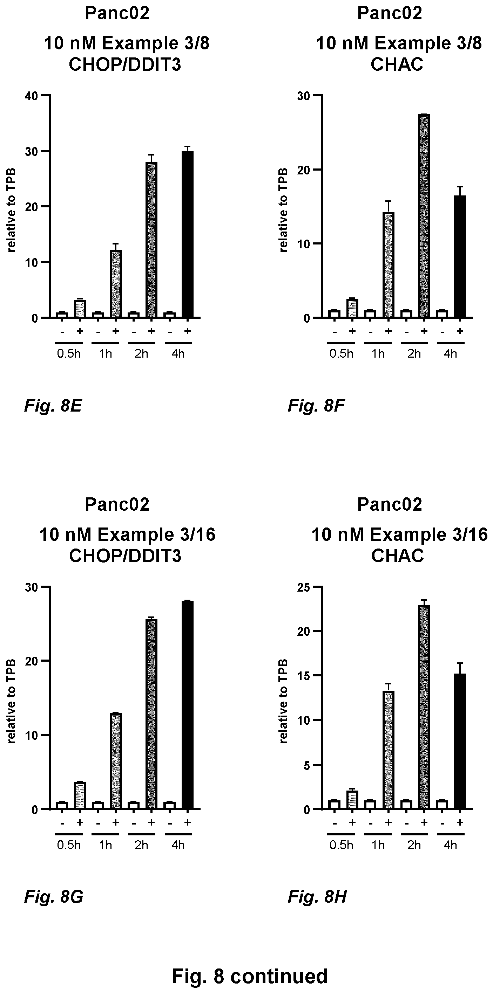

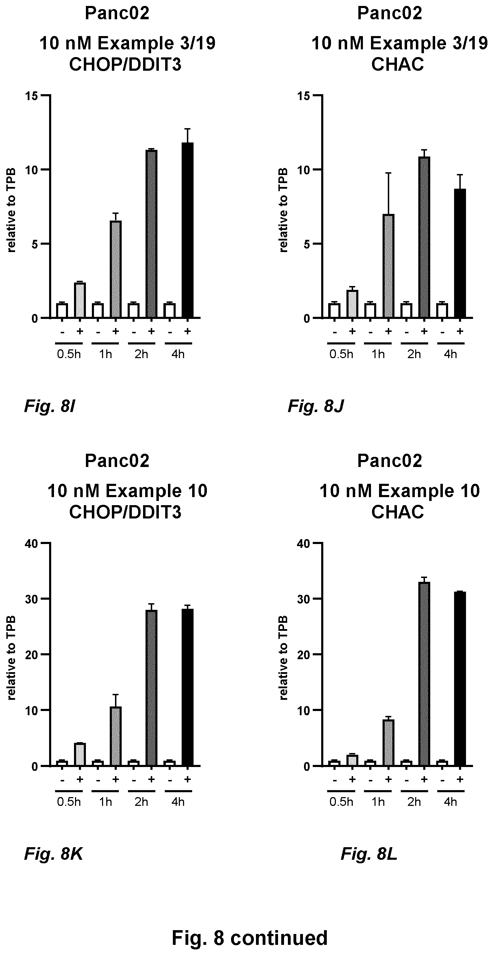

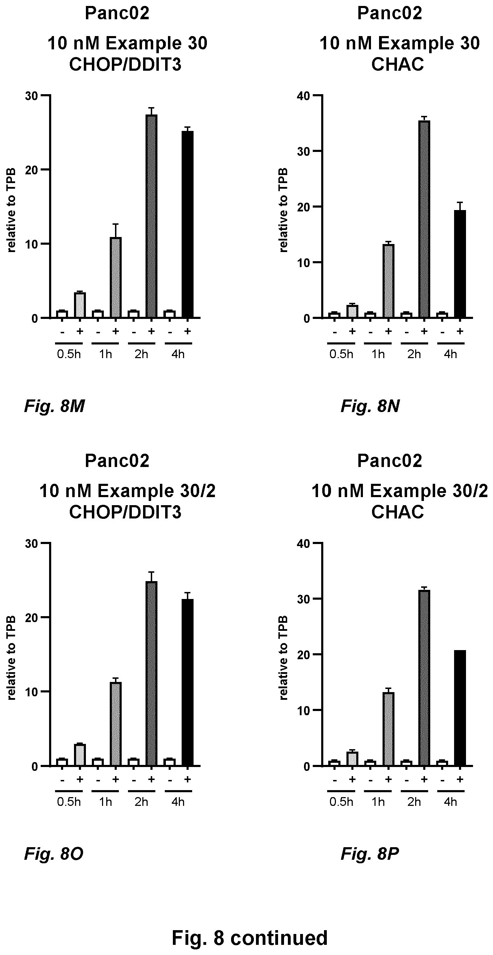

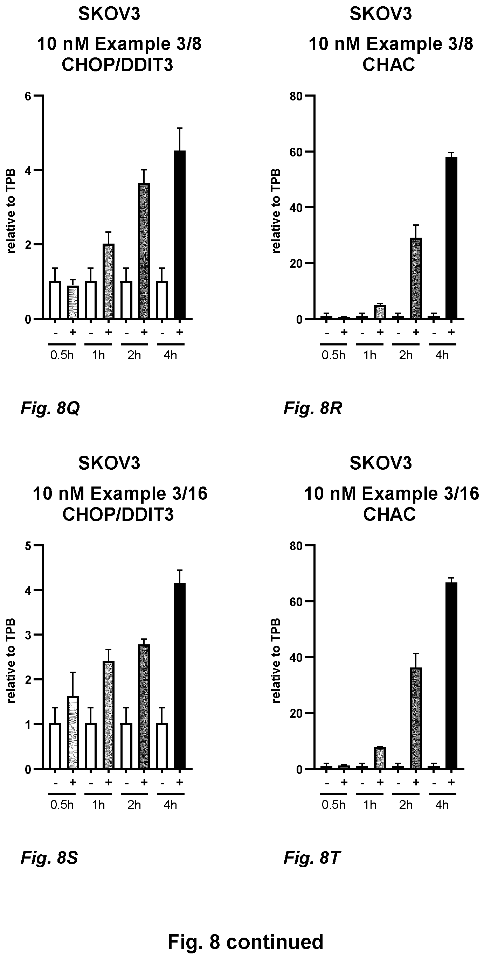

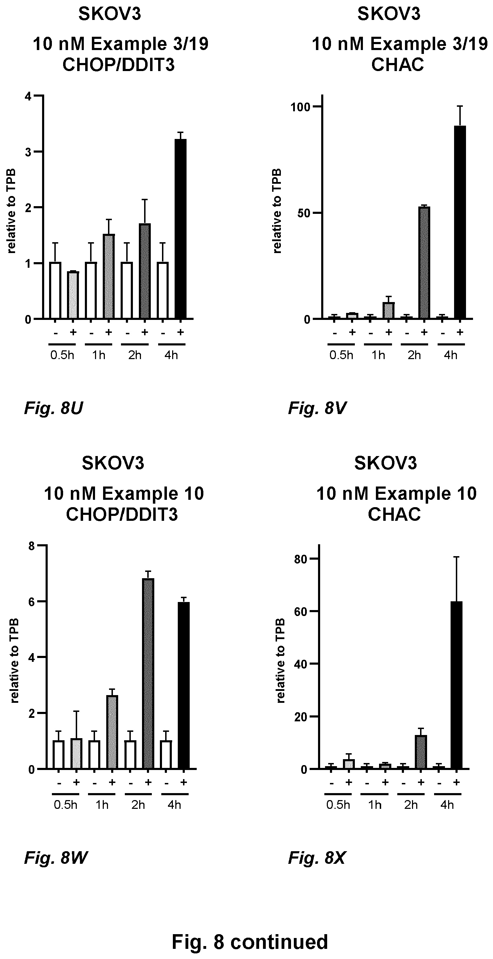

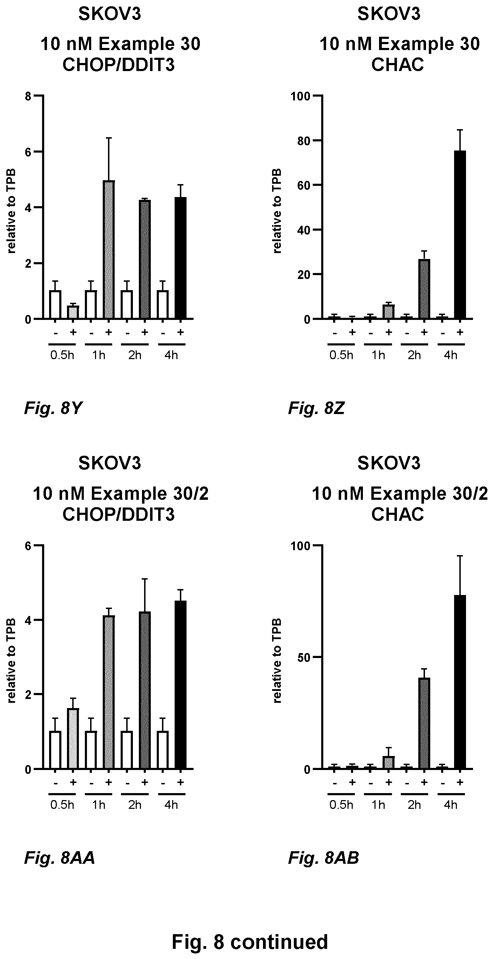

Cells were plated at 4,000 per well in 96-well plate in a medium of high glucose DMEM (PAN) supplemented with 10% FBS (PAN), 2 mM glutamine-alanine (PAN), 2 mM pyruvate (Sigma) and 10 mL/L penicillin-streptomycin and grown at 37 °C in 0.5% O₂, 5 % CO₂ and 75% humidity. For hypoxia, cells were grown and treated in the Whitley H35 Hypoxystation (Don Whitley Scientific) at 37 °C in 0.5% O₂, 5 % CO₂ and 75% humidity. The next day, cells were induced with test compounds (Figure 1: compounds of examples 3/16, 3/8, 10 at 10000, 3333, 1111, 370, 123, 41, 14, 5 and 1.5 nM; Figure 8A and 8B: compounds of example 3/19, 30 and 30/2 at 10000, 3333, 1111, 370, 123, 41, 14, 5 and 1.5 nM), as well as with vehicle (DMSO) only, for 48 hours. After this time, the medium was removed from the cells and intracellular ATP levels were determined using the Promega CellTiter-Glo 2.0 assay protocol (Promega, G9241). To estimate the effect of compounds on the ATP production, sample values were compared to the values of DMSO only. IC₅₀ values were generated by plotting the luciferase counts against compound concentration.

Fig. 1 encompasses several graphs which show the inhibition activity of the compounds of examples 3/16, 3/8 and 10 in HeLa, 4T1, LLC1, A549, MDA-MB-231 and MiaPaCa2 cells after 48 hours treatment. In all cases, a dose-dependent reduction of the ATP production level is observed.

Fig. 8A and 8B show the inhibition activity of the compounds of compounds of example 3/16, 3/8, 10, 3/19, 30 and 30/2 in Panc02 and SKOV3 cells after 48 hours treatment. In all cases, a dose-dependent reduction of the ATP production level is observed.

Numerical results are shown in Table 1 below, in which compound activities are grouped according to the following IC₅₀ ranges:

| | |
|---|---|
| + | 1 µM - 10 µM, |
| ++ | 100 nM - < 1 µM |
| +++ | < 100 nM |

### Lactate production inhibition assay

Recombinant Lactate Dehydrogenase A (LDHA) was used to convert lactate in tissue culture supernatants to pyruvate, with the concomitant reduction of NAD⁺ to NADH. The electron acceptor 1-methoxyphenazine methosulfate (MPMS) was then reduced by the generated NADH, thereby regenerating NAD⁺. Reduced 1-methoxyphenazine methosulfate is used in turn to reduce Water Soluble Tetrazolium 8 (WST8) to an orange WST8 formazan product. The assay is adapted from the publication "Application of WST-8 based colorimetric NAD(P)H detection for quantitative dehydrogenase assays" described in Chamchoy et al., BMC Biochemistry (2019) 20(1):4.

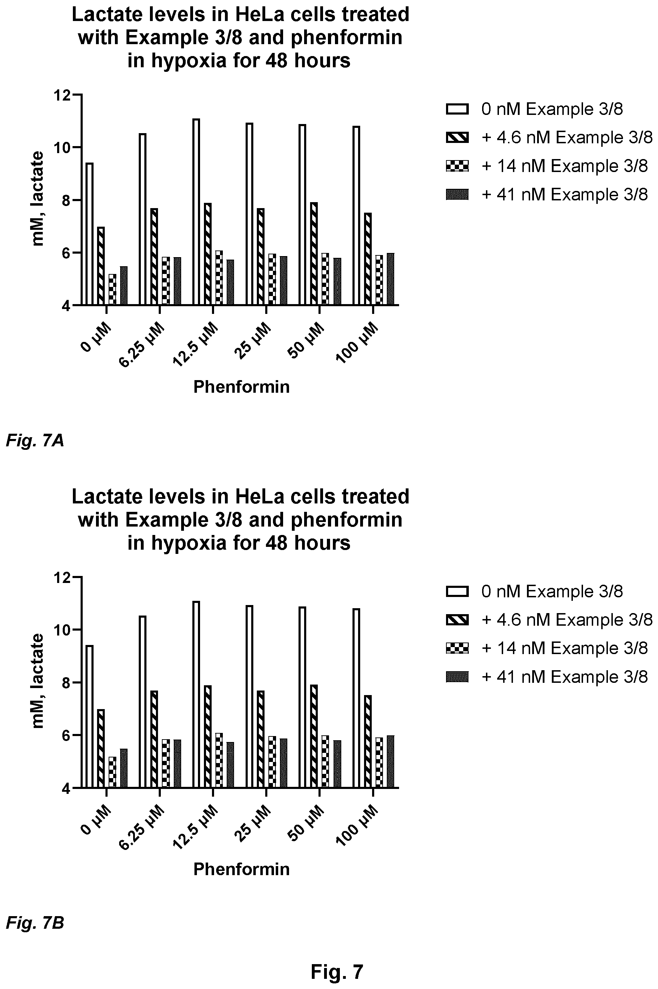

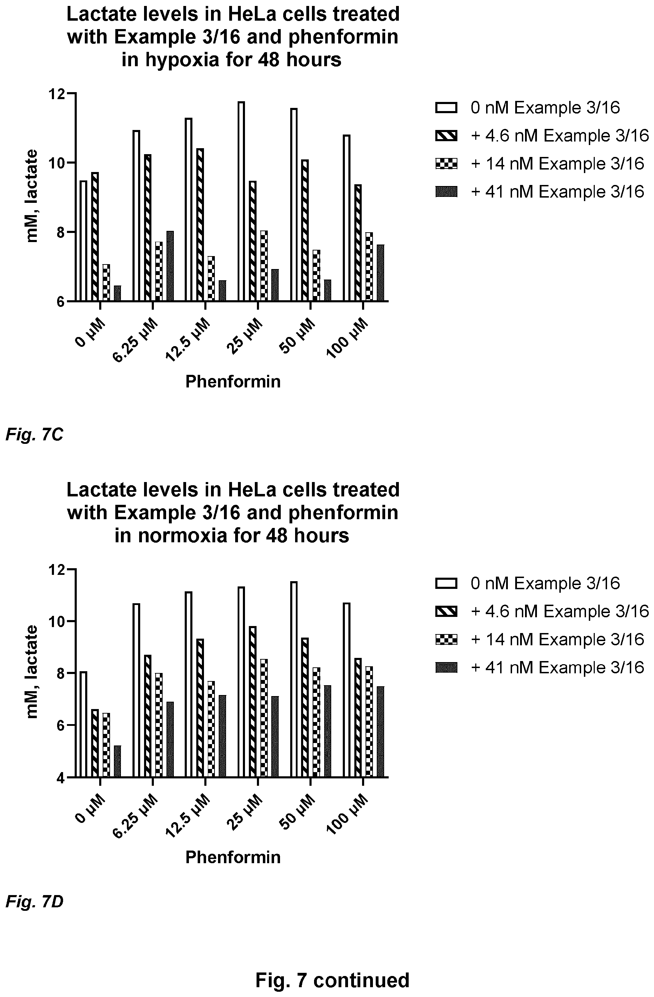

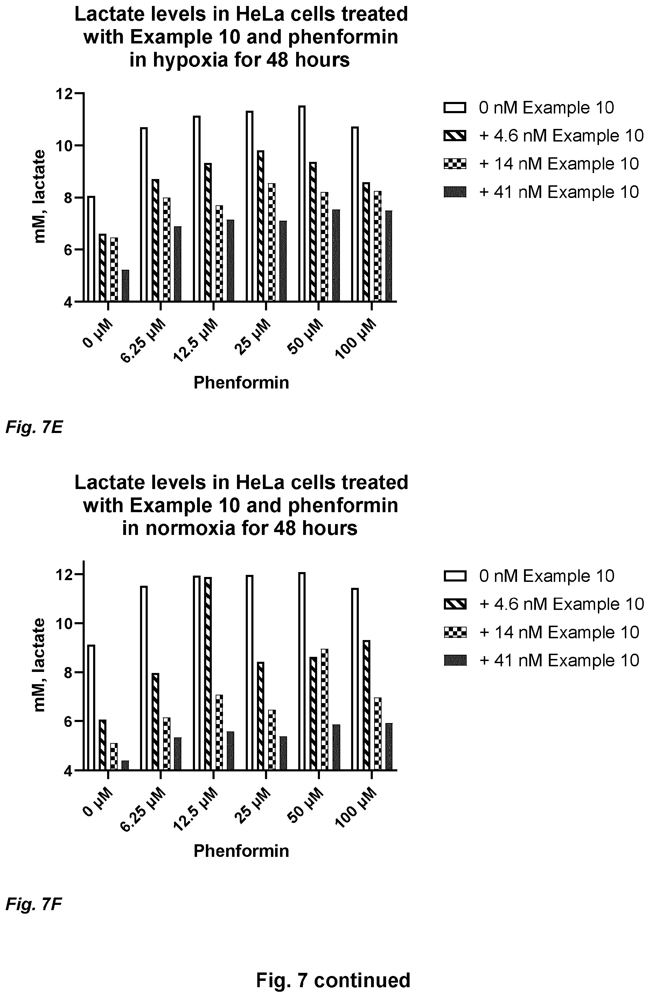

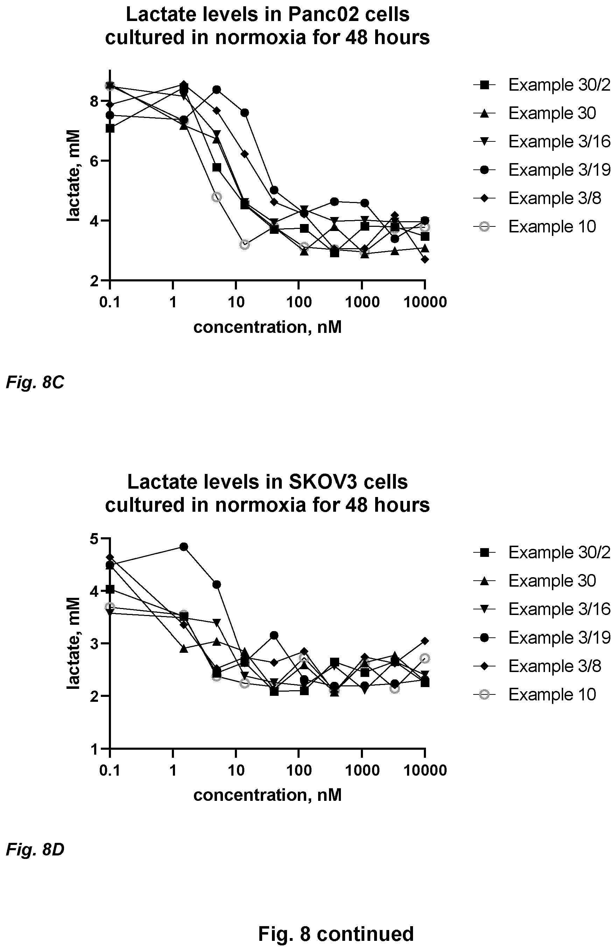

Cells were plated at 4,000 per well in 96-well plate in a medium of high glucose DMEM (PAN) supplemented with 10% FBS (PAN), 2 mM glutamine-alanine (PAN), 2 mM pyruvate (Sigma) and 10 mL/L penicillin-streptomycin (PAN) and grown at 37 °C in 0.5% O₂, 5 % CO₂ and 75% humidity. For hypoxia, cells were grown and treated in the Whitley H35 Hypoxystation (Don Whitley Scientific) at 37 °C in 0.5% O₂, 5 % CO₂ and 75% humidity. The next day, cells were induced with test compounds (Figure 2: compounds of examples 3/16, 3/8, 10 at 10000, 3333, 1111, 370, 123, 41, 14, 5 and 1.5 nM; Figure 7: compounds of examples 3/16, 3/8, 10 at 41, 14, 4.6 nM and Phenformin at 100, 50, 25, 12.5, 6.25 µM; Figure 8C and 8D: compounds of example 3/16, 3/8, 10, 3/19, 30 and 30/2 at 10000, 3333, 1111, 370, 123, 41, 14, 5 and 1.5 nM), as well as with vehicle (DMSO) only, for 48 hours. After this time, the medium was removed from the cells to measure extracellular lactate. To detect extracellular lactate, cell supernatants are diluted 1:5 in assay buffer (0.2 M tris, pH 8.2) by adding 10 µl cell supernatant + 40 µl buffer in a 96-well plate. Assay substrate mix is prepared by adding NAD+ (3.75 mM), WST-8 (0.9 mM), MPMS (10 mM) and recombinant LDHA, expressed in E. coli as an N-terminal hexahistidine tagged fusion protein, is added to a final concentration of 0.8 µg/ml in assay buffer. Thereafter, 80 µl of substrate mix is added to 20 µl of diluted cell supernatant in a 96-well flat-bottomed plate and the absorbance at 460 nm determined at one minute intervals over a 30 minute period. A standard curve of known lactate concentrations of 10, 8, 6, 4, 1, 0.5 and 0.25 mM was constructed for each assay plate.

Fig. 2 shows downregulation of lactate levels in HeLa, 4T1, LLC1, A549, MDA-MB-231 and MiaPaCa2 cells after 48 hours treatment with compounds of examples 3/16, 3/8, 10. In all cases, a dose-dependent reduction of the lactate production level is observed.

Fig. 7 shows downregulation of lactate levels in HeLa cells after 48 hours co-treatment of phenformin with compounds of examples 3/16, 3/8, 10.

Fig. 8C and 8D show a dose-dependent downregulation of lactate levels in Panc02 and SKOV3 cell after 48 hours treatment with compounds of example 3/16, 3/8, 10, 3/19, 30 and 30/2.

Numerical results are shown in Table 1 below, in which compound activities are grouped according to the following IC₅₀ ranges:

| | |
|---|---|
| + | 1 µM - 10 µM, |
| ++ | 100 nM - < 1000 nM |
| +++ | < 100 nM |

**Table 1**

| Above Example in which tested compound is described | IC₅₀ measured in assay measuring inhibition of lactate production | IC₅₀ measured in assay measuring inhibition of intracellular ATP |
|---|---|---|
| 1 | +++ | +++ |
| 1/1 | ++ | ++ |
| 1/2 | + | + |
| 1/3 | ++ | ++ |
| 1/4 | ++ | ++ |
| 1/5 | +++ | +++ |
| 1/6 | +++ | +++ |
| 1/7 | ++ | ++ |
| 1/8 | +++ | +++ |
| 1/9 | +++ | +++ |
| 1/10 | +++ | +++ |
| 1/11 | +++ | +++ |
| 1/12 | +++ | +++ |
| 1/13 | +++ | +++ |
| 1/14 | +++ | +++ |
| 1/15 | +++ | +++ |
| 1/16 | + | + |
| 1/17 | +++ | +++ |
| 1/18 | +++ | +++ |
| 1/19 | + | + |
| 2 | ++ | ++ |
| 2/1 | ++ | +++ |
| 3 | +++ | +++ |
| 3/1 | +++ | +++ |
| 3/2 | +++ | +++ |
| 3/3 | +++ | ++ |
| 3/4 | +++ | +++ |
| 3/5 | +++ | +++ |
| 3/6 | +++ | +++ |
| 3/7 | +++ | +++ |
| 3/8 | +++ | +++ |
| 3/9 | +++ | +++ |
| 3/10 | +++ | +++ |
| 3/11 | +++ | +++ |
| 3/12 | ++ | ++ |
| 3/13 | +++ | +++ |
| 3/14 | +++ | +++ |
| 3/15 | +++ | +++ |
| 3/16 | +++ | +++ |
| 3/17 | ++ | ++ |
| 3/18 | +++ | +++ |
| 3/19 | +++ | +++ |
| 3/20 | +++ | +++ |
| 3/21 | +++ | +++ |
| 3/22 | +++ | +++ |
| 3/23 | +++ | +++ |
| 3/24 | +++ | +++ |
| 3/25 | +++ | +++ |
| 3/26 | +++ | +++ |
| 3/27 | +++ | +++ |
| 3/28 | ++ | +++ |
| 3/29 | +++ | +++ |
| 3/30 | +++ | +++ |
| 3/31 | +++ | +++ |
| 3/32 | +++ | +++ |
| 3/33 | +++ | +++ |
| 3/34 | ++ | ++ |
| 3/37 | +++ | +++ |
| 3/38 | +++ | +++ |
| 3/39 | +++ | +++ |
| 3/40 | ++ | ++ |
| 3/42 | +++ | +++ |
| 3/44 | ++ | ++ |
| 3/45 | +++ | +++ |
| 4 | ++ | +++ |
| 5 | +++ | +++ |
| 6 | +++ | +++ |
| 7 | +++ | +++ |
| 8 | +++ | +++ |
| 9 | +++ | +++ |
| 10 | +++ | +++ |
| 11 | +++ | +++ |
| 12 | +++ | +++ |
| 13 | +++ | +++ |
| 13/1 | +++ | +++ |
| 13/2 | ++ | ++ |
| 13/3 | + | ++ |
| 14 | ++ | ++ |
| 15 | + | ++ |
| 16 | +++ | +++ |
| 17 | +++ | +++ |
| 18 | ++ | +++ |
| 18/1 | ++ | ++ |
| 18/2 | ++ | +++ |
| 19 | +++ | +++ |
| 19/1 | +++ | +++ |
| 19/2 | +++ | +++ |
| 19/3 | +++ | +++ |
| 20 | + | ++ |
| 20/1 | +++ | +++ |
| 21 | +++ | +++ |
| 22 | +++ | +++ |
| 23 | +++ | +++ |
| 24 | +++ | +++ |
| 25 | ++ | +++ |
| 26 | +++ | +++ |
| 27 | +++ | +++ |
| 27/1 | +++ | +++ |
| 27/2 | +++ | +++ |
| 27/3 | +++ | +++ |
| 28 | ++ | ++ |
| 29 | +++ | +++ |
| 30 | +++ | +++ |
| 30/1 | +++ | +++ |
| 30/2 | +++ | +++ |

As can be seen in the above table, all tested inventive compounds showed activity in inhibiting production of ATP and/or lactate. In particular, the inventive compounds of example 10, 3/8, 3/16, 3/19, 30 and 30/2 demonstrated high activity in inhibiting production of either one or both of ATP and lactate.

### Gene expression analysis by qPCR

Cells were plated at 40,000 cells per ml in medium of high glucose DMEM (PAN) supplemented with 10% FBS (PAN), 2 mM glutamine-alanine (PAN), 2 mM pyruvate (Sigma) and10 mL/L penicillin-streptomycin (PAN), and grown at 37 °C in 0.5% O₂, 5 % CO₂ and 75% humidity. Next day, cells were induced with test compounds (Figure 3: compounds of example 3/16, 3/8, 10 at 10 nM; Figure 8E-8AB: compounds of example 3/16, 3/8, 10, 3/19, 30 and 30/2 at 10 nM) 0.5, 1, 2 and 4 hours, as well as with vehicle (DMSO) only. After this time, the cells were lysed according to protocol (Promega, SV 96 Total RNA Isolation System, #Z3505). After transferring the lysate to a 96-well-RNA binding plate, RNA was isolated and put at approx. 1 µg into cDNA synthesis using LunaScript RT SuperMix Kit (NEB, #E3010). The resulting cDNA was diluted 1:5 and used for qPCR Analysis (Taqman). Luna Universal Probe qPCR Master Mix (NEB, #M3004) is used as 2x reaction mix to which the diluted cDNA is added. The qPCR analysis was performed using QuantStudio^{™} 6 Flex real-time PCR systems (Thermo Fisher Scientific).

A set of primers plus probe for each gene of interest was purchased at IDT (Integrated DNA Technologies). The corresponding sequences are summarized in Table 2:

**Table 2:**

| **Gene** | **Primer 1** | **Primer 2** | **Probe** |
|---|---|---|---|
| **CHOP (human)** | TGACCAGGGAAGTAGAGGC (SEQ ID NO:1) | AGTGAGAGGGTAGTCAGTAGC (SEQ ID NO:2) | TGGGAAAGGTGGGTAGTGTGGC (SEQ ID NO:3) |
| **CHAC1 (human)** | GGAGACACCTTCCATCGG (SEQ ID NO:4) | TGCCTCTCGCACATTCAG (SEQ ID NO:5) | CCTTGCTTACCTGCTCCCCTTGC (SEQ ID NO:6) |
| **XBP1 (human)** | CTGGAACAGCAAGTGGTAGA (SEQ ID NO:7) | CGCTGTCTTAACTCCTGGTTC (SEQ ID NO:8) | AGCTTTTACGAGAGAAAACTCATGGCCT (SEQ ID NO:9) |
| **SLC7A11 (human)** | TCATTGGAGCAGGAATCTTCATC (SEQ ID NO:10) | GTTCCCAATTCAGCATAAGACAA (SEQ ID NO:11) | TGTTCTGGAGCACGCCCTTAGG (SEQ ID NO:12) |
| **PPIA (human)** | CCGAGGAAAACCGTGTACTATTAG (SEQ ID NO:13) | TGCTGTCTTTGGGACCTTG (SEQ ID NO:14) | CGCGTCTCCTTTGAGCTGTTTGC (SEQ ID NO:15) |
| **TBP (human)** | GAGAGTTCTGGGATTGTACCG (SEQ ID NO:16) | ATCCTCATGATTACCGCAGC (SEQ ID NO:17) | TGGGATTATATTCGGCGTTTCGGGC (SEQ ID NO:18) |
| **TBP (mouse)** | AAGAAAGGGAGAATCATGGACC (SEQ ID NO:19) | GAGTAAGTCCTGTGCCGTAAG (SEQ ID NO:20) | CCTGAGCATAAGGTGGAAGGCTGTT (SEQ ID NO:21) |
| **CHOP (mouse)** | TGTTGAAGATGAGCGGGTG (SEQ ID NO:22) | AGGTTCTGCTTTCAGGTGTG (SEQ ID NO:23) | AGCGACAGAGCCAGAATAACAGCC (SEQ ID NO:24) |
| **CHAC1 (mouse)** | ACAAGATGAGCACCTGGAAG (SEQ ID NO:25) | GTACTGCATACTGATGTCCACC (SEQ ID NO:26) | ACCCTGCTACCCTGCTCTTACCT (SEQ ID NO:27) |
| **SLC7A11 (mouse)** | CAGGTTATTCTACGTCGCATCT (SEQ ID NO:28) | GAGCATCACCATCGTCAGA (SEQ ID NO:29) | TCATGTCCACAAGCACACTCCTCTG (SEQ ID NO:30) |
| **XBP1 (mouse)** | AGTGGTGGATTTGGAAGAAGAG (SEQ ID NO:31) | TCTGGAACCTCGTCAGGAT (SEQ ID NO:32) | AGGAGTTAAGAACACGCTTGGGAATGG (SEQ ID NO:33) |

Fig. 3 shows time-dependent upregulation of the ER stress markers CHAC, CHOP/DDIT3, XBP1 and SLC7A11 in HeLa, 4T1, LLC1, A549, MDA-MB-231 and MiaPaCa2 cells induced with compounds of example 3/16, 3/8 and 10.

Fig. 8E-8AB show time-dependent upregulation of the ER stress markers CHAC and CHOP/DDIT3 in Panc02 and SKOV3 cells induced with compounds of example 3/16, 3/8, 10, 3/19, 30 and 30/2.

### Western Blot Analysis

Western Blot analysis was used to detect amounts of specific protein in differently treated cells. Proteins are separated by size, transferred onto a nitrocellulose membrane and incubated with protein specific antibodies. The density of the observed band is proportional to the amount of the specific protein inside the cell.

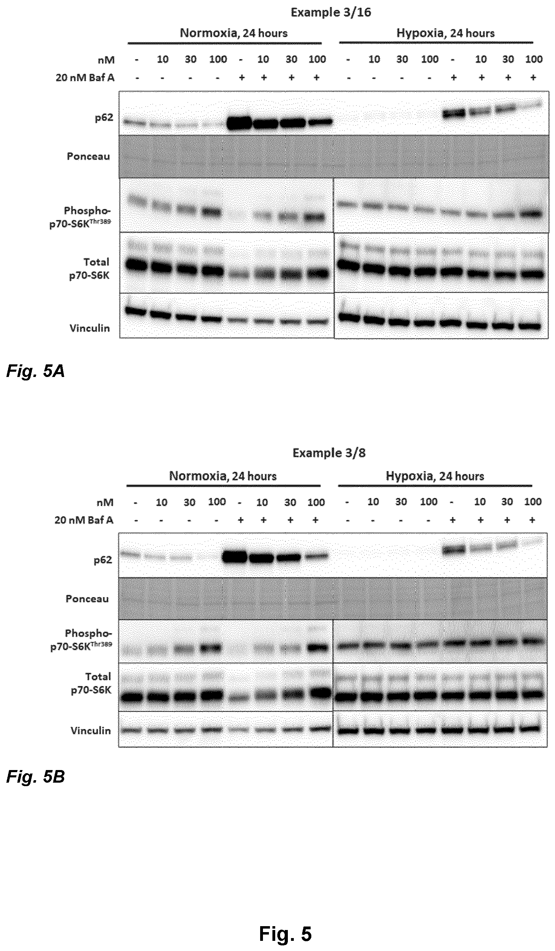

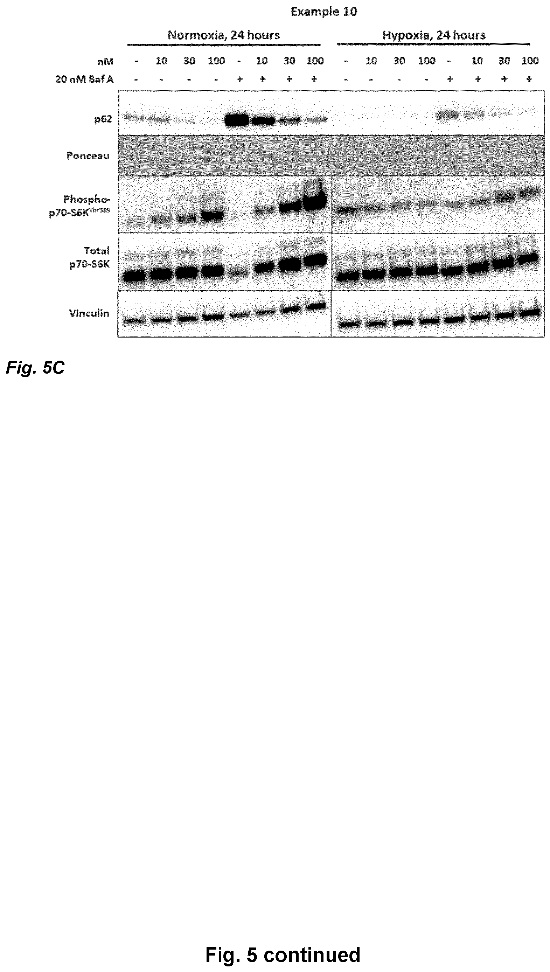

Cells were plated at 4x10⁶ (for hypoxia) or 3x10⁶ (for normoxia) cells per 10cm² plate in medium of high glucose DMEM (PAN) supplemented with 10% FBS (PAN), 2 mM glutamine-alanine (PAN), 2 mM pyruvate (Sigma) and 10 ml/L penicillin-streptomycin (PAN), and grown at 37 °C in 0.5% O₂, 5 % CO₂ and 75% humidity. For hypoxia, cells were grown and treated in the Whitley H35 Hypoxystation (Don Whitley Scientific) at 37 °C in 0.5% O₂, 5 % CO₂ and 75% humidity. Next day, the cells were treated with test compounds (Figure 4:compounds of xample 3/8 and 10 at concentrations 0.1, 0.3 and 1 µM, Bafilomycin A at 20 nM; Figure 5: compounds of example 3/16, 3/8 and 10 at concentrations 10, 30 and 100 nM, Bafilomycin A at 20 nM), as well as with vehicle (DMSO) only, for 24h. After 24 hours, the cells were washed with PBS and scraped to collect. The lysate was centrifuged at 2,000 rpm for 3 min at 4 °C. The cell pellet was resuspended in 250 µl Lysis Buffer (20 mM Tris pH 7.8, 150 mM NaCl, 0.05% Tween20, 2 mM MgCl2, protease inhibitors (Roche), 1 mM NaF, 1 mM Na₃VO₄) and sonicated at 50% for 20 sec. The sample was centrifuged at 14,000 rpm for 4 min at 4 °C. Protein concentration of the sample was measured using the DC Protein Assay Kit (Biorad, 5000112), 22 µg of protein extract per well was loaded onto a precast SDS page gel (Biorad, 1704273). Proteins were then transferred onto a PVDF-membrane (Biorad, 10026933) using Biorad Trans-Blot Turbo Transfer System. The membrane then was incubated in the blocking buffer (5 % dry milk, 0.1 % Tween20, Tris-buffered saline buffer) for 45 min, and after with the 1:1000 dilution of primary antibody against HIF1a (#36169, Cell Signaling), 1:10000 dilution of vinculin (7F9) (#sc-73614, Santa Cruz Biotechnology), 1:1000 dilution of SQSTM1/p62 (D-3) (#sc-28359, Santa Cruz Biotechnology), 1:2000 dilution of p70 S6 Kinase (#9202, Cell Signaling), 1:1000 dilution of phospho-p70 S6 Kinase (Thr389) (#9205, Cell Signaling), in the blocking buffer, overnight. Next day, the membrane was washed with 0.1 % Tween20 in TBS buffer three times for 10 min and incubated with the secondary antibody anti-rabbit IgG HRP (#sc-2004, Santa Cruz Biotechnology) or anti-mouse IgG HRP (#sc-2005, Santa Cruz Biotechnology) in the blocking buffer for 45 min. The membrane was then washed three times for 10 min. The chemiluminescence was measured using chemiluminescent substrate (#34580, Thermo Scientific).

Fig. 4 shows a dose-dependent reduction of p62 and HIF1α protein levels in HeLa cells, treated for 24 hours with the compounds of example 3/8 and 10 under normoxic and hypoxic conditions.

Fig. 5 shows a dose-dependent reduction of p62 in HeLa cells, treated for 24 hours with the compounds of example 3/16, 3/8 and 10, also in cells concomitantly treated with Bafilomycin A, under normoxic and hypoxic conditions.

Fig. 5 also shows a dose-dependent induction of p70-S6K phosphorylation (Thr389) in HeLa cells, treated for 24 hours with the compounds of example 3/16, 3/8 and 10, and in cells concomitantly treated with Bafilomycin A under normoxic condition.

### Determination of Autophagic flux

A HeLa cell line stably expressing an artificial protein construct consisting of Green Fluorescent Protein (GFP) as an N-terminal fusion to the human full-length LC3 protein, placed in front of a Red Fluorescent Protein (RFP) as an N terminal fusion to human LC3 lacking its C-terminal Glycine residue (LC3DG), was used to determine autophagic flux (Kaizuka et al., Mol. Cell (2016) 17 835-849) This construct is cleaved in cells by ATG4 at the C-terminus of the full-length LC3 protein, whereupon the GFP-LC3 fragment becomes conjugated to phosphatidyl ethanolamine. The RFP-LC3DG cannot be lipidated, and can be used as an internal reference to produce ratiometric values reporting autophagic flux. The lipidated GFP-LC3 protein becomes incorporated into the membrane of autophagosomes, and upon fusion with lysosomes, is degraded. Consequently, cells with a high autophagic flux have less GFP-LC3, whereas a low autophagic flux results in a relatively higher intracellular amount of GFP-LC3.

Reporter cells were grown as sub-confluent cultures and test compounds were applied at various concentrations. Thapsigargin, a known inducer of autophagy, was used as a positive control. After 24 hours of treatment, a single-cell suspension was prepared by trypsinization, and the GFP fluorescence determined by flow cytometry. A minimum of 10,000 single cells were evaluated for each condition.

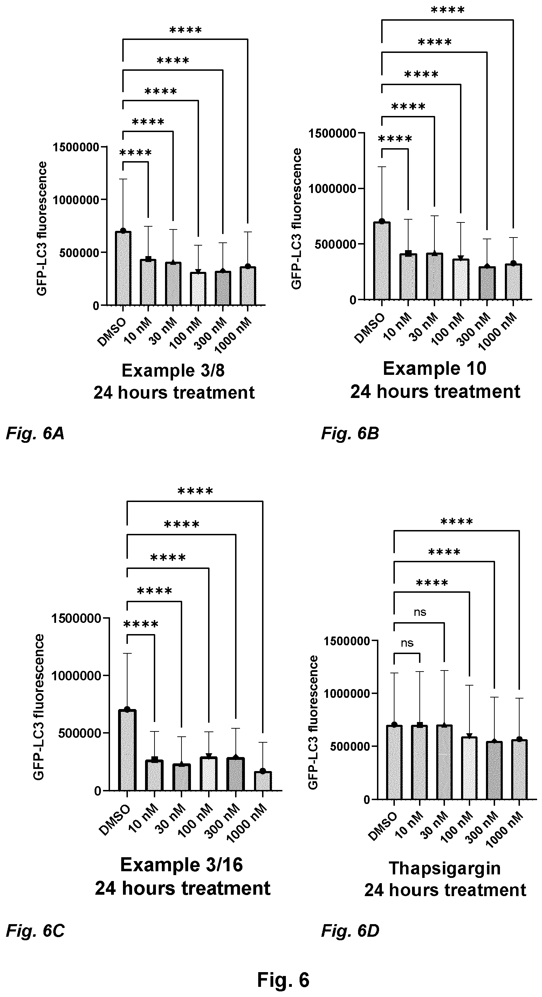

Figure 6 shows that the tested compounds of examples 3/8, 3/16 and 10 significantly enhance authophagic flux in a HeLa cell line expressing the artificial protein construct consisting of GFP-LC3.

## Claims

1. A compound of formula (I) 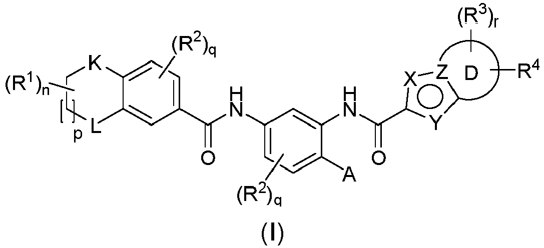
or an enantiomer, diastereomer, N-oxide, solvate or pharmaceutically acceptable salt thereof, wherein
K is O, S or C(R⁵)₂;
L is O, S or C(R⁵)₂;
n is 0, 1, 2, 3 or 4;
p is 1 or 2;
q is independently from each other 0, 1 or 2;
r is 1 or 2;
A is C₁₋₆-alkyl, halogen, CN, halo-C₁₋₆-alkyl, C₃₋₆-cycloalkyl, halo-C₃₋₆-cycloalkyl, OH, -OC₁₋₆-alkyl or O-halo-C₁₋₆-alkyl;
X is S, O, NR⁶, N or CR⁷;
Y is S, O, NR⁶, N or CR⁷;
Z is C or N;
D represents a 6-membered aryl or 5- to 6-membered heteroaryl containing 1 to 4 heteroatoms independently selected from O, N and S;
R¹ is F, C₁₋₃-alkyl or spirocyclic fused C₃₋₆-cycloalkyl;
R² is independently halogen or C₁₋₆-alkyl;
R³ is hydrogen, halogen, C₁₋₆-alkyl, halo-C₁₋₆-alkyl, C₃₋₆-cycloalkyl, halo-C₃₋₆-cycloalkyl, -OC₁₋₆-alkyl or O-halo-C₁₋₆-alkyl;
R⁴ is hydrogen, halogen or V¹-V²-V³-V⁴-V⁵
wherein
V¹ is absent, a bond or C₁₋₆-alkylene;
V² is absent, a bond or O, S, S(O)ₓ, S(O)(=NR^{a}), S(O)₂NR^{a}, NR^{a}, NR^{a}C(O) or C(O)NR^{a};
V³ is absent, a bond or C₁₋₆-alkylene;
wherein C₁₋₆-alkylene in V¹ or V³ is unsubstituted or substituted with 1 to 4 substituents independently selected from the group consisting of OH, C₁₋₆-alkyl, C₁₋₆-haloalkyl, halogen and oxo,
or
wherein two C₁₋₆-alkyl groups on the C₁₋₆-alkylene in V¹ or V³, together with the carbon atom to which they are attached, form a C₃₋₆-cycloalkyl group;
V⁴ is absent, a bond or O, S, S(O)ₓ, S(O)(=NR^{a}), S(O)₂NR^{a}, NR^{a}, NR^{a}C(O) or C(O)NR^{a};
V⁵ is hydrogen, halogen, OH, CN, CO₂H, CO₂-C₁₋₆-alkyl, N(R^{a})₂, C₁₋₆-alkyl, C₃₋₈-cycloalkyl, 4- to 8-membered mono or bicyclic heterocycloalkyl containing 1, 2 or 3 heteroatoms independently selected from the group consisting of O, S and N, phenyl or 5- to 6-membered heteroaryl containing 1, 2 or 3 heteroatoms independently selected from the group consisting of O, S and N,
wherein alkyl, cycloalkyl, heterocycloalkyl, phenyl or heteroaryl are unsubstituted or substituted with 1 to 4 substituents R^{V5} independently selected from the group consisting of CN, CO₂H, CO₂-C₁₋₆-alkyl, N(R^{a})₂, OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N;
R⁵ is hydrogen, F, C₁₋₃-alkyl or spirocyclic fused C₃₋₆-cycloalkyl;
R⁶ is hydrogen or C₁₋₆-alkyl;
R⁷ is hydrogen, halogen or C₁₋₆-alkyl;
R^{a} is hydrogen or C₁₋₃-alkyl; and
x is 1 or 2.

2. The compound according to claim 1, which is represented by formula (I) 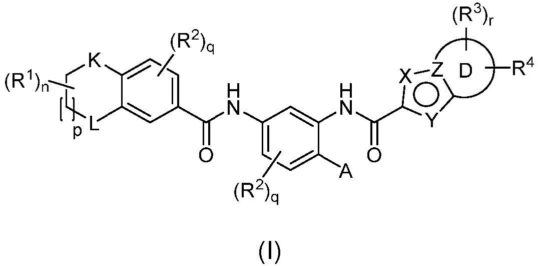
or an enantiomer, diastereomer, N-oxide, solvate or pharmaceutically acceptable salt thereof, wherein
K is O, S or C(R⁵)₂;
L is O, S or C(R⁵)₂;
n is 0, 1, 2, 3 or 4;
p is 1 or 2;
q is independently from each other 0, 1 or 2;
r is 1 or 2;
A is C₁₋₆-alkyl, halogen, CN, halo-C₁₋₆-alkyl, C₃₋₆-cycloalkyl, halo-C₃₋₆-cycloalkyl, OH, -OC₁₋₆-alkyl or O-halo-C₁₋₆-alkyl;
X is S, O, NR⁶, N or CR⁷;
Y is S, O, NR⁶, N or CR⁷;
Z is C or N;
D represents a 6-membered aryl or 5- to 6-membered heteroaryl containing 1 to 4 heteroatoms independently selected from O, N and S;
R¹ is F, C₁₋₃-alkyl or spirocyclic fused C₃₋₆-cycloalkyl;
R² is independently halogen or C₁₋₆-alkyl;
R³ is hydrogen, halogen, C₁₋₆-alkyl, halo-C₁₋₆-alkyl, C₃₋₆-cycloalkyl, halo-C₃₋₆-cycloalkyl, -OC₁₋₆-alkyl or O-halo-C₁₋₆-alkyl;
R⁴ is hydrogen, halogen or V¹-V²-V³-V⁴-V⁵
wherein
V¹ is absent, a bond or C₁₋₆-alkylene;
V² is absent, a bond or O, S, S(O)ₓ, S(O)(=NR^{a}), S(O)₂NR^{a}, NR^{a}, NR^{a}C(O) or C(O)NR^{a};
V³ is absent, a bond or C₁₋₆-alkylene;
wherein C₁₋₆-alkylene in V¹ or V³ is unsubstituted or substituted with 1 to 4 substituents independently selected from the group consisting of C₁₋₆-alkyl, C₁₋₆-haloalkyl, halogen and oxo,
or
wherein two C₁₋₆-alkyl groups on the C₁₋₆-alkylene in V¹ or V³, together with the carbon atom to which they are attached, form a C₃₋₆-cycloalkyl group;
V⁴ is absent, a bond or O, S, S(O)ₓ, S(O)(=NR^{a}), S(O)₂NR^{a}, NR^{a}, NR^{a}C(O) or C(O)NR^{a};
V⁵ is hydrogen, halogen, OH, CN, CO₂H, CO₂-C₁₋₆-alkyl, N(R^{a})₂, C₁₋₆-alkyl, C₃₋₈-cycloalkyl, 4- to 8-membered mono or bicyclic heterocycloalkyl containing 1, 2 or 3 heteroatoms independently selected from the group consisting of O, S and N, phenyl or 5- to 6-membered heteroaryl containing 1, 2 or 3 heteroatoms independently selected from the group consisting of O, S and N,
wherein alkyl, cycloalkyl, heterocycloalkyl, phenyl or heteroaryl are unsubstituted or substituted with 1 to 4 substituents R^{V5} independently selected from the group consisting of CN, CO₂H, CO₂-C₁₋₆-alkyl, N(R^{a})₂, OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N;
R⁵ is hydrogen, F, C₁₋₃-alkyl or spirocyclic fused C₃₋₆-cycloalkyl;
R⁶ is hydrogen or C₁₋₆-alkyl;
R⁷ is hydrogen, halogen or C₁₋₆-alkyl;
R^{a} is hydrogen or C₁₋₃-alkyl; and
x is 1 or 2.

3. The compound according to claim 1 or 2, wherein the compound is represented by formula (II) 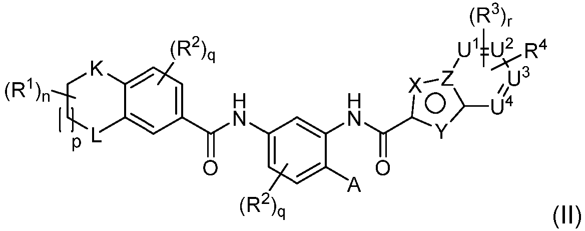 wherein U¹, U² U³ and U⁴ are independently selected from the group consisting of N and C.

4. The compound according to any one of claims 1 to 3, wherein the compound is represented by formula (III) 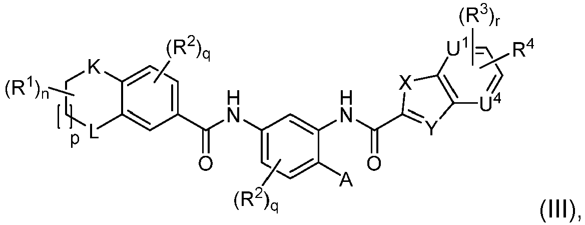 wherein
U¹ and U⁴ are independently selected from N and C;
X is S, O, NR⁶ or N; and
Y is CR⁷ or N, and/or
wherein n is 0, and/or
wherein p is 1, and/or
wherein the compound is represented by formula (IV) 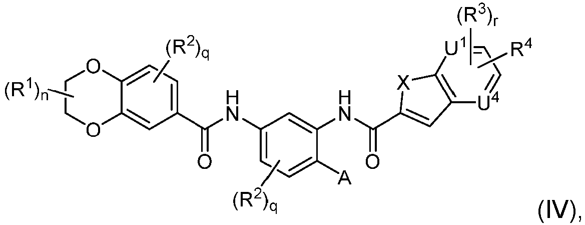 wherein
A is C₁₋₆-alkyl or halogen; and
X is S, O or NH.

5. The compound according to any one of claims 1 to 4, wherein:
R⁴ is hydrogen, halogen or V¹-V²-V³-V⁴-V⁵, wherein
V¹ is absent, a bond or C₁₋₄-alkylene;
V² is absent, a bond or O, S, S(O)ₓ, NR^{a};
V³ is absent, a bond or C₁₋₄-alkylene,
V⁴ is absent, a bond or O, S, S(O)ₓ, NR^{a},
wherein C₁₋₄-alkylene in V¹ or V³ is unsubstituted or substituted with 1 to 4 substituents independently selected from the group consisting of C₁₋₃-alkyl, C₁₋₃-haloalkyl, halogen and oxo,
or
wherein two C₁₋₆-alkyl groups on the C₁₋₄-alkylene in V¹ or V³, together with the carbon atom to which they are attached, form a C₃₋₆-cycloalkyl group;
V⁵ is hydrogen, halogen, OH, CN, C₁₋₆-alkyl, C₃₋₆-cycloalkyl, 4- to 8-membered mono or bicyclic heterocycloalkyl containing 1, 2 or 3 heteroatoms independently selected from the group consisting of O, S and N, or 5- to 6-membered heteroaryl containing 1, 2 or 3 heteroatoms independently selected from the group consisting of O, S and N,
wherein alkyl, cycloalkyl, heterocycloalkyl or heteroaryl are unsubstituted or substituted with 1 to 4 substituents R^{V5} independently selected from the group consisting of OH, C₁₋₆-alkyl, O-C₁₋₆-alkyl, C(O)-C₁₋₆-alkyl, C₁₋₆-haloalkyl, halogen, oxo, spirocyclicly fused C₃₋₆-cycloalkyl and spirocyclicly fused 3-7 membered heterocycloalkyl containing 1 heteroatom selected from the group consisting of O, S and N; and/or
the compound is selected from the group consisting of 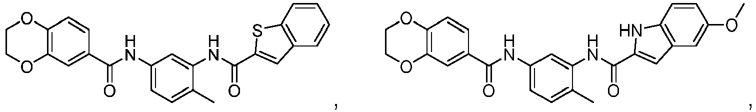 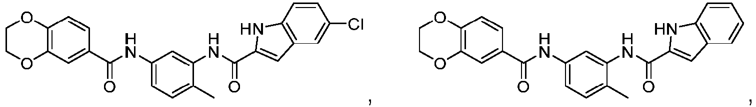 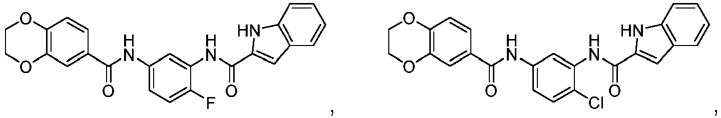 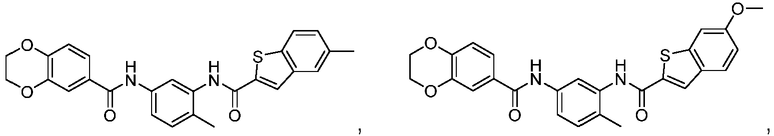 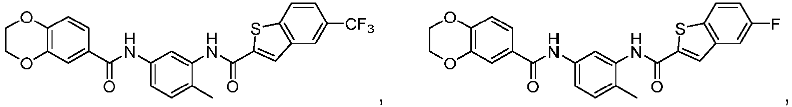 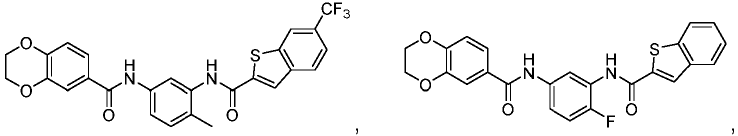 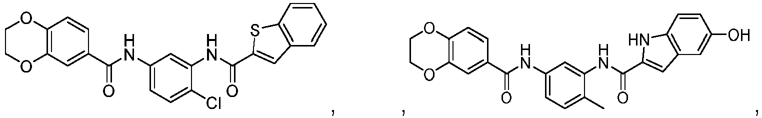 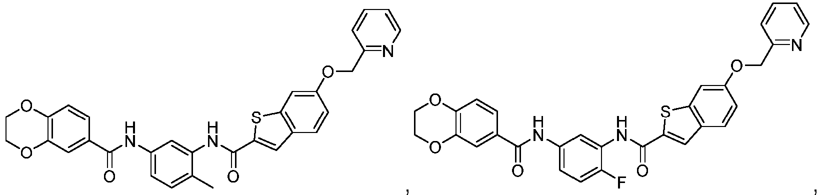 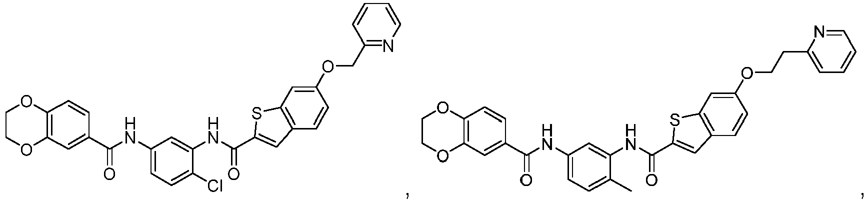 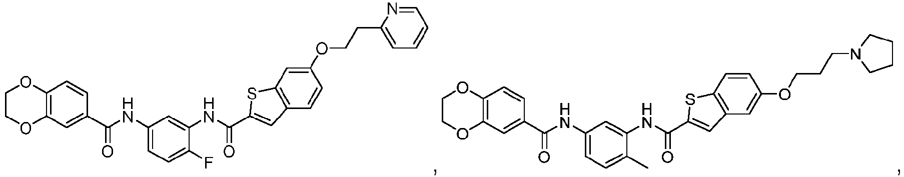 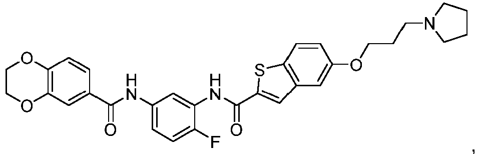 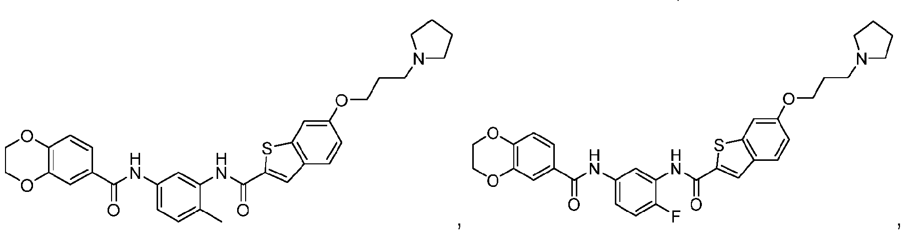 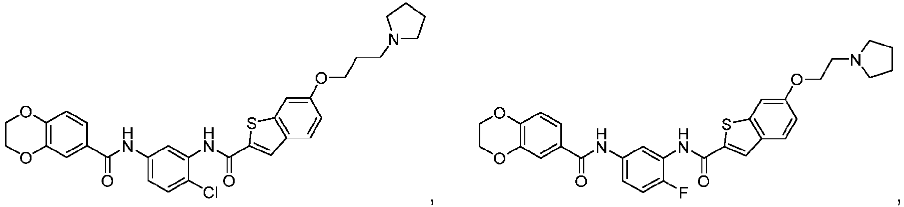 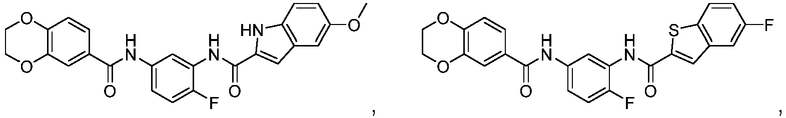 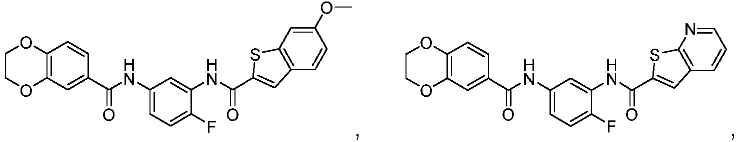 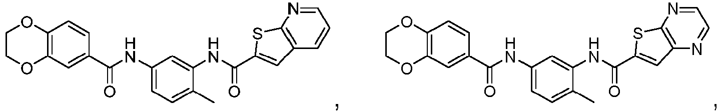 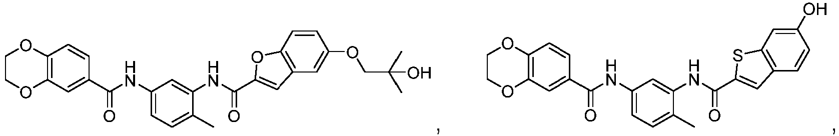 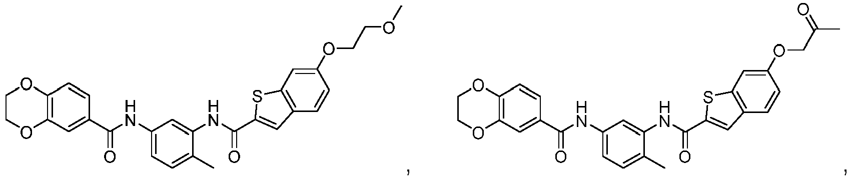 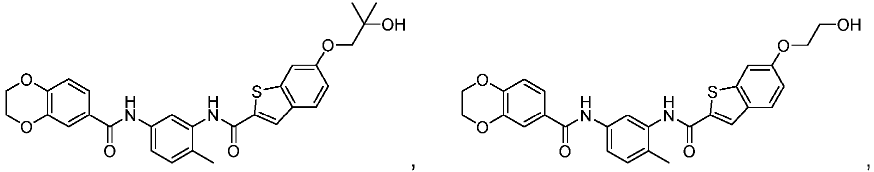 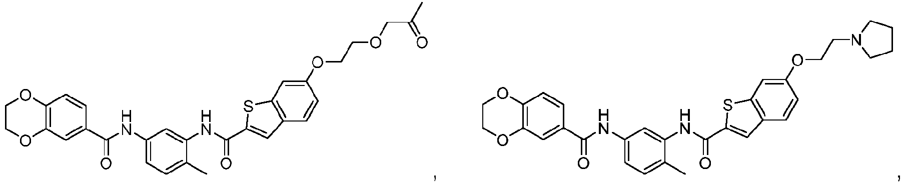 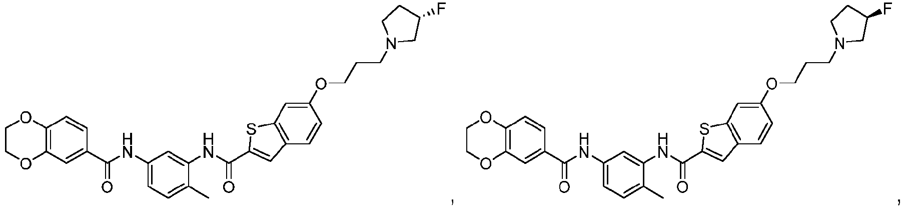 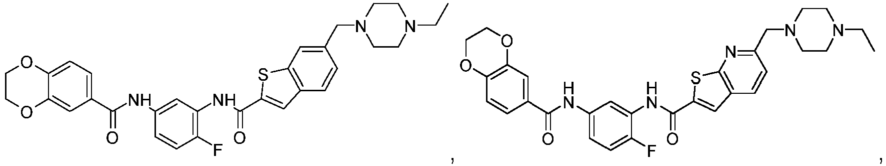 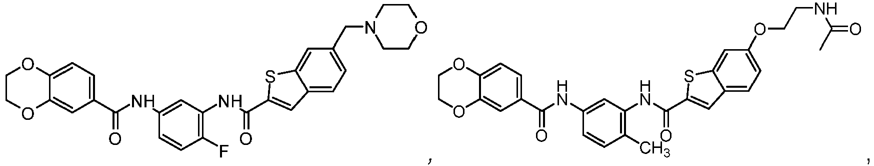 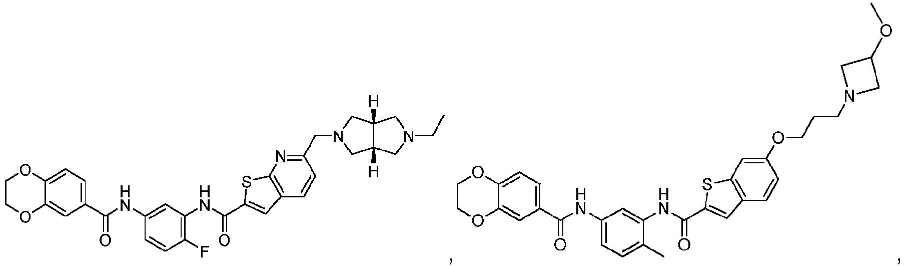 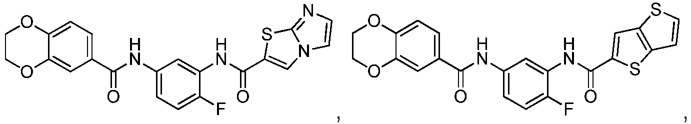 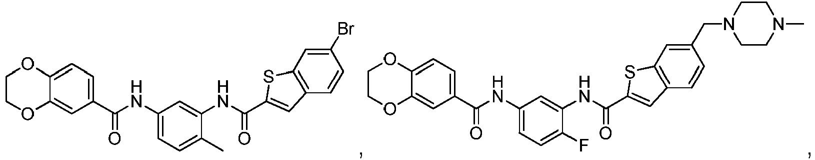 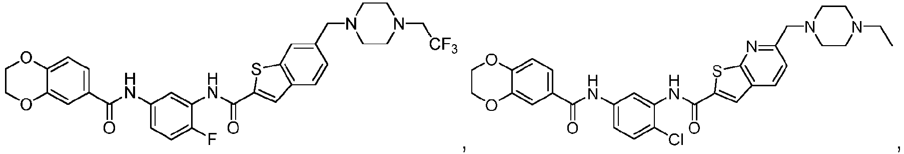 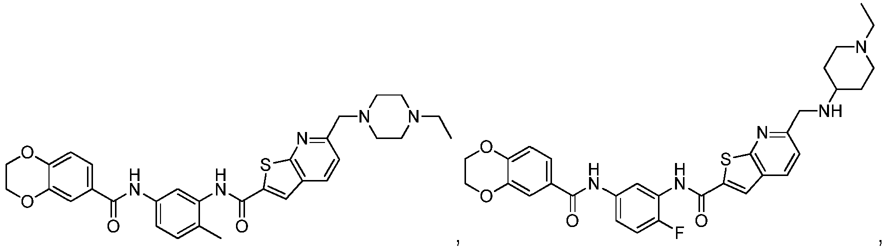 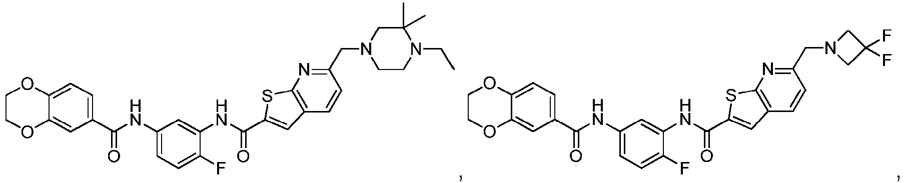 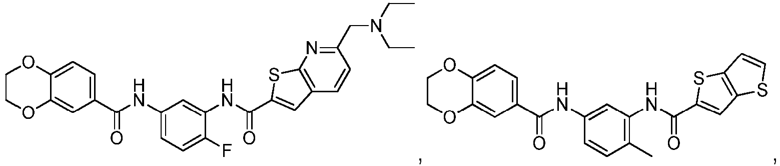 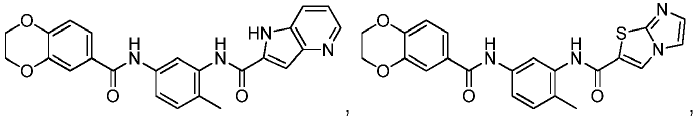 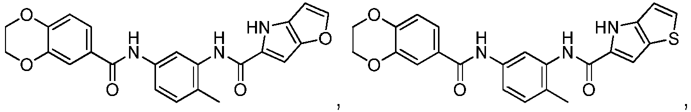 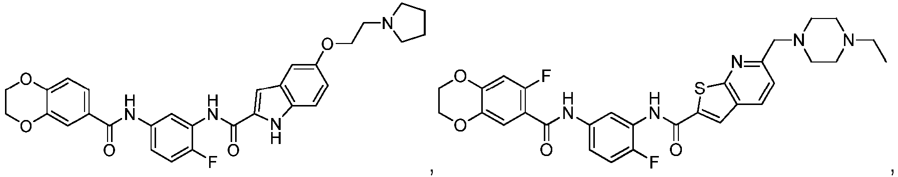 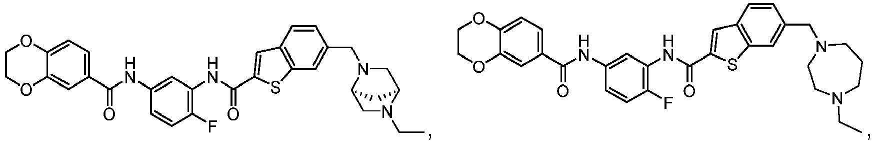 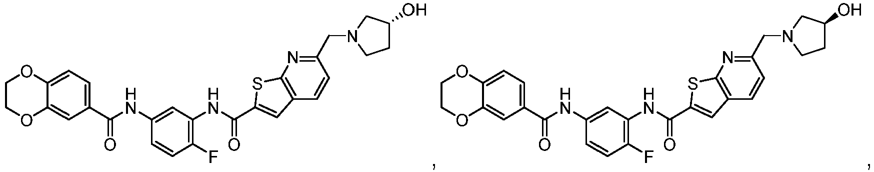 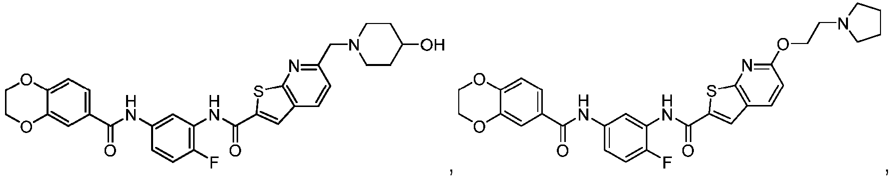 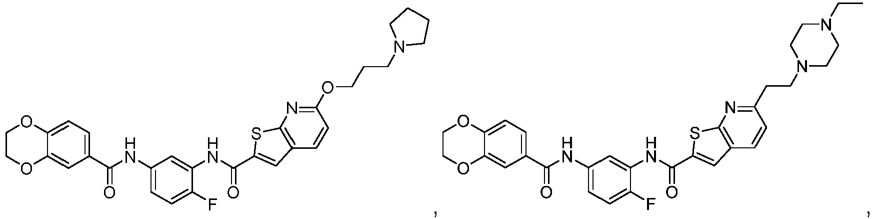 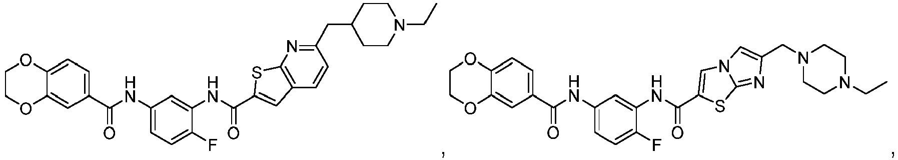 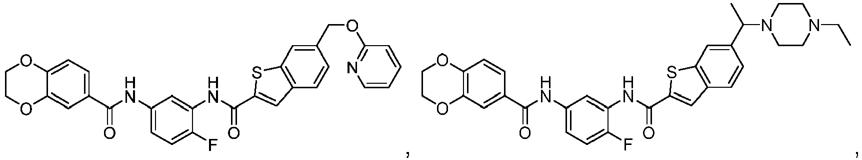 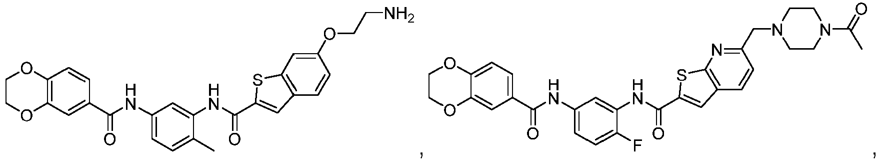 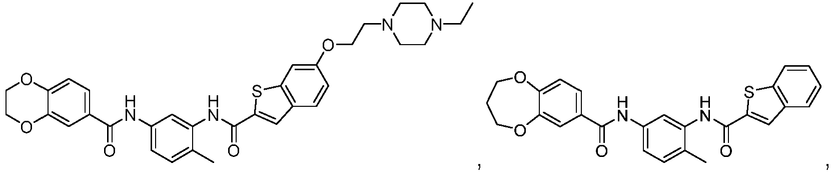 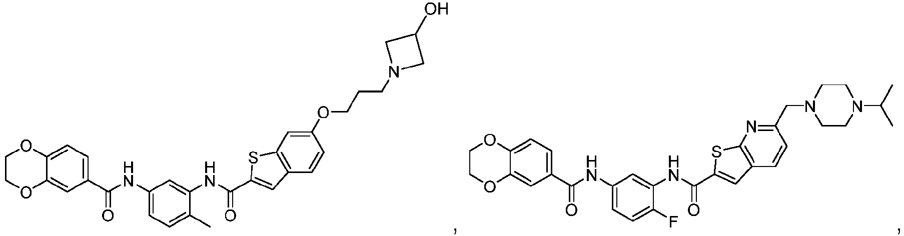 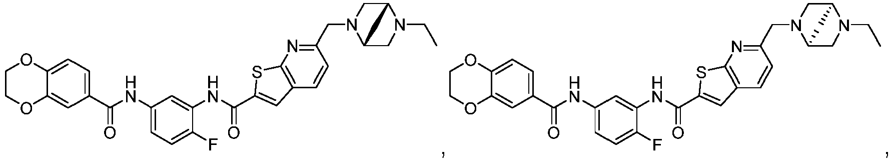 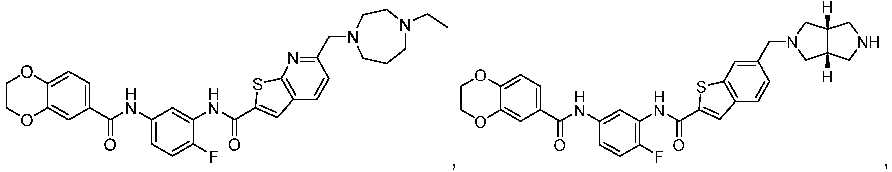 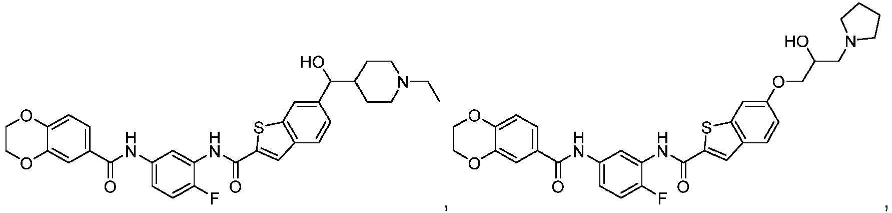 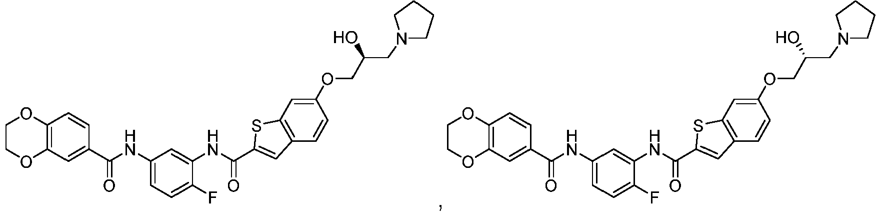
and the N-oxide, solvate or pharmaceutically acceptable salt thereof.

6. A pharmaceutical composition comprising the compound according to any one of claims 1 to 5 and a pharmaceutically acceptable excipient.

7. The compound according to any one of claims 1 to 5 for use as a medicament.

8. The compound according to any one of claims 1 to 5 or the pharmaceutical composition according to claim 6 for use in a method of preventing and/or treating a disease or condition mediated by the lactate/ATP mechanism,
wherein, optionally, the disease or condition mediated by the lactate/ATP mechanism is cancer and wherein, preferably, the compound or the pharmaceutical composition is administered
• together with one or more therapeutic agents for cancer selected from the group consisting of a PD-1 agent, a PD-L1 agent, a CTLA-4 agent, an IDO1 inhibitor, and an anticancer vaccine, or
• together with a cytokine therapy or a known chemo- or pharmacotherapy, or during irradiation therapy.

9. A compound according to any one of claims 1 to 5 or a pharmaceutical composition according to claim 6 for use:
in a method of preventing and/or treating a disease or condition, preferably cancer, by inducing endoplasmic reticulum stress in cells affected by said disease or condition, thereby reducing intracellular ATP and lactate production in said cells; or
in a method of preventing and/or treating cancer, preferably a cancer cell and/or a cancerous tumor, wherein said compound or said pharmaceutical composition reduces Hif1-alpha at the protein level in hypoxic areas of said cancer, thereby downregulating ATP and lactate production in these areas and ultimately leading to reduction, preferably efficient reduction, of hypoxic areas and of growth of said cancer; or
in a method of preventing and/or treating a disease or condition which is susceptible to the induction of endoplasmic reticulum stress, wherein administration of said compound or said pharmaceutical composition to a subject having or suspected of having said disease or condition results in a reduction of the protein level of Hif-1alpha; or
in a method of preventing and/or treating autophagy-related diseases selected from neuro-degenerative diseases such as Parkinson's disease, Alzheimer's disease and Huntington's disease, amyotrophic lateral sclerosis, metabolic disorders such as obesity and diabetes type I and II, viral infections and diseases which lead to accumulation of certain debris particles such as fibrotic lung diseases.

10. A composition comprising a compound according to any one of claims 1 to 5 and a biguanide.

11. A kit comprising a compound according to any one of claims 1 to 5 and a biguanide.

12. The composition according to claim 9, or the kit according to claim 11, for use as a medicament.

13. A combination of a compound according to any one of claims 1 to 5 and a biguanide, for use in therapy.

14. A combination of a compound according to any one of claims 1 to 5 and a biguanide, or a kit according to claim 11, for use in a method of preventing and/or treating cancer, preferably preventing and/or treating advanced stage cancer, such as, optionally, preventing and/or treating advanced stage glycolytic cancer, and/or
wherein the cancer is a LKB-1-deficient cancer, and/or
wherein the cancer is an ARID1a-deficient cancer, and/or
wherein, optionally, the cancer is chosen from the group consisting of non-small-cell lung cancer, sarcoma, gynecological cancers such as cervix, ovarian cell or uterine carcinoma, adenoid cystic carcinoma, pancreatic cancer, other gastrointestinal cancers such as stomach or esophagus cancers.

15. The composition according to claim 8, the kit according to claim 11, the composition for use, or the kit for use, according to claim 12, the combination for use according to claim 13, or the combination for use, or the kit for use, according claim 14, wherein the biguanide is chosen from the group consisting of metformin, buformin and phenformin.

## Patentansprüche

1. Eine Verbindung der Formel (I) 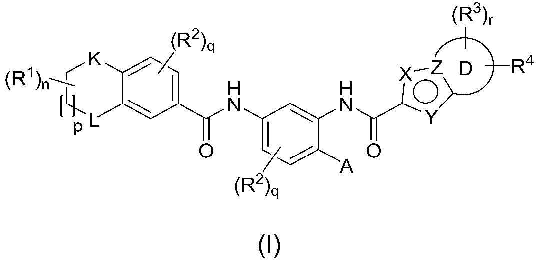
oder ein Enantiomer, Diastereomer, N-Oxid, Solvat oder pharmazeutisch verträgliches Salz davon, wobei
K ist O, S oder C(R⁵)₂;
L ist O, S oder C(R⁵)₂;
n ist 0, 1, 2, 3 oder 4;
p ist 1 oder 2;
q ist jeweils unabhängig voneinander 0, 1 oder 2;
r ist 1 oder 2;
A ist C₁₋₆-Alkyl, Halogen, CN, Halogen-C₁₋₆-alkyl, C₃₋₆-Cycloalkyl, Halogen-C₃₋₆-cycloalkyl, OH, -OC₁₋₆-Alkyl oder O-Halogen-C₁₋₆-alkyl;
X ist S, O, NR⁶, N oder CR⁷;
Y ist S, O, NR⁶, N oder CR⁷;
Z ist C oder N;
D für ein 6-gliedriges Aryl oder ein 5- bis 6-gliedriges Heteroaryl steht, das 1 bis 4 Heteroatome enthält, die unabhängig voneinander aus O, N und S ausgewählt sind;
R¹ ist F, C₁₋₃-Alkyl oder spirocyclisch kondensiertes C₃₋₆-Cycloalkyl;
R² ist jeweils unabhängig voneinander Halogen oder C₁₋₆-Alkyl;
R³ ist Wasserstoff, Halogen, C₁₋₆-Alkyl, Halogen-C₁₋₆-alkyl, C₃₋₆-Cycloalkyl, Halogen-C₃₋₆-cycloalkyl, -OC₁₋₆-Alkyl oder O-Halogen-C₁₋₆-alkyl;
R⁴ ist Wasserstoff, Halogen oder V¹-V²-V³-V⁴-V⁵
wobei
V¹ fehlt, eine Bindung oder C₁₋₆-Alkylen ist;
V² fehlt, eine Bindung, O, S, S(O)ₓ, S(O)(=NR^{a}), S(O)₂NR^{a}, NR^{a}, NR^{a}C(O) oder C(O)NR^{a} ist;
V³ fehlt, eine Bindung oder C₁₋₆-Alkylen ist;
wobei das C₁₋₆-Alkylen in V¹ oder V³ unsubstituiert oder mit 1 bis 4 Substituenten substituiert ist, die unabhängig voneinander aus der Gruppe bestehend aus OH, C₁₋₆-Alkyl, C₁₋₆-Halogenalkyl, Halogen und Oxo ausgewählt sind,
oder
wobei zwei C₁₋₆-Alkylgruppen an dem C₁₋₆-Alkylen in V¹ oder V³ zusammen mit dem Kohlenstoffatom, an das sie gebunden sind, eine C₃₋₆-Cycloalkylgruppe bilden;
V⁴ fehlt, eine Bindung, O, S, S(O)ₓ, S(O)(=NR^{a}), S(O)₂NR^{a}, NR^{a}, NR^{a}C(O) oder C(O)NR^{a} ist;
V⁵ Wasserstoff, Halogen, OH, CN, CO₂H, CO₂-C₁₋₆-Alkyl, N(R^{a})₂, C₁₋₆-Alkyl, C₃₋₈-Cycloalkyl, ein 4- bis 8-gliedriges mono- oder bicyclisches Heterocycloalkyl mit 1, 2 oder 3 Heteroatomen, die unabhängig voneinander aus O, S und N ausgewählt sind, Phenyl oder ein 5- bis 6-gliedriges Heteroaryl mit 1, 2 oder 3 Heteroatomen ist, die unabhängig voneinander aus O, S und N ausgewählt sind,
wobei Alkyl, Cycloalkyl, Heterocycloalkyl, Phenyl oder Heteroaryl unsubstituiert oder mit 1 bis 4 Substituenten R^{v⁵} substituiert sind, die unabhängig voneinander aus der Gruppe bestehend aus CN, CO₂H, CO₂-C₁₋₆-Alkyl, N(R^{a})₂, OH, C₁₋₆-Alkyl, O-C₁₋₆-Alkyl, C(O)-C₁₋₆-alkyl, C₁₋₆-Halogenalkyl, Halogen, Oxo, spirocyclisch kondensiertem C₃₋₆-Cycloalkyl und spirocyclisch kondensiertem 3- bis 7-gliedrigem Heterocycloalkyl mit 1 Heteroatom, das aus O, S und N ausgewählt ist, ausgewählt sind;
R⁵ ist Wasserstoff, F, C₁₋₃-Alkyl oder spirocyclisch kondensiertes C₃₋₆-Cycloalkyl; R⁶ ist Wasserstoff oder C₁₋₆-Alkyl;
R⁷ ist Wasserstoff, Halogen oder C₁₋₆-Alkyl;
R^{a} ist Wasserstoff oder C₁₋₃-Alkyl; und
x ist 1 oder 2.

2. Verbindung nach Anspruch 1, die durch die Formel (I) dargestellt ist 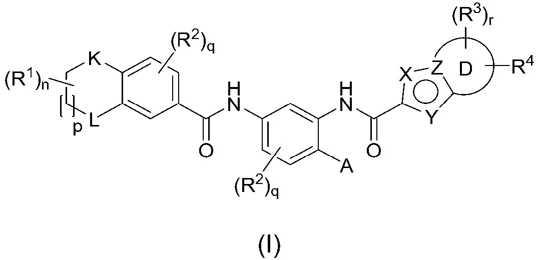
oder ein Enantiomer, Diastereomer, N-Oxid, Solvat oder pharmazeutisch verträgliches Salz davon, wobei
K ist O, S oder C(R⁵)₂;
L ist O, S oder C(R⁵)₂;
n ist 0, 1, 2, 3 oder 4;
p ist 1 oder 2;
q ist jeweils unabhängig voneinander 0, 1 oder 2;
r ist 1 oder 2;
A ist C₁₋₆-Alkyl, Halogen, CN, Halogen-C₁₋₆-alkyl, C₃₋₆-Cycloalkyl, Halogen-C₃₋₆-cycloalkyl, OH, -OC₁₋₆-Alkyl oder O-Halogen-C₁₋₆-alkyl;
X ist S, O, NR⁶, N oder CR⁷;
Y ist S, O, NR⁶, N oder CR⁷;
Z ist C oder N;
D für ein 6-gliedriges Aryl oder ein 5- bis 6-gliedriges Heteroaryl steht, das 1 bis 4 Heteroatome enthält, die unabhängig voneinander aus O, N und S ausgewählt sind;
R¹ ist F, C₁₋₃-Alkyl oder spirocyclisch kondensiertes C₃₋₆-Cycloalkyl;
R² ist jeweils unabhängig voneinander Halogen oder C₁₋₆-Alkyl;
R³ ist Wasserstoff, Halogen, C₁₋₆-Alkyl, Halogen-C₁₋₆-alkyl, C₃₋₆-Cycloalkyl, Halogen-C₃₋₆-cycloalkyl, -OC₁₋₆-Alkyl oder O-Halogen-C₁₋₆-alkyl;
R⁴ ist Wasserstoff, Halogen oder V¹-V²-V³-V⁴-V⁶
wobei
V¹ fehlt, eine Bindung oder C₁₋₆-Alkylen ist;
V² fehlt, eine Bindung, O, S, S(O)ₓ, S(O)(=NR^{a}), S(O)₂NR^{a}, NR^{a}, NR^{a}C(O) oder C(O)NR^{a} ist;
V³ fehlt, eine Bindung oder C₁₋₆-Alkylen ist;
wobei das C₁₋₆-Alkylen in V¹ oder V³ unsubstituiert oder mit 1 bis 4 Substituenten substituiert ist, die unabhängig voneinander aus der Gruppe bestehend aus C₁₋₆-Alkyl, C₁₋₆-Halogenalkyl, Halogen und Oxo ausgewählt sind,
oder
wobei zwei C₁₋₆-Alkylgruppen an dem C₁₋₆-Alkylen in V¹ oder V³ zusammen mit dem Kohlenstoffatom, an das sie gebunden sind, eine C₃₋₆-Cycloalkylgruppe bilden;
V⁴ fehlt, eine Bindung, O, S, S(O)ₓ, S(O)(=NR^{a}), S(O)₂NR^{a}, NR^{a}, NR^{a}C(O) oder C(O)NR^{a} ist;
V⁵ ist Wasserstoff, Halogen, OH, CN, CO₂H, CO₂-C₁₋₆-Alkyl, N(R^{a})₂, C₁₋₆-Alkyl, C₃₋₈-Cycloalkyl, ein 4- bis 8-gliedriges mono- oder bicyclisches Heterocycloalkyl mit 1, 2 oder 3 Heteroatomen, die unabhängig voneinander aus O, S und N ausgewählt sind, Phenyl oder ein 5- bis 6-gliedriges Heteroaryl mit 1, 2 oder 3 Heteroatomen, die unabhängig voneinander aus O, S und N ausgewählt sind,
wobei Alkyl, Cycloalkyl, Heterocycloalkyl, Phenyl oder Heteroaryl unsubstituiert oder mit 1 bis 4 Substituenten R^{v⁵} substituiert sind, die unabhängig voneinander aus der Gruppe bestehend aus CN, CO₂H, CO₂-C₁₋₆-Alkyl, N(R^{a})₂, OH, C₁₋₆-Alkyl, O-C₁₋₆-Alkyl, C(O)-C₁₋₆-alkyl, C₁₋₆-Halogenalkyl, Halogen, Oxo, spirocyclisch kondensiertem C₃₋₆-Cycloalkyl und spirocyclisch kondensiertem 3- bis 7-gliedrigem Heterocycloalkyl mit 1 Heteroatom, das aus O, S und N ausgewählt ist, ausgewählt sind;
R⁵ ist Wasserstoff, F, C₁₋₃-Alkyl oder spirocyclisch kondensiertes C₃₋₆-Cycloalkyl;
R⁶ ist Wasserstoff oder C₁₋₆-Alkyl;
R⁷ ist Wasserstoff, Halogen oder C₁₋₆-Alkyl;
R^{a} ist Wasserstoff oder C₁₋₃-Alkyl; und
x ist 1 oder 2.

3. Verbindung nach Anspruch 1 oder 2, wobei die Verbindung durch die Formel (II) dargestellt ist 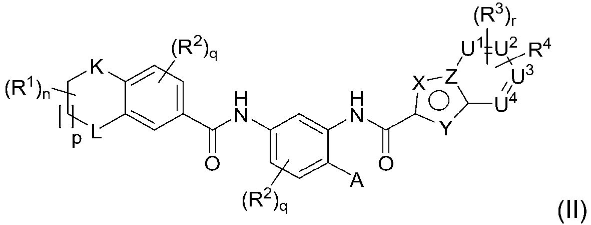 wobei U¹, U², U³ und U⁴ unabhängig voneinander aus N und C ausgewählt sind.

4. Verbindung nach einem der Ansprüche 1 bis 3, wobei die Verbindung durch die Formel (III) dargestellt ist 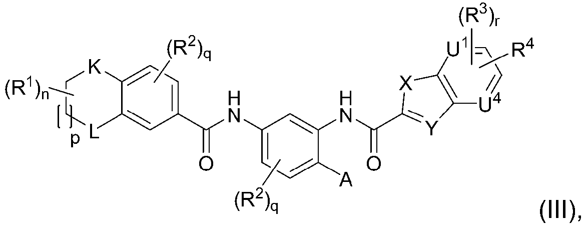 wobei
U¹ und U⁴ unabhängig voneinander aus N und C ausgewählt sind;
X ist S, O, NR⁶ oder N; und
Y st CR⁷ oder N, und/oder
wobei n ist 0, und/oder
wobei p ist 1, und/oder
wobei die Verbindung durch die Formel (IV) dargestellt ist 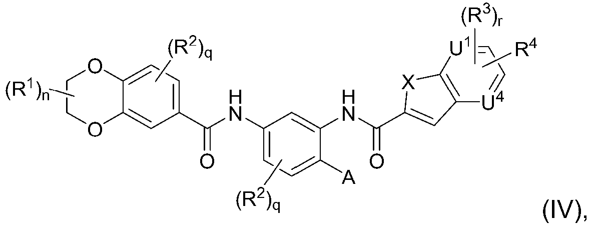 wobei
A ist C₁₋₆-Alkyl oder Halogen; und
X ist S, O oder NH.

5. Verbindung nach einem der Ansprüche 1 bis 4, wobei:
R⁴ Wasserstoff, Halogen oder V¹-V²-V³-V⁴-V⁵ ist, wobei
V¹ fehlt, eine Bindung oder C₁₋₄-Alkylen ist;
V² fehlt, eine Bindung, O, S, S(O)ₓ oder NR^{a} ist;
V³ fehlt, eine Bindung oder C₁₋₄-Alkylen ist;
V⁴ fehlt, eine Bindung, O, S, S(O)ₓ oder NR^{a} ist;
wobei das C₁₋₄-Alkylen in V¹ oder V³ unsubstituiert oder mit 1 bis 4 Substituenten substituiert ist, die unabhängig voneinander aus der Gruppe bestehend aus C₁₋₃-Alkyl, C₁₋₃-Halogenalkyl, Halogen und Oxo ausgewählt sind,
oder
wobei zwei C₁₋₆-Alkylgruppen an dem C₁₋₄-Alkylen in V¹ oder V³ zusammen mit dem Kohlenstoffatom, an das sie gebunden sind, eine C₃₋₆-Cycloalkylgruppe bilden;
V⁵ ist Wasserstoff, Halogen, OH, CN, C₁₋₆-Alkyl, C₃₋₆-Cycloalkyl, ein 4- bis 8-gliedriges mono- oder bicyclisches Heterocycloalkyl mit 1, 2 oder 3 Heteroatomen, die unabhängig voneinander aus O, S und N ausgewählt sind, oder ein 5- bis 6-gliedriges Heteroaryl mit 1, 2 oder 3 Heteroatomen, die unabhängig voneinander aus O, S und N ausgewählt sind,
wobei Alkyl, Cycloalkyl, Heterocycloalkyl oder Heteroaryl unsubstituiert oder mit 1 bis 4 Substituenten R^{v⁵} substituiert sind, die unabhängig voneinander aus der Gruppe bestehend aus OH, C₁₋₆-Alkyl, O-C₁₋₆-Alkyl, C(O)-C₁₋₆-alkyl, C₁₋₆-Halogenalkyl, Halogen, Oxo, spirocyclisch kondensiertem C₃₋₆-Cycloalkyl und spirocyclisch kondensiertem 3- bis 7-gliedrigem Heterocycloalkyl mit 1 Heteroatom, das aus O, S und N ausgewählt ist, ausgewählt sind; und/oder
die Verbindung ist ausgewählt aus der Gruppe bestehend aus 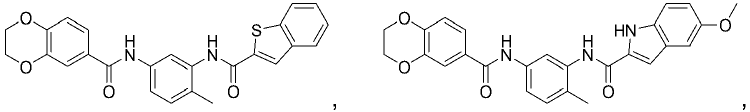 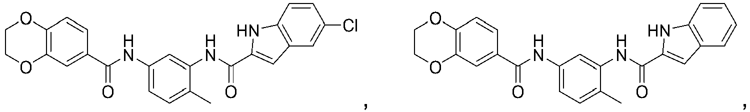 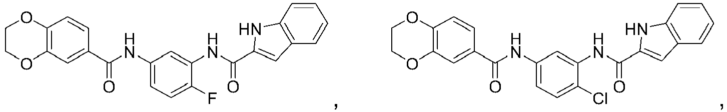 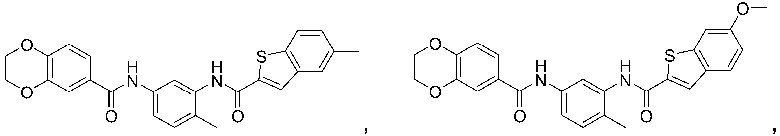 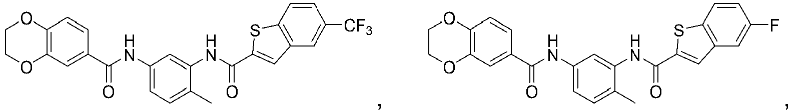 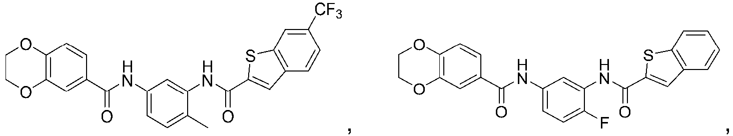 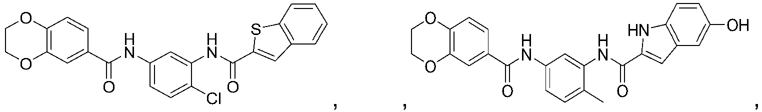 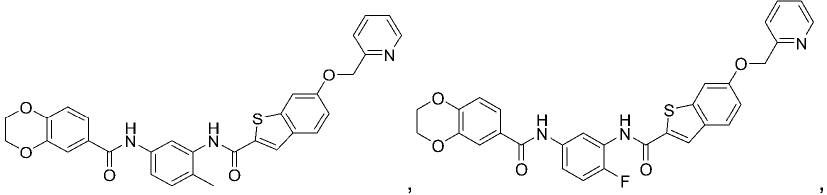 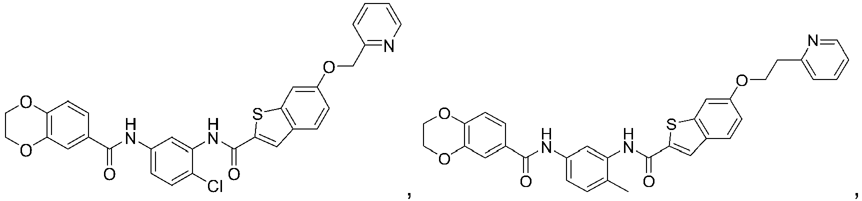 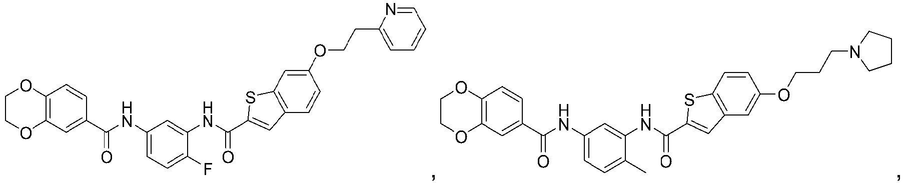 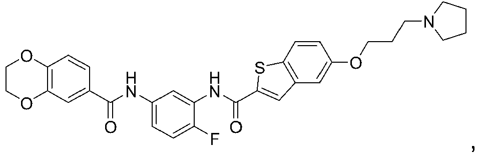 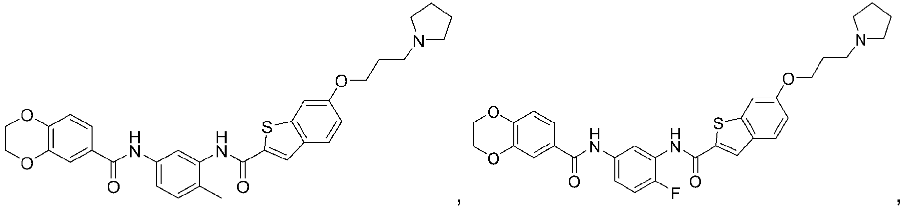 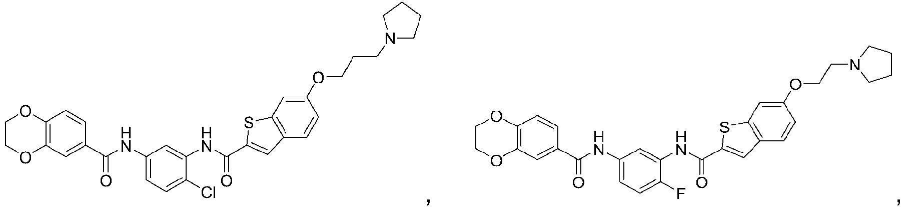 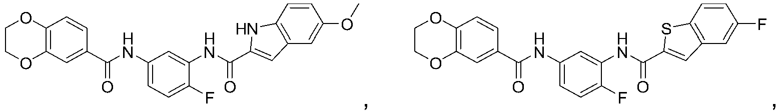 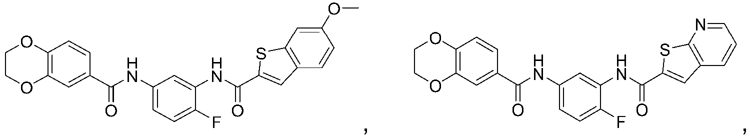 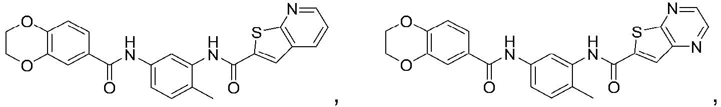 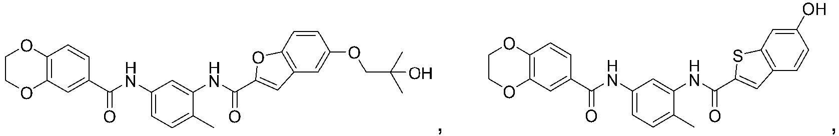 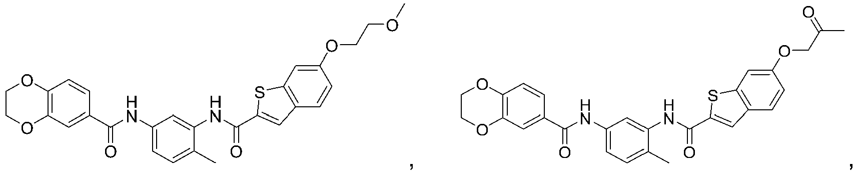 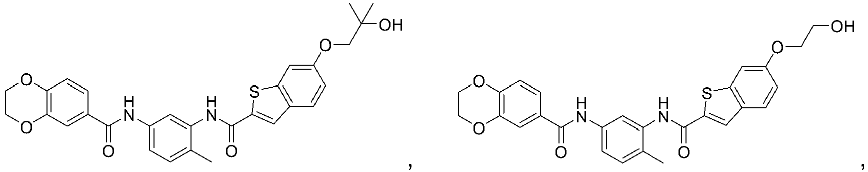 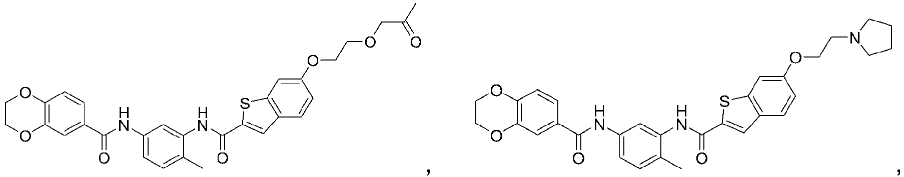 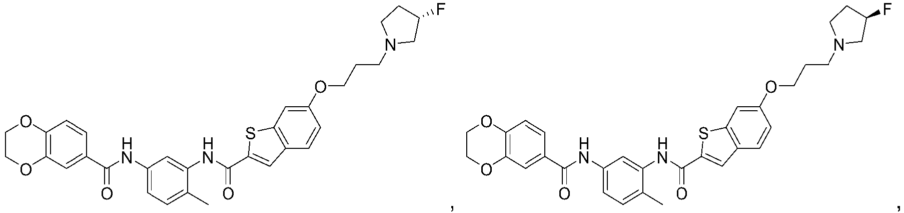 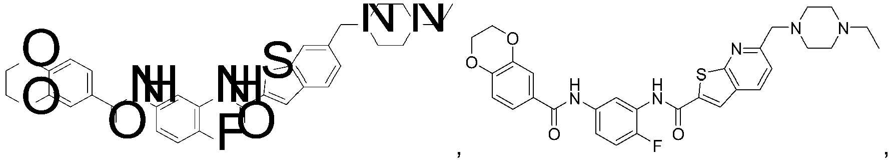 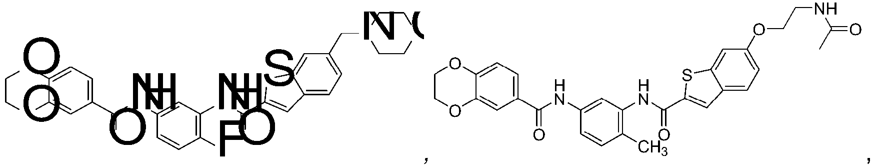 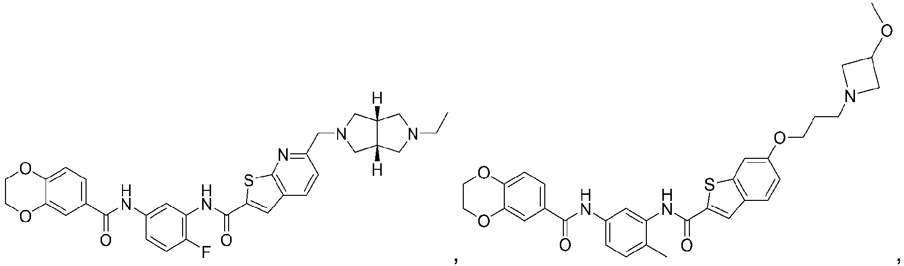 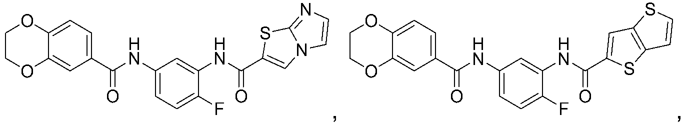 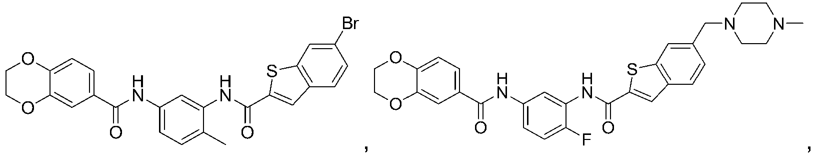 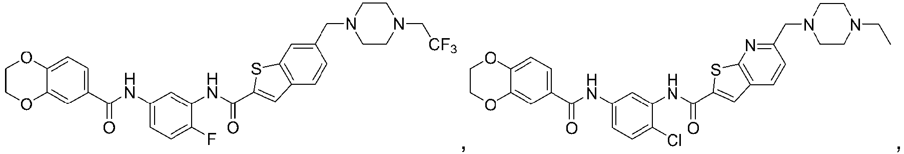 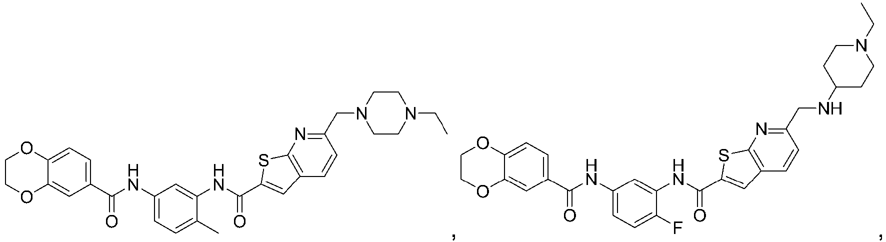 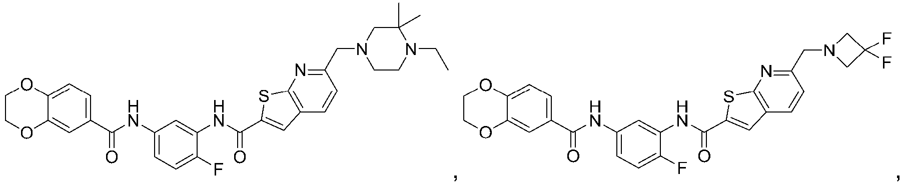 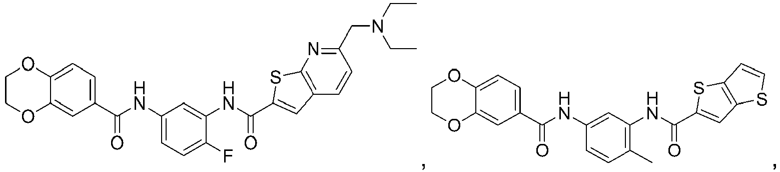 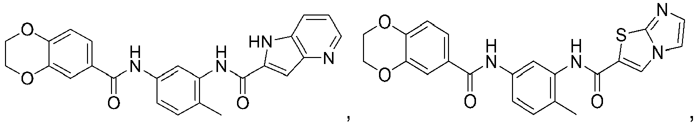 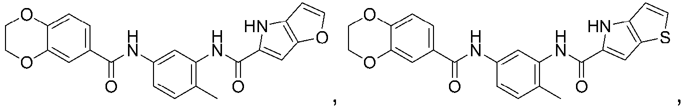 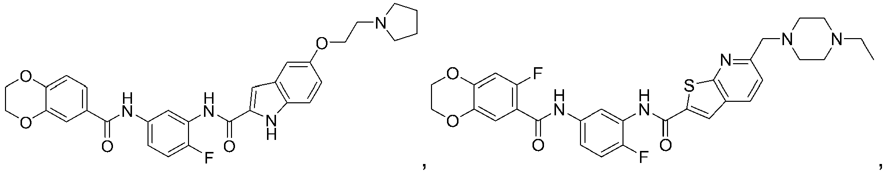 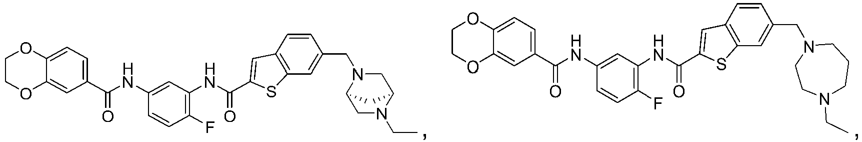 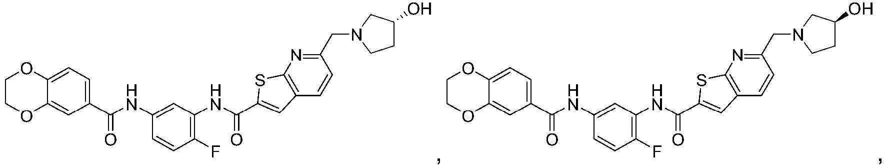 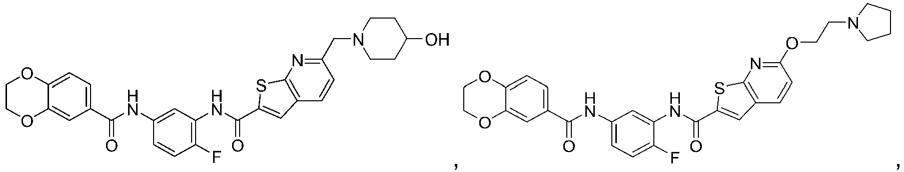 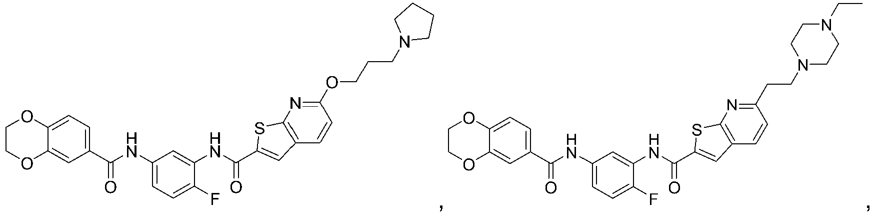 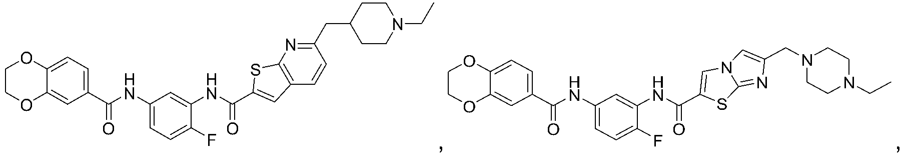 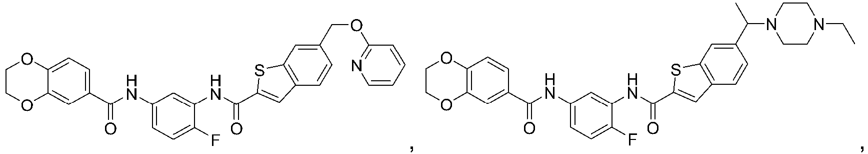 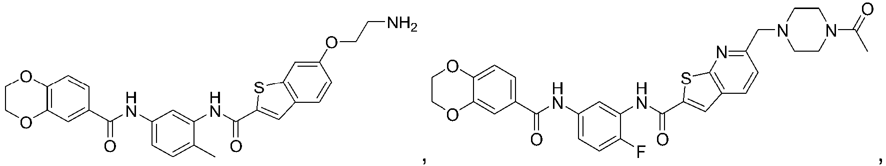 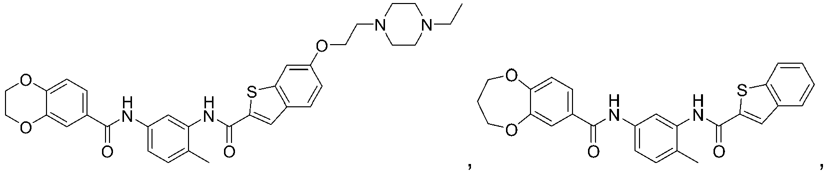 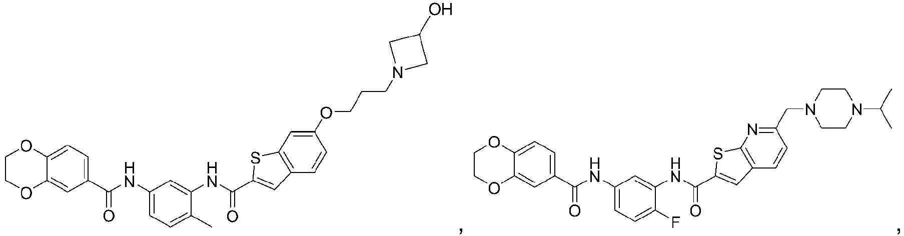 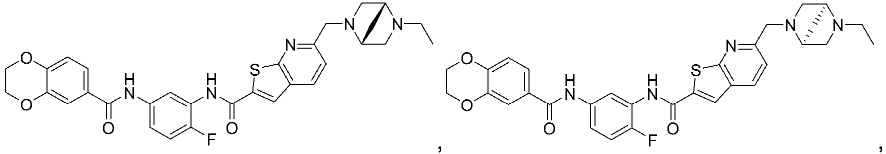 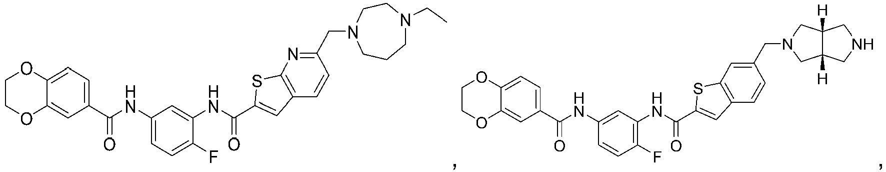 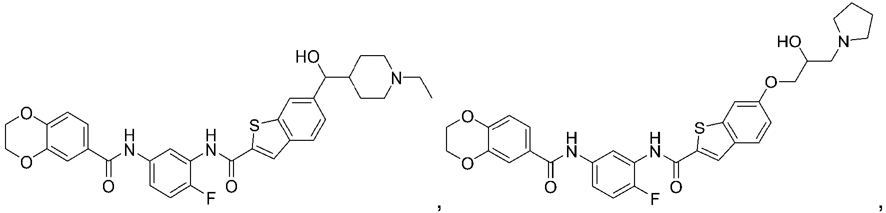 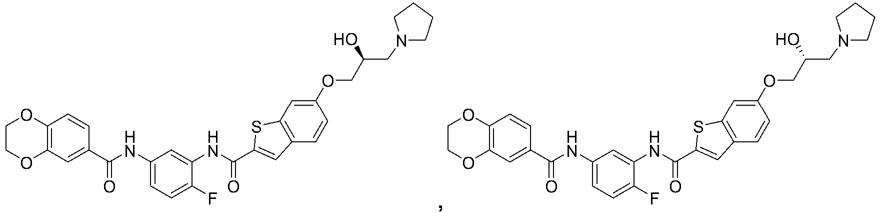 und dem N-Oxid, Solvat oder pharmazeutisch verträglichen Salz davon.

6. Pharmazeutische Zusammensetzung, umfassend die Verbindung nach einem der Ansprüche 1 bis 5 und einen pharmazeutisch verträglichen Hilfsstoff.

7. Verbindung nach einem der Ansprüche 1 bis 5 zur Verwendung als Arzneimittel.

8. Verbindung nach einem der Ansprüche 1 bis 5 oder pharmazeutische Zusammensetzung nach Anspruch 6 zur Verwendung in einem Verfahren zur Prävention und/oder Behandlung einer Krankheit oder eines Zustands, die bzw. der durch den Laktat/ATP-Mechanismus vermittelt wird,
wobei die durch den Laktat/ATP-Mechanismus vermittelte Krankheit oder der dadurch vermittelte Zustand gegebenenfalls Krebs ist und wobei die Verbindung oder die pharmazeutische Zusammensetzung vorzugsweise verabreicht wird
• zusammen mit einem oder mehreren therapeutischen Wirkstoffen gegen Krebs, die aus der Gruppe bestehend aus einem PD-1-Wirkstoff, einem PD-L1-Wirkstoff, einem CTLA-4-Wirkstoff, einem IDO1-Inhibitor und einem Antikrebsimpfstoff ausgewählt sind, oder
• zusammen mit einer Zytokintherapie oder einer bekannten Chemo- oder Pharmakotherapie oder während einer Strahlentherapie.

9. Verbindung nach einem der Ansprüche 1 bis 5 oder pharmazeutische Zusammensetzung nach Anspruch 6 zur Verwendung:
in einem Verfahren zur Prävention und/oder Behandlung einer Krankheit oder eines Zustands, vorzugsweise Krebs, durch Induktion von endoplasmatischem Retikulum-Stress in von der Krankheit oder dem Zustand betroffenen Zellen, wodurch die intrazelluläre ATP- und Laktatproduktion in diesen Zellen verringert wird; oder
in einem Verfahren zur Prävention und/oder Behandlung von Krebs, vorzugsweise einer Krebszelle und/oder eines Krebstumors, wobei die Verbindung oder die pharmazeutische Zusammensetzung Hif1-alpha auf Proteinebene in hypoxischen Bereichen des Krebses reduziert, wodurch die ATP- und Laktatproduktion in diesen Bereichen herunterreguliert wird und letztlich eine Verringerung, vorzugsweise eine wirksame Verringerung, der hypoxischen Bereiche und des Wachstums des Krebses bewirkt wird; oder
in einem Verfahren zur Prävention und/oder Behandlung einer Krankheit oder eines Zustands, der bzw. dem die Induktion von endoplasmatischem Retikulum-Stress zugänglich ist, wobei die Verabreichung der Verbindung oder der pharmazeutischen Zusammensetzung an ein Subjekt, das die Krankheit oder den Zustand aufweist oder bei dem der Verdacht besteht, dass es die Krankheit oder den Zustand aufweist, zu einer Verringerung des Proteinspiegels von Hif-1alpha führt; oder
in einem Verfahren zur Prävention und/oder Behandlung von mit Autophagie zusammenhängenden Krankheiten, ausgewählt aus neurodegenerativen Krankheiten wie der Parkinson-Krankheit, der Alzheimer-Krankheit und der Huntington-Krankheit, amyotropher Lateralsklerose, Stoffwechselstörungen wie Adipositas und Diabetes mellitus Typ I und Typ II, Virusinfektionen und Krankheiten, die zur Ansammlung bestimmter Debris-Partikel führen, wie fibrotischen Lungenerkrankungen.

10. Zusammensetzung, umfassend eine Verbindung nach einem der Ansprüche 1 bis 5 und ein Biguanid.

11. Ein Kit umfassend eine Verbindung nach eine der Ansprüche 1 bis 5 und ein Biguanid

12. Zusammensetzung nach Anspruch 9 oder Kit nach Anspruch 11 zur Verwendung als Arzneimittel.

13. Kombination eine Verbindung nach einem der Ansprüche 1 bis 5 und eines Biguanids zur Verwendung in einer Therapie

14. Kombination einer Verbindung nach einem der Ansprüche 1 bis 5 und eines Biguanids oder Kit nach Anspruch 11 zur Verwendung in einem Verfahren zur Prävention und/oder Behandlung von Krebs, vorzugsweise zur Prävention und/oder Behandlung von Krebs im fortgeschrittenen Stadium, wie gegebenenfalls zur Prävention und/oder Behandlung von glykolytischem Krebs im fortgeschrittenen Stadium, und/oder
wobei der Krebs ein LKB-1-defizienter Krebs ist
wobei der Krebs ein ARID1a-defizienter Krebs ist, und/oder
wobei der Kreis optional aus der Gruppe bestehend aus nicht-kleinzelligem Lungenkarzinom, Sarkom, gynäkologischen Krebsarten wie Zervix-, Ovarialzell- oder Uteruskarzinom, adenoidzystischem Karzinom, Bauchspeicheldrüsenkrebs und anderen gastrointestinalen Krebsarten wie Magen- oder Speiseröhrenkrebs ausgewählt ist.

15. Zusammensetzung nach Anspruch 8, Kit nach Anspruch 11, Zusammensetzung zur Verwendung oder Kit zur Verwendung nach Anspruch 12, Kombination zur Verwendung nach Anspruch 13 oder Kombination zur Verwendung oder Kit zur Verwendung nach Anspruch 14, wobei das Biguanid aus der Gruppe bestehend aus Metformin, Buformin und Phenformin ausgewählt ist.

## Revendications

1. Composé de formule (I) 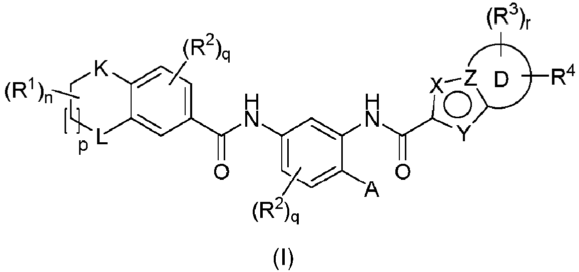 ou un énantiomère, diastéréoisomère, N-oxyde, solvate, ou sel pharmaceutiquement acceptables, de celui-ci, dans lequel
K représente O, S ou C(R⁵)₂ ;
L représente O, S ou C(R⁵)₂ ;
n est égal à 0, 1, 2, 3 ou 4 ;
p est égal à 1 ou 2 ;
q sont égaux, indépendamment les uns des autres, à 0, 1 ou 2 ;
r est égal à 1 ou 2 ;
A représente un alkyle en C₁ à C₆, un halogène, CN, halogénoalkyle en C₁ à C₆, cycloalkyle en C₃ à C₆, halogénocycloalkyle en C₃ à C₆, OH, -O-alkyle en C₁ à C₆ ou O-halogénoalkyle en C₁ à C₆ ;
X représente S, O, NR⁶, N ou CR⁷ ;
Y représente S, O, NR⁶, N ou CR⁷ ;
Z représente C ou N ;
D représente un aryle hexagonal ou hétéroaryle penta- ou hexagonal contenant 1 à 4 hétéroatomes choisis indépendamment parmi O, N et S ;
R¹ représente F, un alkyle en C₁ à C₃ ou cycloalkyle en C₃ à C₆ condensé spirocyclique ;
R² représente, indépendamment, un halogène ou un alkyle en C₁ à C₆ ;
R³ représente l'hydrogène, un halogène, alkyle en C₁ à C₆, halogénoalkyle en C₁ à C₆, cycloalkyle en C₃ à C₆, halogénocycloalkyle en C₃ à C₆, -O-alkyle en C₁ à C₆ ou O-halogénoalkyle en C₁ à C₆ ;
R⁴ représente l'hydrogène, un halogène ou V¹-V²-V³-V⁴-V⁵
dans lequel
V¹ est absent, représente une liaison ou un alkylène en C₁ à C₆ ;
V² est absent, représente une liaison ou O, S, S(O)ₓ, S(O)(=NR^{a}), S(O)₂NR^{a}, NR^{a}, NR^{a}C(O) ou C(O)NR^{a} ;
V³ est absent, représente une liaison ou un groupe alkylène en C₁ à C₆ ;
où l'alkylène en C₁ à C₆ dans V¹ ou V³ est non substitué ou substitué par 1 à 4 substituants choisis indépendamment dans le groupe consistant en OH, alkyle en C₁ à C₆, halogénoalkyle en C₁ à C₆, halogène et oxo,
ou
où deux groupes alkyle en C₁ à C₆ sur l'alkylène en C₁ à C₆ dans V¹ ou V³, conjointement avec l'atome de carbone auquel ils sont fixés, forment un groupe cycloalkyle en C₃ à C₆ ;
V⁴ est absent, représente une liaison ou O, S, S(O)ₓ, S(O)(=NR^{a}), S(O)₂NR^{a}, NR^{a}, NR^{a}C(O) ou C(O)NR^{a} ;
V⁵ représente l'hydrogène, un halogène, OH, CN, CO₂H, CO₂-alkyle en C₁ à C₆, N(R^{a})₂, alkyle en C₁ à C₆, cycloalkyle en C₃ à C₈, hétérocycloalkyle mono- ou bicyclique tétra- à octogonal contenant 1, 2 ou 3 hétéroatomes choisis indépendamment dans le groupe consistant en O, S et N, phényle ou hétéroaryle penta- ou hexagonal contenant 1, 2 ou 3 hétéroatomes choisis indépendamment dans le groupe consistant en O, S et N,
où les alkyle, cycloalkyle, hétérocycloalkyle, phényle ou hétéroaryle sont non substitués ou substitués par 1 à 4 substituants R^{V5} choisis indépendamment dans le groupe consistant en CN, CO₂H, CO₂-alkyle en C₁ à C₆, N(R^{a})₂, OH, alkyle en C₁ à C₆, O-alkyle en C₁ à C₆, C(O)-alkyle en C₁ à C₆, halogénoalkyle en C₁ à C₆, halogène, oxo, cycloalkyle en C₃ à C₆ condensé de manière spirocyclique et hétérocycloalkyle tri- à heptagonal condensé de manière spirocyclique contenant 1 hétéroatome choisi dans le groupe consistant en O, S et N ;
R⁵ représente l'hydrogène, F, un alkyle en C₁ à C₃ ou cycloalkyle en C₃ à C₆ condensé spirocyclique ;
R⁶ représente l'hydrogène ou un alkyle en C₁ à C₆ ;
R⁷ représente l'hydrogène, un halogène ou alkyle en C₁ à C₆ ;
R^{a} représente l'hydrogène ou un alkyle en C₁ à C₃ ; et
x est égal à 1 ou 2.

2. Composé selon la revendication 1, qui est représentés par la formule (I) 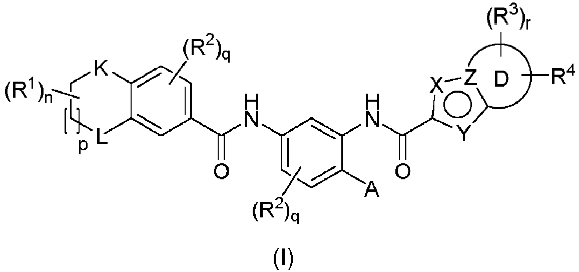
ou un des ses énantiomères, diastéréoisomères, N-oxydes, solvates, ou sels pharmaceutiquement acceptables, dans lequel
K représente O, S ou C(R⁵)₂ ;
L représente O, S ou C(R⁵)₂ ;
n est égal à 0, 1, 2, 3 ou 4 ;
p est égal à 1 ou 2 ;
q sont égaux, indépendamment les uns des autres, à 0, 1 ou 2 ;
r est égal à 1 ou 2 ;
A représente un alkyle en C₁ à C₆, un halogène, CN, halogénoalkyle en C₁ à C₆, cycloalkyle en C₃ à C₆, halogénocycloalkyle en C₃ à C₆, OH, -O-alkyle en C₁ à C₆ ou O-halogénoalkyle en C₁ à C₆ ;
X représente S, O, NR⁶, N ou CR⁷ ;
Y représente S, O, NR⁶, N ou CR⁷ ;
Z représente C ou N ;
D représente un aryle hexagonal ou hétéroaryle penta- ou hexagonal contenant 1 à 4 hétéroatomes choisis indépendamment parmi O, N et S ;
R¹ représente F, un alkyle en C₁ à C₃ ou cycloalkyle en C₃ à C₆ condensé spirocyclique ;
R² représente, indépendamment, un halogène ou alkyle en C₁ à C₆ ;
R³ représente l'hydrogène, un halogène, alkyle en C₁ à C₆, halogénoalkyle en C₁ à C₆, cycloalkyle en C₃ à C₆, halogénocycloalkyle en C₃ à C₆, -O-alkyle en C₁ à C₆ ou O-halogénoalkyle en C₁ à C₆ ;
R⁴ représente l'hydrogène, un halogène ou V¹-V²-V³-V⁴-V⁵
dans lequel
V¹ est absent, représente une liaison ou un alkylène en C₁ à C₆ ;
V² est absent, représente une liaison ou O, S, S(O)ₓ, S(O)(=NR^{a}), S(O)₂NR^{a}, NR^{a}, NR^{a}C(O) ou C(O)NR^{a} ;
V³ est absent, représente une liaison ou un groupe alkylène en C₁ à C₆ ;
où l'alkylène en C₁ à C₆ dans V¹ ou V³ est non substitué ou substitué par 1 à 4 substituants choisis indépendamment dans le groupe consistant en alkyle en C₁ à C₆, halogénoalkyle en C₁ à C₆, halogène et oxo,
ou
où deux groupes alkyle en C₁ à C₆ sur l'alkylène en C₁ à C₆ dans V¹ ou V³, conjointement avec l'atome de carbone auquel ils sont fixés, forment un groupe cycloalkyle en C₃ à C₆ ;
V⁴ est absent, représente une liaison ou O, S, S(O)ₓ, S(O)(=NR^{a}), S(O)₂NR^{a}, NR^{a}, NR^{a}C(O) ou C(O)NR^{a} ;
V⁵ représente l'hydrogène, un halogène, OH, CN, CO₂H, CO₂-alkyle en C₁ à C₆, N(R^{a})₂, alkyle en C₁ à C₆, cycloalkyle en C₃ à C₈, hétérocycloalkyle mono- ou bicyclique tétra- à octogonal contenant 1, 2 ou 3 hétéroatomes choisis indépendamment dans le groupe consistant en O, S et N, phényle ou hétéroaryle penta- ou hexagonal contenant 1, 2 ou 3 hétéroatomes choisis indépendamment dans le groupe consistant en O, S et N,
où les alkyle, cycloalkyle, hétérocycloalkyle, phényle ou hétéroaryle sont non substitués ou substitués par 1 à 4 substituants R^{V5} choisis indépendamment dans le groupe consistant en CN, CO₂H, CO₂-alkyle en C₁ à C₆, N(R^{a})₂, OH, alkyle en C₁ à C₆, O-alkyle en C₁ à C₆, C(O)-alkyle en C₁ à C₆, halogénoalkyle en C₁ à C₆, halogène, oxo, cycloalkyle en C₃ à C₆ condensé de manière spirocyclique et hétérocycloalkyle tri- à heptagonal condensé de manière spirocyclique contenant 1 hétéroatome choisi dans le groupe consistant en O, S et N ;
R⁵ représente l'hydrogène, F, un alkyle en C₁ à C₃ ou cycloalkyle en C₃ à C₆ condensé de manière spirocyclique ;
R⁶ représente l'hydrogène ou un alkyle en C₁ à C₆ ;
R⁷ représente l'hydrogène, un halogène ou alkyle en C₁ à C₆ ;
R³ représente l'hydrogène ou un alkyle en C₁ à C₃ ; et
x est égal à 1 ou 2.

3. Composé selon la revendication 1 ou 2, le composé étant représenté par la formule (II) 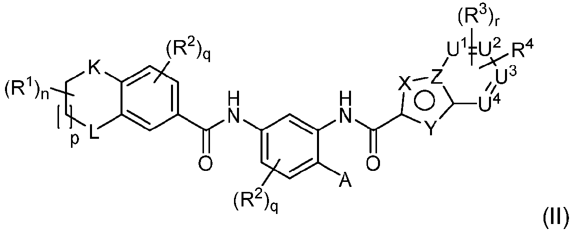 dans laquelle U¹, U², U³ et U⁴ sont choisis indépendamment dans le groupe consistant en N et C.

4. Composé selon l'une quelconque des revendications 1 à 3, le composé étant représenté par la formule (III) 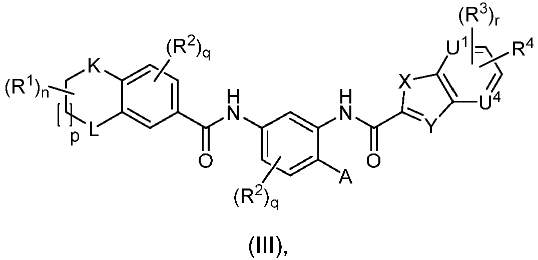 dans laquelle
U¹ et U⁴ sont choisis indépendamment parmi N et C ;
X représente S, O, NR⁶ ou N ; et
Y représente CR⁷ ou N, et/ou
où n est égal à 0, et/ou
où p est égal à 1, et/ou
le composé étant représenté par la formule (IV) 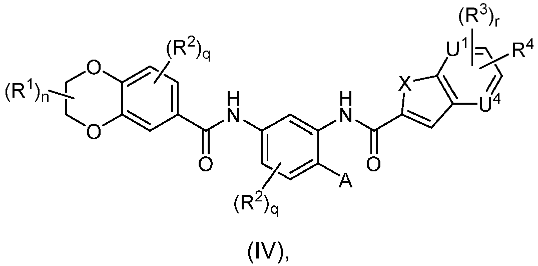 dans laquelle
A représente un alkyle en C₁ à C₆ ou un halogène ; et
X représente S, O ou NH.

5. Composé selon l'une quelconque des revendications 1 à 4, dans lequel :
R⁴ représente l'hydrogène, un halogène ou V¹-V²-V³-V⁴-V⁵, dans lequel
V¹ est absent, représente une liaison ou un alkylène en C₁ à ;
V² est absent, représente une liaison ou O, S, S(O)ₓ ou NR^{a} ;
V³ est absent, représente une liaison ou un groupe alkylène en C₁ à C₄, V⁴ est absent, représente une liaison ou O, S, S(O)ₓ ou NR^{a},
où l'alkylène en C₁ à dans V¹ ou V³ est non substitué ou substitué par 1 à 4 substituants choisis indépendamment dans le groupe consistant en alkyle en C₁ à C₃, halogénoalkyle en C₁ à C₃, halogène et oxo,
ou
où deux groupes alkyle en C₁ à C₆ sur l'alkylène en C₁ à dans V¹ ou V³, conjointement avec l'atome de carbone auquel ils sont fixés, forment un groupe cycloalkyle en C₃ à C₆ ;
V⁵ représente l'hydrogène, un halogène, OH, CN, alkyle en C₁ à C₆, cycloalkyle en C₃ à C₆, hétérocycloalkyle mono- ou bicyclique tétra- à octogonal contenant 1, 2 ou 3 hétéroatomes choisis indépendamment dans le groupe consistant en O, S et N, ou hétéroaryle penta- ou hexagonal contenant 1, 2 ou 3 hétéroatomes choisis indépendamment dans le groupe consistant en O, S et N,
où les alkyle, cycloalkyle, hétérocycloalkyle ou hétéroaryle sont non substitués ou substitués par 1 à 4 substituants R^{V5} choisis indépendamment dans le groupe consistant en OH, alkyle en C₁ à C₆, O-alkyle en C₁ à C₆, C(O)-alkyle en C₁ à C₆, halogénoalkyle en C₁ à C₆, halogène, oxo, cycloalkyle en C₃ à C₆ condensé de manière spirocyclique et hétérocycloalkyle tri- à heptagonal condensé de manière spirocyclique contenant 1 hétéroatome choisi dans le groupe consistant en O, S et N ; et/ou
le composé étant choisi dans le groupe consistant en 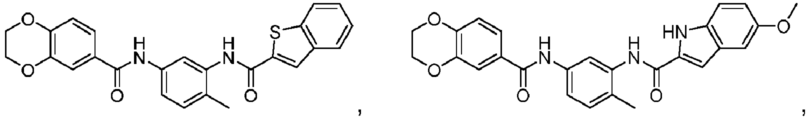 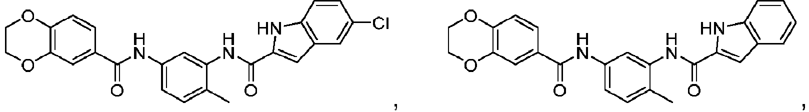 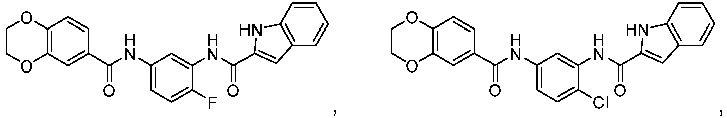 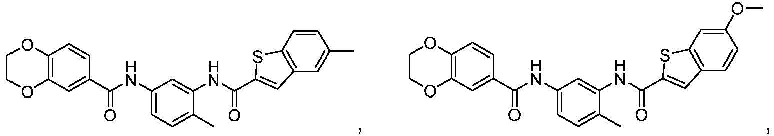 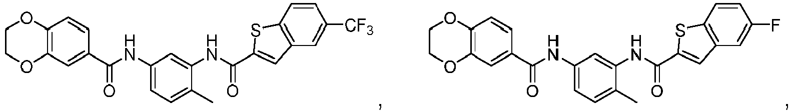 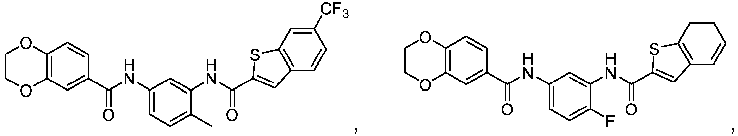 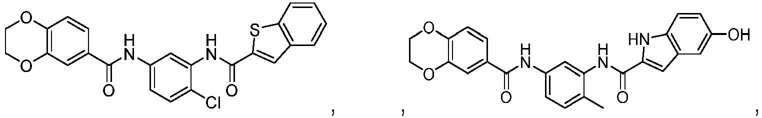 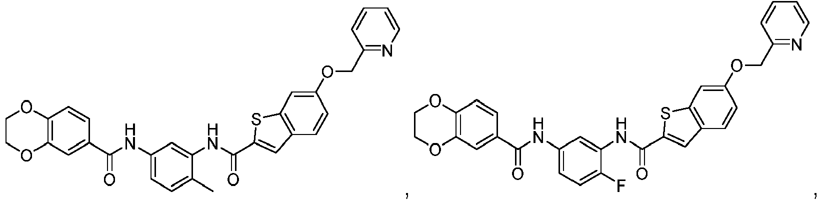 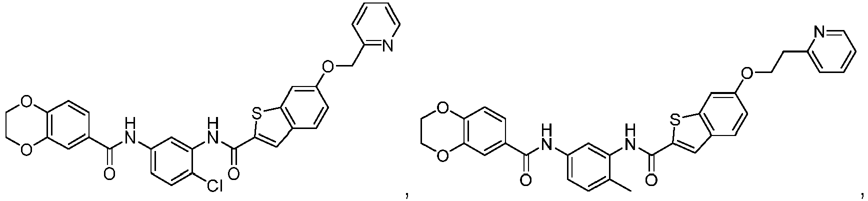 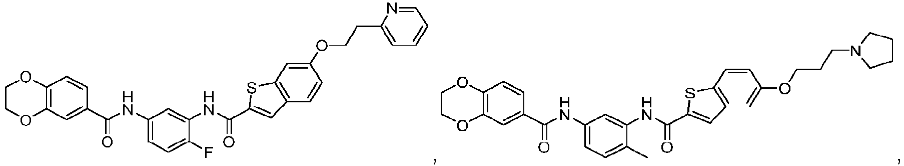 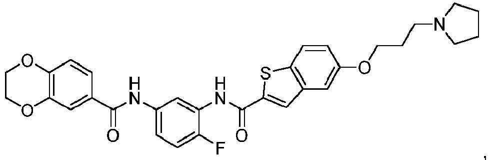 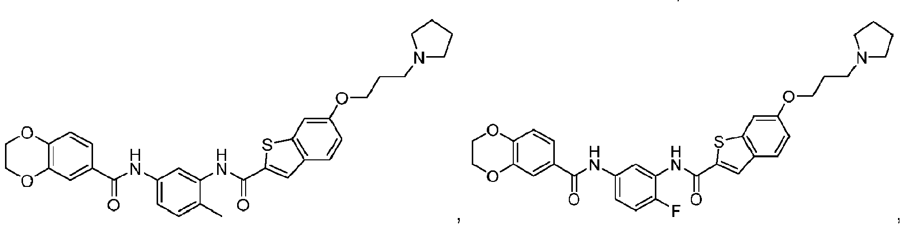 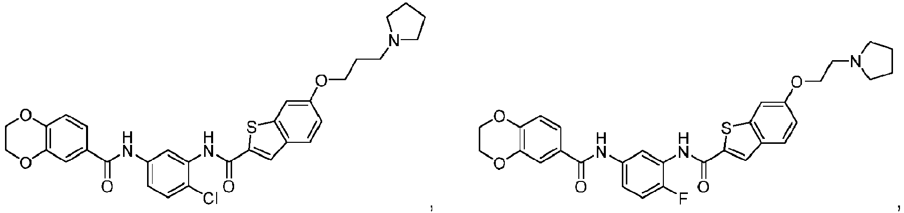 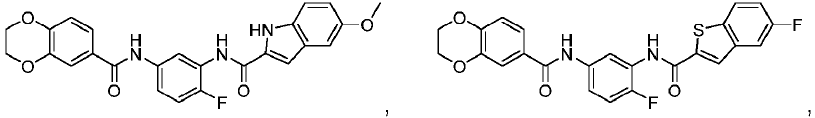 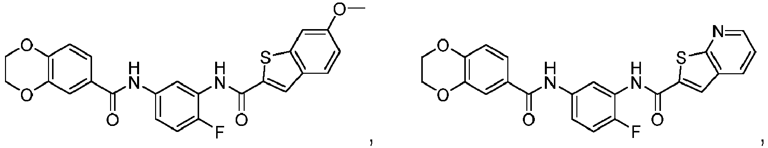 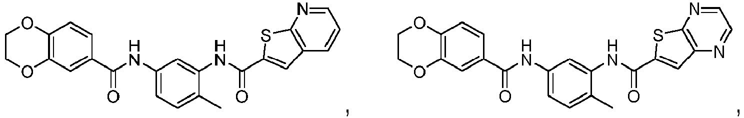 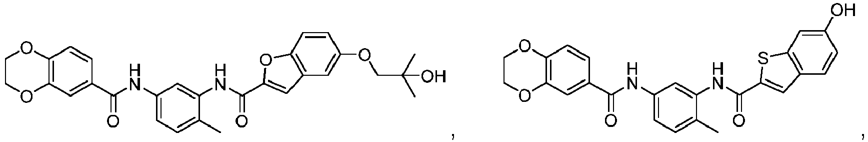 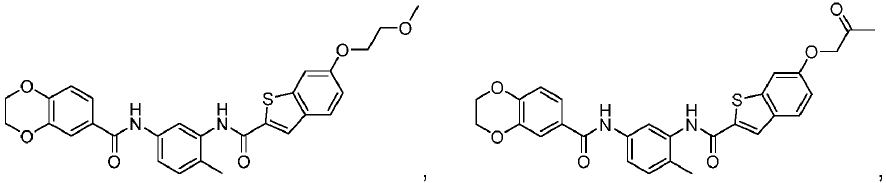 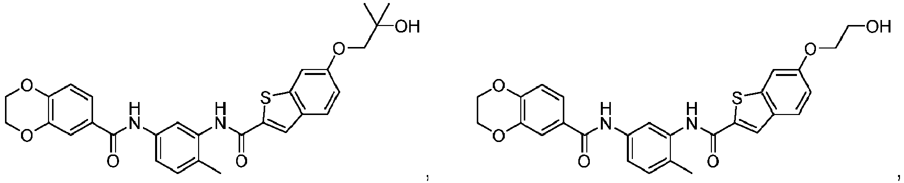 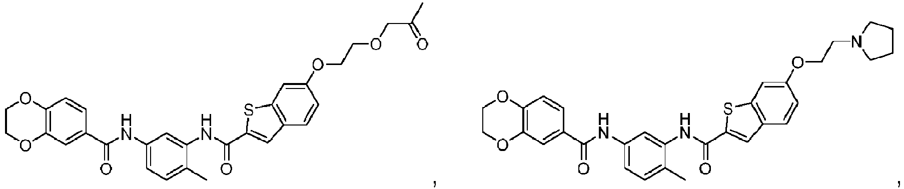 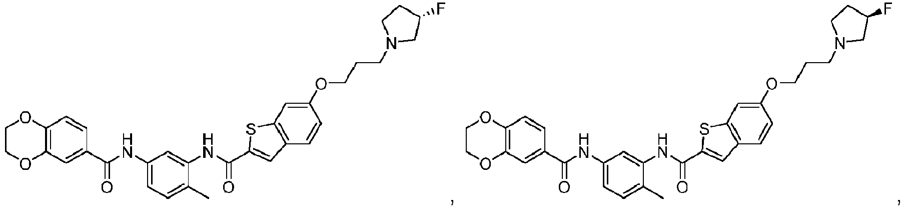 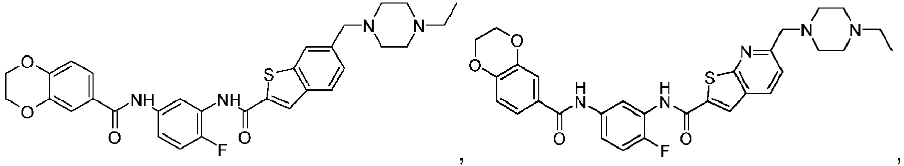 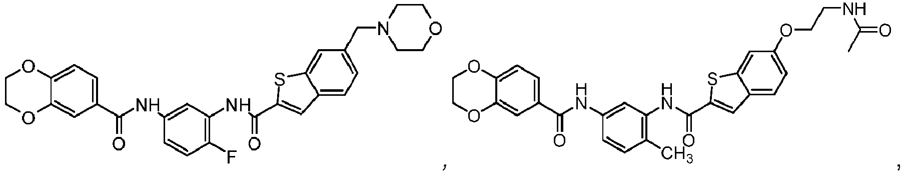 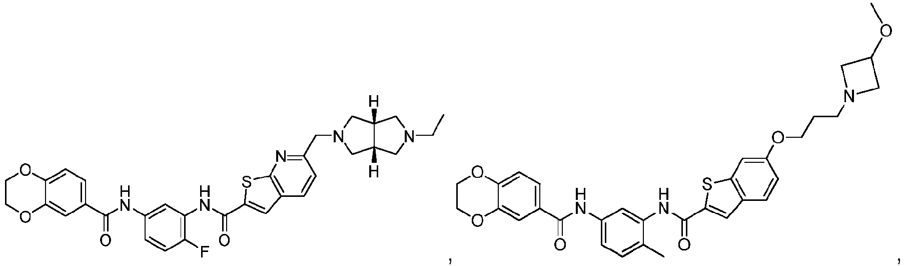 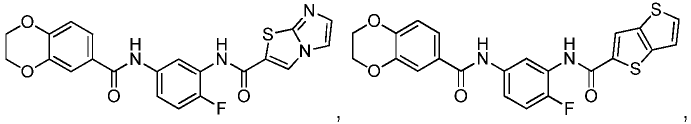 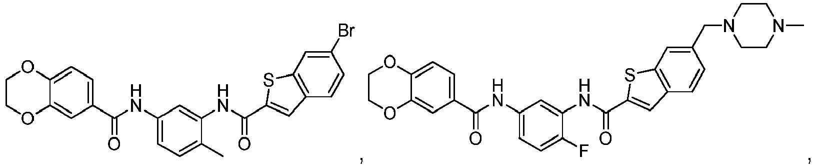 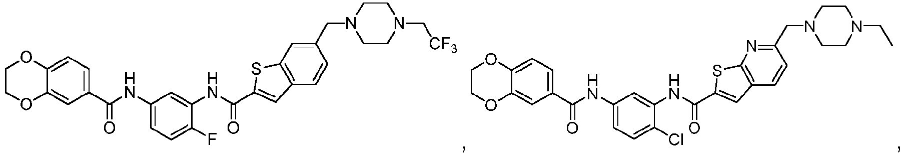 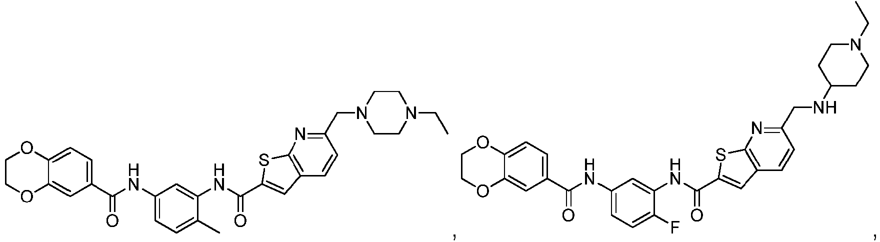 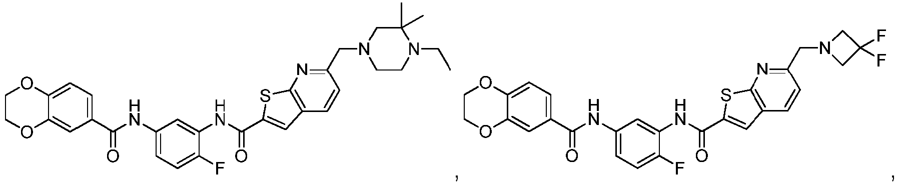 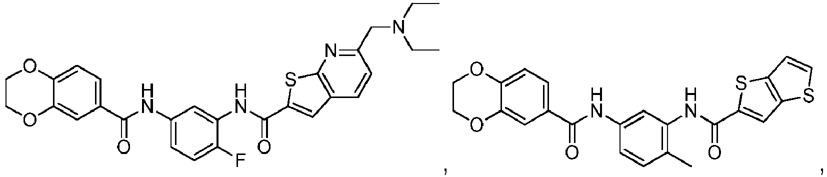 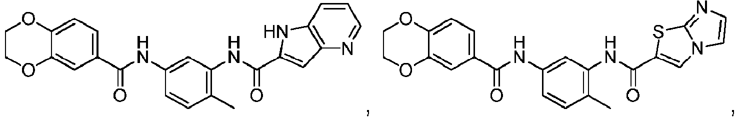 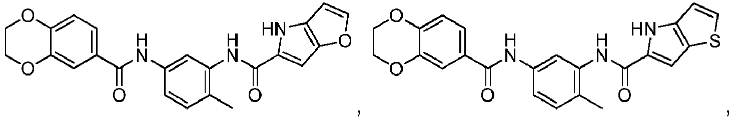 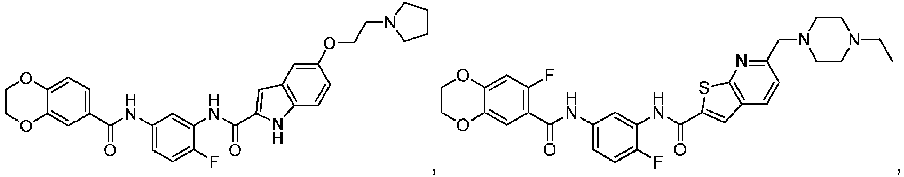 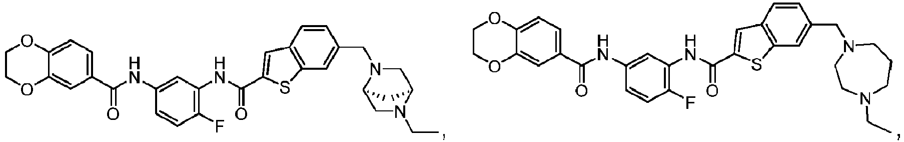 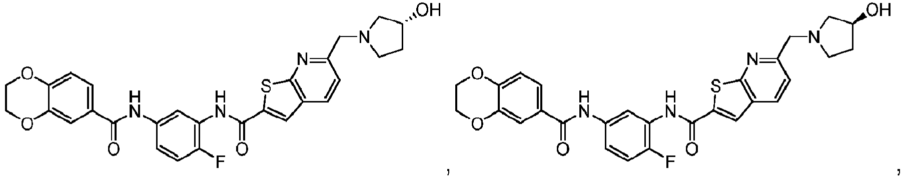 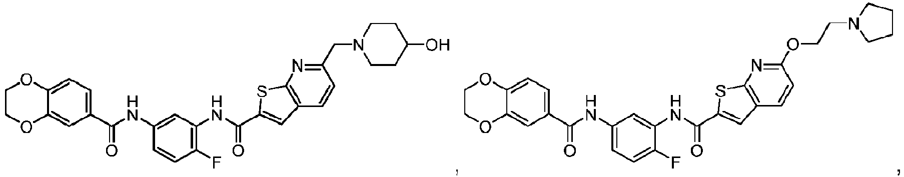 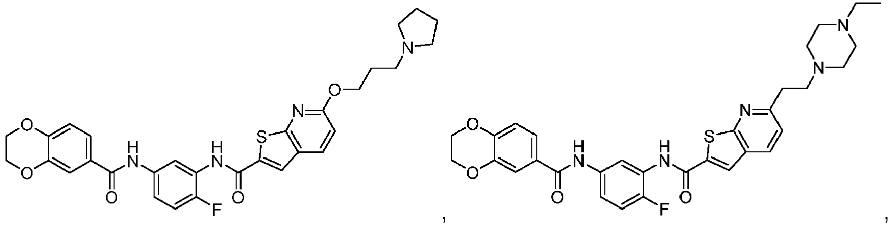 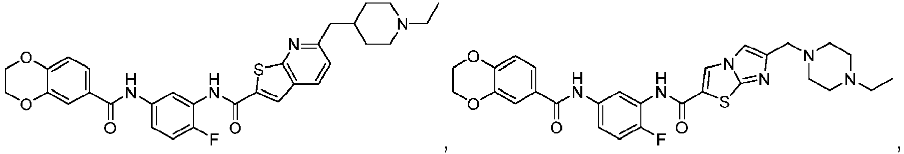 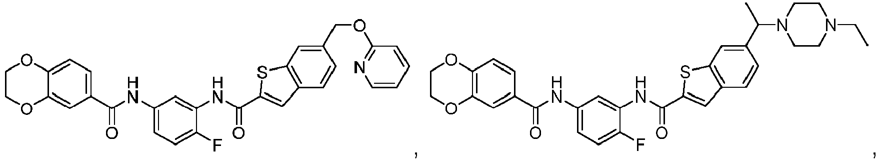 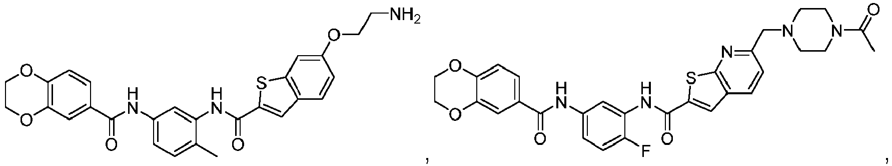 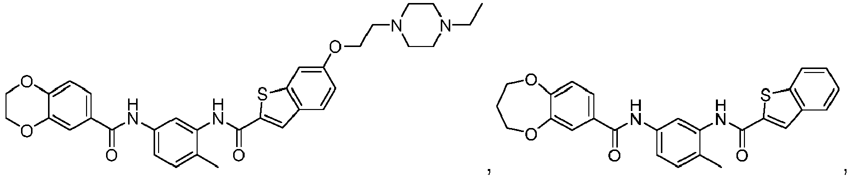 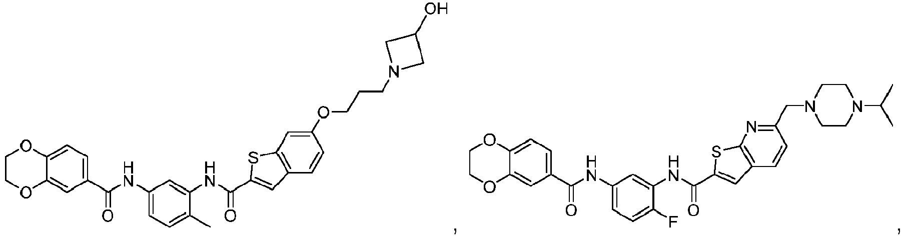 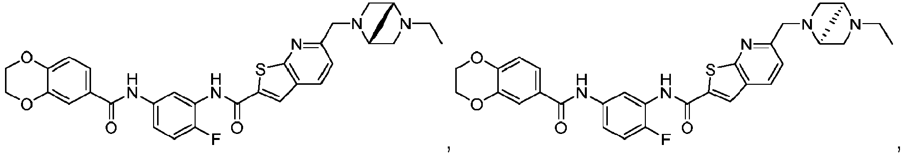 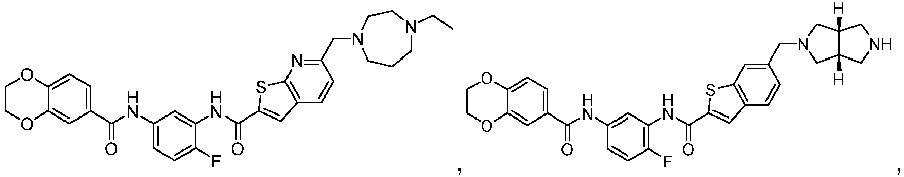 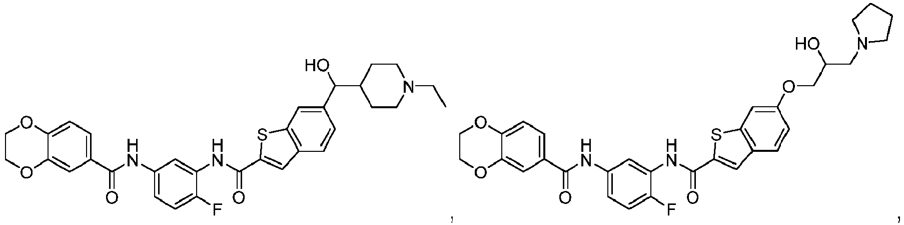 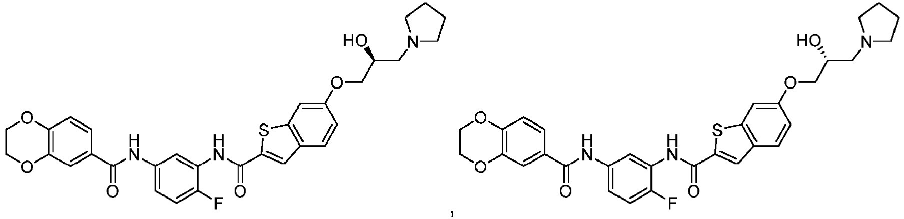 et le N-oxyde, solvate, ou sel pharmaceutiquement acceptable de celui-ci.

6. Composition pharmaceutique comprenant le composé selon l'une quelconque des revendications 1 à 5 et un excipient pharmaceutiquement acceptables.

7. Composé selon l'une quelconque des revendications 1 à 5 pour une utilisation comme médicament.

8. Composé selon l'une quelconque des revendications 1 à 5 ou composition pharmaceutique selon la revendication 6, pour une utilisation dans un procédé de prévention et/ou de traitement d'une maladie ou d'un état médié par le mécanisme lactate/ATP,
où, facultativement, la maladie ou l'état médié par le mécanisme lactate/ATP est un cancer et où, de préférence, le composé ou la composition pharmaceutique est administré(e)
• conjointement avec un ou plusieurs agents thérapeutiques anticancéreux choisis dans le groupe consistant en agent PD-1, agent PD-L1, agent CTLA-4, inhibiteur de l'IDO1 et vaccin anticancéreux, ou
• conjointement avec une thérapie à base de cytokine ou une chimiothérapie ou pharmacothérapie connue, ou au cours d'une radiothérapie.

9. Composé selon l'une quelconque des revendications 1 à 5 ou composition pharmaceutique selon la revendication 6 pour une utilisation :
dans un procédé de prévention et/ou de traitement d'une maladie ou d'un état, de préférence un cancer, en induisant un stress du réticulum endoplasmique dans les cellules atteintes par ladite maladie ou ledit état, réduisant ainsi la production intracellulaire d'ATP et de lactate dans lesdites cellules ; ou
dans un procédé de prévention et/ou de traitement du cancer, de préférence d'une cellule cancéreuse et/ou d'une tumeur cancéreuse, ledit composé ou ladite composition pharmaceutique réduisant le Hif-1alpha au niveau protéique dans les zones hypoxiques dudit cancer, régulant ainsi à la baisse la production d'ATP et de lactate dans ces zones et conduisant finalement à une réduction, de préférence une réduction efficace, de zones hypoxiques et de la croissance dudit cancer ; ou
dans un procédé de prévention et/ou de traitement d'une maladie ou d'un état qui est susceptible d'induire un stress du réticulum endoplasmique, dans lequel l'administration dudit composé ou de ladite composition pharmaceutique à un sujet qui présente ou est suspecté de présenter ladite maladie ou ledit état entraîne une réduction du taux de protéine du Hif-1alpha ; ou
dans un procédé de prévention et/ou de traitement de maladies liées à l'autophagie, choisies parmi les maladies neurodégénératives telles que la maladie de Parkinson, la maladie d'Alzheimer et la maladie de Huntington, la sclérose latérale amyotrophique, les troubles métaboliques tels que l'obésité et les diabètes de type I et II, les infections virales et les maladies conduisant à une accumulation de certaines particules de débris, telles que les maladies pulmonaires fibrotiques.

10. Composition comprenant un composé selon l'une quelconque des revendications 1 à 5 et un biguanide.

11. Trousse comprenant un composé selon l'une quelconque des revendications 1 à 5 et un biguanide.

12. Composition selon la revendications 9 ou trousse selon la revendication 11, pour une utilisation comme médicament.

13. Combinaison d'un composé selon l'une quelconque des revendications 1 à 5 et d'un biguanide, pour une utilisation en thérapie.

14. Combinaison d'un composé selon l'une quelconque des revendications 1 à 5 et d'un biguanide, ou trousse selon la revendication 11, pour une utilisation dans un procédé de prévention et/ou de traitement du cancer, de préférence de prévention et/ou de traitement d'un cancer à un stade avancé, tel que, facultativement, la prévention et/ou le traitement d'un cancer à métabolisme glycolytique à un stade avancé, et/ou
où le cancer est un cancer LKB-1-déficient, et/ou
où le cancer est un cancer ARID1a-déficient, et/ou
où, facultativement, le cancer est choisi dans le groupe consistant en cancer du poumon non à petites cellules, sarcome, cancers gynécologiques tels que le carcinome du col de l'utérus, de l'ovaire ou de l'utérus, carcinome adénoïde kystique, cancer du pancréas, autres cancers gastro-intestinaux tels que les cancers de l'estomac ou de l'œsophage.

15. Composition selon la revendication 8, trousse selon la revendication 11, composition pour une utilisation ou trousse pour une utilisation selon la revendication 12, combinaison pour une utilisation selon la revendication 13, ou combinaison pour une utilisation ou trousse pour une utilisation selon la revendication 14, où le biguanide est choisi dans le groupe consistant en metformine, buformine et phenformine.
